# EUROPÄISCHE PATENTSCHRIFT

(11) **EP 2 751 106 B1**
(45) Veröffentlichungstag und Bekanntmachung des Hinweises auf die Patenterteilung: **18.10.2017**
(21) Anmeldenummer: 12766292.2
(22) Anmeldetag: 30.08.2012
(51) Int. Cl.: C07D 471/04, C07D 487/04, C07D 519/00, A61K 31/519, A61P 9/00

(54) **SUBSTITUIERTE ANNELLIERTE PYRIMIDINE UND IHRE VERWENDUNG**
SUBSTITUTED ANNELLATED PYRIMIDINE AND THE USE THEREOF
PYRIMIDINES ANNELÉES SUBSTITUÉES ET LEUR UTILISATION

(30) Priorität: 02.09.2011 DE 102011082041; 11.01.2012 DE 102012200351
(43) Veröffentlichungstag der Anmeldung: 09.07.2014
(73) Patentinhaber: Bayer Intellectual Property GmbH, 40789 Monheim (DE); Bayer Pharma Aktiengesellschaft, 13353 Berlin (DE)
(72) Erfinder: FOLLMANN, Markus, 50895 Köln (DE); STASCH, Johannes-Peter, 42651 Solingen (DE); REDLICH, Gorden, 44799 Bochum (DE); GRIEBENOW, Nils, 41541 Dormagen (DE); LANG, Dieter, 42553 Velbert (DE); WUNDER, Frank, 42117 Wuppertal (DE); HÜBSCH, Walter, 42113 Wuppertal (DE); LINDNER, Niels, 42115 Wuppertal (DE); VAKALOPOULOS, Alexandros, 40721 Hilden (DE); TERSTEEGEN, Adrian, 42111 Wuppertal (DE)
(74) Vertreter: BIP Patents
(86) Internationale Anmeldenummer: PCT/EP2012/066876
(87) Internationale Veröffentlichungsnummer: WO 2013/030288

(56) Entgegenhaltungen:
- WO-A1-2010/065275
- WO-A1-2011/149921

## Beschreibung

Die vorliegende Anmeldung betrifft neue substituierte annellierte Pyrimidine, Verfahren zu ihrer Herstellung, ihre Verwendung allein oder in Kombinationen zur Behandlung und/oder Prophylaxe von Krankheiten sowie ihre Verwendung zur Herstellung von Arzneimitteln zur Behandlung und/oder Prophylaxe von Krankheiten, insbesondere zur Behandlung und/oder Prophylaxe von Herz-Kreislauf-Erkrankungen.

Eines der wichtigsten zellulären Übertragungssysteme in Säugerzellen ist das cyclische Guanosinmonophosphat (cGMP). Zusammen mit Stickstoffmonoxid (NO), das aus dem Endothel freigesetzt wird und hormonelle und mechanische Signale überträgt, bildet es das NO/cGMP-System. Die Guanylatcyclasen katalysieren die Biosynthese von cGMP aus Guanosintriphosphat (GTP). Die bisher bekannten Vertreter dieser Familie lassen sich sowohl nach strukturellen Merkmalen als auch nach der Art der Liganden in zwei Gruppen aufteilen: Die partikulären, durch natriuretische Peptide stimulierbaren Guanylatcyclasen und die löslichen, durch NO stimulierbaren Guanylatcyclasen. Die löslichen Guanylatcyclasen bestehen aus zwei Untereinheiten und enthalten höchstwahrscheinlich ein Häm pro Heterodimer, das ein Teil des regulatorischen Zentrums ist. Dieses hat eine zentrale Bedeutung für den Aktivierungsmechanismus. NO kann an das Eisenatom des Häms binden und so die Aktivität des Enzyms deutlich erhöhen. Hämfreie Präparationen lassen sich hingegen nicht durch NO stimulieren. Auch Kohlenmonoxid (CO) ist in der Lage, an das Eisen-Zentralatom des Häms zu binden, wobei die Stimulierung durch CO deutlich geringer ist als die durch NO.

Durch die Bildung von cGMP und der daraus resultierenden Regulation von Phosphodiesterasen, Ionenkanälen und Proteinkinasen spielt die Guanylatcyclase eine entscheidende Rolle bei unterschiedlichen physiologischen Prozessen, insbesondere bei der Relaxation und Proliferation glatter Muskelzellen, der Plättchenaggregation und -adhäsion, der neuronalen Signalübertragung sowie bei Erkrankungen, welche auf einer Störung der vorstehend genannten Vorgänge beruhen. Unter pathophysiologischen Bedingungen kann das NO/cGMP-System supprimiert sein, was zum Beispiel zu Bluthochdruck, einer Plättchenaktivierung, einer vermehrten Zellproliferation, endothelialer Dysfunktion, Arteriosklerose, Angina pectoris, Herzinsuffizienz, Myokardinfarkt, Thrombosen, Schlaganfall und sexueller Dysfunktion führen kann.

Eine auf die Beeinflussung des cGMP-Signalweges in Organismen abzielende NO-unabhängige Behandlungsmöglichkeit für derartige Erkrankungen ist aufgrund der zu erwartenden hohen Effizienz und geringen Nebenwirkungen ein vielversprechender Ansatz.

Zur therapeutischen Stimulation der löslichen Guanylatcyclase wurden bisher ausschließlich Verbindungen wie organische Nitrate verwendet, deren Wirkung auf NO beruht. Dieses wird durch Biokonversion gebildet und aktiviert die lösliche Guanylatcyclase durch Angriff am Eisen-Zentralatom des Häms. Neben den Nebenwirkungen gehört die Toleranzentwicklung zu den entscheidenden Nachteilen dieser Behandlungsweise.

Vor einigen Jahren wurden einige Substanzen beschrieben, die die lösliche Guanylatcyclase direkt, d.h. ohne vorherige Freisetzung von NO stimulieren, wie beispielsweise 3-(5'-Hydroxymethyl-2'-furyl)-1-benzylindazol [YC-1; Wu et al., Blood 84 (1994), 4226; Mülsch et al., Brit. J. Pharmacol. 120 (1997), 681]. Zu den neueren Stimulatoren der löslichen Guanylatzyklase gehören u.a. BAY 41-2272, BAY 41-8543 und Riociguat (BAY 63-2521) (siehe z.B. Stasch J.-P. et al., Nat. Rev. Drug Disc. 2006; 5: 755-768; Stasch J.-P. et al., ChemMedChem 2009; 4: 853-865. Stasch J.-P. et al., Circulation 2011; 123: 2263-2273). Interessanterweise zeigen einige dieser sGC Stimulatoren, wie beispielsweise YC-1 oder BAY 41-2272 zusätzlich zur direkten Guanylatzyklasestimulation auch eine PDE5-inhibibitorische Wirkung. Um den cGMPpathway zu maximieren, ist es pharmakologisch wünschenswert, die Synthese von cGMP zu stimulieren und gleichzeitig den Abbau über PDE-5 zu inhibieren. Dieses duale Prinzip ist pharmakologisch besonders vorteilhaft (s. z.B. Oudout et al., Eur. Urol. 2011, 60, 1020-1026.). Das duale Prinzip wird im Sinne der vorliegenden Erfindung erfüllt, wenn die erfindungsgemäßen Verbindungen eine Wirkung an rekombinanter Guanylatcyclase-Reporterzellinien gemäß der Untersuchung unter B-2 als minimal effective concentration (MEC) von ≤ 3 µm zeigen und eine Inhibition der humanen Phosphodiesterase 5 (PDE5) gemäß der Untersuchung unter B-6 als IC50 < 100 nm zeigen.

Phosphodiesterase-5 (PDE5) ist der Name für eines der Enzyme, die die Phosphorsäureesterbindung in cGMP spalten, wobei 5'-Guanosinmonophosphat (5'-GMP) entsteht. Beim Menschen kommt die Phosphodiesterase-5 vorwiegend in der glatten Muskulatur des Penisschwellkörpers (Corpus cavernosum penis) und der Lungenarterien vor. Blockierung des cGMP-Abbaus durch Hemmung von PDE5 (mit beispielsweise Sildenafil, Vardenafil oder Tadalafil) führt zu vermehrten Signalen der Entspannungs-Signalwege und speziell zu erhöhter Blutzufuhr in den Penisschwellkörper und Druckerniedrigung in den Blutgefäßen der Lunge. Sie werden zur Behandlung der erektilen Dysfunktion und der pulmonalen arteriellen Hypertonie eingesetzt. Neben PDE5 gibt es weitere, ausschließlich cGMP spaltende Phosphodiesterasen (Stasch J.-P. et al. Circulation 2011).

Als Stimulatoren der löslichen Guanylatcyclase werden in WO 00/06568 und WO 00/06569 annellierte Pyrazol-Derivate und in WO 03/095451 Carbamat-substitutierte 3-Pyrimidinyl-Pyrazolopyridine offenbart. 3-Pyrimidinyl-Pyrazolopyridine mit Phenylamid-Substituenten werden in E. M. Becker et al., BMC Pharmacology 1 (13), 2001 beschrieben. WO 2004/009590 beschreibt Pyrazolopyridine mit substituierten 4-Aminopyrimidinen zur Behandlung von ZNS-Erkrankungen. WO 2010/065275 und WO 2011/149921 offenbaren substituierte Pyrrolo- und Dihydropyridopyrimidine als sGC Aktivatoren. Als sGC Stimulatoren werden in WO 2012/004259 annellierte Aminopyrimidine und in WO 2012/004258 annellierte Pyrimidine und Triazine beschrieben. WO 2012/28647 offenbart Pyrazolopyridine mit verschiedenen Azaheterocyclen zur Behandlung kardiovaskulärer Erkrankungen.

Aufgabe der vorliegenden Erfindung war die Bereitstellung neuer Substanzen, die als Stimulatoren der löslichen Guanylatcyclase und als Stimulatoren der löslichen Guanylatcyclase und Phosphodiesterase-5-Inhibitoren (duales Prinzip) wirken und ein gleiches oder verbessertes therapeutisches Profil gegenüber den aus dem Stand der Technik bekannten Verbindungen aufweisen, wie beispielsweise hinsichtlich ihrer *in-vivo* Eigenschaften, wie beispielsweise ihrem pharmakokinetischem und pharmakodynamischem Verhalten und/oder ihres Metabolismus-Profils und/oder ihrer Dosis-Wirkungsbeziehung.

Offenbarungsgegenstand sind Verbindungen der allgemeinen Formel (I) 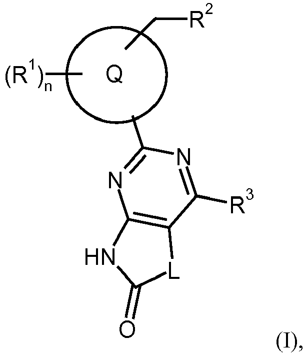 in welcher
- L: für eine Gruppe #¹-CR^{7A}R^{7B}-(CR^{8A}R^{8B})ₘ-#² steht, wobei
#¹ für die Anknüpfstelle an die Carbonylgruppe steht,
#² für die Anknüpfstelle an den Pyrimidinring steht,
m für eine Zahl 0, 1 oder 2 steht,
- R^{7A}: für Wasserstoff, Fluor, (C₁-C₄)-Alkyl, Hydroxy oder Amino steht,
worin (C₁-C₄)-Alkyl mit 1 bis 3 Substituenten unabhängig voneinander ausgewählt aus der Gruppe Fluor, Trifluormethyl, Hydroxy, Hydroxycarbonyl, (C₁-C₄)-Alkoxycarbonyl und Amino substituiert sein kann,
- R^{7B}: für Wasserstoff, Fluor, Difluormethyl, Trifluormethyl, (C₁-C₆)-Alkyl, (C₁-C₄)-Alkoxycarbonylamino, Cyano, (C₃-C₇)-Cycloalkyl, Difluormethoxy, Trifluormethoxy, Phenyl oder eine Gruppe der Formel -M-R¹³ steht,
worin (C₁-C₆)-Alkyl mit 1 bis 3 Substituenten unabhängig voneinander ausgewählt aus der Gruppe Fluor, Cyano, Trifluormethyl, (C₃-C₇)-Cycloalkyl, Hydroxy, Difluormethoxy, Trifluormethoxy, (C₁-C₄)-Alkoxy, Hydroxycarbonyl, (C₁-C₄)-Alkoxycarbonyl und Amino substituiert sein kann,
und worin
M für eine Bindung oder (C₁-C₄)-Alkandiyl steht,
R¹³ für -(C=O)ᵣ-OR¹⁴, -(C=O)ᵣ-NR¹⁴R¹⁵, -C(=S)-NR¹⁴R¹⁵, -NR¹⁴-(C=O)-R¹⁷, -NR¹⁴-(C=O)-NR¹⁵R¹⁶, -NR¹⁴-SO₂-NR¹⁵R¹⁶, -NR¹⁴-SO₂-R¹⁷, -S(O)ₛ-R¹⁷, -SO₂-NR¹⁴R¹⁵, 4- bis 7-gliedriges Heterocyclyl, Phenyl oder 5- oder 6-gliedriges Heteroaryl steht,
worin
r die Zahl 0 oder 1 bedeutet,
s die Zahl 0, 1 oder 2 bedeutet,
R¹⁴, R¹⁵ und R¹⁶ unabhängig voneinander jeweils für Wasserstoff, (C₁-C₆)-Alkyl, (C₃-C₈)-Cycloalkyl, 4- bis 7-gliedriges Heterocyclyl, Phenyl oder 5- oder 6-gliedriges Heteroaryl stehen, oder
R¹⁴ und R¹⁵ bilden zusammen mit dem/den Atom/-en, an die sie jeweils gebunden sind, einen 4- bis 7-gliedrigen Heterocyclus,
worin der 4- bis 7-gliedrigen Heterocyclus seinerseits mit 1 oder 2 Substituenten unabhängig voneinander ausgewählt aus der Gruppe Cyano, Trifluormethyl, (C₁-C₆)-Alkyl, Hydroxy, Oxo, (C₁-C₆)-Alkoxy, Trifluormethoxy, (C₁-C₆)-Alkoxycarbonyl, Amino, Mono-(C₁-C₆)-alkylamino und Di-(C₁-C₆)-alkylamino substituiert sein kann,
oder
R¹⁵ und R¹⁶ bilden zusammen mit dem/den Atom/-en, an die sie jeweils gebunden sind, einen 4- bis 7-gliedrigen Heterocyclus,
worin der 4- bis 7-gliedrigen Heterocyclus seinerseits mit 1 oder 2 Substituenten unabhängig voneinander ausgewählt aus der Gruppe Cyano, Trifluormethyl, (C₁-C₆)-Alkyl, Hydroxy, Oxo, (C₁-C₆)-Alkoxy, Trifluormethoxy, (C₁-C₆)-Alkoxycarbonyl, Amino, Mono-(C₁-C₆)-alkylamino und Di-(C₁-C₆)-alkylamino substituiert sein kann,
R¹⁷ für (C₁-C₆)-Alkyl oder (C₃-C₇)-Cycloalkyl steht,
oder
R¹⁴ und R¹⁷ bilden zusammen mit dem/den Atom/-en, an die sie jeweils gebunden sind, einen 4- bis 7-gliedrigen Heterocyclus,
worin der 4- bis 7-gliedrigen Heterocyclus seinerseits mit 1 oder 2 Substituenten unabhängig voneinander ausgewählt aus der Gruppe Cyano, Trifluormethyl, (C₁-C₆)-Alkyl, Hydroxy, Oxo, (C₁-C₆)-Alkoxy, Trifluormethoxy, (C₁-C₆)-Alkoxycarbonyl, Amino, Mono-(C₁-C₆)-alkylamino und Di-(C₁-C₆)-alkylamino substituiert sein kann,
und worin 4- bis 7-gliedriges Heterocyclyl, Phenyl und 5- oder 6-gliedriges Heteroaryl ihrerseits mit 1 bis 3 Substituenten unabhängig voneinander ausgewählt aus der Gruppe Halogen, Cyano, Difluormethyl, Trifluormethyl, (C₁-C₆)-Alkyl, (C₃-C₇)-Cycloalkyl, Hydroxy, Oxo, Thiooxo und (C₁-C₄)-Alkoxy substituiert sein können,
und worin die zuvor genannten (C₁-C₄)-Alkyl-, (C₁-C₆)-Alkyl-, (C₃-C₈)-Cycloalkyl-, (C₃-C₇)-Cycloalkyl- und 4- bis 7-gliedriges Heterocyclyl-Gruppen sofern nicht anders angegeben jeweils unabhängig voneinander weiterhin mit 1 bis 3 Substituenten unabhängig voneinander ausgewählt aus der Gruppe Fluor, Difluormethyl, Trifluormethyl, (C₁-C₄)-Alkyl, (C₃-C₇)-Cycloalkyl, Hydroxy, Difluormethoxy, Trifluormethoxy, (C₁-C₄)-Alkoxy, Hydroxycarbonyl, (C₁-C₄)-Alkoxycarbonyl, Amino, Phenyl, 4- bis 7-gliedriges Heterocyclyl und 5- oder 6-gliedriges Heteroaryl substituiert sein können,
oder
R^{7A} und R^{7B} zusammen mit dem Kohlenstoffatom, an das sie gebunden sind, eine (C₂-C₄)-Alkenyl-Gruppe, eine Oxo-Gruppe, einen 3- bis 6-gliedrigen Carbocyclus oder einen 4- bis 7-gliedrigen Heterocyclus bilden,
worin der 3- bis 6-gliedrigen Carbocyclus und der 4- bis 7-gliedrigen Heterocyclus mit 1 oder 2 Substituenten unabhängig voneinander ausgewählt aus der Gruppe Fluor und (C₁-C₄)-Alkyl substituiert sein können,
R^{8A} für Wasserstoff, Fluor, (C₁-C₄)-Alkyl oder Hydroxy steht,
R^{8B} für Wasserstoff, Fluor, (C₁-C₄)-Alkyl oder Trifluormethyl steht,
der Ring Q für 8- oder 9-gliedriges Heteroaryl steht,
- R³: für -OR⁴ oder -NR⁵R⁶ steht,
wobei
R⁴ für Wasserstoff, (C₁-C₆)-Alkyl, (C₃-C₇)-Cycloalkyl, 4- bis 7-gliedriges Heterocyclyl, Phenyl oder 5- oder 6-gliedriges Heteroaryl steht,
worin (C₁-C₆)-Alkyl, (C₃-C₇)-Cycloalkyl, 4- bis 7-gliedriges Heterocyclyl, Phenyl und 5- oder 6-gliedriges Heteroaryl mit 1 bis 3 Substituenten unabhängig voneinander ausgewählt aus der Gruppe Fluor, Difluormethyl, Trifluormethyl, (C₁-C₆)-Alkyl, (C₃-C₆)-Cycloalkyl, Difluormethoxy, Trifluormethoxy, Oxo, -(C=O)ₚ-OR⁹, -C(=O)ₚ-NR⁹R¹⁰, -NR⁹-(C=O)-R¹⁰, -NR⁹-(C=O)-OR¹⁰, -NR⁹-(C=O)-NR¹⁰R¹¹, -NR⁹-SO₂-R¹⁰, -S(O)_{q}-R¹² und -SO₂-NR⁹R¹⁰ substituiert sein können,
worin
   p die Zahl 0 oder 1 bedeutet,
   q die Zahl 0, 1 oder 2 bedeutet,
   R⁹, R¹⁰ und R¹¹ unabhängig voneinander jeweils für Wasserstoff, (C₁-C₆)-Alkyl oder (C₃-C₈)-Cycloalkyl stehen,
   worin (C₁-C₆)-Alkyl seinerseits mit 1 oder 2 Substituenten unabhängig voneinander ausgewählt aus der Gruppe Fluor, Difluormethyl, Trifluormethyl, Hydroxy, (C₁-C₆)-Alkoxy, Difluormethoxy, Trifluormethoxy, (C₁-C₆)-Alkoxycarbonyl, Amino, Mono-(C₁-C₆)-alkylamino, Di-(C₁-C₆)-alkylamino und 4- bis 7-gliedriges Heterocyclyl substituiert sein kann,
   oder
   R⁹ und R¹⁰ bilden zusammen mit dem/den Atom/-en, an die sie jeweils gebunden sind, einen 4- bis 7-gliedrigen Heterocyclus,
   worin der 4- bis 7-gliedrigen Heterocyclus seinerseits mit 1 oder 2 Substituenten unabhängig voneinander ausgewählt aus der Gruppe Fluor, Trifluormethyl, (C₁-C₆)-Alkyl, Hydroxy, Oxo, (C₁-C₆)-Alkoxy, Trifluormethoxy, (C₁-C₆)-Alkoxycarbonyl, Amino, Mono-(C₁-C₆)-alkylamino und Di-(C₁-C₆)-alkylamino substituiert sein kann,
   oder
   R¹⁰ und R¹¹ bilden zusammen mit dem/den Atom/-en, an die sie jeweils gebunden sind, einen 4- bis 7-gliedrigen Heterocyclus,
   worin der 4- bis 7-gliedrigen Heterocyclus seinerseits mit 1 oder 2 Substituenten unabhängig voneinander ausgewählt aus der Gruppe Fluor, Trifluormethyl, (C₁-C₆)-Alkyl, Hydroxy, Oxo, (C₁-C₆)-Alkoxy, Trifluormethoxy, (C₁-C₆)-Alkoxycarbonyl, Amino, Mono-(C₁-C₆)-alkylamino und Di-(C₁-C₆)-alkylamino substituiert sein kann,
   und worin
   R¹² für (C₁-C₆)-Alkyl oder (C₃-C₇)-Cycloalkyl steht,
und
worin die zuvor genannten (C₁-C₄)-Alkyl-, (C₁-C₆)-Alkyl-, (C₃-C₈)-Cycloalkyl-, (C₃-C₇)-Cycloalkyl- und 4- bis 7-gliedriges Heterocyclyl-Gruppen sofern nicht anders angegeben jeweils unabhängig voneinander weiterhin mit 1 bis 3 Substituenten unabhängig voneinander ausgewählt aus der Gruppe Fluor, Difluormethyl, Trifluormethyl, (C₁-C₄)-Alkyl, (C₃-C₇)-Cycloalkyl, Hydroxy, Difluormethoxy, Trifluormethoxy, (C₁-C₄)-Alkoxy, Hydroxycarbonyl, (C₁-C₄)-Alkoxycarbonyl, Amino, Phenyl, 4- bis 7-gliedriges Heterocyclyl und 5- oder 6-gliedriges Heteroaryl substituiert sein können,
R⁵ für Wasserstoff oder (C₁-C₄)-Alkyl steht,
R⁶ für (C₁-C₆)-Alkyl, (C₃-C₇)-Cycloalkyl, 4- bis 7-gliedriges Heterocyclyl, Phenyl oder 5- oder 6-gliedriges Heteroaryl steht,
worin (C₁-C₆)-Alkyl (C₃-C₇)-Cycloalkyl, 4- bis 7-gliedriges Heterocyclyl, Phenyl und 5- oder 6-gliedriges Heteroaryl mit 1 bis 3 Substituenten unabhängig voneinander ausgewählt aus der Gruppe Fluor, Difluormethyl, Trifluormethyl, (C₁-C₆)-Alkyl, (C₃-C₇)-Cycloalkyl, Difluormethoxy, Trifluormethoxy, Oxo,-(C=O)ₚ-OR⁹, -(C=O)ₚ-NRR⁹R¹⁰, -NR⁹-(C=O)-R¹⁰, -NR⁹-(C=O)-OR¹⁰, -NR⁹-(C=O)-NR¹⁰R¹¹, -NR⁹-SO₂-R¹⁰, -S(O)_{q}-R¹², -SO₂-NR⁹R¹⁰, Phenyl, 4-bis 7-gliedriges Heterocyclyl und 5- oder 6-gliedriges Heteroaryl substituiert sein können,
worin
   p die Zahl 0 oder 1 bedeutet,
   q die Zahl 0, 1 oder 2 bedeutet,
   R⁹, R¹⁰ und R¹¹ unabhängig voneinander jeweils für Wasserstoff, (C₁-C₆)-Alkyl oder (C₃-C₈)-Cycloalkyl stehen,
   worin (C₁-C₆)-Alkyl seinerseits mit 1 bis 3 Substituenten unabhängig voneinander ausgewählt aus der Gruppe Fluor, Difluormethyl, Trifluormethyl, (C₃-C₇)-Cycloalkyl, Difluormethoxy, Trifluormethoxy und (C₁-C₄)-Alkoxy substituiert sein kann,
   oder
   R⁹ und R¹⁰ bilden zusammen mit dem/den Atom/-en, an die sie jeweils gebunden sind, einen 4- bis 7-gliedrigen Heterocyclus,
   worin der 4- bis 7-gliedrigen Heterocyclus seinerseits mit 1 oder 2 Substituenten unabhängig voneinander ausgewählt aus der Gruppe Fluor, Cyano, Trifluormethyl, (C₁-C₆)-Alkyl, Hydroxy, Oxo, (C₁-C₆)-Alkoxy, Trifluormethoxy, (C₁-C₆)-Alkoxycarbonyl, Amino, Mono-(C₁-C₆)-alkylamino und Di-(C₁-C₆)-alkylamino substituiert sein kann,
   oder
   R¹⁰ und R¹¹ bilden zusammen mit dem/den Atom/-en, an die sie jeweils gebunden sind, einen 4- bis 7-gliedrigen Heterocyclus,
   worin der 4- bis 7-gliedrigen Heterocyclus seinerseits mit 1 oder 2 Substituenten unabhängig voneinander ausgewählt aus der Gruppe Cyano, Trifluormethyl, (C₁-C₆)-Alkyl, Hydroxy, Oxo, (C₁-C₆)-Alkoxy, Trifluormethoxy, (C₁-C₆)-Alkoxycarbonyl, Amino, Mono-(C₁-C₆)-alkylamino und Di-(C₁-C₆)-alkylamino substituiert sein kann,
   worin
   R¹² für (C₁-C₆)-Alkyl oder (C₃-C₇)-Cycloalkyl steht,
   und
   worin Phenyl, 4- bis 7-gliedriges Heterocyclyl und 5- oder 6-gliedriges Heteroaryl ihrerseits mit 1 bis Substituenten unabhängig voneinander ausgewählt aus der Gruppe Halogen, Cyano, Difluormethyl, Trifluormethyl, (C₁-C₄)-Alkyl, (C₃-C₇)-Cycloalkyl, Hydroxy, Oxo, Difluormethoxy, Trifluormethoxy und (C₁-C₄)-Alkoxy substituiert sein können,
   oder
   R⁵ und R⁶ bilden zusammen mit dem Stickstoffatom, an das sie gebunden sind, einen 4- bis 7-gliedrigen Heterocyclus oder ein 5- oder 6-gliedriges Heteroaryl,
   worin der 4- bis 7-gliedrigen Heterocyclus und das 5- oder 6-gliedriges Heteroaryl mit 1 bis 3 Substituenten unabhängig voneinander ausgewählt aus der Gruppe Fluor, Cyano, Difluormethyl, Trifluormethyl, (C₁-C₆)-Alkyl, (C₃-C₇)-Cycloalkyl, Hydroxy, Oxo, (C₁-C₆)-Alkoxy, Difluormethoxy, Trifluormethoxy, (C₁-C₆)-Alkoxycarbonyl, (C₁-C₆)-Alkylcarbonylamino, Amino, Mono-(C₁-C₆)-alkylamino, Di-(C₁-C₆)-alkylamino und 4- bis 7-gliedriges Heterocyclyl substituiert sein können,
   und
   worin die zuvor genannten (C₁-C₄)-Alkyl-, (C₁-C₆)-Alkyl-, (C₃-C₈)-Cycloalkyl-, (C₃-C₇)-Cycloalkyl- und 4- bis 7-gliedriges Heterocyclyl-Gruppen sofern nicht anders angegeben jeweils unabhängig voneinander weiterhin mit 1 bis 3 Substituenten unabhängig voneinander ausgewählt aus der Gruppe Fluor, Difluormethyl, Trifluormethyl, (C₁-C₄)-Alkyl, (C₃-C₇)-Cycloalkyl, Hydroxy, Difluormethoxy, Trifluormethoxy, (C₁-C₄)-Alkoxy, Hydroxycarbonyl, (C₁-C₄)-Alkoxycarbonyl, Amino, Phenyl, 4- bis 7-gliedriges Heterocyclyl und 5- oder 6-gliedriges Heteroaryl substituiert sein können,

R¹ für Fluor, Chlor, Cyano, Difluormethyl, Trifluormethyl, (C₁-C₄)-Alkyl, (C₃-C₇)-Cycloalkyl oder (C₁-C₄)-Alkoxy steht,
n für eine Zahl 0, 1 oder 2 steht,
R² für Trifluormethyl, (C₁-C₆)-Alkyl, (C₃-C₈)-Cycloalkyl, Phenyl oder 5- oder 6-gliedriges Heteroaryl steht,
wobei (C₁-C₆)-Alkyl mit einem Substituenten ausgewählt aus der Gruppe Difluormethyl und Trifluormethyl substituiert ist,
wobei (C₁-C₆)-Alkyl mit 1 bis 3 Substituenten Fluor substituiert sein kann,
wobei (C₃-C₈)-Cycloalkyl mit 1 oder 2 Substituenten unabhängig voneinander ausgewählt aus der Gruppe Fluor, Methyl und Methoxy substituiert sein kann,
wobei Phenyl mit 1 bis 3 Substituenten Fluor substituiert ist,
wobei Phenyl mit 1 oder 2 Substituenten unabhängig voneinander ausgewählt aus der Gruppe Methyl und Methoxy substituiert sein kann,
und
wobei 5- und 6-gliedriges Heteroaryl mit 1 oder 2 Substituenten unabhängig voneinander ausgewählt aus der Gruppe Fluor, Trifluormethyl und Methyl substituiert sein können,
sowie ihre *N*-Oxide, Salze, Solvate, Salze der *N*-Oxide und Solvate der *N*-Oxide und Salze.

Erfindungsgemäße Verbindungen sind die Verbindungen der Formel (I) und deren *N*-Oxide, Salze, Solvate und Solvate der *N*-Oxide und Salze, die von Formel (I) umfassten Verbindungen der nachfolgend genannten Formeln und deren *N*-Oxide, Salze, Solvate und Solvate der *N*-Oxide und Salze sowie die von Formel (I) umfassten, nachfolgend als Ausführungsbeispiele genannten Verbindungen und deren *N*-Oxide, Salze, Solvate und Solvate der *N*-Oxide und Salze, soweit es sich bei den von Formel (I) umfassten, nachfolgend genannten Verbindungen nicht bereits um *N-*Oxide, Salze, Solvate und Solvate der *N*-Oxide und Salze handelt.

Als Salze sind im Rahmen der vorliegenden Erfindung physiologisch unbedenkliche Salze der erfindungsgemäßen Verbindungen bevorzugt. Umfasst sind auch Salze, die für pharmazeutische Anwendungen selbst nicht geeignet sind, jedoch beispielsweise für die Isolierung oder Reinigung der erfindungsgemäßen Verbindungen verwendet werden können.

Physiologisch unbedenkliche Salze der erfindungsgemäßen Verbindungen umfassen Säureadditionssalze von Mineralsäuren, Carbonsäuren und Sulfonsäuren, z.B. Salze der Chlorwasserstoffsäure, Bromwasserstoffsäure, Schwefelsäure, Phosphorsäure, Methansulfonsäure, Ethansulfonsäure, Toluolsulfonsäure, Benzolsulfonsäure, Naphthalindisulfonsäure, Ameisensäure, Essigsäure, Trifluoressigsäure, Propionsäure, Milchsäure, Weinsäure, Äpfelsäure, Zitronensäure, Fumarsäure, Maleinsäure und Benzoesäure.

Physiologisch unbedenkliche Salze der erfindungsgemäßen Verbindungen umfassen auch Salze üblicher Basen, wie beispielhaft und vorzugsweise Alkalimetallsalze (z.B. Natrium- und Kaliumsalze), Erdalkalisalze (z.B. Calcium- und Magnesiumsalze) und Ammoniumsalze, abgeleitet von Ammoniak oder organischen Aminen mit 1 bis 16 C-Atomen, wie beispielhaft und vorzugsweise Ethylamin, Diethylamin, Triethylamin, Ethyldiisopropylamin, Monoethanolamin, Diethanolamin, Triethanolamin, Dicyclohexylamin, Dimethylaminoethanol, Prokain, Dibenzylamin, N-Methylmorpholin, Arginin, Lysin, Ethylendiamin und N-Methylpiperidin.

Als Solvate werden im Rahmen der Erfindung solche Formen der erfindungsgemäßen Verbindungen bezeichnet, welche in festem oder flüssigem Zustand durch Koordination mit Lösungsmittelmolekülen einen Komplex bilden. Hydrate sind eine spezielle Form der Solvate, bei denen die Koordination mit Wasser erfolgt. Als Solvate sind im Rahmen der vorliegenden Erfindung Hydrate bevorzugt.

Die erfindungsgemäßen Verbindungen können in Abhängigkeit von ihrer Struktur in unterschiedlichen stereoisomeren Formen existieren, d.h. in Gestalt von Konfigurationsisomeren oder gegebenenfalls auch als Konformationsisomere (Enantiomere und/oder Diastereomere, einschließlich solcher bei Atropisomeren). Die vorliegende Erfindung umfasst deshalb die Enantiomere und Diastereomere und ihre jeweiligen Mischungen. Aus solchen Mischungen von Enantiomeren und/ oder Diastereomeren lassen sich die stereoisomer einheitlichen Bestandteile in bekannter Weise isolieren; vorzugsweise werden hierfür chromatographische Verfahren verwendet, insbesondere die HPLC-Chromatographie an achiraler bzw. chiraler Phase.

Sofern die erfindungsgemäßen Verbindungen in tautomeren Formen vorkommen können, umfasst die vorliegende Erfindung sämtliche tautomere Formen.

Die vorliegende Erfindung umfasst auch alle geeigneten isotopischen Varianten der erfindungsgemäßen Verbindungen. Unter einer isotopischen Variante einer erfindungsgemäßen Verbindung wird hierbei eine Verbindung verstanden, in welcher mindestens ein Atom innerhalb der erfindungsgemäßen Verbindung gegen ein anderes Atom der gleichen Ordnungszahl, jedoch mit einer anderen Atommasse als der gewöhnlich oder überwiegend in der Natur vorkommenden Atommasse ausgetauscht ist. Beispiele für Isotope, die in eine erfindungsgemäße Verbindung inkorporiert werden können, sind solche von Wasserstoff, Kohlenstoff, Stickstoff, Sauerstoff, Phosphor, Schwefel, Fluor, Chlor, Brom und Iod, wie ²H (Deuterium), ³H (Tritium), ¹³C, ¹⁴C, ¹⁵N, ¹⁷O, ¹⁸O ³²P, ³³P, ³³S, ³⁴S, ³⁵S, ³⁶S, ¹⁸F, ³⁶Cl, ⁸²Br, ¹²³I, ¹²⁴I, ¹²⁹I und ¹³¹I. Bestimmte isotopische Varianten einer erfindungsgemäßen Verbindung, wie insbesondere solche, bei denen ein oder mehrere radioaktive Isotope inkorporiert sind, können von Nutzen sein beispielsweise für die Untersuchung des Wirkmechanismus oder der Wirkstoff-Verteilung im Körper; aufgrund der vergleichsweise leichten Herstell- und Detektierbarkeit sind hierfür insbesondere mit ³H- oder ¹⁴C-Isotopen markierte Verbindungen geeignet. Darüber hinaus kann der Einbau von Isotopen, wie beispielsweise von Deuterium, zu bestimmten therapeutischen Vorteilen als Folge einer größeren metabolischen Stabilität der Verbindung führen, wie beispielsweise eine Verlängerung der Halbwertszeit im Körper oder eine Reduktion der erforderlichen Wirkdosis; solche Modifikationen der erfindungsgemäßen Verbindungen können daher gegebenenfalls auch eine bevorzugte Ausführungsform der vorliegenden Erfindung darstellen. Isotopische Varianten der erfindungsgemäßen Verbindungen können nach den dem Fachmann bekannten Verfahren hergestellt werden, so beispielsweise nach den weiter unten beschriebenen Methoden und den bei den Ausführungsbeispielen wiedergegebenen Vorschriften, indem entsprechende isotopische Modifikationen der jeweiligen Reagentien und/oder Ausgangsverbindungen eingesetzt werden.

Außerdem umfasst die vorliegende Erfindung auch Prodrugs der erfindungsgemäßen Verbindungen. Der Begriff "Prodrugs" bezeichnet hierbei Verbindungen, welche selbst biologisch aktiv oder inaktiv sein können, jedoch während ihrer Verweilzeit im Körper zu erfindungsgemäßen Verbindungen umgesetzt werden (beispielsweise metabolisch oder hydrolytisch).

Im Rahmen der vorliegenden Erfindung haben die Substituenten, soweit nicht anders spezifiziert, die folgende Bedeutung:
Alkyl steht im Rahmen der Erfindung für einen linearen oder verzweigten Alkylrest mit der jeweils angegebenen Anzahl an Kohlenstoffatomen. Beispielhaft und vorzugsweise seien genannt: Methyl, Ethyl, n-Propyl, Isopropyl, n-Butyl, iso-Butyl, 1-Methylpropyl, tert.-Butyl, n-Pentyl, iso-Pentyl, 1-Ethylpropyl, 1-Methylbutyl, 2-Methylbutyl, 3-Methylbutyl, n-Hexyl, 1-Methylpentyl, 2-Methylpentyl, 3-Methylpentyl, 4-Methylpentyl, 3,3-Dimethylbutyl, 1-Ethylbutyl und 2-Ethylbutyl.
Cycloalkyl bzw. Carbocyclus steht in Rahmen der Erfindung für einen monocyclischen, gesättigten Alkylrest mit der jeweils angegebenen Anzahl an Kohlenstoffatomen. Beispielhaft und vorzugsweise seien genannt: Cyclopropyl, Cyclobutyl, Cyclopentyl, Cyclohexyl und Cycloheptyl.
5- bis 7-gliedrige gesättigter oder teilweise ungesättigte Carbocyclus steht in Rahmen der Erfindung für einen gesättigten oder teilweise ungesättigten cyclischen Alkylrest mit der jeweils angegebenen Anzahl an Kohlenstoffatomen. Beispielhaft und vorzugsweise seien genannt: Cyclopentyl, Cyclohexyl, Cycloheptyl, Cyclopentenyl, Cyclohexenyl und Cycloheptenyl.
Alkandiyl steht im Rahmen der Erfindung für einen linearen oder verzweigten divalenten Alkylrest mit 1 bis 4 Kohlenstoffatomen. Beispielhaft und vorzugsweise seien genannt: Methylen, Ethan-1,2-diyl, Ethan-1,1-diyl, Propan-1,3-diyl, Propan-1,1-diyl, Propan-1,2-diyl, Propan-2,2-diyl, Butan-1,4-diyl, Butan-1,2-diyl, Butan-1,3-diyl und Butan-2,3-diyl.
Alkenyl steht im Rahmen der Erfindung für einen linearen oder verzweigten Alkenylrest mit 2 bis 4 Kohlenstoffatomen und einer Doppelbindung. Beispielhaft und vorzugsweise seien genannt: Vinyl, Allyl, Isopropenyl und n-But-2-en-1-yl.
Alkoxy steht im Rahmen der Erfindung für einen linearen oder verzweigten Alkoxyrest mit 1 bis 6 bzw. 1 bis 4 Kohlenstoffatomen. Beispielhaft seien genannt: Methoxy, Ethoxy, n-Propoxy, Isopropoxy, 1-Methylpropoxy, n-Butoxy, iso-Butoxy, tert.-Butoxy, n-Pentoxy, iso-Pentoxy, 1-Ethylpropoxy, 1-Methylbutoxy, 2-Methylbutoxy, 3-Methylbutoxy und n-Hexoxy. Bevorzugt ist ein linearer oder verzweigter Alkoxyrest mit 1 bis 4 Kohlenstoffatomen. Beispielhaft und vorzugsweise seien genannt: Methoxy, Ethoxy, n-Propoxy, Isopropoxy, 1-Methylpropoxy, n-Butoxy, iso-Butoxy, tert.-Butoxy,
Alkoxycarbonyl steht im Rahmen der Erfindung für einen linearen oder verzweigten Alkoxyrest mit 1 bis 4 Kohlenstoffatomen und einer am Sauerstoff angebundenen Carbonylgruppe. Beispielhaft und vorzugsweise seien genannt: Methoxycarbonyl, Ethoxycarbonyl, n-Propoxycarbonyl, Isopropoxycarbonyl und tert.-Butoxycarbonyl.
Alkoxycarbonylamino steht im Rahmen der Erfindung für eine Amino-Gruppe mit einem linearen oder verzweigten Alkoxycarbonyl-Substituenten, der 1 bis 4 Kohlenstoffatome in der Alkylkette aufweist und über die Carbonylgruppe mit dem N-Atom verknüpft ist. Beispielhaft und vorzugsweise seien genannt: Methoxycarbonylamino, Ethoxycarbonylamino, Propoxycarbonylamino, n-Butoxycarbonylamino, iso-Butoxycarbonylamino und tert.-Butoxycarbonylamino.
Mono-alkylamino steht im Rahmen der Erfindung für eine Amino-Gruppe mit einem linearen oder verzweigten Alkylsubstituenten, der 1 bis 6 Kohlenstoffatome aufweist. Beispielhaft und vorzugsweise seien genannt: Methylamino, Ethylamino, n-Propylamino, Isopropylamino und tert.-Butylamino.
Di-alkylamino steht im Rahmen der Erfindung für eine Amino-Gruppe mit zwei gleichen oder verschiedenen linearen oder verzweigten Alkylsubstituenten, die jeweils 1 bis 6 Kohlenstoffatome aufweisen. Beispielhaft und vorzugsweise seien genannt: *N,N*-Dimethylamino, *N,N*-Diethylamino, *N*-Ethyl-*N*-methylamino, *N*-Methyl-*N*-n-propylamino, *N*-Isopropyl-*N*-n-propylamino, *N*-tert.-Butyl-*N*-methylamino, *N*-Ethyl-*N*-n-pentylamino und *N*-n-Hexyl-*N*-methylamino.
5- bis 7-gliedrige gesättigter oder teilweise ungesättigter Heterocyclus steht im Rahmen der Erfindung für einen gesättigten oder teilweise ungesättigten Heterocyclus mit insgesamt 5 bis 7 Ringatomen, der ein Ring-Heteroatome aus der Reihe N, O, S, SO und/oder SO₂ enthält. Beispielhaft seien genannt: Pyrrolidinyl, Tetrahydrofuranyl, Piperidinyl, Tetrahydropyranyl, Dihydropyrrolyl, Dihydropyridyl.
Heterocyclyl bzw. Heterocyclus steht im Rahmen der Erfindung für einen gesättigten Heterocyclus mit insgesamt 4 bis 7 Ringatomen, der ein oder zwei Ring-Heteroatome aus der Reihe N, O, S, SO und/oder SO₂ enthält. Beispielhaft seien genannt: Azetidinyl, Oxetanyl, Pyrrolidinyl, Pyrazolidinyl, Imidazolinyl, Tetrahydrofuranyl, Piperidinyl, Piperazinyl, Tetrahydropyranyl, Morpholinyl, Thiomorpholinyl und Dioxidothiomorpholinyl. Bevorzugt sind Azetidinyl, Oxetanyl, Pyrrolidinyl, Tetrahydrofuranyl, Piperidinyl, Tetrahydropyranyl und Morpholinyl.
5- oder 6-gliedrige Heteroaryl steht im Rahmen der Erfindung für einen monocyclischen aromatischen Heterocyclus (Heteroaromaten) mit insgesamt 5 oder 6 Ringatomen, der bis zu drei gleiche oder verschiedene Ring-Heteroatome aus der Reihe N, O und/oder S enthält und über ein Ring-Kohlenstoffatom oder gegebenenfalls über ein Ring-Stickstoffatom verknüpft ist. Beispielhaft und vorzugsweise seien genannt: Furyl, Pyrrolyl, Thienyl, Pyrazolyl, Imidazolyl, Thiazolyl, Oxazolyl, Isoxazolyl, Isothiazolyl, Triazolyl, Oxadiazolyl, Thiadiazolyl, Pyridyl, Pyrimidinyl, Pyridazinyl, Pyrazinyl und Triazinyl. Bevorzugt sind: Pyrazolyl, Oxazolyl, Thiazolyl, Triazolyl, Oxadiazolyl, Thiadiazolyl, Pyridyl und Pyrimidinyl.
8- oder 9-gliedrige Heteroaryl steht im Rahmen der Erfindung für einen bicyclischen aromatischen oder teilweise ungesättigten Heterocyclus mit insgesamt 8 oder 9 Ringatomen, der mindestens zwei Stickstoffatome und bis zu zwei weitere, gleiche oder verschiedene Ring-Heteroatome aus der Reihe N, O und/oder S enthält. Beispielhaft seien genannt: Dihydrothienopyrazolyl, Thienopyrazolyl, Pyrazolopyrazolyl, Imidazothiazolyl, Tetrahydrocyclopentapyrazolyl, Dihydrocyclopentapyrazolyl, Tetrahydroindazolyl, Dihydroindazolyl, Indazolyl, Pyrazolopyridyl, Tetrahydropyrazolopyridyl, Pyrazolopyrimidinyl, Imidazopyridyl und Imidazopyridazinyl. Bevorzugt sind: Indazolyl, Pyrazolo[3,4-b]pyridyl, Pyrazolo[3,4-b]pyrimidinyl und Imidazo[1,5-a]pyridyl.
Halogen steht im Rahmen der Erfindung für Fluor, Chlor, Brom und Iod. Bevorzugt sind Brom und Iod.
Eine Oxo-Gruppe steht im Rahmen der Erfindung für ein Sauerstoffatom, das über eine Doppelbindung an ein Kohlenstoffatom gebunden ist.
Eine Thiooxo-Gruppe steht im Rahmen der Erfindung für ein Schwefelatom, das über eine Doppelbindung an ein Kohlenstoffatom gebunden ist.

In der Formel der Gruppe, für die L bzw. Q stehen kann, steht der Endpunkt der Linie, an dem das Zeichen #¹, #², * und ** steht, nicht für ein Kohlenstoffatom beziehungsweise eine CH₂-Gruppe, sondern ist Bestandteil der Bindung zu dem jeweils bezeichneten Atom, an das L bzw. Q gebunden ist.

Wenn Reste in den erfindungsgemäßen Verbindungen substituiert sind, können die Reste, soweit nicht anders spezifiziert, ein- oder mehrfach substituiert sein. Im Rahmen der vorliegenden Erfindung gilt, dass für alle Reste, die mehrfach auftreten, deren Bedeutung unabhängig voneinander ist. Eine Substitution mit ein, zwei oder drei gleichen oder verschiedenen Substituenten ist bevorzugt.

Im Sinne der vorliegenden Erfindung umfasst der Begriff "Behandlung" oder "behandeln" ein Hemmen, Verzögern, Aufhalten, Lindern, Abschwächen, Einschränken, Verringern, Unterdrücken, Zurückdrängen oder Heilen einer Krankheit, eines Leidens, einer Erkrankung, einer Verletzung oder einer gesundheitlichen Störung, der Entfaltung, des Verlaufs oder des Fortschreitens solcher Zustände und/oder der Symptome solcher Zustände. Der Begriff "Therapie" wird hierbei als synonym mit dem Begriff "Behandlung" verstanden.

Die Begriffe "Prävention", "Prophylaxe" oder "Vorbeugung" werden im Rahmen der vorliegenden Erfindung synonym verwendet und bezeichnen das Vermeiden oder Vermindern des Risikos, eine Krankheit, ein Leiden, eine Erkrankung, eine Verletzung oder eine gesundheitliche Störung, eine Entfaltung oder ein Fortschreiten solcher Zustände und/oder die Symptome solcher Zustände zu bekommen, zu erfahren, zu erleiden oder zu haben.

Die Behandlung oder die Prävention einer Krankheit, eines Leidens, einer Erkrankung, einer Verletzung oder einer gesundheitlichen Störung können teilweise oder vollständig erfolgen.

Offenbarungsgegenstand sind weiterhin Verbindungen der Formel (I), in welcher
- L: für eine Gruppe #¹-CR^{7A}R^{7B}-(CR^{8A}R^{8B})ₘ-#² steht,
wobei
#¹ für die Anknüpfstelle an die Carbonylgruppe steht,
#² für die Anknüpfstelle an den Pyrimidinring steht,
m für eine Zahl 0, 1 oder 2 steht,
R^{7A} für Wasserstoff, Fluor, (C₁-C₄)-Alkyl, Hydroxy oder Amino steht,
worin (C₁-C₄)-Alkyl mit 1 bis 3 Substituenten unabhängig voneinander ausgewählt aus der Gruppe Fluor, Trifluormethyl, Hydroxy, Hydroxycarbonyl, (C₁-C₄)-Alkoxycarbonyl und Amino substituiert sein kann,
R^{7B} für Wasserstoff, Fluor, Difluormethyl, Trifluormethyl, (C₁-C₆)-Alkyl, (C₁-C₄)-Alkoxycarbonylamino, Cyano, (C₃-C₇)-Cycloalkyl, Difluormethoxy, Trifluormethoxy, Phenyl oder eine Gruppe der Formel -M-R¹³ steht,
worin (C₁-C₆)-Alkyl mit 1 bis 3 Substituenten unabhängig voneinander ausgewählt aus der Gruppe Fluor, Cyano, Trifluormethyl, (C₃-C₇)-Cycloalkyl, Hydroxy, Difluormethoxy, Trifluormethoxy, (C₁-C₄)-Alkoxy, Hydroxycarbonyl, (C₁-C₄)-Alkoxycarbonyl und Amino substituiert sein kann,
und worin
M für eine Bindung oder (C₁-C₄)-Alkandiyl steht,
R¹³ für -(C=O)ᵣ-OR¹⁴, -(C=O)ᵣ-NR¹⁴R¹⁵, -C(=S)-NR¹⁴R¹⁵, -NR¹⁴-(C=O)-R¹⁷, -NR¹⁴-(C=O)-NR¹⁵R¹⁶, -NR¹⁴-SO₂-NR¹⁵R¹⁶, -NR¹⁴-SO²-R¹⁷, -S(O)ₛ-Erz, -SO₂-NR¹⁴R¹⁵, 4- bis 7-gliedriges Heterocyclyl, Phenyl oder 5- oder 6-gliedriges Heteroaryl steht, worin
r die Zahl 0 oder 1 bedeutet,
s die Zahl 0, 1 oder 2 bedeutet,
R¹⁴, R¹⁵ und R¹⁶ unabhängig voneinander jeweils für Wasserstoff, (C₁-C₆)-Alkyl, (C₃-C₈)-Cycloalkyl, 4- bis 7-gliedriges Heterocyclyl, Phenyl oder 5- oder 6-gliedriges Heteroaryl stehen,
oder
R¹⁴ und R¹⁵ bilden zusammen mit dem/den Atom/-en, an die sie jeweils gebunden sind, einen 4- bis 7-gliedrigen Heterocyclus, worin der 4- bis 7-gliedrigen Heterocyclus seinerseits mit 1 oder 2 Substituenten unabhängig voneinander ausgewählt aus der Gruppe Cyano, Trifluormethyl, (C₁-C₆)-Alkyl, Hydroxy, Oxo, (C₁-C₆)-Alkoxy, Trifluormethoxy, (C₁-C₆)-Alkoxycarbonyl, Amino, Mono-(C₁-C₆)-alkylamino und Di-(C₁-C₆)-alkylamino substituiert sein kann,
oder
R¹⁵ und R¹⁶ bilden zusammen mit dem/den Atom/-en, an die sie jeweils gebunden sind, einen 4- bis 7-gliedrigen Heterocyclus, worin der 4- bis 7-gliedrigen Heterocyclus seinerseits mit 1 oder 2 Substituenten unabhängig voneinander ausgewählt aus der Gruppe Cyano, Trifluormethyl, (C₁-C₆)-Alkyl, Hydroxy, Oxo, (C₁-C₆)-Alkoxy, Trifluormethoxy, (C₁-C₆)-Alkoxycarbonyl, Amino, Mono-(C₁-C₆)-alkylamino und Di-(C₁-C₆)-alkylamino substituiert sein kann,
R¹⁷ für (C₁-C₆)-Alkyl oder (C₃-C₇)-Cycloalkyl steht,
oder
R¹⁴ und R¹⁷ bilden zusammen mit dem/den Atom/-en, an die sie jeweils gebunden sind, einen 4- bis 7-gliedrigen Heterocyclus, worin der 4- bis 7-gliedrigen Heterocyclus seinerseits mit 1 oder 2 Substituenten unabhängig voneinander ausgewählt aus der Gruppe Cyano, Trifluormethyl, (C₁-C₆)-Alkyl, Hydroxy, Oxo, (C₁-C₆)-Alkoxy, Trifluormethoxy, (C₁-C₆)-Alkoxycarbonyl, Amino, Mono-(C₁-C₆)-alkylamino und Di-(C₁-C₆)-alkylamino substituiert sein kann,
und
worin 4- bis 7-gliedriges Heterocyclyl, Phenyl und 5- oder 6-gliedriges Heteroaryl ihrerseits mit 1 bis 3 Substituenten unabhängig voneinander ausgewählt aus der Gruppe Halogen, Cyano, Difluormethyl, Trifluormethyl, (C₁-C₆)-Alkyl, (C₃-C₇)-Cycloalkyl, Hydroxy, Oxo, Thiooxo und (C₁-C₄)-Alkoxy substituiert sein können,
und
worin die zuvor genannten (C₁-C₄)-Alkyl-, (C₁-C₆)-Alkyl-, (C₃-C₈)-Cycloalkyl- und 4- bis 7-gliedriges Heterocyclyl-Gruppen sofern nicht anders angegeben jeweils unabhängig voneinander weiterhin mit 1 bis 3 Substituenten unabhängig voneinander ausgewählt aus der Gruppe Fluor, Difluormethyl, Trifluormethyl, (C₁-C₆)-Alkyl, (C₃-C₇)-Cycloalkyl, Hydroxy, Difluormethoxy, Trifluormethoxy, (C₁-C₄)-Alkoxy, Hydroxycarbonyl, (C₁-C₄)-Alkoxycarbonyl, Amino, Phenyl, 4- bis 7-gliedriges Heterocyclyl und 5- oder 6-gliedriges Heteroaryl substituiert sein können,
oder R^{7A} und R^{7B} zusammen mit dem Kohlenstoffatom, an das sie gebunden sind, eine (C₂-C₄)-Alkenyl-Gruppe, eine Oxo-Gruppe, einen 3- bis 6-gliedrigen Carbocyclus oder einen 4- bis 7-gliedrigen Heterocyclus bilden,
worin der 3- bis 6-gliedrigen Carbocyclus und der 4- bis 7-gliedrigen Heterocyclus mit 1 oder 2 Substituenten unabhängig voneinander ausgewählt aus der Gruppe Fluor und (C₁-C₄)-Alkyl substituiert sein können,
R^{8A} für Wasserstoff, Fluor, (C₁-C₄)-Alkyl oder Hydroxy steht,
R^{8B} für Wasserstoff, Fluor, (C₁-C₄)-Alkyl oder Trifluormethyl steht,
der Ring Q für 8- oder 9-gliedriges Heteroaryl steht,
- R³: für -OR⁴ oder -NR⁵R⁶ steht,
wobei
R⁴ für Wasserstoff, (C₁-C₆)-Alkyl, (C₃-C₇)-Cycloalkyl, 4- bis 7-gliedriges Heterocyclyl, Phenyl oder 5- oder 6-gliedriges Heteroaryl steht,
worin (C₁-C₆)-Alkyl, (C₃-C₇)-Cycloalkyl, 4- bis 7-gliedriges Heterocyclyl, Phenyl und 5- oder 6-gliedriges Heteroaryl mit 1 bis 3 Substituenten unabhängig voneinander ausgewählt aus der Gruppe Fluor, Difluormethyl, Trifluormethyl, (C₁-C₆)-Alkyl, (C₃-C₆)-Cycloalkyl, Difluormethoxy, Trifluormethoxy, Oxo, -(C=O)ₚ-OR⁹, -C(=O)ₚNR⁹R¹⁰, -NR⁹-(C=O)-R¹⁰, -NR⁹-(C=O)-OR¹⁰, -NR⁹-(C=O)-NR¹⁰R¹¹, -NR⁹-SO₂-R¹⁰, -S(O)_{q}-R¹² und -SO₂-NR⁹R¹⁰ substituiert sein können,
worin
   p die Zahl 0 oder 1 bedeutet,
   q die Zahl 0, 1 oder 2 bedeutet,
   R⁹, R¹⁰ und R¹¹ unabhängig voneinander jeweils für Wasserstoff, (C₁-C₆)-Alkyl oder (C₃-C₈)-Cycloalkyl stehen,
   worin (C₁-C₆)-Alkyl seinerseits mit 1 oder 2 Substituenten unabhängig voneinander ausgewählt aus der Gruppe Fluor, Difluormethyl, Trifluormethyl, Hydroxy, (C₁-C₆)-Alkoxy, Difluormethoxy, Trifluormethoxy, (C₁-C₆)-Alkoxycarbonyl, Amino, Mono-(C₁-C₆)-alkylamino, Di-(C₁-C₆)-alkylamino und 4- bis 7-gliedriges Heterocyclyl substituiert sein kann,
   oder
   R⁹ und R¹⁰ bilden zusammen mit dem/den Atom/-en, an die sie jeweils gebunden sind, einen 4- bis 7-gliedrigen Heterocyclus,
   worin der 4- bis 7-gliedrigen Heterocyclus seinerseits mit 1 oder 2 Substituenten unabhängig voneinander ausgewählt aus der Gruppe Fluor, Trifluormethyl, (C₁-C₆)-Alkyl, Hydroxy, Oxo, (C₁-C₆)-Alkoxy, Trifluormethoxy, (C₁-C₆)-Alkoxycarbonyl, Amino, Mono-(C₁-C₆)-alkylamino und Di-(C₁-C₆)-alkylamino substituiert sein kann,
   oder
   R¹⁰ und R¹¹ bilden zusammen mit dem/den Atom/-en, an die sie jeweils gebunden sind, einen 4- bis 7-gliedrigen Heterocyclus,
   worin der 4- bis 7-gliedrigen Heterocyclus seinerseits mit 1 oder 2 Substituenten unabhängig voneinander ausgewählt aus der Gruppe Fluor, Trifluormethyl, (C₁-C₆)-Alkyl, Hydroxy, Oxo, (C₁-C₆)-Alkoxy, Trifluormethoxy, (C₁-C₆)-Alkoxycarbonyl, Amino, Mono-(C₁-C₆)-alkylamino und Di-(C₁-C₆)-alkylamino substituiert sein kann,
   und worin
   R¹² für (C₁-C₆)-Alkyl oder (C₃-C₇)-Cycloalkyl steht,
und
worin die zuvor genannten (C₁-C₄)-Alkyl-, (C₁-C₆)-Alkyl-, (C₃-C₈)-Cycloalkyl- und 4- bis 7-gliedriges Heterocyclyl-Gruppen sofern nicht anders angegeben jeweils unabhängig voneinander weiterhin mit 1 bis 3 Substituenten unabhängig voneinander ausgewählt aus der Gruppe Fluor, Difluormethyl, Trifluormethyl, (C₃-C₇)-Cycloalkyl, Hydroxy, Difluormethoxy, Trifluormethoxy, (C₁-C₄)-Alkoxy, Hydroxycarbonyl, (C₁-C₄)-Alkoxycarbonyl, Amino, Phenyl, 4- bis 7-gliedriges Heterocyclyl und 5- oder 6-gliedriges Heteroaryl substituiert sein können,
R⁵ für Wasserstoff oder (C₁-C₄)-Alkyl steht,
R⁶ für (C₁-C₆)-Alkyl, (C₃-C₇)-Cycloalkyl, 4- bis 7-gliedriges Heterocyclyl, Phenyl oder 5- oder 6-gliedriges Heteroaryl steht,
worin (C₁-C₆)-Alkyl (C₃-C₇)-Cycloalkyl, 4- bis 7-gliedriges Heterocyclyl, Phenyl und 5- oder 6-gliedriges Heteroaryl mit 1 bis 3 Substituenten unabhängig voneinander ausgewählt aus der Gruppe Fluor, Difluormethyl, Trifluormethyl, (C₁-C₆)-Alkyl, (C₃-C₇)-Cycloalkyl, Difluormethoxy, Trifluormethoxy, Oxo,-(C=O)ₚ-OR⁹, -(C=O)ₚ-NRR⁹R¹⁰, -NR⁹-(C=O)R¹⁰, -NR⁹-(C=O)-OR¹⁰, -NR⁹-(C=O)-NR¹⁰R¹¹, -NR⁹-SO²-R¹⁰, -S(O)_{q}-R¹², -SO₂-NR⁹R¹⁰, Phenyl, 4-bis 7-gliedriges Heterocyclyl und 5- oder 6-gliedriges Heteroaryl substituiert sein können,
worin
   p die Zahl 0 oder 1 bedeutet,
   q die Zahl 0, 1 oder 2 bedeutet,
   R⁹, R¹⁰ und R¹¹ unabhängig voneinander jeweils für Wasserstoff, (C₁-C₆)-Alkyl oder (C₃-C₈)-Cycloalkyl stehen,
   worin (C₁-C₆)-Alkyl seinerseits mit 1 bis 3 Substituenten unabhängig voneinander ausgewählt aus der Gruppe Fluor, Difluormethyl, Trifluormethyl, (C₃-C₇)-Cycloalkyl, Difluormethoxy, Trifluormethoxy und (C₁-C₄)-Alkoxy substituiert sein kann,
   oder
   R⁹ und R¹⁰ bilden zusammen mit dem/den Atom/-en, an die sie jeweils gebunden sind, einen 4- bis 7-gliedrigen Heterocyclus,
   worin der 4- bis 7-gliedrigen Heterocyclus seinerseits mit 1 oder 2 Substituenten unabhängig voneinander ausgewählt aus der Gruppe Fluor, Cyano, Trifluormethyl, (C₁-C₆)-Alkyl, Hydroxy, Oxo, (C₁-C₆)-Alkoxy, Trifluormethoxy, (C₁-C₆)-Alkoxycarbonyl, Amino, Mono-(C₁-C₆)-alkyl-amino und Di-(C₁-C₆)-alkylamino substituiert sein kann,
   oder
   R¹⁰ und R¹¹ bilden zusammen mit dem/den Atom/-en, an die sie jeweils gebunden sind, einen 4- bis 7-gliedrigen Heterocyclus,
   worin der 4- bis 7-gliedrigen Heterocyclus seinerseits mit 1 oder 2 Substituenten unabhängig voneinander ausgewählt aus der Gruppe Cyano, Trifluormethyl, (C₁-C₆)-Alkyl, Hydroxy, Oxo, (C₁-C₆)-Alkoxy, Trifluormethoxy, (C₁-C₆)-Alkoxycarbonyl, Amino, Mono-(C₁-C₆)-alkylamino und Di-(C₁-C₆)-alkylamino substituiert sein kann, worin
   R¹² für (C₁-C₆)-Alkyl oder (C₃-C₇)-Cycloalkyl steht,
   und
   worin Phenyl, 4- bis 7-gliedriges Heterocyclyl und 5- oder 6-gliedriges Heteroaryl ihrerseits mit 1 bis Substituenten unabhängig voneinander ausgewählt aus der Gruppe Halogen, Cyano, Difluormethyl, Trifluormethyl, (C₁-C₄)-Alkyl, (C₃-C₇)-Cycloalkyl, Hydroxy, Oxo, Difluormethoxy, Trifluormethoxy und (C₁-C₄)-Alkoxy substituiert sein können,
oder
R⁵ und R⁶ bilden zusammen mit dem Stickstoffatom, an das sie gebunden sind, einen 4- bis 7-gliedrigen Heterocyclus oder ein 5- oder 6-gliedriges Heteroaryl,
worin der 4- bis 7-gliedrigen Heterocyclus und das 5- oder 6-gliedriges Heteroaryl mit 1 bis 3 Substituenten unabhängig voneinander ausgewählt aus der Gruppe Fluor, Cyano, Difluormethyl, Trifluormethyl, (C₁-C₆)-Alkyl, (C₃-C₇)-Cycloalkyl, Hydroxy, Oxo, (C₁-C₆)-Alkoxy, Difluormethoxy, Trifluormethoxy, (C₁-C₆)-Alkoxycarbonyl, Amino, Mono-(C₁-C₆)-alkylamino und Di-(C₁-C₆)-alkylamino substituiert sein können, und
worin die zuvor genannten (C₁-C₄)-Alkyl-, (C₁-C₆)-Alkyl-, (C₃-C₈)-Cycloalkyl- und 4- bis 7-gliedriges Heterocyclyl-Gruppen sofern nicht anders angegeben jeweils unabhängig voneinander weiterhin mit 1 bis 3 Substituenten unabhängig voneinander ausgewählt aus der Gruppe Fluor, Difluormethyl, Trifluormethyl, (C₃-C₇)-Cycloalkyl, Hydroxy, Difluormethoxy, Trifluormethoxy, (C₁-C₄)-Alkoxy, Hydroxycarbonyl, (C₁-C₄)-Alkoxycarbonyl, Amino, Phenyl, 4- bis 7-gliedriges Heterocyclyl und 5- oder 6-gliedriges Heteroaryl substituiert sein können,
- R¹: für Fluor, Chlor, Cyano, Difluormethyl, Trifluormethyl, (C₁-C₄)-Alkyl, (C₃-C₇)-Cycloalkyl oder (C₁-C₄)-Alkoxy steht,
- n: für eine Zahl 0, 1 oder 2 steht,
- R²: für Trifluormethyl, (C₁-C₆)-Alkyl, (C₃-C₈)-Cycloalkyl, Phenyl oder 5- oder 6-gliedriges Heteroaryl steht,
wobei (C₁-C₆)-Alkyl mit einem Substituenten ausgewählt aus der Gruppe Difluormethyl und Trifluormethyl substituiert ist,
wobei (C₁-C₆)-Alkyl mit 1 bis 3 Substituenten Fluor substituiert sein kann,
wobei (C₃-C₈)-Cycloalkyl mit 1 oder 2 Substituenten unabhängig voneinander ausgewählt aus der Gruppe Fluor, Methyl und Methoxy substituiert sein kann,
wobei Phenyl mit 1 bis 3 Substituenten Fluor substituiert ist,
wobei Phenyl mit 1 oder 2 Substituenten unabhängig voneinander ausgewählt aus der Gruppe Methyl und Methoxy substituiert sein kann,
und
wobei 5- und 6-gliedriges Heteroaryl mit 1 oder 2 Substituenten unabhängig voneinander ausgewählt aus der Gruppe Fluor, Trifluormethyl und Methyl substituiert sein können,
sowie ihre *N*-Oxide, Salze, Solvate, Salze der *N*-Oxide und Solvate der *N*-Oxide und Salze.

Offenbarungsgegenstand sind weiterhin Verbindungen der Formel (I), in welcher
- L: für eine Gruppe #¹-CR^{7A}R^{7B}-(CR^{8A}R^{8B})ₘ-#² steht,
wobei
#¹ für die Anknüpfstelle an die Carbonylgruppe steht,
#² für die Anknüpfstelle an den Pyrimidinring steht,
m für eine Zahl 0 oder 1 steht,
R^{7A} für Wasserstoff, Fluor, Methyl, Ethyl, Hydroxy oder Amino steht,
R^{7B} für Wasserstoff, Fluor, Difluormethyl, Trifluormethyl, (C₁-C₄)-Alkyl, Methoxycarbonylamino, Cyano, Cyclopropyl, Cyclobutyl, Cyclopentyl, Phenyl oder eine Gruppe der Formel -M-R¹³ steht,
worin (C₁-C₄)-Alkyl mit 1 bis 3 Substituenten unabhängig voneinander ausgewählt aus der Gruppe Fluor, Cyano, Trifluormethyl, Cyclopropyl, Cyclobutyl, Cyclopentyl, Hydroxy, Difluormethoxy, Trifluormethoxy, Methoxy, Ethoxy, Hydroxycarbonyl, Methoxycarbonyl, Ethoxycarbonyl und Amino substituiert sein kann, und worin
M für eine Bindung oder Methylen steht,
R¹³ für -(C=O)ᵣ-NR¹⁴R¹⁵, -C(=S)-NR¹⁴R¹⁵, Oxadiazolonyl, Oxadiazolthionyl, Phenyl, Oxazolyl, Thiazolyl, Pyrazolyl, Triazolyl, Oxadiazolyl, Thiadiazolyl, Pyridyl, Pyrimidinyl oder Pyrazinyl steht,
worin
r die Zahl 0 oder 1 bedeutet,
R¹⁴ und R¹⁵ unabhängig voneinander jeweils für Wasserstoff, Methyl, Ethyl, iso-Propyl, Cyclopropyl, Cyclobutyl, Cyclopentyl, Oxetanyl, Azetidinyl, Tetrahydrofuranyl, Pyrrolidinyl, Tetrahydropyranyl, Piperidinyl, Piperazinyl, Morpholinyl, Phenyl, Pyrazolyl oder Pyridyl stehen,
worin Methyl, Ethyl und iso-Propyl weiterhin mit 1 oder 2 Substituenten unabhängig voneinander ausgewählt aus der Gruppe Fluor, Difluormethyl, Trifluormethyl, Cyclopropyl, Cyclobutyl, Cyclopentyl, Hydroxy, Difluormethoxy, Trifluormethoxy, Methoxy, Ethoxy, Hydroxycarbonyl, Methoxycarbonyl, Ethoxycarbonyl und Amino substituiert sein können, und
worin Oxadiazolonyl, Oxadiazolthionyl, Phenyl, Oxazolyl, Thiazolyl, Pyrazolyl, Triazolyl, Oxadiazolyl, Thiadiazolyl, Pyridyl, Pyrimidinyl und Pyrazinyl ihrerseits mit 1 oder 2 Substituenten unabhängig voneinander ausgewählt aus der Gruppe Fluor, Chlor, Cyano, Difluormethyl, Trifluormethyl, Methyl, Ethyl, iso-Propyl, 2,2,2-Trifluorethyl, 1,1,2,2,2-Pentafluorethyl, Cyclopropyl, Cyclobutyl, Cyclopropylmetyl, Cyclobutylmethyl, Hydroxy, Methoxy und Ethoxy substituiert sein können, oder
R^{7A} und R^{7B} zusammen mit dem Kohlenstoffatom, an das sie gebunden sind, einen Cyclopropyl-, Cyclobutyl-, Cyclopentyl-, Azetidinyl-, Tetrahydrofuranyl-, Pyrrolidinyl- oder Tetrahydropyranyl-Ring bilden, worin der Cyclopropyl-, Cyclobutyl-, Cyclopentyl-, Azetidinyl-, Tetrahydrofuranyl-, Pyrrolidinyl- und Tetrahydropyranyl-Ring mit 1 oder 2 Substituenten unabhängig voneinander ausgewählt aus der Gruppe Fluor und Methyl substituiert sein können,
R^{8A} für Wasserstoff, Fluor, Methyl, Ethyl oder Hydroxy steht,
R^{8B} für Wasserstoff, Fluor, Methyl, Ethyl oder Trifluormethyl steht,
der Ring Q für eine Gruppe der Formel 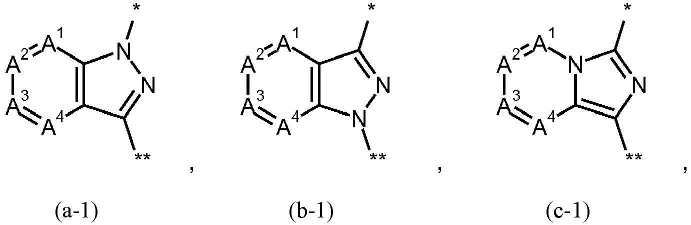 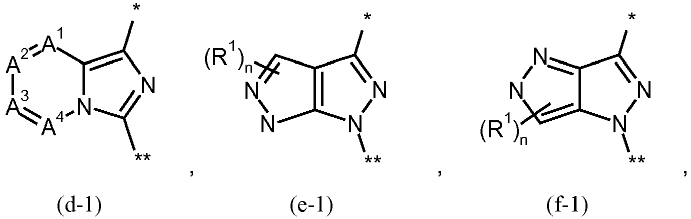 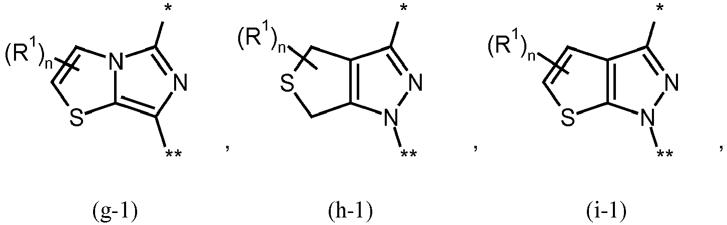 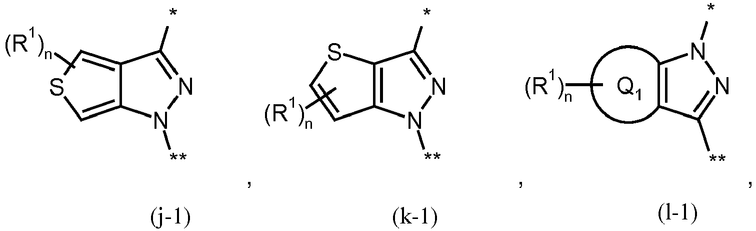 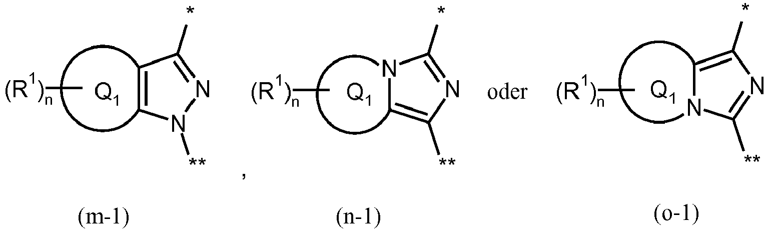 steht, wobei
- *: für die Anknüpfungsstelle an -CH₂-R² steht,
- **: für die Anknüpfungsstelle an das Pyrimidin steht,
der Ring Q₁ zusammen mit den Atomen, an die er gebunden ist, einen 5- bis 7-gliedrigen gesättigten oder teilweise ungesättigten Carbocyclus oder einen 5- bis 7-gliedrigen gesättigten oder teilweise ungesättigten Heterocyclus bildet,
- R¹: für Fluor, Chlor oder Methyl steht,
- n: für eine Zahl 0, 1 oder 2 steht,
A¹, A², A³ und A⁴ unabhängig voneinander jeweils für N, CH oder CR¹ stehen, mit der Maßgabe, dass maximal zwei der Gruppen A¹, A², A³ und A⁴ für N stehen,
- R³: für -OR⁴ oder -NR⁵R⁶ steht,
wobei
R⁴ für (C₁-C₆)-Alkyl, Cyclopropyl, Cyclobutyl, Cyclopentyl, Oxetanyl, Azetidinyl, Tetrahydrofuranyl, Pyrrolidinyl, Tetrahydropyranyl, Piperidinyl, Piperazinyl, Morpholinyl, Phenyl, Pyrazolyl, Oxazolyl, Thiazolyl, Triazolyl, Oxadiazolyl, Thiadiazolyl, Pyridyl oder Pyrimidinyl steht,
worin (C₁-C₆)-Alkyl mit 1 bis 3 Substituenten unabhängig voneinander ausgewählt aus der Gruppe Fluor, Difluormethyl, Trifluormethyl, Cyclopropyl, Cyclobutyl, Cyclopentyl, Difluormethoxy, Trifluormethoxy, -(C=O)ₚ-OR⁹, -(C=O)ₚ-NR⁹R¹⁰, und -NR⁹-(C=O)-R¹⁰ substituiert sein kann,
und worin Cyclopropyl, Cyclobutyl, Cyclopentyl, Oxetanyl, Azetidinyl, Tetrahydrofuranyl, Pyrrolidinyl, Tetrahydropyranyl, Piperidinyl, Piperazinyl, Morpholinyl, Phenyl, Pyrazolyl, Oxazolyl, Thiazolyl, Triazolyl, Oxadiazolyl, Thiadiazolyl, Pyridyl oder Pyrimidinyl mit 1 bis 3 Substituenten unabhängig voneinander ausgewählt aus der Gruppe Fluor, Difluormethyl, Trifluormethyl, 2,2,2-Trifluorethyl, 1,1,2,2,2-Pentafluorethyl, Methyl, Ethyl, Isopropyl, Cyclopropyl, Cyclobutyl, Cyclopentyl, Difluormethoxy, Trifluormethoxy, Oxo, -(C=O)ₚ-OR⁹ und -(C=O)ₚ-NR⁹R¹⁰ substituiert sein können,
worin
   p die Zahl 0 oder 1 bedeutet,
   R⁹ und R¹⁰ unabhängig voneinander jeweils für Wasserstoff, Methyl, Ethyl, Isopropyl, Trifluormethyl, 2,2,2-Trifluorethyl, 1,1,2,2,2-Pentafluorethyl, Cyclopropyl, Cyclobutyl oder Cyclopentyl stehen,
   oder
   R⁹ und R¹⁰ bilden zusammen mit dem/den Atom/-en, an die sie jeweils gebunden sind, einen Azetidinyl-, Pyrrolidinyl-, Piperidinyl-, Piperazinyl- oder Morpholinyl-Ring, worin der Azetidinyl-, Pyrrolidinyl-, Piperidinyl-, Piperazinyl- und Morpholinyl-Ring seinerseits mit 1 oder 2 Substituenten unabhängig voneinander ausgewählt aus der Gruppe Fluor, Trifluormethyl, Methyl, Ethyl, Hydroxy, Oxo, Methoxy, Ethoxy, Amino, Methylamino, Ethylamino, Dimethylamino und Diethylamino substituiert sein kann,
- R⁵: für Wasserstoff, Methyl oder Ethyl steht,
- R⁶: für (C₁-C₆)-Alkyl, Cyclopropyl, (C₃-C₆)-Cycloalkyl, Oxetanyl, Azetidinyl, Tetrahydrofuranyl, Pyrrolidinyl, Tetrahydropyranyl, Piperidinyl, Piperazinyl, Morpholinyl, Phenyl, Pyrazolyl, Oxazolyl, Thiazolyl, Triazolyl, Oxadiazolyl, Thiadiazolyl, Pyridyl oder Pyrimidinyl steht,
worin (C₁-C₆)-Alkyl und (C₃-C₆)-Cycloalkyl mit 1 bis 3 Substituenten unabhängig voneinander ausgewählt aus der Gruppe Fluor, Difluormethyl, Trifluormethyl Methyl, Ethyl, Cyclopropyl, Cyclobutyl, Cyclopentyl, Difluormethoxy, Trifluormethoxy, -(C=O)ₚ-OR⁹, -(C=O)ₚ-NR⁹R¹⁰, -NR⁹-(C=O)-R¹⁰, Oxetanyl, Azetidinyl, Tetrahydrofuranyl, Pyrrolidinyl, Tetrahydropyranyl, Piperidinyl, Piperazinyl, Morpholinyl, Phenyl, Furanyl, Pyrazolyl, Imidazolyl, Oxazolyl, Thiazolyl, Triazolyl, Oxadiazolyl, Thiadiazolyl, Pyridyl und Pyrimidinyl substituiert sind,
worin Cyclopropyl, Cyclobutyl, Cyclopentyl, Oxetanyl, Azetidinyl, Tetrahydrofuranyl, Pyrrolidinyl, Tetrahydropyranyl, Piperidinyl, Piperazinyl, Morpholinyl, Phenyl, Furanyl, Pyrazolyl, Imidazolyl, Oxazolyl, Thiazolyl, Triazolyl, Oxadiazolyl, Thiadiazolyl, Pyridyl und Pyrimidinyl ihrerseits mit 1 oder 2 Substituenten unabhängig voneinander ausgewählt aus der Gruppe Fluor, Chlor, Cyano, Difluormethyl, Trifluormethyl, Methyl, Ethyl, Cyclopropyl, Cyclobutyl, Cyclopentyl, Hydroxy, Oxo, Difluormethoxy, Trifluormethoxy, Methoxy und Ethoxy substituiert sein können,
worin Oxetanyl, Azetidinyl, Tetrahydrofuranyl, Pyrrolidinyl, Tetrahydropyranyl, Piperidinyl, Piperazinyl, Morpholinyl, Phenyl, Pyrazolyl, Oxazolyl, Thiazolyl, Triazolyl, Oxadiazolyl, Thiadiazolyl, Pyridyl und Pyrimidinyl mit 1 bis 3 Substituenten unabhängig voneinander ausgewählt aus der Gruppe Fluor, Difluormethyl, Trifluormethyl, Methyl, Ethyl, Cyclopropyl, Cyclobutyl, Cyclopentyl, Difluormethoxy, Trifluormethoxy, Oxo, -(C=O)ₚ-OR⁹ und -(C=O)ₚ-NR⁹R¹⁰ substituiert sein können,
worin
   p die Zahl 0 oder 1 bedeutet,
   R⁹ und R¹⁰ unabhängig voneinander jeweils für Wasserstoff, Methyl, Ethyl, Isopropyl, Trifluormethyl, 2,2,2-Trifluorethyl, 1,1,2,2,2-Pentafluorethyl, Cyclopropyl, Cyclobutyl oder Cyclopentyl stehen,
   oder
   R⁹ und R¹⁰ bilden zusammen mit dem/den Atom/-en, an die sie jeweils gebunden sind, einen Azetidinyl-, Pyrrolidinyl-, Piperidinyl-, Piperazinyl- oder Morpholinyl-Ring,
   worin der Azetidinyl-, Pyrrolidinyl-, Piperidinyl-, Piperazinyl- und Morpholinyl-Ring seinerseits mit 1 oder 2 Substituenten unabhängig voneinander ausgewählt aus der Gruppe Fluor, Trifluormethyl, Methyl, Ethyl, Hydroxy, Oxo, Methoxy, Ethoxy, Amino, Methylamino, Ethylamino, Dimethylamino und Diethylamino substituiert sein kann,
   oder
   R⁵ und R⁶ bilden zusammen mit dem Stickstoffatom, an das sie gebunden sind, einen Azetidinyl-, Pyrrolidinyl-, Imidazolidinyl-, Piperidinyl-, Dihydropiperdinyl-, Piperazinyl-, Morpholinyl-, Pyrazolyl-, Imidazolyl- oder Triazolyl-Ring,
   worin der Azetidinyl-, Pyrrolidinyl-, Imidazolidinyl-, Piperidinyl-, Dihydropiperdinyl-, Piperazinyl-, Morpholinyl-, Pyrazolyl-, Imidazolyl- und Triazolyl-Ring mit 1 oder 2 Substituenten unabhängig voneinander ausgewählt aus der Gruppe Fluor, Cyano, Difluormethyl, Trifluormethyl, 2,2,2-Trifluorethyl, 1,1,2,2,2-Pentafluorethyl, Methyl, Ethyl, 1-Hydroxyethyl, Cyclopropyl, Cyclobutyl, Cyclopentyl, Hydroxy, Oxo, Methoxy, Ethoxy, Difluormethoxy, Trifluormethoxy, Oxetanyl, Azetidinyl, Tetrahydrofuranyl, Pyrrolidinyl, Tetrahydropyranyl, Piperidinyl, Piperazinyl und Morpholinyl substituiert sein können,
- R²: für Trifluormethyl, 2,2,2-Trifluorethyl, 3,3,3-Trifluorprop-1-yl, 2,2,3,3,3-Pentafluorprop-1-yl, Cyclopropyl, Cyclobutyl, Cyclopentyl, Cyclohexyl, Phenyl, Pyridyl, Pyrimidinyl, Pyrazinyl oder Pyridazinyl steht,
wobei Phenyl mit 1 bis 3 Substituenten Fluor substituiert ist,
und
wobei Cyclopropyl, Cyclobutyl, Cyclopentyl, Cyclohexyl, Pyridyl, Pyrimidinyl, Pyrazinyl und Pyridazinyl mit 1 oder 2 Substituenten Fluor substituiert sein können,
sowie ihre Salze, Solvate und Solvate der Salze.

Offenbarungsgegenstand sind weiterhin Verbindungen der Formel (I), in welcher
- L: für eine Gruppe #¹-CR^{7A}R^{7B}-(CR^{8A}R^{8B})ₘ-#² steht,
wobei
#¹ für die Anknüpfstelle an die Carbonylgruppe steht,
#² für die Anknüpfstelle an den Pyrimidinring steht,
m für eine Zahl 0 oder 1 steht,
R^{7A} für Wasserstoff, Fluor, Methyl, Ethyl, Hydroxy oder Amino steht,
R^{7B} für Wasserstoff, Fluor, Difluormethyl, Trifluormethyl, (C₁-C₄)-Alkyl, Methoxycarbonylamino, Cyano, Cyclopropyl, Cyclobutyl, Cyclopentyl, Phenyl oder eine Gruppe der Formel -M-R¹³ steht,
worin (C₁-C₄)-Alkyl mit 1 bis 3 Substituenten unabhängig voneinander ausgewählt aus der Gruppe Fluor, Cyano, Trifluormethyl, Cyclopropyl, Cyclobutyl, Cyclopentyl, Hydroxy, Difluormethoxy, Trifluormethoxy, Methoxy, Ethoxy, Hydroxycarbonyl, Methoxycarbonyl, Ethoxycarbonyl und Amino substituiert sein kann,
und worin
M für eine Bindung oder Methylen steht,
R¹³ für -(C=O)ᵣ-NR¹⁴R¹⁵, -C(=S)-NR¹⁴R¹⁵, Oxadiazolonyl, Oxadiazolthionyl, Phenyl, Oxazolyl, Thiazolyl, Pyrazolyl, Triazolyl, Oxadiazolyl, Thiadiazolyl, Pyridyl, Pyrimidinyl oder Pyrazinyl steht,
worin
r die Zahl 0 oder 1 bedeutet,
R¹⁴ und R¹⁵ unabhängig voneinander jeweils für Wasserstoff, Methyl, Ethyl, iso-Propyl, Cyclopropyl, Cyclobutyl, Cyclopentyl, Oxetanyl, Azetidinyl, Tetrahydrofuranyl, Pyrrolidinyl, Tetrahydropyranyl, Piperidinyl, Piperazinyl, Morpholinyl, Phenyl, Pyrazolyl oder Pyridyl stehen,
worin Methyl, Ethyl und iso-Propyl weiterhin mit 1 oder 2 Substituenten unabhängig voneinander ausgewählt aus der Gruppe Fluor, Difluormethyl, Trifluormethyl, Cyclopropyl, Cyclobutyl, Cyclopentyl, Hydroxy, Difluormethoxy, Trifluormethoxy, Methoxy, Ethoxy, Hydroxycarbonyl, Methoxycarbonyl, Ethoxycarbonyl und Amino substituiert sein können, und
worin Oxadiazolonyl, Oxadiazolthionyl, Phenyl, Oxazolyl, Thiazolyl, Pyrazolyl, Triazolyl, Oxadiazolyl, Thiadiazolyl, Pyridyl, Pyrimidinyl und Pyrazinyl ihrerseits mit 1 oder 2 Substituenten unabhängig voneinander ausgewählt aus der Gruppe Fluor, Chlor, Cyano, Difluormethyl, Trifluormethyl, Methyl, Ethyl, iso-Propyl, 2,2,2-Trifluorethyl, 1,1,2,2,2-Pentafluorethyl, Cyclopropyl, Cyclobutyl, Cyclopropylmetyl, Cyclobutylmethyl, Hydroxy, Methoxy und Ethoxy substituiert sein können, oder
R^{7A} und R^{7B} zusammen mit dem Kohlenstoffatom, an das sie gebunden sind, einen Cyclopropyl-, Cyclobutyl-, Cyclopentyl-, Azetidinyl-, Tetrahydrofuranyl-, Pyrrolidinyl- oder Tetrahydropyranyl-Ring bilden,
worin der Cyclopropyl-, Cyclobutyl-, Cyclopentyl-, Azetidinyl-, Tetrahydrofuranyl-, Pyrrolidinyl- und Tetrahydropyranyl-Ring mit 1 oder 2 Substituenten unabhängig voneinander ausgewählt aus der Gruppe Fluor und Methyl substituiert sein können,
R^{8A} für Wasserstoff, Fluor, Methyl, Ethyl oder Hydroxy steht,
R^{8B} für Wasserstoff, Fluor, Methyl, Ethyl oder Trifluormethyl steht,
der Ring Q für eine Gruppe der Formel 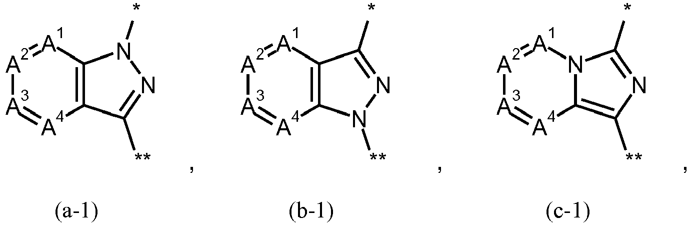 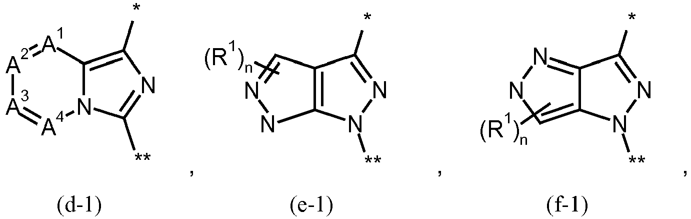 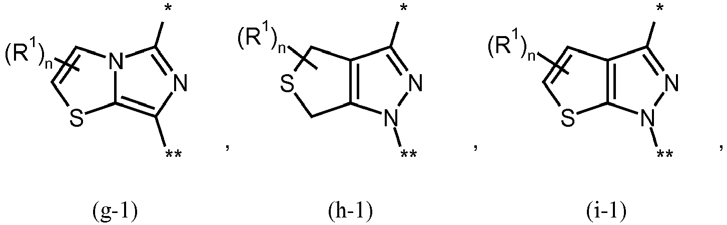 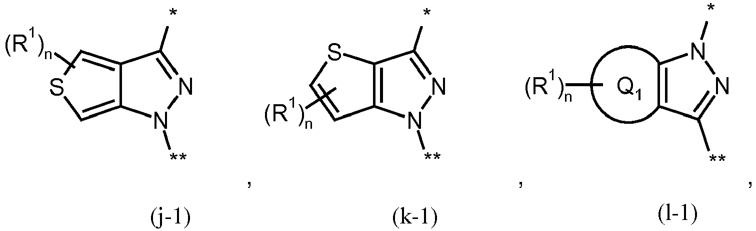 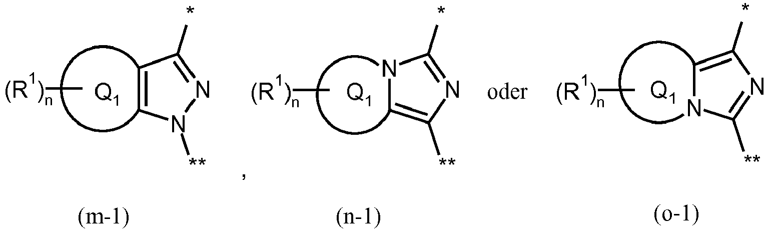 steht, wobei
- *: für die Anknüpfungsstelle an -CH₂-R² steht,
- **: für die Anknüpfungsstelle an das Pyrimidin steht,
der Ring Q₁ zusammen mit den Atomen, an die er gebunden ist, einen 5- bis 7-gliedrigen gesättigten oder teilweise ungesättigten Carbocyclus oder einen 5- bis 7-gliedrigen gesättigten oder teilweise ungesättigten Heterocyclus bildet,
- R¹: für Fluor, Chlor oder Methyl steht,
- n: für eine Zahl 0, 1 oder 2 steht,
A¹, A², A³ und A⁴ unabhängig voneinander jeweils für N, CH oder CR¹ stehen, mit der Maßgabe, dass maximal zwei der Gruppen A¹, A², A³ und A⁴ für N stehen,
- R³: für -OR⁴ oder -NR⁵R⁶ steht, wobei
- R⁴: für (C₁-C₆)-Alkyl, Cyclopropyl, Cyclobutyl, Cyclopentyl, Oxetanyl, Azetidinyl, Tetrahydrofuranyl, Pyrrolidinyl, Tetrahydropyranyl, Piperidinyl, Piperazinyl, Morpholinyl, Phenyl, Pyrazolyl, Oxazolyl, Thiazolyl, Triazolyl, Oxadiazolyl, Thiadiazolyl, Pyridyl oder Pyrimidinyl steht,
worin (C₁-C₆)-Alkyl mit 1 bis 3 Substituenten unabhängig voneinander ausgewählt aus der Gruppe Fluor, Difluormethyl, Trifluormethyl, Cyclopropyl, Cyclobutyl, Cyclopentyl, Difluormethoxy, Trifluormethoxy, -(C=O)ₚ-OR⁹, -(C=O)ₚ-NR⁹R¹⁰, und -NR⁹-(C=O)-R¹⁰ substituiert sein kann,
und
worin Cyclopropyl, Cyclobutyl, Cyclopentyl, Oxetanyl, Azetidinyl, Tetrahydrofuranyl, Pyrrolidinyl, Tetrahydropyranyl, Piperidinyl, Piperazinyl, Morpholinyl, Phenyl, Pyrazolyl, Oxazolyl, Thiazolyl, Triazolyl, Oxadiazolyl, Thiadiazolyl, Pyridyl oder Pyrimidinyl mit 1 bis 3 Substituenten unabhängig voneinander ausgewählt aus der Gruppe Fluor, Difluormethyl, Trifluormethyl, 2,2,2-Trifluorethyl, 1,1,2,2,2-Pentafluorethyl, Methyl, Ethyl, Isopropyl, Cyclopropyl, Cyclobutyl, Cyclopentyl, Difluormethoxy, Trifluormethoxy, Oxo, -(C=O)ₚ-OR⁹ und-(C=O)ₚ-NR⁹R¹⁰ substituiert sein können,
worin
   p die Zahl 0 oder 1 bedeutet,
   R⁹ und R¹⁰ unabhängig voneinander jeweils für Wasserstoff, Methyl, Ethyl, Isopropyl, Trifluormethyl, 2,2,2-Trifluorethyl, 1,1,2,2,2-Pentafluorethyl, Cyclopropyl, Cyclobutyl oder Cyclopentyl stehen,
   oder
   R⁹ und R¹⁰ bilden zusammen mit dem/den Atom/-en, an die sie jeweils gebunden sind, einen Azetidinyl-, Pyrrolidinyl-, Piperidinyl-, Piperazinyl- oder Morpholinyl-Ring,
   worin der Azetidinyl-, Pyrrolidinyl-, Piperidinyl-, Piperazinyl- und Morpholinyl-Ring seinerseits mit 1 oder 2 Substituenten unabhängig voneinander ausgewählt aus der Gruppe Fluor, Trifluormethyl, Methyl, Ethyl, Hydroxy, Oxo, Methoxy, Ethoxy, Amino, Methylamino, Ethylamino, Dimethylamino und Diethylamino substituiert sein kann,
- R⁵: für Wasserstoff, Methyl oder Ethyl steht,
- R⁶: für (C₁-C₆)-Alkyl, Cyclopropyl, Cyclobutyl, Cyclopentyl, Oxetanyl, Azetidinyl, Tetrahydrofuranyl, Pyrrolidinyl, Tetrahydropyranyl, Piperidinyl, Piperazinyl, Morpholinyl, Phenyl, Pyrazolyl, Oxazolyl, Thiazolyl, Triazolyl, Oxadiazolyl, Thiadiazolyl, Pyridyl oder Pyrimidinyl steht,
worin (C₁-C₆)-Alkyl, Cyclopropyl, Cyclobutyl und Cyclopentyl mit 1 bis 3 Substituenten unabhängig voneinander ausgewählt aus der Gruppe Fluor, Difluormethyl, Trifluormethyl Methyl, Ethyl, Cyclopropyl, Cyclobutyl, Cyclopentyl, Difluormethoxy, Trifluormethoxy, -(C=O)ₚ-OR⁹, -(C=O)ₚ-NRR⁹R¹⁰, -NR⁹-(C=O)-R¹⁰, Oxetanyl, Azetidinyl, Tetrahydrofuranyl, Pyrrolidinyl, Tetrahydropyranyl, Piperidinyl, Piperazinyl, Morpholinyl, Phenyl, Furanyl, Pyrazolyl, Imidazolyl, Oxazolyl, Thiazolyl, Triazolyl, Oxadiazolyl, Thiadiazolyl, Pyridyl und Pyrimidinyl substituiert sind,
worin Oxetanyl, Azetidinyl, Tetrahydrofuranyl, Pyrrolidinyl, Tetrahydropyranyl, Piperidinyl, Piperazinyl, Morpholinyl, Phenyl, Furanyl, Pyrazolyl, Imidazolyl, Oxazolyl, Thiazolyl, Triazolyl, Oxadiazolyl, Thiadiazolyl, Pyridyl und Pyrimidinyl ihrerseits mit 1 oder 2 Substituenten unabhängig voneinander ausgewählt aus der Gruppe Fluor, Chlor, Cyano, Difluormethyl, Trifluormethyl, Methyl, Ethyl, Cyclopropyl, Cyclobutyl, Cyclopentyl, Hydroxy, Oxo, Difluormethoxy, Trifluormethoxy, Methoxy und Ethoxy substituiert sein können,
worin Oxetanyl, Azetidinyl, Tetrahydrofuranyl, Pyrrolidinyl, Tetrahydropyranyl, Piperidinyl, Piperazinyl, Morpholinyl, Phenyl, Pyrazolyl, Oxazolyl, Thiazolyl, Triazolyl, Oxadiazolyl, Thiadiazolyl, Pyridyl und Pyrimidinyl mit 1 bis 3 Substituenten unabhängig voneinander ausgewählt aus der Gruppe Fluor, Difluormethyl, Trifluormethyl, Methyl, Ethyl, Cyclopropyl, Cyclobutyl, Cyclopentyl, Difluormethoxy, Trifluormethoxy, Oxo, -(C=O)ₚ-OR⁹ und -(C=O)ₚ-NR⁹R¹⁰ substituiert sein können,
worin
   p die Zahl 0 oder 1 bedeutet,
   R⁹ und R¹⁰ unabhängig voneinander jeweils für Wasserstoff, Methyl, Ethyl, Isopropyl, Trifluormethyl, 2,2,2-Trifluorethyl, 1,1,2,2,2-Pentafluorethyl, Cyclopropyl, Cyclobutyl oder Cyclopentyl stehen,
   oder
   R⁹ und R¹⁰ bilden zusammen mit dem/den Atom/-en, an die sie jeweils gebunden sind, einen Azetidinyl-, Pyrrolidinyl-, Piperidinyl-, Piperazinyl- oder Morpholinyl-Ring, worin der Azetidinyl-, Pyrrolidinyl-, Piperidinyl-, Piperazinyl- und Morpholinyl-Ring seinerseits mit 1 oder 2 Substituenten unabhängig voneinander ausgewählt aus der Gruppe Fluor, Trifluormethyl, Methyl, Ethyl, Hydroxy, Oxo, Methoxy, Ethoxy, Amino, Methylamino, Ethylamino, Dimethylamino und Diethylamino substituiert sein kann,
oder
- R⁵ und R⁶: bilden zusammen mit dem Stickstoffatom, an das sie gebunden sind, einen Azetidinyl-, Pyrrolidinyl-, Imidazolidinyl-, Piperidinyl-, Dihydropiperdinyl-, Piperazinyl-, Morpholinyl-, Pyrazolyl-, Imidazolyl-oder Triazolyl-Ring,
worin der Azetidinyl-, Pyrrolidinyl-, Imidazolidinyl-, Piperidinyl-, Dihydropiperdinyl-, Piperazinyl-, Morpholinyl-, Pyrazolyl-, Imidazolyl- und Triazolyl-Ring mit 1 oder 2 Substituenten unabhängig voneinander ausgewählt aus der Gruppe Fluor, Cyano, Difluormethyl, Trifluormethyl, 2,2,2-Trifluorethyl, 1,1,2,2,2-Pentafluorethyl, Methyl, Ethyl, 1-Hydroxyethyl, Cyclopropyl, Cyclobutyl, Cyclopentyl, Hydroxy, Oxo, Methoxy, Ethoxy, Difluormethoxy und Trifluormethoxy substituiert sein können,
- R²: für Trifluormethyl, 2,2,2-Trifluorethyl, 3,3,3-Trifluorprop-1-yl, 2,2,3,3,3-Pentafluorprop-1-yl, Cyclopropyl, Cyclobutyl, Cyclopentyl, Cyclohexyl, Phenyl, Pyridyl, Pyrimidinyl, Pyrazinyl oder Pyridazinyl steht,
wobei Phenyl mit 1 bis 3 Substituenten Fluor substituiert ist,
und wobei Cyclopropyl, Cyclobutyl, Cyclopentyl, Cyclohexyl, Pyridyl, Pyrimidinyl, Pyrazinyl und Pyridazinyl mit 1 oder 2 Substituenten Fluor substituiert sein können,
sowie ihre Salze, Solvate und Solvate der Salze.

Offenbarungsgegestand sind Verbindungen der Formel (I), in welcher
- L: für eine Gruppe #¹-CR^{7A}R^{7B}-(CR^{8A}R^{8B})ₘ-#² steht,
wobei
#¹ für die Anknüpfstelle an die Carbonylgruppe steht,
#² für die Anknüpfstelle an den Pyrimidinring steht,
m für eine Zahl 0 steht,
R^{7A} für Wasserstoff, Fluor, Methyl, Ethyl, Hydroxy oder Amino steht,
R^{7B} für Wasserstoff, Fluor, Difluormethyl, Trifluormethyl, Methyl, Ethyl, Methoxycarbonylamino, Cyclopropyl, Cyclobutyl, Cyclopentyl, oder eine Gruppe der Formel -M-R¹³ steht,
worin Methyl und Ethyl mit 1 bis 3 Substituenten unabhängig voneinander ausgewählt aus der Gruppe Fluor, Cyano, Trifluormethyl, Cyclopropyl, Cyclobutyl, Hydroxy, Difluormethoxy, Trifluormethoxy, Methoxy, Ethoxy, Hydroxycarbonyl, Methoxycarbonyl, Ethoxycarbonyl und Amino substituiert sein kann,
und worin
M für eine Bindung steht,
R¹³ für -(C=O)ᵣ-NR¹⁴R¹⁵, Phenyl, Thiazolyl, Triazolyl, Oxadiazolyl, Thiadiazolyl oder Pyrimidinyl steht,
worin
r die Zahl 1 bedeutet,
R¹⁴ und R¹⁵ unabhängig voneinander jeweils für Wasserstoff oder Cyclopropyl stehen, und worin Phenyl, Thiazolyl, Triazolyl, Oxadiazolyl, Thiadiazolyl und Pyrimidinyl ihrerseits mit 1 oder 2 Substituenten unabhängig voneinander ausgewählt aus der Gruppe Fluor, Difluormethyl, Trifluormethyl, Methyl, Ethyl, iso-Propyl, 2,2,2-Trifluorethyl, 1,1,2,2,2-Pentafluorethyl, Cyclopropyl, Cyclobutyl, Cyclopropylmetyl und Cyclobutylmethyl substituiert sein können, oder
R^{7A} und R^{7B} zusammen mit dem Kohlenstoffatom, an das sie gebunden sind, einen Cyclopropyl-, Cyclobutyl-, Cyclopentyl-, Azetidinyl-, Tetrahydrofuranyl-, Pyrrolidinyl- oder Tetrahydropyranyl-Ring bilden,
worin der Cyclopropyl-, Cyclobutyl-, Cyclopentyl-, Azetidinyl-, Tetrahydrofuranyl-, Pyrrolidinyl- und Tetrahydropyranyl-Ring mit 1 oder 2 Substituenten unabhängig voneinander ausgewählt aus der Gruppe Fluor und Methyl substituiert sein können,
der Ring Q für eine Gruppe der Formel 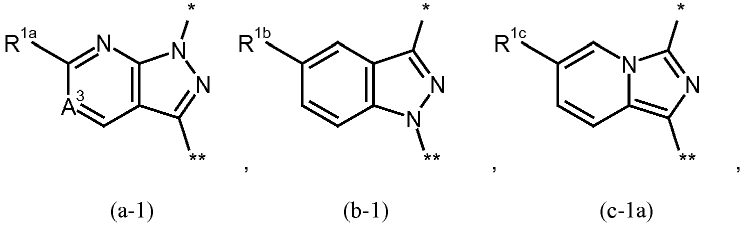 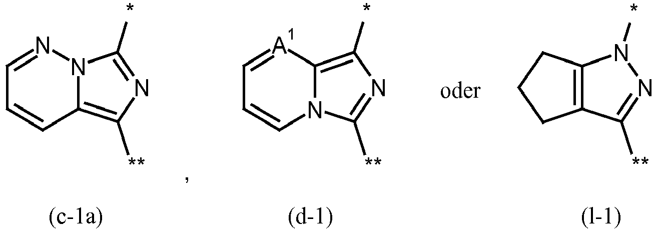 steht, wobei
- *: für die Anknüpfungsstelle an -CH₂-R² steht,
- **: für die Anknüpfungsstelle an das Pyrimidin steht,
- R^{1a}: für Wasserstoff oder Methyl steht,
- R^{1b}: für Wasserstoff oder Fluor steht,
- R^{1c}: für Wasserstoff oder Chlor steht,
- A¹: für N oder CH steht,
- A³: für N, CH oder C-F steht,
- R³: für -OR⁴ oder -NR⁵R⁶ steht,
wobei
R⁴ für (C₁-C₆)-Alkyl oder Pyrazolyl steht,
worin (C₁-C₆)-Alkyl mit 1 bis 3 Substituenten unabhängig voneinander ausgewählt aus der Gruppe Fluor, Trifluormethyl, -(C=O)ₚ-OR⁹ und -(C=O)ₚ-NR⁹R¹⁰ substituiert sein kann,
worin
   p die Zahl 0 oder 1 bedeutet,
   R⁹ und R¹⁰ unabhängig voneinander jeweils für Wasserstoff, oder Methyl stehen,
und
worin Pyrazolyl mit 1 oder 2 Substituenten unabhängig voneinander ausgewählt aus der Gruppe Fluor, Difluormethyl, Trifluormethyl, 2,2,2-Trifluorethyl, 1,1,2,2,2-Pentafluorethyl, Methyl, Cyclopropyl, Cyclobutyl, Cyclopentyl substituiert sein kann,
- R⁵: für Wasserstoff, Methyl oder Ethyl steht,
- R⁶: für (C₁-C₆)-Alkyl, Oxetanyl, Azetidinyl, Tetrahydrofuranyl, Pyrrolidinyl, Tetrahydropyranyl, Piperidinyl, Piperazinyl, Morpholinyl, Phenyl, Pyrazolyl, Oxazolyl, Thiazolyl, Triazolyl, Oxadiazolyl, Thiadiazolyl, Pyridyl oder Pyrimidinyl steht,
worin (C₁-C₆)-Alkyl mit 1 oder 2 Substituenten unabhängig voneinander ausgewählt aus der Gruppe Fluor, Difluormethyl, Trifluormethyl Cyclopropyl, Cyclobutyl, Cyclopentyl, Trifluormethoxy, -(C=O)ₚ-OR⁹, -(C=O)ₚ-NRR⁹R¹⁰, Tetrahydrofuranyl, Pyrrolidinyl, Tetrahydropyranyl, Phenyl, Furanyl, Pyrazolyl, Imidazolyl, Triazolyl und Pyridyl substituiert ist,
worin
   p die Zahl 0 oder 1 bedeutet,
   R⁹ und R¹⁰ unabhängig voneinander jeweils für Wasserstoff, Methyl, Ethyl, Trifluormethyl, 2,2,2-Trifluorethyl, 1,1,2,2,2-Pentafluorethyl, Cyclopropyl oder Cyclobutyl stehen, und
worin Tetrahydrofuranyl, Pyrrolidinyl, Tetrahydropyranyl, Phenyl, Furanyl, Pyrazolyl, Imidazolyl, Triazolyl und Pyridyl ihrerseits mit 1 oder 2 Substituenten unabhängig voneinander ausgewählt aus der Gruppe Fluor, Chlor, Cyano, Difluormethyl, Trifluormethyl, Methyl, Ethyl und Oxo sein können,
worin Oxetanyl, Azetidinyl, Tetrahydrofuranyl, Pyrrolidinyl, Tetrahydropyranyl, Piperidinyl, Piperazinyl, Morpholinyl, Phenyl, Pyrazolyl, Oxazolyl, Thiazolyl, Triazolyl, Oxadiazolyl, Thiadiazolyl, Pyridyl und Pyrimidinylmit 1 oder 2 Substituenten unabhängig voneinander ausgewählt aus der Gruppe Fluor, Difluormethyl, Trifluormethyl, Methyl, Ethyl und Oxo substituiert sein können,
oder
- R⁵ und R⁶: bilden zusammen mit dem Stickstoffatom, an das sie gebunden sind, einen Azetidinyl-, Pyrrolidinyl-, Imidazolidinyl-, Piperidinyl-, Dihydropiperdinyl-, Piperazinyl-, Morpholinyl-, Pyrazolyl- oder Imidazolyl-Ring,
worin der Azetidinyl-, Pyrrolidinyl-, Imidazolidinyl-, Piperidinyl-, Dihydropiperdinyl-, Piperazinyl-, Morpholinyl-, Pyrazolyl- und Imidazolyl-Ring mit 1 oder 2 Substituenten unabhängig voneinander ausgewählt aus der Gruppe Fluor, Cyano, Difluormethyl, Trifluormethyl, 2,2,2-Trifluorethyl, 1,1,2,2,2-Pentafluorethyl, Methyl, Ethyl, 1-Hydroxyethyl, Cyclopropyl, Cyclobutyl, Cyclopentyl, Hydroxy, Oxo, Methoxy, Ethoxy, Difluormethoxy und Trifluormethoxy substituiert sein können,
- R²: für 3,3,3-Trifluorprop-1-yl, 2,2,3,3-Tetrafluorprop-1-yl, 2,2,3,3,3-Pentafluorprop-1-yl, Phenyl oder Pyridyl steht,
wobei Phenyl mit 1 bis 3 Substituenten Fluor substituiert ist,
und
wobei Pyridyl mit 1 Substituenten Fluor substituiert sein kann,
sowie ihre Salze, Solvate und Solvate der Salze.

Offenbarungsgegenstand Verbindungen der Formel (I), in welcher
- L: für eine Gruppe #¹-CR^{7A}R^{7B}-(CR^{8A}R^{8B})ₘ-#² steht,
wobei
#¹ für die Anknüpfstelle an die Carbonylgruppe steht,
#² für die Anknüpfstelle an den Pyrimidinring steht,
m für eine Zahl 0 steht,
R^{7B} für Wasserstoff, Fluor, Methyl oder Hydroxy steht,
R^{7B} für Wasserstoff, Fluor, Trifluormethyl, 2,2,2-Trifluorethyl oder Methyl steht,
der Ring Q für eine Gruppe der Formel 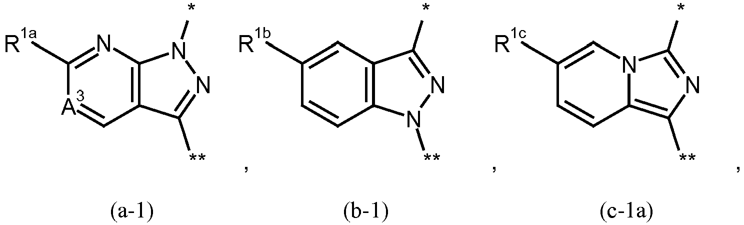 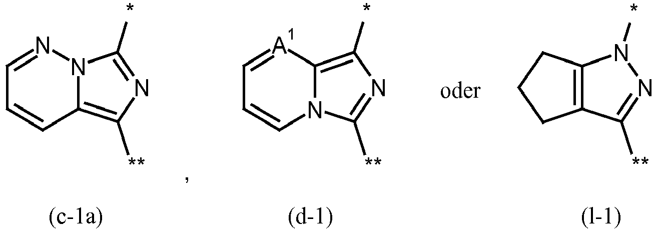 steht, wobei
- *: für die Anknüpfungsstelle an -CH₂-R² steht,
- **: für die Anknüpfungsstelle an das Pyrimidin steht,
- R^{1a}: für Wasserstoff oder Methyl steht,
- R^{1b}: für Wasserstoff oder Fluor steht,
- R^{1c}: für Wasserstoff oder Chlor steht,
- A¹: für N oder CH steht,
- A³: für N, CH oder C-F steht,
- R³: für -OR⁴ oder -NR⁵R⁶ steht,
wobei
R⁴ für (C₁-C₆)-Alkyl oder Pyrazolyl steht,
worin (C₁-C₆)-Alkyl mit 1 bis 3 Substituenten unabhängig voneinander ausgewählt aus der Gruppe Fluor, Trifluormethyl, -(C=O)ₚ-OR⁹ und -(C=O)ₚ-NR⁹R¹⁰ substituiert sein kann,
worin
   p die Zahl 0 oder 1 bedeutet,
   R⁹ und R¹⁰ unabhängig voneinander jeweils für Wasserstoff oder Methyl stehen,
und
worin Pyrazolyl mit 1 oder 2 Substituenten unabhängig voneinander ausgewählt aus der Gruppe Fluor, Difluormethyl, Trifluormethyl, 2,2,2-Trifluorethyl, 1,1,2,2,2-Pentafluorethyl, Methyl, Cyclopropyl, Cyclobutyl, Cyclopentyl substituiert sein kann,
R⁵ für Wasserstoff, Methyl oder Ethyl steht,
R⁶ für (C₁-C₆)-Alkyl, Oxetanyl, Azetidinyl, Tetrahydrofuranyl, Pyrrolidinyl, Tetrahydropyranyl, Piperidinyl, Piperazinyl, Morpholinyl, Phenyl, Pyrazolyl, Oxazolyl, Thiazolyl, Triazolyl, Oxadiazolyl, Thiadiazolyl, Pyridyl oder Pyrimidinyl steht,
worin (C₁-C₆)-Alkyl mit 1 oder 2 Substituenten unabhängig voneinander ausgewählt aus der Gruppe Fluor, Difluormethyl, Trifluormethyl Cyclopropyl, Cyclobutyl, Cyclopentyl, Trifluormethoxy, -(C=O)ₚ-OR⁹, -(C=O)ₚ-NRR⁹R¹⁰, Tetrahydrofuranyl, Pyrrolidinyl, Tetrahydropyranyl, Phenyl, Furanyl, Pyrazolyl, Imidazolyl, Triazolyl und Pyridyl substituiert ist,
worin
   p die Zahl 0 oder 1 bedeutet,
   R⁹ und R¹⁰ unabhängig voneinander jeweils für Wasserstoff, Methyl, Ethyl, Trifluormethyl, 2,2,2-Trifluorethyl, 1,1,2,2,2-Pentafluorethyl, Cyclopropyl oder Cyclobutyl stehen, und
worin Tetrahydrofuranyl, Pyrrolidinyl, Tetrahydropyranyl, Phenyl, Furanyl, Pyrazolyl, Imidazolyl, Triazolyl und Pyridyl ihrerseits mit 1 oder 2 Substituenten unabhängig voneinander ausgewählt aus der Gruppe Fluor, Chlor, Cyano, Difluormethyl, Trifluormethyl, Methyl, Ethyl und Oxo sein können,
worin Oxetanyl, Azetidinyl, Tetrahydrofuranyl, Pyrrolidinyl, Tetrahydropyranyl, Piperidinyl, Piperazinyl, Morpholinyl, Phenyl, Pyrazolyl, Oxazolyl, Thiazolyl, Triazolyl, Oxadiazolyl, Thiadiazolyl, Pyridyl und Pyrimidinylmit 1 oder 2 Substituenten unabhängig voneinander ausgewählt aus der Gruppe Fluor, Difluormethyl, Trifluormethyl, Methyl, Ethyl und Oxo substituiert sein können,
oder R⁵ und R⁶ bilden zusammen mit dem Stickstoffatom, an das sie gebunden sind, einen Azetidinyl-, Pyrrolidinyl-, Imidazolidinyl-, Piperidinyl-, Dihydropiperdinyl-, Piperazinyl-, Morpholinyl-, Pyrazolyl- oder Imidazolyl-Ring,
worin der Azetidinyl-, Pyrrolidinyl-, Imidazolidinyl-, Piperidinyl-, Dihydropiperdinyl-, Piperazinyl-, Morpholinyl-, Pyrazolyl- und Imidazolyl-Ring mit 1 oder 2 Substituenten unabhängig voneinander ausgewählt aus der Gruppe Fluor, Cyano, Difluormethyl, Trifluormethyl, 2,2,2-Trifluorethyl, 1,1,2,2,2-Pentafluorethyl, Methyl, Ethyl, 1-Hydroxyethyl, Cyclopropyl, Cyclobutyl, Cyclopentyl, Hydroxy, Oxo, Methoxy, Ethoxy, Difluormethoxy und Trifluormethoxy substituiert sein können,
R² für 3,3,3-Trifluorprop-1-yl, 2,2,3,3,3-Pentafluorprop-1-yl, Phenyl oder Pyridyl steht,
wobei Phenyl mit 1 bis 3 Substituenten Fluor substituiert ist,
und
wobei Pyridyl mit 1 Substituenten Fluor substituiert sein kann,
sowie ihre Salze, Solvate und Solvate der Salze.

Offenbarungsgegenstand sind auch Verbindungen der Formel (I), in welcher
- L: für eine Gruppe #¹-CR^{7A}R^{7B}-(CR^{8A}R^{8B})ₘ-#² steht,
wobei
#¹ für die Anknüpfstelle an die Carbonylgruppe steht,
#² für die Anknüpfstelle an den Pyrimidinring steht,
m für eine Zahl 0 steht,
R^{7A} für Wasserstoff, Fluor, Methyl oder Hydroxy steht,
R^{7B} für Wasserstoff, Fluor, Trifluormethyl, Methyl oder 2,2,2-Trifluorethyl steht,
oder
R^{7A} und R^{7B} zusammen mit dem Kohlenstoffatom, an das sie gebunden sind, einen Tetrahydrofuranyl-Ring bilden,
der Ring Q für eine Gruppe der Formel 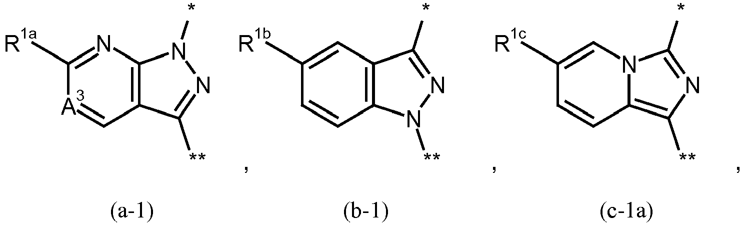 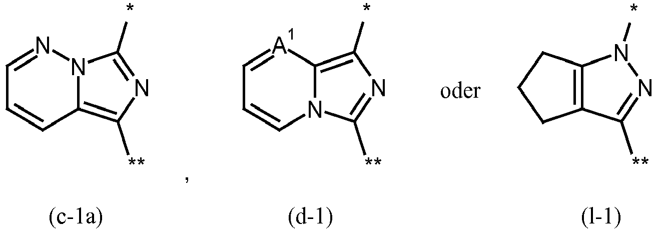 steht, wobei
- *: für die Anknüpfungsstelle an -CH₂-R² steht,
- **: für die Anknüpfungsstelle an das Pyrimidin steht,
- R^{1a}: für Wasserstoff oder Methyl steht,
- R^{1b}: für Wasserstoff oder Fluor steht,
- R^{1c}: für Wasserstoff oder Chlor steht,
- A¹: für N oder CH steht,
- A³: für N, CH oder C-F steht,
- R³: für -NR⁵R⁶ steht,
wobei
R⁵ für Wasserstoff steht,
R⁶ für (C₁-C₆)-Alkyl steht,
worin (C₁-C₆)-Alkyl mit 1 oder 2 Substituenten unabhängig voneinander ausgewählt aus der Gruppe Fluor, Difluormethyl, Trifluormethyl oder -(C=O)ₚ-OR⁹ substituiert ist,
worin
   p die Zahl 0 bedeutet,
   R⁹ für Wasserstoff steht,
- R²: für 2-Fluorphenyl, 2,3-Difluorphenyl oder 3-Fluorpyrid-2-yl steht,
sowie ihre Salze, Solvate und Solvate der Salze.

Offenbarungegenstand sind auch Verbindungen der Formel (I), in welcher
- L: für eine Gruppe #¹-CR^{7A}R^{7B}-(CR^{8A}R^{8B})ₘ-#² steht,
wobei
#¹ für die Anknüpfstelle an die Carbonylgruppe steht,
#² für die Anknüpfstelle an den Pyrimidinring steht,
m für eine Zahl 0 steht,
R^{7B} für Methyl steht,
R^{7B} für Methyl steht,
der Ring Q für eine Gruppe der Formel 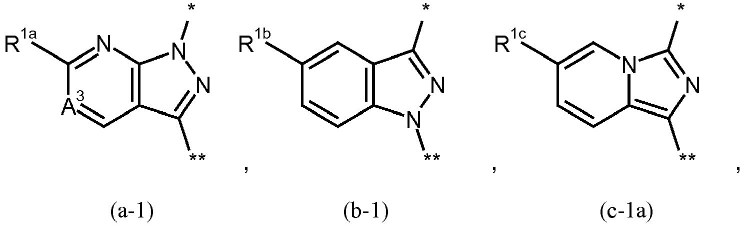 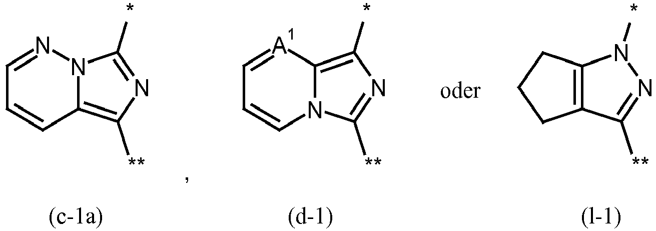 steht, wobei
- *: für die Anknüpfungsstelle an -CH₂-R² steht,
- **: für die Anknüpfungsstelle an das Pyrimidin steht,
- R^{1a}: für Wasserstoff oder Methyl steht,
- R^{1b}: für Wasserstoff oder Fluor steht,
- R^{1c}: für Wasserstoff oder Chlor steht,
- A¹: für N oder CH steht,
- A³: für N, CH oder C-F steht,
- R³: für -NR⁵R⁶ steht,
wobei
R⁵ für Wasserstoff, Methyl oder Ethyl steht,
R⁶ für (C₁-C₆)-Alkyl, Oxetanyl, Azetidinyl, Tetrahydrofuranyl, Pyrrolidinyl, Tetrahydropyranyl, Piperidinyl, Piperazinyl, Morpholinyl, Phenyl, Pyrazolyl, Oxazolyl, Thiazolyl, Triazolyl, Oxadiazolyl, Thiadiazolyl, Pyridyl oder Pyrimidinyl steht,
worin (C₁-C₆)-Alkyl mit 1 oder 2 Substituenten unabhängig voneinander ausgewählt aus der Gruppe Fluor, Difluormethyl, Trifluormethyl Cyclopropyl, Cyclobutyl, Cyclopentyl, Trifluormethoxy, -(C=O)ₚ-OR⁹, -(C=O)ₚ-NRR⁹R¹⁰, Tetrahydrofuranyl, Pyrrolidinyl, Tetrahydropyranyl, Phenyl, Furanyl, Pyrazolyl, Imidazolyl, Triazolyl und Pyridyl substituiert ist,
worin
   p die Zahl 0 oder 1 bedeutet,
   R⁹ und R¹⁰ unabhängig voneinander jeweils für Wasserstoff, Methyl, Ethyl, Trifluormethyl, 2,2,2-Trifluorethyl, 1,1,2,2,2-Pentafluorethyl, Cyclopropyl oder Cyclobutyl stehen, und
worin Tetrahydrofuranyl, Pyrrolidinyl, Tetrahydropyranyl, Phenyl, Furanyl, Pyrazolyl, Imidazolyl, Triazolyl und Pyridyl ihrerseits mit 1 oder 2 Substituenten unabhängig voneinander ausgewählt aus der Gruppe Fluor, Chlor, Cyano, Difluormethyl, Trifluormethyl, Methyl, Ethyl und Oxo sein können,
worin Oxetanyl, Azetidinyl, Tetrahydrofuranyl, Pyrrolidinyl, Tetrahydropyranyl, Piperidinyl, Piperazinyl, Morpholinyl, Phenyl, Pyrazolyl, Oxazolyl, Thiazolyl, Triazolyl, Oxadiazolyl, Thiadiazolyl, Pyridyl und Pyrimidinylmit 1 oder 2 Substituenten unabhängig voneinander ausgewählt aus der Gruppe Fluor, Difluormethyl, Trifluormethyl, Methyl, Ethyl und Oxo substituiert sein können, oder
R⁵ und R⁶ bilden zusammen mit dem Stickstoffatom, an das sie gebunden sind, einen Azetidinyl-, Pyrrolidinyl-, Imidazolidinyl-, Piperidinyl-, Dihydropiperdinyl-, Piperazinyl-, Morpholinyl-, Pyrazolyl- oder Imidazolyl-Ring,
worin der Azetidinyl-, Pyrrolidinyl-, Imidazolidinyl-, Piperidinyl-, Dihydropiperdinyl-, Piperazinyl-, Morpholinyl-, Pyrazolyl- und Imidazolyl-Ring mit 1 oder 2 Substituenten unabhängig voneinander ausgewählt aus der Gruppe Fluor, Cyano, Difluormethyl, Trifluormethyl, 2,2,2-Trifluorethyl, 1,1,2,2,2-Pentafluorethyl, Methyl, Ethyl, 1-Hydroxyethyl, Cyclopropyl, Cyclobutyl, Cyclopentyl, Hydroxy, Oxo, Methoxy, Ethoxy, Difluormethoxy und Trifluormethoxy substituiert sein können,
- R²: für 3,3,3-Trifluorprop-1-yl, 2,2,3,3,3-Pentafluorprop-1-yl, Phenyl oder Pyridyl steht,
wobei Phenyl mit 1 bis 3 Substituenten Fluor substituiert ist,
und
wobei Pyridyl mit 1 Substituenten Fluor substituiert sein kann,
sowie ihre Salze, Solvate und Solvate der Salze.

Besonders bevorzugt sind im Rahmen der vorliegenden Erfindung auch Verbindungen der Formel (I), in welcher
- L: für eine Gruppe #¹-CR^{7A}R^{7B}-(CR^{8A}R^{8B})ₘ-#² steht,
wobei
#¹ für die Anknüpfstelle an die Carbonylgruppe steht,
#² für die Anknüpfstelle an den Pyrimidinring steht,
m für eine Zahl 0 steht,
R^{7B} für Wasserstoff, Fluor, Methyl, Hydroxy steht,
R^{7B} für Wasserstoff, Fluor, Methyl oder Trifluormethyl steht, oder
- R^{7B} und R^{7B}: zusammen mit dem Kohlenstoffatom, an das sie gebunden sind, einen Tetrahydrofuranyl-Ring bilden,
der Ring Q für eine Gruppe der Formel 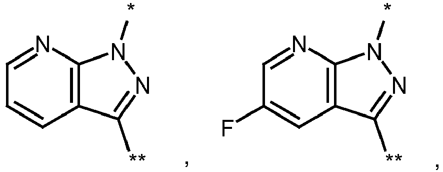 steht, wobei
- *: für die Anknüpfungsstelle an -CH₂-R² steht,
- **: für die Anknüpfungsstelle an das Pyrimidin steht,
- R³: für -OR⁴ oder -NR⁵R⁶ steht,
wobei
R⁴ für (C₁-C₆)-Alkyl oder Pyrazolyl steht,
worin (C₁-C₆)-Alkyl mit 1 bis 3 Substituenten unabhängig voneinander ausgewählt aus der Gruppe Fluor, Trifluormethyl, -(C=O)ₚ-OR⁹ und -(C=O)ₚ-NR⁹R¹⁰ substituiert sein kann,
worin
   p die Zahl 0 oder 1 bedeutet,
   R⁹ und R¹⁰ unabhängig voneinander jeweils für Wasserstoff oder Methyl stehen,
und
worin Pyrazolyl mit 1 oder 2 Substituenten unabhängig voneinander ausgewählt aus der Gruppe Fluor, Difluormethyl, Trifluormethyl, 2,2,2-Trifluorethyl, 1,1,2,2,2-Pentafluorethyl, Methyl, Cyclopropyl, Cyclobutyl, Cyclopentyl substituiert sein kann,
- R⁵: für Wasserstoff, Methyl oder Ethyl steht,
- R⁶: für (C₁-C₆)-Alkyl, Oxetanyl, Azetidinyl, Tetrahydrofuranyl, Pyrrolidinyl, Tetrahydropyranyl, Piperidinyl, Piperazinyl, Morpholinyl, Phenyl, Pyrazolyl, Oxazolyl, Thiazolyl, Triazolyl, Oxadiazolyl, Thiadiazolyl, Pyridyl oder Pyrimidinyl steht,
worin (C₁-C₆)-Alkyl mit 1 oder 2 Substituenten unabhängig voneinander ausgewählt aus der Gruppe Fluor, Difluormethyl, Trifluormethyl Cyclopropyl, Cyclobutyl, Cyclopentyl, Trifluormethoxy, -(C=O)ₚ-OR⁹, -(C=O)ₚ-NRR⁹R¹⁰, Tetrahydrofuranyl, Pyrrolidinyl, Tetrahydropyranyl, Phenyl, Furanyl, Pyrazolyl, Imidazolyl, Triazolyl und Pyridyl substituiert ist,
worin
   p die Zahl 0 oder 1 bedeutet,
   R⁹ und R¹⁰ unabhängig voneinander jeweils für Wasserstoff, Methyl, Ethyl, Trifluormethyl, 2,2,2-Trifluorethyl, 1,1,2,2,2-Pentafluorethyl, Cyclopropyl oder Cyclobutyl stehen, und
worin Cyclopropyl, Cyclobutyl, Cyclopentyl, Tetrahydrofuranyl, Pyrrolidinyl, Tetrahydropyranyl, Phenyl, Furanyl, Pyrazolyl, Imidazolyl, Triazolyl und Pyridyl ihrerseits mit 1 oder 2 Substituenten unabhängig voneinander ausgewählt aus der Gruppe Fluor, Chlor, Cyano, Difluormethyl, Trifluormethyl, Methyl, Ethyl, Oxo und Hydroxy sein können,
worin Oxetanyl, Azetidinyl, Tetrahydrofuranyl, Pyrrolidinyl, Tetrahydropyranyl, Piperidinyl, Piperazinyl, Morpholinyl, Phenyl, Pyrazolyl, Oxazolyl, Thiazolyl, Triazolyl, Oxadiazolyl, Thiadiazolyl, Pyridyl und Pyrimidinyl mit 1 oder 2 Substituenten unabhängig voneinander ausgewählt aus der Gruppe Fluor, Difluormethyl, Trifluormethyl, Methyl, Ethyl, Oxo, Azetidinyl und Pyrrolidinyl substituiert sein können,
oder
- R⁵ und R⁶: bilden zusammen mit dem Stickstoffatom, an das sie gebunden sind, einen Azetidinyl-, Pyrrolidinyl-, Imidazolidinyl-, Piperidinyl-, Dihydropiperdinyl-, Piperazinyl-, Morpholinyl-, Pyrazolyl- oder Imidazolyl-Ring,
worin der Azetidinyl-, Pyrrolidinyl-, Imidazolidinyl-, Piperidinyl-, Dihydropiperdinyl-, Piperazinyl-, Morpholinyl-, Pyrazolyl- und Imidazolyl-Ring mit 1 oder 2 Substituenten unabhängig voneinander ausgewählt aus der Gruppe Fluor, Cyano, Difluormethyl, Trifluormethyl, 2,2,2-Trifluorethyl, 1,1,2,2,2-Pentafluorethyl, Methyl, Ethyl, 1-Hydroxyethyl, Cyclopropyl, Cyclobutyl, Cyclopentyl, Hydroxy, Oxo, Methoxy, Ethoxy, Difluormethoxy und Trifluormethoxy substituiert sein können,
- R²: für 2-Fluorphenyl, 2,3-Difluorphenyl oder 3-Fluorpyrid-2-yl steht,
sowie ihre Salze, Solvate und Solvate der Salze.

Offenbarungsgegenstand sind auch Verbindungen der Formel (I), in welcher
- L: für eine Gruppe #¹-CR^{7A}R^{7B}-(CR^{8A}R^{8B})ₘ-#² steht,
wobei
#¹ für die Anknüpfstelle an die Carbonylgruppe steht,
#² für die Anknüpfstelle an den Pyrimidinring steht,
m für eine Zahl 0, 1 oder 2 steht,
R^{7A} für Wasserstoff, Fluor, (C₁-C₄)-Alkyl, Hydroxy oder Amino steht,
worin (C₁-C₄)-Alkyl mit 1 bis 3 Substituenten unabhängig voneinander ausgewählt aus der Gruppe Fluor, Trifluormethyl, Hydroxy, Hydroxycarbonyl, (C₁-C₄)-Alkoxycarbonyl und Amino substituiert sein kann,
R^{7B} für Wasserstoff, Fluor, (C₁-C₄)-Alkyl, Trifluormethyl, (C₁-C₄)-Alkoxycarbonylamino oder Phenyl steht,
worin (C₁-C₄)-Alkyl mit 1 bis 3 Substituenten unabhängig voneinander ausgewählt aus der Gruppe Fluor, Trifluormethyl, Hydroxy, Hydroxycarbonyl, (C₁-C₄)-Alkoxycarbonyl und Amino substituiert sein kann,
oder
R^{7B} und R^{7B} zusammen mit dem Kohlenstoffatom, an das sie gebunden sind, eine (C₂-C₄)-Alkenyl-Gruppe, eine Oxo-Gruppe, einen 3- bis 6-gliedrigen Carbocyclus oder einen 4- bis 7-gliedrigen Heterocyclus bilden,
worin der 3- bis 6-gliedrigen Carbocyclus und der 4- bis 7-gliedrigen Heterocyclus mit 1 oder 2 Substituenten unabhängig voneinander ausgewählt aus der Gruppe Fluor und (C₁-C₄)-Alkyl substituiert sein können,
R^{8A} für Wasserstoff, Fluor, (C₁-C₄)-Alkyl oder Hydroxy steht,
R^{8B} für Wasserstoff, Fluor, (C₁-C₄)-Alkyl oder Trifluormethyl steht, der Ring Q für 8- oder 9-gliedriges Heteroaryl steht,
R³ für -OR⁴ oder -NR⁵R⁶ steht,
wobei
R⁴ für Wasserstoff, (C₁-C₆)-Alkyl, (C₃-C₇)-Cycloalkyl, 4- bis 7-gliedriges Heterocyclyl oder 5- oder 6-gliedriges Heteroaryl steht,
worin (C₁-C₆)-Alkyl, (C₃-C₇)-Cycloalkyl, 4- bis 7-gliedriges Heterocyclyl und 5-oder 6-gliedriges Heteroaryl mit 1 bis 3 Substituenten unabhängig voneinander ausgewählt aus der Gruppe Fluor, Difluormethyl, Trifluormethyl, (C₃-C₆)-Cycloalkyl, Difluormethoxy, Trifluormethoxy, -(C=O)p OR⁹, -C(=O)ₚ-NR⁹R¹⁰, -NR⁹-(C=O)-R¹⁰, -NR⁹-(C=O)-OR¹⁰, -NR⁹-(C=O)-NR¹⁰R¹¹, -NR⁹-SO₂-R¹⁰, -S(O)_{q}-R¹² und -SO₂-NR⁹R¹⁰ substituiert sein können,
worin
   p die Zahl 0 oder 1 bedeutet,
   q die Zahl 0, 1 oder 2 bedeutet,
   R⁹, R¹⁰ und R¹¹ unabhängig voneinander jeweils für Wasserstoff, (C₁-C₆)-Alkyl oder (C₃-C₈)-Cycloalkyl stehen, worin (C₁-C₆)-Alkyl seinerseits mit 1 oder 2 Substituenten unabhängig voneinander ausgewählt aus der Gruppe Fluor, Difluormethyl, Trifluormethyl, Hydroxy, (C₁-C₆)-Alkoxy, Difluormethoxy, Trifluormethoxy, (C₁-C₆)-Alkoxycarbonyl, Amino, Mono-(C₁-C₆)-alkylamino, Di-(C₁-C₆)-alkylamino und 4- bis 7-gliedriges Heterocyclyl substituiert sein kann,
   oder
   R⁹ und R¹⁰ bilden zusammen mit dem/den Atom/-en, an die sie jeweils gebunden sind, einen 4- bis 7-gliedrigen Heterocyclus,
   worin der 4- bis 7-gliedrigen Heterocyclus seinerseits mit 1 oder 2 Substituenten unabhängig voneinander ausgewählt aus der Gruppe Fluor, Trifluormethyl, (C₁-C₆)-Alkyl, Hydroxy, Oxo, (C₁-C₆)-Alkoxy, Trifluormethoxy, (C₁-C₆)-Alkoxycarbonyl, Amino, Mono-(C₁-C₆)-alkylamino und Di-(C₁-C₆)-alkylamino substituiert sein kann, oder
   R¹⁰ und R¹¹ bilden zusammen mit dem/den Atom/-en, an die sie jeweils gebunden sind, einen 4- bis 7-gliedrigen Heterocyclus,
   worin der 4- bis 7-gliedrigen Heterocyclus seinerseits mit 1 oder 2 Substituenten unabhängig voneinander ausgewählt aus der Gruppe Fluor, Trifluormethyl, (C₁-C₆)-Alkyl, Hydroxy, Oxo, (C₁-C₆)-Alkoxy, Trifluormethoxy, (C₁-C₆)-Alkoxycarbonyl, Amino, Mono-(C₁-C₆)-alkylamino und Di-(C₁-C₆)-alkylamino substituiert sein kann,
   und worin
   R¹² für (C₁-C₆)-Alkyl oder (C₃-C₇)-Cycloalkyl steht,

R⁵ für Wasserstoff oder (C₁-C₄)-Alkyl steht,
R⁶ für (C₁-C₆)-Alkyl, (C₃-C₇)-Cycloalkyl, 4- bis 7-gliedriges Heterocyclyl oder 5- oder 6-gliedriges Heteroaryl steht,
worin (C₁-C₆)-Alkyl (C₃-C₇)-Cycloalkyl, 4- bis 7-gliedriges Heterocyclyl und 5- oder 6-gliedriges Heteroaryl mit 1 bis 3 Substituenten unabhängig voneinander ausgewählt aus der Gruppe Fluor, Difluormethyl, Trifluormethyl, Difluormethoxy, Trifluormethoxy, -(C=O)ₚ-OR⁹, -C(=O)ₚ-NR⁹R¹⁰, -NR⁹-(C=O)-R¹⁰, -NR⁹-(C=O)-OR¹⁰, -NR⁹-(C=O)-NR¹⁰R¹¹, -NR⁹-SO₂-R¹⁰, -S(O)_{q}-R¹² und -SO₂-NR⁹R¹⁰ substituiert sein können,
worin
   p die Zahl 0 oder 1 bedeutet,
   q die Zahl 0, 1 oder 2 bedeutet,
   R⁹, R¹⁰ und R¹¹ unabhängig voneinander jeweils für Wasserstoff, (C₁-C₆)-Alkyl oder (C₃-C₈)-Cycloalkyl stehen,
   oder
   R⁹ und R¹⁰ bilden zusammen mit dem/den Atom/-en, an die sie jeweils gebunden sind, einen 4- bis 7-gliedrigen Heterocyclus,
   worin der 4- bis 7-gliedrigen Heterocyclus seinerseits mit 1 oder 2 Substituenten unabhängig voneinander ausgewählt aus der Gruppe Cyano, Trifluormethyl, (C₁-C₆)-Alkyl, Hydroxy, Oxo, (C₁-C₆)-Alkoxy, Trifluormethoxy, (C₁-C₆)-Alkoxycarbonyl, Amino, Mono-(C₁-C₆)-alkylamino und Di-(C₁-C₆)-alkylamino substituiert sein kann,
   oder
   R¹⁰ und R¹¹ bilden zusammen mit dem/den Atom/-en, an die sie jeweils gebunden sind, einen 4- bis 7-gliedrigen Heterocyclus,
   worin der 4- bis 7-gliedrigen Heterocyclus seinerseits mit 1 oder 2 Substituenten unabhängig voneinander ausgewählt aus der Gruppe Cyano, Trifluormethyl, (C₁-C₆)-Alkyl, Hydroxy, Oxo, (C₁-C₆)-Alkoxy, Trifluormethoxy, (C₁-C₆)-Alkoxycarbonyl, Amino, Mono-(C₁-C₆)-alkylamino und Di-(C₁-C₆)-alkylamino substituiert sein kann,
   und worin
   R¹² für (C₁-C₆)-Alkyl oder (C₃-C₇)-Cycloalkyl steht,
oder
R⁵ und R⁶ bilden zusammen mit dem Stickstoffatom, an das sie gebunden sind, einen 4- bis 7-gliedrigen Heterocyclus,
worin der 4- bis 7-gliedrigen Heterocyclus mit 1 oder 2 Substituenten unabhängig voneinander ausgewählt aus der Gruppe Fluor, Trifluormethyl, (C₁-C₆)-Alkyl, Hydroxy, Oxo, (C₁-C₆)-Alkoxy, Trifluormethoxy, (C₁-C₆)-Alkoxycarbonyl, Amino, Mono-(C₁-C₆)-alkylamino und Di-(C₁-C₆)-alkylamino substituiert sein kann,
- R¹: für Fluor, Chlor oder Methyl steht,
- n: für eine Zahl 0, 1 oder 2 steht,
- R²: für (C₁-C₆)-Alkyl, (C₃-C₈)-Cycloalkyl, Phenyl oder 5- oder 6-gliedriges Heteroaryl steht,
wobei (C₁-C₆)-Alkyl mit einem Substituenten Trifluormethyl substituiert ist,
wobei (C₁-C₆)-Alkyl mit 1 bis 3 Substituenten Fluor substituiert sein kann,
wobei Phenyl mit 1 bis 3 Substituenten Fluor substituiert ist,
und
wobei 5- und 6-gliedriges Heteroaryl mit 1 oder 2 Substituenten unabhängig voneinander ausgewählt aus der Gruppe Fluor und Methyl substituiert sein können,
sowie ihre *N*-Oxide, Salze, Solvate, Salze der *N*-Oxide und Solvate der *N*-Oxide und Salze.

Offenbarungsgegenstand sind auch Verbindungen der Formel (I), in welcher
- L: für eine Gruppe #¹-CR^{7A}R^{7B}-(CR^{8A}R^{8B})ₘ-#² steht,
wobei
#¹ für die Anknüpfstelle an die Carbonylgruppe steht,
#² für die Anknüpfstelle an den Pyrimidinring steht,
m für eine Zahl 0 oder 1 steht,
R^{7A} für Wasserstoff, Fluor, Methyl, Ethyl oder Hydroxy steht,
R^{7B} für Wasserstoff, Fluor, Methyl, Ethyl, Trifluormethyl, Methoxycarbonylamino oder Phenyl steht, worin Methyl und Ethyl mit 1 bis 3 Substituenten unabhängig voneinander ausgewählt aus der Gruppe Fluor, Trifluormethyl und Hydroxy substituiert sein können,
oder
R^{7A} und R^{7B} zusammen mit dem Kohlenstoffatom, an das sie gebunden sind, einen Cyclopropyl-, Cyclobutyl-, Cyclopentyl-, Azetidinyl-, Pyrrolidinyl-, Tetrahydrofuranyl-, Piperidinyl- oder Tetrahydropyranyl-Ring bilden,
worin der Cyclopropyl-, Cyclobutyl-, Cyclopentyl-, Azetidinyl-, Pyrrolidinyl-, Tetrahydrofuranyl-, Piperidinyl- und Tetrahydropyranyl-Ring mit 1 oder 2 Substituenten unabhängig voneinander ausgewählt aus der Gruppe Fluor und Methyl substituiert sein können,
R^{8A} für Wasserstoff, Fluor, Methyl, Ethyl oder Hydroxy steht,
R^{8B} für Wasserstoff, Fluor, Methyl, Ethyl oder Trifluormethyl steht,
der Ring Q für eine Gruppe der Formel 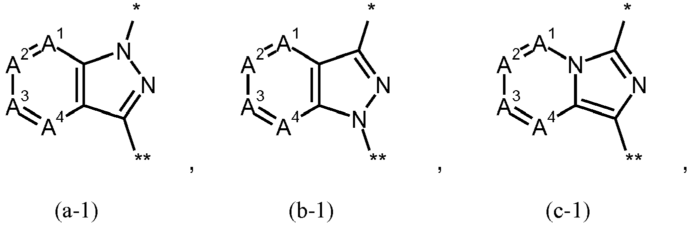 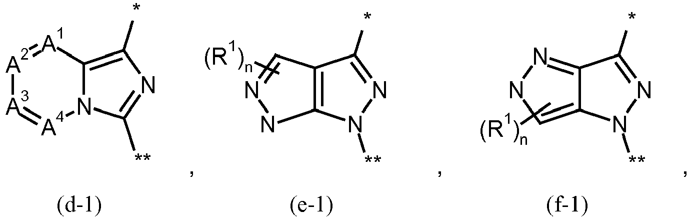 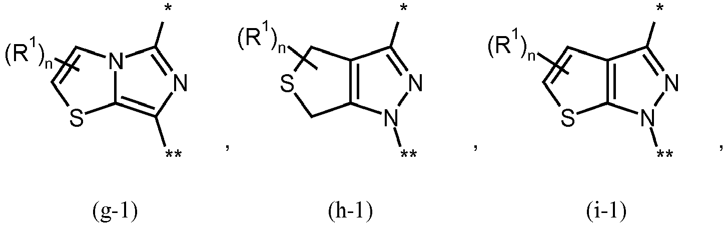 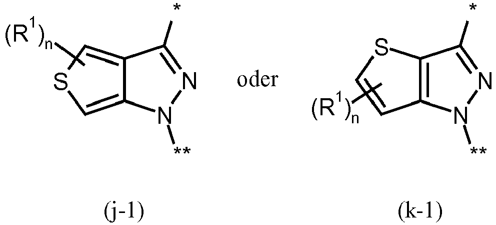 steht, wobei
- *: für die Anknüpfungsstelle an -CH₂-R² steht,
- **: für die Anknüpfungsstelle an das Pyrimidin steht,
- R¹: für Fluor, Chlor oder Methyl steht,
- n: für eine Zahl 0, 1 oder 2 steht,
- A¹, A², A³ und A⁴: unabhängig voneinander jeweils für N, CH oder CR¹ stehen,
mit der Maßgabe, dass maximal zwei der Gruppen A¹, A², A³ und A⁴ für N stehen,
- R³: für -OR⁴ oder -NR⁵R⁶ steht,
wobei
R⁴ für Wasserstoff, (C₁-C₆)-Alkyl oder (C₃-C₇)-Cycloalkyl steht,
worin (C₁-C₆)-Alkyl mit 1 oder 2 Substituenten unabhängig voneinander ausgewählt aus der Gruppe Fluor, Difluormethyl, Trifluormethyl, Difluormethoxy, Trifluormethoxy, -(C=O)ₚ-OR⁹ und -C(=O)ₚ-NR⁹R¹⁰ substituiert sein kann,
worin
   p die Zahl 0 bedeutet,
   R⁹ und R¹⁰ unabhängig voneinander jeweils für Wasserstoff, Methyl oder Ethyl stehen,
   oder
   R⁹ und R¹⁰ bilden zusammen mit dem Stickstoffatom, an das sie gebunden sind, einen Azetidinyl-, Pyrrolidinyl-, Piperidinyl-, Piperazinyl- oder Morpholinyl-Ring, worin der Azetidinyl-, Pyrrolidinyl-, Piperidinyl-, Piperazinyl- und Morpholinyl-Ring seinerseits mit 1 oder 2 Substituenten unabhängig voneinander ausgewählt aus der Gruppe Fluor, Trifluormethyl, Methyl, Ethyl, Hydroxy, Oxo, Methoxy, Ethoxy, Amino, Methylamino, Ethylamino, Dimethylamino und Diethylamino substituiert sein können,

R⁵ für Wasserstoff oder Methyl steht,
R⁶ für (C₁-C₆)-Alkyl oder (C₃-C₇)-Cycloalkyl steht,
worin (C₁-C₆)-Alkyl mit 1 oder 2 Substituenten unabhängig voneinander ausgewählt aus der Gruppe Fluor, Difluormethyl, Trifluormethyl, Difluormethoxy, Trifluormethoxy, -(C=O)ₚ-OR⁹ und -C(=O)ₚ-NR⁹R¹⁰ substituiert sein kann,
worin
p die Zahl 0 bedeutet,
R⁹ und R¹⁰ unabhängig voneinander jeweils für Wasserstoff, Methyl oder Ethyl stehen,
oder
R⁹ und R¹⁰ bilden zusammen mit dem Stickstoffatom, an das sie gebunden sind, einen Azetidinyl-, Pyrrolidinyl-, Piperidinyl-, Piperazinyl- oder Morpholinyl-Ring,
worin der Azetidinyl-, Pyrrolidinyl-, Piperidinyl-, Piperazinyl- und Morpholinyl-Ring seinerseits mit 1 oder 2 Substituenten unabhängig voneinander ausgewählt aus der Gruppe Fluor, Trifluormethyl, Methyl, Ethyl, Hydroxy, Oxo, Methoxy, Ethoxy, Amino, Methylamino, Ethylamino, Dimethylamino und Diethylamino substituiert sein können,
oder
R⁵ und R⁶ bilden zusammen mit dem Stickstoffatom, an das sie gebunden sind, einen Azetidinyl-, Pyrrolidinyl-, Piperidinyl-, Piperazinyl- oder Morpholinyl-Ring, worin der Azetidinyl-, Pyrrolidinyl-, Piperidinyl-, Piperazinyl- und Morpholinyl-Ring seinerseits mit 1 oder 2 Substituenten unabhängig voneinander ausgewählt aus der Gruppe Fluor, Trifluormethyl, Methyl, Ethyl, Hydroxy, Oxo, Methoxy, Ethoxy, Amino, Methylamino, Ethylamino, Dimethylamino und Diethylamino substituiert sein können,
R² für Trifluormethyl, 2,2,2-Trifluorethyl, 3,3,3-Trifluorprop-1-yl, 2,2,3,3,3-Pentafluorprop-1-yl, Cyclopropyl, Cyclobutyl, Cyclopentyl, Cyclohexyl, Phenyl, Pyridyl, Pyrimidinyl, Pyrazinyl oder Pyridazinyl steht,
wobei Phenyl mit 1 bis 3 Substituenten Fluor substituiert ist,
und
wobei Pyridyl, Pyrimidinyl, Pyrazinyl und Pyridazinyl mit 1 oder 2 Substituenten Fluor substituiert sein können,
sowie ihre Salze, Solvate und Solvate der Salze.

Offenbarungsgegenstand sind auch Verbindungen der Formel (I), in welcher
- L: für eine Gruppe #¹-CR^{7A}R^{7B}-(CR^{8A}R^{8B})ₘ-#² steht,
wobei
#¹ für die Anknüpfstelle an die Carbonylgruppe steht,
#² für die Anknüpfstelle an den Pyrimidinring steht,
m für eine Zahl 0 steht,
R^{7A} für Wasserstoff, Fluor, Methyl oder Hydroxy steht,
R^{7B} für Wasserstoff, Fluor, Methyl oder Trifluormethyl steht,
oder
R^{7A} und R^{7B} zusammen mit dem Kohlenstoffatom, an das sie gebunden sind, einen Cyclopropyl- oder Cyclobutyl-Ring bilden,
worin der Cyclopropyl- und der Cyclobutyl-Ring mit 1 oder 2 Substituenten unabhängig voneinander ausgewählt aus der Gruppe Fluor und Methyl substituiert sein können,
der Ring Q für eine Gruppe der Formel 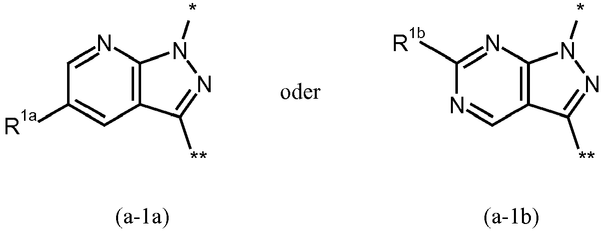 steht, wobei
- *: für die Anknüpfungsstelle an -CH₂-R² steht,
- **: für die Anknüpfungsstelle an das Pyrimidin steht,
- R^{1a}: für Wasserstoff oder Fluor steht,
- R^{1b}: für Wasserstoff oder Methyl steht,
- R³: für -OR⁴ oder -NR⁵R⁶ steht,
wobei
R⁴ für Wasserstoff oder (C₁-C₆)-Alkyl steht,
worin (C₁-C₆)-Alkyl mit 1 oder 2 Substituenten unabhängig voneinander ausgewählt aus der Gruppe Fluor, Difluormethyl, Trifluormethyl, Difluormethoxy, Trifluormethoxy, -(C=O)ₚ-OR⁹ und -C(=O)ₚ-NR⁹R¹⁰ substituiert sein kann,
worin
   p die Zahl 0 bedeutet,
   R⁹ und R¹⁰ unabhängig voneinander jeweils für Wasserstoff stehen,

R⁵ für Wasserstoff steht,
R⁶ für (C₁-C₆)-Alkyl steht,
worin (C₁-C₆)-Alkyl mit 1 oder 2 Substituenten unabhängig voneinander ausgewählt aus der Gruppe Fluor, Difluormethyl, Trifluormethyl, Difluormethoxy, Trifluormethoxy, -(C=O)ₚ-OR⁹ und -C(=O)ₚ-NR⁹R¹⁰ substituiert ist, worin
p die Zahl 0 bedeutet,
R⁹ und R¹⁰ unabhängig voneinander jeweils für Wasserstoff stehen,
- R²: für 2,2,3,3,3-Pentafluorprop-1-yl, 2-Fluorphenyl oder 2,3,6-Trifluorphenyl steht,
sowie ihre Salze, Solvate und Solvate der Salze.

Offenbarungsgegenstand sind auch Verbindungen der Formel (I), in welcher
- R³: für -OR⁴ oder -NR⁵R⁶ steht,
wobei
R⁴ für (C₁-C₆)-Alkyl oder (C₃-C₇)-Cycloalkyl steht,
worin (C₁-C₆)-Alkyl mit 1 oder 2 Substituenten unabhängig voneinander ausgewählt aus der Gruppe Fluor, Difluormethyl, Trifluormethyl, Difluormethoxy, Trifluormethoxy, -(C=O)ₚ-OR⁹ und -C(=O)ₚ-NR⁹R¹⁰ substituiert sein kann,
worin
   p die Zahl 0 bedeutet,
   R⁹ und R¹⁰ unabhängig voneinander jeweils für Wasserstoff, Methyl oder Ethyl stehen,
   oder
   R⁹ und R¹⁰ bilden zusammen mit dem Stickstoffatom, an das sie gebunden sind, einen Azetidinyl-, Pyrrolidinyl-, Piperidinyl-, Piperazinyl- oder Morpholinyl-Ring, worin der Azetidinyl-, Pyrrolidinyl-, Piperidinyl-, Piperazinyl- und Morpholinyl-Ring seinerseits mit 1 oder 2 Substituenten unabhängig voneinander ausgewählt aus der Gruppe Fluor, Trifluormethyl, Methyl, Ethyl, Hydroxy, Oxo, Methoxy, Ethoxy, Amino, Methylamino, Ethylamino, Dimethylamino und Diethylamino substituiert sein können,

R⁵ für Wasserstoff oder Methyl steht,
R⁶ für (C₁-C₆)-Alkyl oder (C₃-C₇)-Cycloalkyl steht,
worin (C₁-C₆)-Alkyl mit 1 oder 2 Substituenten unabhängig voneinander ausgewählt aus der Gruppe Fluor, Difluormethyl, Trifluormethyl, Difluormethoxy, Trifluormethoxy, -(C=O)ₚ-OR⁹ und -C(=O)ₚ-NR⁹R¹⁰ substituiert sein kann,
worin
   p die Zahl 0 bedeutet,
   R⁹ und R¹⁰ unabhängig voneinander jeweils für Wasserstoff, Methyl oder Ethyl stehen,
   oder
   R⁹ und R¹⁰ bilden zusammen mit dem Stickstoffatom, an das sie gebunden sind, einen Azetidinyl-, Pyrrolidinyl-, Piperidinyl-, Piperazinyl- oder Morpholinyl-Ring,
   worin der Azetidinyl-, Pyrrolidinyl-, Piperidinyl-, Piperazinyl- und Morpholinyl-Ring seinerseits mit 1 oder 2 Substituenten unabhängig voneinander ausgewählt aus der Gruppe Fluor, Trifluormethyl, Methyl, Ethyl, Hydroxy, Oxo, Methoxy, Ethoxy, Amino, Methylamino, Ethylamino, Dimethylamino und Diethylamino substituiert sein können,
oder
R⁵ und R⁶ bilden zusammen mit dem Stickstoffatom, an das sie gebunden sind, einen Azetidinyl-, Pyrrolidinyl-, Piperidinyl-, Piperazinyl- oder Morpholinyl-Ring,
worin der Azetidinyl-, Pyrrolidinyl-, Piperidinyl-, Piperazinyl- und Morpholinyl-Ring seinerseits mit 1 oder 2 Substituenten unabhängig voneinander ausgewählt aus der Gruppe Fluor, Trifluormethyl, Methyl, Ethyl, Hydroxy, Oxo, Methoxy, Ethoxy, Amino, Methylamino, Ethylamino, Dimethylamino und Diethylamino substituiert sein können,
n, L, Q, R¹ und R² jeweils die oben angegebenen Bedeutungen haben,
sowie ihre Salze, Solvate und Solvate der Salze.

Offenbarungsgegenstand sind auch Verbindungen der Formel (I), in welcher
- R³: für -NR⁵R⁶ steht,
wobei
R⁵ für Wasserstoff oder Methyl steht,
R⁶ für (C₁-C₆)-Alkyl oder (C₃-C₇)-Cycloalkyl steht,
worin (C₁-C₆)-Alkyl mit 1 oder 2 Substituenten unabhängig voneinander ausgewählt aus der Gruppe Fluor, Difluormethyl, Trifluormethyl, Difluormethoxy, Trifluormethoxy, -(C=O)ₚ-OR⁹ und -C(=O)ₚ-NR⁹R¹⁰ substituiert sein kann,
worin
   p die Zahl 0 bedeutet,
   R⁹ und R¹⁰ unabhängig voneinander jeweils für Wasserstoff, Methyl oder Ethyl stehen,
   oder
   R⁹ und R¹⁰ bilden zusammen mit dem Stickstoffatom, an das sie gebunden sind, einen Azetidinyl-, Pyrrolidinyl-, Piperidinyl-, Piperazinyl- oder Morpholinyl-Ring,
   worin der Azetidinyl-, Pyrrolidinyl-, Piperidinyl-, Piperazinyl- und Morpholinyl-Ring seinerseits mit 1 oder 2 Substituenten unabhängig voneinander ausgewählt aus der Gruppe Fluor, Trifluormethyl, Methyl, Ethyl, Hydroxy, Oxo, Methoxy, Ethoxy, Amino, Methylamino, Ethylamino, Dimethylamino und Diethylamino substituiert sein können,
oder
R⁵ und R⁶ bilden zusammen mit dem Stickstoffatom, an das sie gebunden sind, einen Azetidinyl-, Pyrrolidinyl-, Piperidinyl-, Piperazinyl- oder Morpholinyl-Ring,
worin der Azetidinyl-, Pyrrolidinyl-, Piperidinyl-, Piperazinyl- und Morpholinyl-Ring seinerseits mit 1 oder 2 Substituenten unabhängig voneinander ausgewählt aus der Gruppe Fluor, Trifluormethyl, Methyl, Ethyl, Hydroxy, Oxo, Methoxy, Ethoxy, Amino, Methylamino, Ethylamino, Dimethylamino und Diethylamino substituiert sein können,
n, L, Q, R¹ und R² jeweils die oben angegebenen Bedeutungen haben,
sowie ihre Salze, Solvate und Solvate der Salze.

Offenbarungsgegenstand sind auch Verbindungen der Formel (I), in welcher
- R³: für -NR⁵R⁶ steht,
wobei
R⁵ für Wasserstoff oder Methyl steht,
R⁶ für (C₁-C₆)-Alkyl oder (C₃-C₇)-Cycloalkyl steht,
worin (C₁-C₆)-Alkyl und (C₃-C₇)-Cycloalkyl mit 1 oder 2 Substituenten unabhängig voneinander ausgewählt aus der Gruppe Fluor, Difluormethyl, Trifluormethyl, Difluormethoxy, Trifluormethoxy, -(C=O)ₚ-OR⁹ und -C(=O)ₚ-NR⁹R¹⁰ substituiert sind,
worin
   p die Zahl 0 bedeutet,
   R⁹ und R¹⁰ unabhängig voneinander jeweils für Wasserstoff, Methyl oder Ethyl stehen,
   oder
   R⁹ und R¹⁰ bilden zusammen mit dem Stickstoffatom, an das sie gebunden sind, einen Azetidinyl-, Pyrrolidinyl-, Piperidinyl-, Piperazinyl- oder Morpholinyl-Ring,
   worin der Azetidinyl-, Pyrrolidinyl-, Piperidinyl-, Piperazinyl- und Morpholinyl-Ring seinerseits mit 1 oder 2 Substituenten unabhängig voneinander ausgewählt aus der Gruppe Fluor, Trifluormethyl, Methyl, Ethyl, Hydroxy, Oxo, Methoxy, Ethoxy, Amino, Methylamino, Ethylamino, Dimethylamino und Diethylamino substituiert sein können,
oder
R⁵ und R⁶ bilden zusammen mit dem Stickstoffatom, an das sie gebunden sind, einen Azetidinyl-, Pyrrolidinyl-, Piperidinyl-, Piperazinyl- oder Morpholinyl-Ring,
worin der Azetidinyl-, Pyrrolidinyl-, Piperidinyl-, Piperazinyl- und Morpholinyl-Ring seinerseits mit 1 oder 2 Substituenten unabhängig voneinander ausgewählt aus der Gruppe Fluor, Trifluormethyl, Methyl, Ethyl, Hydroxy, Oxo, Methoxy, Ethoxy, Amino, Methylamino, Ethylamino, Dimethylamino und Diethylamino substituiert sein können,
n, L, Q, R¹ und R² jeweils die oben angegebenen Bedeutungen haben,
sowie ihre Salze, Solvate und Solvate der Salze.

Offenbarungsgegenstand sind auch Verbindungen der Formel (I), in welcher
der Ring Q für eine Gruppe der Formel 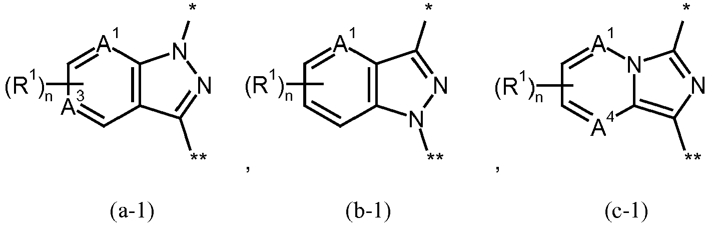 oder 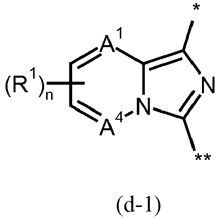 steht, wobei
- *: für die Anknüpfungsstelle an -CH₂-R² steht,
- **: für die Anknüpfungsstelle an das Pyrimidin steht,
- A¹, A³ und A⁴: unabhängig voneinander jeweils für N, CH oder CR¹ stehen, worin
R¹ für Fluor, Chlor oder Methyl steht,
n für eine Zahl 0, 1 oder 2 steht,
mit der Maßgabe, dass maximal zwei der Gruppen A¹, A³ und A⁴ für N stehen,
- R³: für -NR⁵R⁶ steht,
wobei
R⁵ für Wasserstoff oder Methyl steht,
R⁶ für (C₁-C₆)-Alkyl steht,
worin (C₁-C₆)-Alkyl mit 1 oder 2 Substituenten unabhängig voneinander ausgewählt aus der Gruppe Fluor, Difluormethyl und Trifluormethyl substituiert ist,
L und R² jeweils die oben angegebenen Bedeutungen haben,
sowie ihre Salze, Solvate und Solvate der Salze.

Bevorzugt sind im Rahmen der vorliegenden Erfindung auch Verbindungen der Formel (I), in welcher
der Ring Q für eine Gruppe der Formel 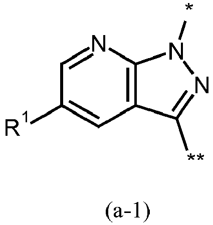 steht, wobei
- *: für die Anknüpfungsstelle an -CH₂-R² steht,
- **: für die Anknüpfungsstelle an das Pyrimidin steht,
- R¹: für Wasserstoff oder Fluor steht,
- R³: für -NR⁵R⁶ steht,
wobei
- R⁵: für Wasserstoff oder Methyl steht,
- R⁶: für (C₁-C₆)-Alkyl steht,
worin (C₁-C₆)-Alkyl mit 1 oder 2 Substituenten unabhängig voneinander ausgewählt aus der Gruppe Fluor, Difluormethyl und Trifluormethyl substituiert ist,
- für: 2-Fluorphenyl oder 3-Fluorpyrid-2-yl steht,
und
L und R² jeweils die oben angegebenen Bedeutungen haben,
sowie ihre Salze, Solvate und Solvate der Salze.

Bevorzugt sind im Rahmen der vorliegenden Erfindung auch Verbindungen der Formel (I), in welcher
- R³: für -NR⁵R⁶ steht,
wobei
R⁵ für Wasserstoff oder Methyl steht,
R⁶ für (C₁-C₆)-Alkyl steht,
worin (C₁-C₆)-Alkyl mit 1 oder 2 Substituenten unabhängig voneinander ausgewählt aus der Gruppe Fluor, Difluormethyl und Trifluormethyl substituiert ist,
n, L, Q, R¹ und R² jeweils die oben angegebenen Bedeutungen haben,
sowie ihre Salze, Solvate und Solvate der Salze.

Bevorzugt sind im Rahmen der vorliegenden Erfindung auch Verbindungen der Formel (I), in welcher
- R³: für -NR⁵R⁶ steht,
wobei
R⁵ für Wasserstoff oder Methyl steht,
R⁶ für (C₁-C₆)-Alkyl steht, worin (C₁-C₆)-Alkyl mit 1 oder 2 Substituenten unabhängig voneinander ausgewählt aus der Gruppe Fluor, Difluormethyl, Trifluormethyl und -(C=O)ₚ-OR⁹ substituiert ist,
worin
   p die Zahl 0 bedeutet,
   R⁹ für Wasserstoff steht,
n, L, Q, R¹ und R² jeweils die oben angegebenen Bedeutungen haben,
sowie ihre Salze, Solvate und Solvate der Salze.

Bevorzugt sind im Rahmen der vorliegenden Erfindung auch Verbindungen der Formel (I), in welcher
- L: für eine Gruppe #¹-CR^{7A}R^{7B}-(CR^{8A}R^{8B})ₘ-#² steht,
wobei
#¹ für die Anknüpfstelle an die Carbonylgruppe steht,
#² für die Anknüpfstelle an den Pyrimidinring steht,
m für eine Zahl 0 steht,
R^{7A} für Methyl steht,
R^{7B} für Methyl steht,
- R³: für -NR⁵R⁶ steht,
wobei
R⁵ für Wasserstoff oder Methyl steht,
R⁶ für (C₁-C₆)-Alkyl steht,
worin (C₁-C₆)-Alkyl mit 1 oder 2 Substituenten unabhängig voneinander ausgewählt aus der Gruppe Fluor, Difluormethyl, Trifluormethyl und -(C=O)ₚ-OR⁹ substituiert ist,
worin
p die Zahl 0 bedeutet,
R⁹ für Wasserstoff steht,
n, Q, R¹ und R² jeweils die oben angegebenen Bedeutungen haben,
sowie ihre Salze, Solvate und Solvate der Salze.

Bevorzugt sind im Rahmen der vorliegenden Erfindung auch Verbindungen der Formel (I), in welcher
- L: für eine Gruppe #¹-CR^{7A}R^{7B}-(CR^{8A}R^{8B})ₘ-#² steht,
wobei
#¹ für die Anknüpfstelle an die Carbonylgruppe steht,
#² für die Anknüpfstelle an den Pyrimidinring steht,
m für eine Zahl 0 steht,
R^{7A} für Wasserstoff, Fluor, Methyl oder Hydroxy steht,
R^{7B} für Wasserstoff, Fluor, Methyl oder Trifluormethyl steht,
- R³: für -NR⁵R⁶ steht,
wobei
R⁵ für Wasserstoff oder Methyl steht,
R⁶ für (C₁-C₆)-Alkyl steht,
worin (C₁-C₆)-Alkyl mit 1 oder 2 Substituenten unabhängig voneinander ausgewählt aus der Gruppe Fluor, Difluormethyl, Trifluormethyl und -(C=O)ₚ-OR⁹ substituiert ist,
worin
   p die Zahl 0 bedeutet,
   R⁹ für Wasserstoff steht,
n, Q, R¹ und R² jeweils die oben angegebenen Bedeutungen haben,
sowie ihre Salze, Solvate und Solvate der Salze.

Offenbarungsgegenstand sind auch Verbindungen der Formel (I), in welcher
- R³: für -OR⁴ steht,
wobei
R⁴ für Wasserstoff, (C₁-C₆)-Alkyl oder (C₃-C₇)-Cycloalkyl steht,
worin (C₁-C₆)-Alkyl mit 1 oder 2 Substituenten unabhängig voneinander ausgewählt aus der Gruppe Fluor, Difluormethyl, Trifluormethyl, Difluormethoxy, Trifluormethoxy, -(C=O)ₚ-OR⁹ und -C(=O)ₚ-NR⁹R¹⁰ substituiert sein kann,
worin
   p die Zahl 0 bedeutet,
   R⁹ und R¹⁰ unabhängig voneinander jeweils für Wasserstoff, Methyl oder Ethyl stehen,
   oder
   R⁹ und R¹⁰ bilden zusammen mit dem Stickstoffatom, an das sie gebunden sind, einen Azetidinyl-, Pyrrolidinyl-, Piperidinyl-, Piperazinyl- oder Morpholinyl-Ring,
   worin der Azetidinyl-, Pyrrolidinyl-, Piperidinyl-, Piperazinyl- und Morpholinyl-Ring seinerseits mit 1 oder 2 Substituenten unabhängig voneinander ausgewählt aus der Gruppe Fluor, Trifluormethyl, Methyl, Ethyl, Hydroxy, Oxo, Methoxy, Ethoxy, Amino, Methylamino, Ethylamino, Dimethylamino und Diethylamino substituiert sein können,
n, L, Q, R¹ und R² jeweils die oben angegebenen Bedeutungen haben,
sowie ihre Salze, Solvate und Solvate der Salze.

Offenbarungsgegenstand sind auch Verbindungen der Formel (I), in welcher
- R³: für -OR⁴ steht,
wobei
- R⁴: für Wasserstoff steht,
n, L, Q, R¹ und R² jeweils die oben angegebenen Bedeutungen haben,
sowie ihre Salze, Solvate und Solvate der Salze.

Offenbarungsgegenstand sind auch Verbindungen der Formel (I), in welcher
der Ring Q für eine Gruppe der Formel 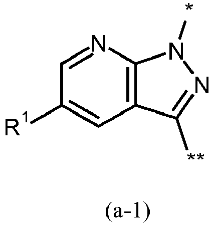 steht, wobei
- *: für die Anknüpfungsstelle an -CH₂-R² steht,
- **: für die Anknüpfungsstelle an das Pyrimidin steht,
- R¹: für Wasserstoff oder Fluor steht,
- R³: für -OR⁴ steht,
wobei
R⁴ für Wasserstoff steht,
n, L, Q, R¹ und R² jeweils die oben angegebenen Bedeutungen haben,
sowie ihre Salze, Solvate und Solvate der Salze.

Bevorzugt sind im Rahmen der vorliegenden Erfindung auch Verbindungen der Formel (I), in welcher R¹ für H steht, sowie ihre Salze, Solvate und Solvate der Salze.

Bevorzugt sind im Rahmen der vorliegenden Erfindung auch Verbindungen der Formel (I), in welcher R¹ für Fluor steht, sowie ihre Salze, Solvate und Solvate der Salze.

Bevorzugt sind im Rahmen der vorliegenden Erfindung auch Verbindungen der Formel (I), in welcher R¹ für Methyl steht, sowie ihre Salze, Solvate und Solvate der Salze.

Offenbarungsgegenstand sind auch Verbindungen der Formel (I), in welcher
- R²: für 2,2,3,3,3-Pentafluorprop-1-yl, 2-Fluorphenyl, 2,3-Difluorphenyl, 2,3,6-Trifluorphenyl, 3-Fluorpyrid-2-yl oder Pyrimidin-2-yl steht,
sowie ihre Salze, Solvate und Solvate der Salze.

Besonders bevorzugt sind im Rahmen der vorliegenden Erfindung auch Verbindungen der Formel (I), in welcher
- R²: für 2-Fluorphenyl oder 3-Fluorpyrid-2-yl steht,
sowie ihre Salze, Solvate und Solvate der Salze.

Besonders bevorzugt sind im Rahmen der vorliegenden Erfindung auch Verbindungen der Formel (I), in welcher
- R²: für 2-Fluorphenyl steht,
sowie ihre Salze, Solvate und Solvate der Salze.

Besonders bevorzugt sind im Rahmen der vorliegenden Erfindung auch Verbindungen der Formel (I), in welcher
- R²: für 3-Fluorpyrid-2-yl oder Pyrimidin-2-yl steht,
sowie ihre Salze, Solvate und Solvate der Salze.

Offenbarungsgegenstand sind auch Verbindungen der Formel (I), in welcher
- L: für eine Gruppe #¹-CR^{7A}R^{7B}-(CR^{8A}R^{8B})ₘ-#² steht,
wobei
#¹ für die Anknüpfstelle an die Carbonylgruppe steht,
#² für die Anknüpfstelle an den Pyrimidinring steht,
m für eine Zahl 0 steht,
R^{7A} für Wasserstoff, Fluor, Methyl oder Hydroxy steht,
R^{7B} für Wasserstoff, Fluor, Methyl oder Trifluormethyl steht, oder

- R^{7A} und R^{7B}: zusammen mit dem Kohlenstoffatom, an das sie gebunden sind, einen Cyclopropyl- oder Cyclobutyl-Ring bilden,
worin der Cyclopropyl- und der Cyclobutyl-Ring mit 1 oder 2 Substituenten unabhängig voneinander ausgewählt aus der Gruppe Fluor und Methyl substituiert sein können,
n, Q, R¹, R² und R³ jeweils die oben angegebenen Bedeutungen haben,
sowie ihre Salze, Solvate und Solvate der Salze.

Bevorzugt sind im Rahmen der vorliegenden Erfindung auch Verbindungen der Formel (I), in welcher
- L: für eine Gruppe #¹-CR^{7A}R^{7B}-(CR^{8A}R^{8B})ₘ-#² steht,
wobei
#¹ für die Anknüpfstelle an die Carbonylgruppe steht,
#² für die Anknüpfstelle an den Pyrimidinring steht,
m für eine Zahl 0 steht,
R^{7A} für Wasserstoff, Fluor, Methyl oder Hydroxy steht,
R^{7B} für Wasserstoff, Fluor, Methyl oder Trifluormethyl steht,
n, Q, R¹, R² und R³ jeweils die oben angegebenen Bedeutungen haben,
sowie ihre Salze, Solvate und Solvate der Salze.

Bevorzugt sind im Rahmen der vorliegenden Erfindung auch Verbindungen der Formel (I), in welcher
- L: für eine Gruppe #¹-CR^{7A}R^{7B}-(CR^{8A}R^{8B})ₘ-#² steht,
wobei
#¹ für die Anknüpfstelle an die Carbonylgruppe steht,
#² für die Anknüpfstelle an den Pyrimidinring steht,
m für eine Zahl 0 steht,
R^{7A} für Methyl steht,
R^{7B} für Methyl steht,
n, Q, R¹, R² und R³ jeweils die oben angegebenen Bedeutungen haben,
sowie ihre Salze, Solvate und Solvate der Salze.

Bevorzugt sind im Rahmen der vorliegenden Erfindung auch Verbindungen der Formel (I), in welcher
- L: für eine Gruppe #¹-CR^{7A}R^{7B}-(CR^{8A}R^{8B})ₘ-#² steht,
wobei
#¹ für die Anknüpfstelle an die Carbonylgruppe steht,
#² für die Anknüpfstelle an den Pyrimidinring steht,
m für eine Zahl 0 steht,
R^{7A} für Methyl steht,
R^{7B} für Methyl steht, oder
R^{7A} und R^{7B} zusammen mit dem Kohlenstoffatom, an das sie gebunden sind, einen Tetrahydrofuranyl-Ring bilden,
n, Q, R¹, R² und R³ jeweils die oben angegebenen Bedeutungen haben,
sowie ihre Salze, Solvate und Solvate der Salze.

Offenbarungsgegenstand sind auch Verbindungen der Formel (I), in welcher
- L: für eine Gruppe #¹-CR^{7A}R^{7B}-(CR^{8A}R^{8B})ₘ-#² steht,
wobei
#¹ für die Anknüpfstelle an die Carbonylgruppe steht,
#² für die Anknüpfstelle an den Pyrimidinring steht,
m für eine Zahl 0 steht,
R^{7A} für Wasserstoff, Fluor, Methyl, Ethyl, Hydroxy oder Amino steht,
R^{7B} für eine Gruppe der Formel -M-R¹³ steht,
worin
M für eine Bindung steht,
R¹³ für -(C=O)ᵣ-NR¹⁴R¹⁵, Phenyl, Thiazolyl, Triazolyl, Oxadiazolyl, Thiadiazolyl oder Pyrimidinyl steht,
worin
r die Zahl 1 bedeutet,
R¹⁴ und R¹⁵ unabhängig voneinander jeweils für Wasserstoff oder Cyclopropyl stehen, und
worin Phenyl, Thiazolyl, Triazolyl, Oxadiazolyl, Thiadiazolyl und Pyrimidinyl ihrerseits mit 1 oder 2 Substituenten unabhängig voneinander ausgewählt aus der Gruppe Fluor, Difluormethyl, Trifluormethyl, Methyl, Ethyl, iso-Propyl, 2,2,2-Trifluorethyl, 1,1,2,2,2-Pentafluorethyl, Cyclopropyl, Cyclobutyl, Cyclopropylmetyl und Cyclobutylmethyl substituiert sein können,
sowie ihre Salze, Solvate und Solvate der Salze.

Offenbarungsgegenstand sind auch Verbindungen der Formel (I), in welcher
- R³: für -NR⁵R⁶ steht,
wobei
R⁵ für Wasserstoff oder Methyl steht,
R⁶ für (C₁-C₆)-Alkyl oder (C₃-C₇)-Cycloalkyl steht,
worin (C₁-C₆)-Alkyl mit 1 oder 2 Substituenten unabhängig voneinander ausgewählt aus der Gruppe Fluor, Difluormethyl, Trifluormethyl, Difluormethoxy, Trifluormethoxy, -(C=O)ₚ-OR⁹ und -C(=O)ₚ-NR⁹R¹⁰ substituiert sein kann,
worin
   p die Zahl 0 bedeutet,
   R⁹ und R¹⁰ unabhängig voneinander jeweils für Wasserstoff, Methyl oder Ethyl stehen, oder
   R⁹ und R¹⁰ bilden zusammen mit dem Stickstoffatom, an das sie gebunden sind, einen Azetidinyl-, Pyrrolidinyl-, Piperidinyl-, Piperazinyl- oder Morpholinyl-Ring,
   oder
   worin der Azetidinyl-, Pyrrolidinyl-, Piperidinyl-, Piperazinyl- und Morpholinyl-Ring seinerseits mit 1 oder 2 Substituenten unabhängig voneinander ausgewählt aus der Gruppe Fluor, Trifluormethyl, Methyl, Ethyl, Hydroxy, Oxo, Methoxy, Ethoxy, Amino, Methylamino, Ethylamino, Dimethylamino und Diethylamino substituiert sein können,

R⁵ und R⁶ bilden zusammen mit dem Stickstoffatom, an das sie gebunden sind, einen Azetidinyl-, Pyrrolidinyl-, Piperidinyl-, Piperazinyl- oder Morpholinyl-Ring,
worin der Azetidinyl-, Pyrrolidinyl-, Piperidinyl-, Piperazinyl- und Morpholinyl-Ring seinerseits mit 1 oder 2 Substituenten unabhängig voneinander ausgewählt aus der Gruppe Fluor, Trifluormethyl, Methyl, Ethyl, Hydroxy, Oxo, Methoxy, Ethoxy, Amino, Methylamino, Ethylamino, Dimethylamino und Diethylamino substituiert sein können,
der Ring Q für eine Gruppe der Formel 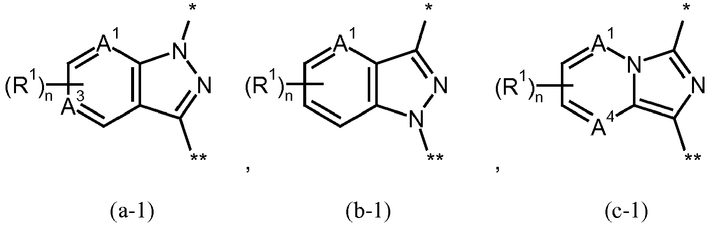 oder 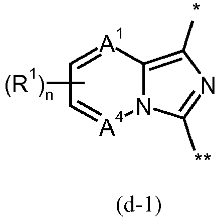 steht, wobei
- *: für die Anknüpfungsstelle an -CH₂-R² steht,
- **: für die Anknüpfungsstelle an das Pyrimidin steht,
- A¹, A³ und A⁴: unabhängig voneinander jeweils für N, CH oder CR¹ stehen,
worin
   R¹ für Fluor, Chlor oder Methyl steht,
   n für eine Zahl 0, 1 oder 2 steht,
mit der Maßgabe, dass maximal zwei der Gruppen A¹, A³ und A⁴ für N stehen,
sowie ihre Salze, Solvate und Solvate der Salze.

Offenbarungsgegenstand sind Erfindung auch Verbindungen der Formel (I), in welcher
der Ring Q für eine Gruppe der Formel 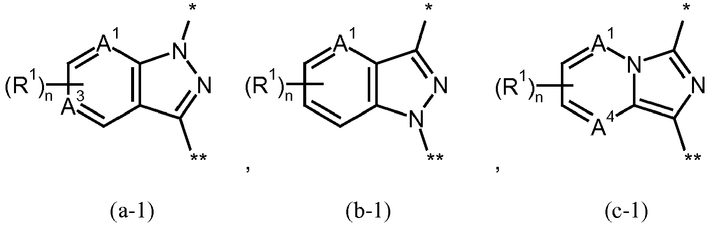 oder 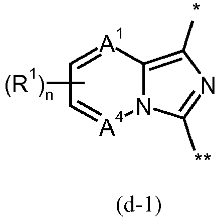 steht, wobei
- *: für die Anknüpfungsstelle an -CH₂-R² steht,
- **: für die Anknüpfungsstelle an das Pyrimidin steht,
- A¹, A³ und A⁴: unabhängig voneinander jeweils für N, CH oder CR¹ stehen,
worin
   R¹ für Fluor, Chlor oder Methyl steht,
   n für eine Zahl 0, 1 oder 2 steht,
mit der Maßgabe, dass maximal zwei der Gruppen A¹, A³ und A⁴ für N stehen,
- R³: für -OR⁴ oder -NR⁵R⁶ steht,
wobei
R⁴ für Wasserstoff oder (C₁-C₆)-Alkyl steht,
worin (C₁-C₆)-Alkyl mit 1 oder 2 Substituenten unabhängig voneinander ausgewählt aus der Gruppe Fluor, Difluormethyl, Trifluormethyl, Difluormethoxy, Trifluormethoxy, -(C=O)ₚ-OR⁹ und -C(=O)ₚ-NR⁹R¹⁰ substituiert sein kann,
worin
   p die Zahl 0 bedeutet,
   R⁹ und R¹⁰ unabhängig voneinander jeweils für Wasserstoff stehen,

R⁵ für Wasserstoff steht,
R⁶ für (C₁-C₆)-Alkyl steht,
worin (C₁-C₆)-Alkyl mit 1 oder 2 Substituenten unabhängig voneinander ausgewählt aus der Gruppe Fluor, Difluormethyl, Trifluormethyl, Difluormethoxy, Trifluormethoxy, -(C=O)ₚ-OR⁹ und -C(=O)ₚ-NR⁹R¹⁰ substituiert ist,
worin
p die Zahl 0 bedeutet,
R⁹ und R¹⁰ unabhängig voneinander jeweils für Wasserstoff stehen,
sowie ihre Salze, Solvate und Solvate der Salze.

Offenbarungsgegenstand sind auch Verbindungen der Formel (I), in welcher
der Ring Q für eine Gruppe der Formel 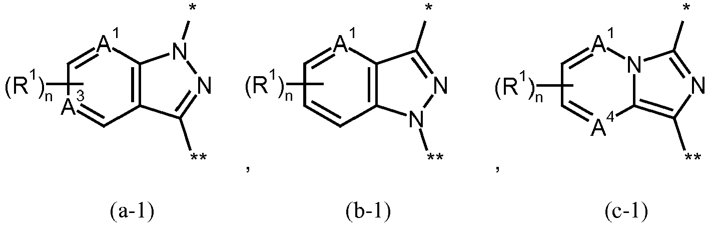 oder 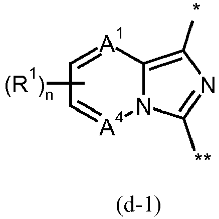 steht, wobei
- *: für die Anknüpfungsstelle an -CH₂-R² steht,
- **: für die Anknüpfungsstelle an das Pyrimidin steht,
- A¹, A³ und A⁴: unabhängig voneinander jeweils für N, CH oder CR¹ stehen,
worin
R¹ für Fluor, Chlor oder Methyl steht,
n für eine Zahl 0, 1 oder 2 steht,
mit der Maßgabe, dass maximal zwei der Gruppen A¹, A³ und A⁴ für N stehen,
sowie ihre Salze, Solvate und Solvate der Salze.

Offenbarungsgegenstand sind auch Verbindungen der Formel (I), in welcher
- L: für eine Gruppe #¹-CR^{7A}R^{7B}-(CR^{8A}R^{8B})ₘ-#² steht,
wobei
#¹ für die Anknüpfstelle an die Carbonylgruppe steht,
#² für die Anknüpfstelle an den Pyrimidinring steht,
m für eine Zahl 0 steht,
R^{7A} für Wasserstoff, Fluor, Methyl oder Hydroxy steht,
R^{7B} für Wasserstoff, Fluor, Trifluormethyl, 2,2,2-Trifluorethyl oder Methyl steht,
der Ring Q für eine Gruppe der Formel 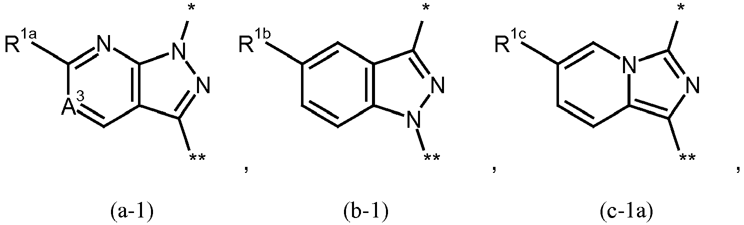 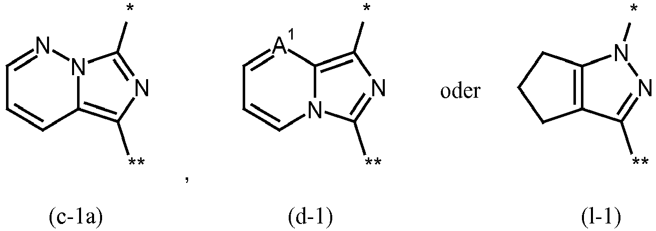 steht, wobei
- *: für die Anknüpfungsstelle an -CH₂-R² steht,
- **: für die Anknüpfungsstelle an das Pyrimidin steht,
- R^{1a}: für Wasserstoff oder Methyl steht,
- R^{1b}: für Wasserstoff oder Fluor steht,
- R^{1c}: für Wasserstoff oder Chlor steht,
- A¹: für N oder CH steht,
- A³: für N, CH oder C-F steht,

R² und R³ jeweils die oben angegebenen Bedeutungen haben,
sowie ihre Salze, Solvate und Solvate der Salze.

Offenbarungsgegenstand sind auch Verbindungen der Formel (I), in welcher
- L: für eine Gruppe #¹-CR^{7A}R^{7B}-(CR^{8A}R^{8B})ₘ-#² steht,
wobei
#¹ für die Anknüpfstelle an die Carbonylgruppe steht,
#² für die Anknüpfstelle an den Pyrimidinring steht,
m für eine Zahl 0 oder 1 steht,
R^{7A} für Wasserstoff, Fluor, Methyl, Ethyl, Hydroxy oder Amino steht,
R^{7B} für Wasserstoff, Fluor, Difluormethyl, Trifluormethyl, Methyl, Ethyl, Methoxycarbonylamino, Cyclopropyl, Cyclobutyl, Cyclopentyl, oder eine Gruppe der Formel -M-R¹³ steht,
worin Methyl und Ethyl mit 1 bis 3 Substituenten unabhängig voneinander ausgewählt aus der Gruppe Fluor, Cyano, Trifluormethyl, Cyclopropyl, Cyclobutyl, Hydroxy, Difluormethoxy, Trifluormethoxy, Methoxy, Ethoxy, Hydroxycarbonyl, Methoxycarbonyl, Ethoxycarbonyl und Amino substituiert sein kann,
und worin
M für eine Bindung steht,
R¹³ für -(C=O)ᵣ-NR¹⁴R¹⁵, Phenyl, Thiazolyl, Triazolyl, Oxadiazolyl, Thiadiazolyl oder Pyrimidinyl steht,
worin
r die Zahl 1 bedeutet,
R¹⁴ und R¹⁵ unabhängig voneinander jeweils für Wasserstoff oder Cyclopropyl stehen, und
worin Phenyl, Thiazolyl, Triazolyl, Oxadiazolyl, Thiadiazolyl und Pyrimidinyl ihrerseits mit 1 oder 2 Substituenten unabhängig voneinander ausgewählt aus der Gruppe Fluor, Difluormethyl, Trifluormethyl, Methyl, Ethyl, iso-Propyl, 2,2,2-Trifluorethyl, 1,1,2,2,2-Pentafluorethyl, Cyclopropyl, Cyclobutyl, Cyclopropylmetyl und Cyclobutylmethyl substituiert sein können, oder
- R^{7A} und R^{7B}: zusammen mit dem Kohlenstoffatom, an das sie gebunden sind, einen Cyclopropyl-, Cyclobutyl-, Cyclopentyl-, Azetidinyl-, Tetrahydrofuranyl-, Pyrrolidinyl- oder Tetrahydropyranyl-Ring bilden,
worin der Cyclopropyl-, Cyclobutyl-, Cyclopentyl-, Azetidinyl-, Tetrahydrofuranyl-, Pyrrolidinyl- und Tetrahydropyranyl-Ring mit 1 oder 2 Substituenten unabhängig voneinander ausgewählt aus der Gruppe Fluor und Methyl substituiert sein können,
- R^{8A}: für Wasserstoff, Fluor, Methyl oder Hydroxy steht,
- R^{8B}: für Wasserstoff, Fluor, Methyl oder Trifluormethyl steht,
der Ring Q für eine Gruppe der Formel 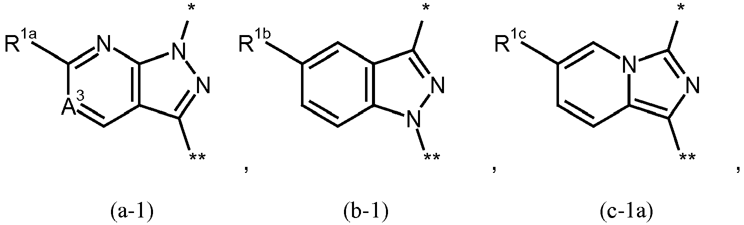 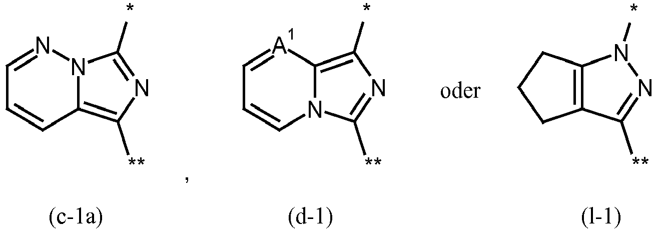 steht, wobei
- *: für die Anknüpfungsstelle an -CH₂-R² steht,
- **: für die Anknüpfungsstelle an das Pyrimidin steht,
- R^{1a}: für Wasserstoff oder Methyl steht,
- R^{1b}: für Wasserstoff oder Fluor steht,
- R^{1c}: für Wasserstoff oder Chlor steht,
- A¹: für N oder CH steht,
- A³: für N, CH oder C-F steht,
R² und R³ jeweils die oben angegebenen Bedeutungen haben,
sowie ihre Salze, Solvate und Solvate der Salze.

Bevorzugt sind im Rahmen der vorliegenden Erfindung auch folgende Verbindungen der Formel (I):
2-[1-(2-Fluorbenzyl)-1H-pyrazolo[3,4-b]pyridin-3-yl]-4-methoxy-5,5-dimethyl-5,7-dihydro-6H-pyrrolo[2,3-d]pyrimidin-6-on,
2-[5-Fluor-1-(2-fluorbenzyl)-1H-pyrazolo[3,4-b]pyridin-3-yl]-4-[(2-hydroxyethyl)amino]-5,5-dimethyl-5,7-dihydro-6H-pyrrolo[2,3-d]pyrimidin-6-on,
4-[(2-Amino-2-methylpropyl)amino]-2-[5-fluor-1-(2-fluorbenzyl)-1H-pyrazolo[3,4-b]pyridin-3-yl]-5,5-dimethyl-5,7-dihydro-6H-pyrrolo[2,3-d]pyrimidin-6-on,
2-[5-Fluor-1-(2-fluorbenzyl)-1H-pyrazolo[3,4-b]pyridin-3-yl]-4-[(2-hydroxy-2-methylpropyl)amino]-5,5-dimethyl-5,7-dihydro-6H-pyrrolo[2,3-d]pyrimidin-6-on,
2-[5-Fluor-1-(2-fluorbenzyl)-1H-pyrazolo[3,4-b]pyridin-3-yl]-5,5-dimethyl-4-[(3,3,3-trifluorpropyl)amino]-5,7-dihydro-6H-pyrrolo[2,3-d]pyrimidin-6-on,
4-[(2,2-Difluorethyl)amino]-2-[5-fluor-1-(2-fluorbenzyl)-1H-pyrazolo[3,4-b]pyridin-3-yl]-5,5-dimethyl-5,7-dihydro-6H-pyrrolo[2,3-d]pyrimidin-6-on,
4-(3-Fluorazetidin-1-yl)-2-[5-fluor-1-(2-fluorbenzyl)-1H-pyrazolo[3,4-b]pyridin-3-yl]-5,5-dimethyl-5,7-dihydro-6H-pyrrolo[2,3-d]pyrimidin-6-on,
4-[(Cyclopropylmethyl)amino]-2-[5-fluor-1-(2-fluorbenzyl)-1H-pyrazolo[3,4-b]pyridin-3-yl]-5,5-dimethyl-5,7-dihydro-6H-pyrrolo[2,3-d]pyrimidin-6-on,
2-[5-Fluor-1-(2-fluorbenzyl)-1H-pyrazolo[3,4-b]pyridin-3-yl]-5,5-dimethyl-4-[(2,2,2-trifluorethyl)amino]-5,7-dihydro-6H-pyrrolo[2,3-d]pyrimidin-6-on,
2-{5-Fluor-1-[(3-fluorpyridin-2-yl)methyl]-1H-pyrazolo[3,4-b]pyridin-3-yl}-5,5-dimethyl-4-[(3,3,3-trifluorpropyl)amino]-5,7-dihydro-6H-pyrrolo[2,3-d]pyrimidin-6-on,
2-{5-Fluor-1-[(3-fluorpyridin-2-yl)methyl]-1H-pyrazolo[3,4-b]pyridin-3-yl}-5,5-dimethyl-4-[(2,2,2-trifluorethyl)amino]-5,7-dihydro-6H-pyrrolo[2,3-d]pyrimidin-6-on,
4-[(Cyclopropylmethyl)amino]-2-{5-fluor-1-[(3-fluorpyridin-2-yl)methyl]-1H-pyrazolo[3,4-b]pyridin-3-yl}-5,5-dimethyl-5,7-dihydro-6H-pyrrolo[2,3-d]pyrimidin-6-on,
4-(3-Ethyl-2-oxoimidazolidin-1-yl)-2-[1-(2-fluorbenzyl)-1H-pyrazolo[3,4-b]pyridin-3-yl]-5,5-dimethyl-5,7-dihydro-6H-pyrrolo[2,3-d]pyrimidin-6-on,
2-[1-(2-Fluorbenzyl)-1H-pyrazolo[3,4-b]pyridin-3-yl]-4-(4-hydroxy-1H-pyrazol-1-yl)-5,5-dimethyl-5,7-dihydro-6H-pyrrolo[2,3-d]pyrimidin-6-on,
2-[1-(2-Fluorbenzyl)-1H-pyrazolo[3,4-b]pyridin-3-yl]-5,5-dimethyl-4-(1H-pyrazol-4-yloxy)-5,7-dihydro-6H-pyrrolo[2,3-d]pyrimidin-6-on,
2-[1-(2-Fluorbenzyl)-1H-pyrazolo[3,4-b]pyridin-3-yl]-4-[3-(1-hydroxyethyl)-1H-pyrazol-1-yl]-5,5-dimethyl-5,7-dihydro-6H-pyrrolo[2,3-d]pyrimidin-6-on,
2-[5-Fluor-3-(2-fluorbenzyl)-1H-indazol-1-yl]-4-hydroxy-5,5-dimethyl-5,7-dihydro-6H-pyrrolo[2,3-d]pyrimidin-6-on,
4-[(Cyclopropylmethyl)amino]-2-[5-fluor-3-(2-fluorbenzyl)-1H-indazol-1-yl]-5,5-dimethyl-5,7-dihydro-6H-pyrrolo[2,3-d]pyrimidin-6-on,
2-[1-(2-Fluorbenzyl)-1H-pyrazolo[3,4-b]pyridin-3-yl]-4-[(2-hydroxypropyl)amino]-5,5-dimethyl-5,7-dihydro-6H-pyrrolo[2,3-d]pyrimidin-6-on,
N-(1-{2-[1-(2-Fluorbenzyl)-1H-pyrazolo[3,4-b]pyridin-3-yl]-5,5-dimethyl-6-oxo-6,7-dihydro-5H-pyrrolo[2,3-d]pyrimidin-4-yl}pyrrolidin-3-yl)acetamid,
2-[1-(2-Fluorbenzyl)-1H-pyrazolo[3,4-b]pyridin-3-yl]-4-[(trans-4-hydroxycyclohexyl)amino]-5,5-dimethyl-5,7-dihydro-6H-pyrrolo[2,3-d]pyrimidin-6-on,
2-[5-Fluor-1-(2-fluorbenzyl)-1H-pyrazolo[3,4-b]pyridin-3-yl]-5,5-dimethyl-4-[(3,3,3-trifluor-2-hydroxypropyl)amino]-5,7-dihydro-6H-pyrrolo[2,3-d]pyrimidin-6-on (Enantiomer 1),
2-[5-Fluor-1-(2-fluorbenzyl)-1H-pyrazolo[3,4-b]pyridin-3-yl]-5,5-dimethyl-4-[(3,3,3-trifluor-2-hydroxypropyl)amino]-5,7-dihydro-6H-pyrrolo[2,3-d]pyrimidin-6-on (Enantiomer 2),
4-{[(2,2-Difluorcyclopropyl)methyl]amino}-2-[5-fluor-1-(2-fluorbenzyl)-1H-pyrazolo[3,4-b]pyridin-3-yl]-5,5-dimethyl-5,7-dihydro-6H-pyrrolo[2,3-d]pyrimidin-6-on,
2-[5-Fluor-1-(2-fluorbenzyl)-1H-pyrazolo[3,4-b]pyridin-3-yl]-4-{[(1-hydroxy-cyclopropyl)methyl]amino}-5,5-dimethyl-5,7-dihydro-6H-pyrrolo[2,3-d]pyrimidin-6-on,
Ethyl-2-[1-(2-fluorbenzyl)-1H-pyrazolo[3,4-b]pyridin-3-yl]-4-hydroxy-5-methyl-6-oxo-6,7-dihydro-5H-pyrrolo[2,3-d]pyrimidin-5-carboxylat,
2-[5-Fluor-1-(2-fluorbenzyl)-1H-pyrazolo[3,4-b]pyridin-3-yl]-4-hydroxy-5,5-dimethyl-5,7-dihydro-6H-pyrrolo[2,3-d]pyrimidin-6-on,
4-(3-Aminopyrrolidin-1-yl)-2-[1-(2-fluorbenzyl)-1H-pyrazolo[3,4-b]pyridin-3-yl]-5,5-dimethyl-5,7-dihydro-6H-pyrrolo[2,3-d]pyrimidin-6-on,
2-[1-(2-Fluorbenzyl)-6-methyl-1H-pyrazolo[3,4-d]pyrimidin-3-yl]-5,5-dimethyl-4-[(3,3,3-trifluoropropyl)amino]-5,7-dihydro-6H-pyrrolo[2,3-d]pyrimidin-6-on,
2-[1-(2-Fluorbenzyl)-1H-pyrazolo[3,4-b]pyridin-3-yl]-4-hydroxy-5,5-dimethyl-5,7-dihydro-6H-pyrrolo[2,3-d]pyrimidin-6-on,
2-[1-(2,3-Difluorbenzyl)-5-fluor-1H-pyrazolo[3,4-b]pyridin-3-yl]-4-hydroxy-5,5-dimethyl-5,7-dihydro-6H-pyrrolo[2,3-d]pyrimidin-6-on,
2-[1-(2-Fluorbenzyl)-1H-pyrazolo[3,4-b]pyridin-3-yl]-4-(2-hydroxyethoxy)-5,5-dimethyl-5,7-dihydro-6H-pyrrolo[2,3-d]pyrimidin-6-on,
sowie ihre Salze, Solvate und Solvate der Salze.

Offenbarungsgegenstand sind Verbindungen gemäß Formel (I) 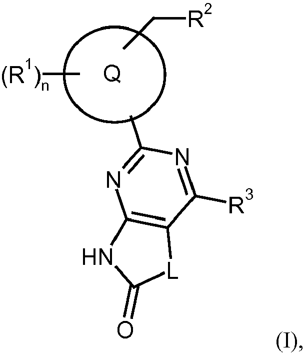 in welcher
- L: für eine Gruppe #¹-CR^{7A}R^{7B}-(CR^{8A}R^{8B})ₘ-#² steht,
wobei
- #¹: für die Anknüpfstelle an die Carbonylgruppe steht,
- #²: für die Anknüpfstelle an den Pyrimidinring steht,
- m: für eine Zahl 0, 1 oder 2 steht,
- R^{7A}: für Wasserstoff, Fluor, (C₁-C₄)-Alkyl, Hydroxy oder Amino steht,
worin (C₁-C₄)-Alkyl mit 1 bis 3 Substituenten unabhängig voneinander ausgewählt aus der Gruppe Fluor, Trifluormethyl, Hydroxy, Hydroxycarbonyl, (C₁-C₄)-Alkoxycarbonyl und Amino substituiert sein kann,
- R^{7B}: für Wasserstoff, Fluor, Difluormethyl, Trifluormethyl, (C₁-C₆)-Alkyl, (C₁-C₄)-Alkoxycarbonylamino, Cyano, (C₃-C₇)-Cycloalkyl, Difluormethoxy, Trifluormethoxy, Phenyl oder eine Gruppe der Formel -M-R¹³ steht,
worin (C₁-C₆)-Alkyl mit 1 bis 3 Substituenten unabhängig voneinander ausgewählt aus der Gruppe Fluor, Cyano, Trifluormethyl, (C₃-C₇)-Cycloalkyl, Hydroxy, Difluormethoxy, Trifluormethoxy, (C₁-C₄)-Alkoxy, Hydroxycarbonyl, (C₁-C₄)- Alkoxycarbonyl und Amino substituiert sein kann,
und worin
M für eine Bindung oder (C₁-C₄)-Alkandiyl steht,
- R¹³: für -(C=O)ᵣ-OR¹⁴, -(C=O)ᵣ-NR¹⁴R¹⁵, -C(=S)-NR¹⁴R¹⁵, -NR¹⁴-(C=O)-R¹⁷, -NR¹⁴-(C=O)-NR¹⁵R¹⁶, -NR¹⁴-SO₂-NR¹⁵R¹⁶, -NR¹⁴-SO₂-R¹⁷, -S(O)ₛ-R¹⁷, -SO₂-NR¹⁴R¹⁵, 4- bis 7-gliedriges Heterocyclyl, Phenyl oder 5- oder 6-gliedriges Heteroaryl steht,
worin
r die Zahl 0 oder 1 bedeutet,
s die Zahl 0, 1 oder 2 bedeutet,
R¹⁴, R¹⁵ und R¹⁶ unabhängig voneinander jeweils für Wasserstoff, (C₁-C₆)-Alkyl, (C₃-C₈)-Cycloalkyl, 4- bis 7-gliedriges Heterocyclyl, Phenyl oder 5- oder 6-gliedriges Heteroaryl stehen, oder
R¹⁴ und R¹⁵ bilden zusammen mit dem/den Atom/-en, an die sie jeweils gebunden sind, einen 4- bis 7-gliedrigen Heterocyclus,
worin der 4- bis 7-gliedrigen Heterocyclus seinerseits mit 1 oder 2 Substituenten unabhängig voneinander ausgewählt aus der Gruppe Cyano, Trifluormethyl, (C₁-C₆)-Alkyl, Hydroxy, Oxo, (C₁-C₆)-Alkoxy, Trifluormethoxy, (C₁-C₆)-Alkoxycarbonyl, Amino, Mono-(C₁-C₆)-alkylamino und Di-(C₁-C₆)-alkylamino substituiert sein kann, oder
R¹⁵ und R¹⁶ bilden zusammen mit dem/den Atom/-en, an die sie jeweils gebunden sind, einen 4- bis 7-gliedrigen Heterocyclus,
worin der 4- bis 7-gliedrigen Heterocyclus seinerseits mit 1 oder 2 Substituenten unabhängig voneinander ausgewählt aus der Gruppe Cyano, Trifluormethyl, (C₁-C₆)-Alkyl, Hydroxy, Oxo, (C₁-C₆)-Alkoxy, Trifluormethoxy, (C₁-C₆)-Alkoxycarbonyl, Amino, Mono-(C₁-C₆)-alkylamino und Di-(C₁-C₆)-alkylamino substituiert sein kann,
R¹⁷ für (C₁-C₆)-Alkyl oder (C₃-C₇)-Cycloalkyl steht,
oder
R¹⁴ und R¹⁷ bilden zusammen mit dem/den Atom/-en, an die sie jeweils gebunden sind, einen 4- bis 7-gliedrigen Heterocyclus,
worin der 4- bis 7-gliedrigen Heterocyclus seinerseits mit 1 oder 2 Substituenten unabhängig voneinander ausgewählt aus der Gruppe Cyano, Trifluormethyl, (C₁-C₆)-Alkyl, Hydroxy, Oxo, (C₁-C₆)-Alkoxy, Trifluormethoxy, (C₁-C₆)-Alkoxycarbonyl, Amino, Mono-(C₁-C₆)-alkylamino und Di-(C₁-C₆)-alkylamino substituiert sein kann, und
worin 4- bis 7-gliedriges Heterocyclyl, Phenyl und 5- oder 6-gliedriges Heteroaryl ihrerseits mit 1 bis 3 Substituenten unabhängig voneinander ausgewählt aus der Gruppe Halogen, Cyano, Difluormethyl, Trifluormethyl, (C₁-C₆)-Alkyl, (C₃-C₇)-Cycloalkyl, Hydroxy, Oxo, Thiooxo und (C₁-C₄)-Alkoxy substituiert sein können,
und
worin die zuvor genannten (C₁-C₄)-Alkyl-, (C₁-C₆)-Alkyl-, (C₃-C₈)-Cycloalkyl-, (C₃-C₇)-Cycloalkyl- und 4- bis 7-gliedriges Heterocyclyl-Gruppen sofern nicht anders angegeben jeweils unabhängig voneinander weiterhin mit 1 bis 3 Substituenten unabhängig voneinander ausgewählt aus der Gruppe Fluor, Difluormethyl, Trifluormethyl, (C₁-C₄)-Alkyl, (C₃-C₇)-Cycloalkyl, Hydroxy, Difluormethoxy, Trifluormethoxy, (C₁-C₄)-Alkoxy, Hydroxycarbonyl, (C₁-C₄)-Alkoxycarbonyl, Amino, Phenyl, 4- bis 7-gliedriges Heterocyclyl und 5- oder 6-gliedriges Heteroaryl substituiert sein können,
oder
- R^{7A} und R^{7B}: zusammen mit dem Kohlenstoffatom, an das sie gebunden sind, eine (C₂-C₄)-Alkenyl-Gruppe, eine Oxo-Gruppe, einen 3- bis 6-gliedrigen Carbocyclus oder einen 4- bis 7-gliedrigen Heterocyclus bilden,
worin der 3- bis 6-gliedrigen Carbocyclus und der 4- bis 7-gliedrigen Heterocyclus mit 1 oder 2 Substituenten unabhängig voneinander ausgewählt aus der Gruppe Fluor und (C₁-C₄)-Alkyl substituiert sein können,
- R^{8A}: für Wasserstoff, Fluor, (C₁-C₄)-Alkyl oder Hydroxy steht,
- R^{8B}: für Wasserstoff, Fluor, (C₁-C₄)-Alkyl oder Trifluormethyl steht,

der Ring Q für 8- oder 9-gliedriges Heteroaryl steht,
- R³: für -OR⁴ oder -NR⁵R⁶ steht,
wobei
R⁴ für Wasserstoff, (C₁-C₆)-Alkyl, (C₃-C₇)-Cycloalkyl, 4- bis 7-gliedriges Heterocyclyl, Phenyl oder 5- oder 6-gliedriges Heteroaryl steht,
worin (C₁-C₆)-Alkyl, (C₃-C₇)-Cycloalkyl, 4- bis 7-gliedriges Heterocyclyl, Phenyl und 5- oder 6-gliedriges Heteroaryl mit 1 bis 3 Substituenten unabhängig voneinander ausgewählt aus der Gruppe Fluor, Difluormethyl, Trifluormethyl, (C₁-C₆)-Alkyl, (C₃-C₆)-Cycloalkyl, Difluormethoxy, Trifluormethoxy, Oxo, -(C=O)ₚ-OR⁹, -C(=O)ₚ-NR⁹R¹⁰, -NR⁹-(C=O)-R¹⁰, -NR⁹-(C=O)-OR¹⁰, -NR⁹-(C=O)-NR¹⁰R¹¹, -NR⁹-SO₂-R¹⁰, -S(O)_{q}-R¹² und -SO₂-NR⁹R¹⁰ substituiert sein können,
worin
   p die Zahl 0 oder 1 bedeutet,
   q die Zahl 0, 1 oder 2 bedeutet,
   R⁹, R¹⁰ und R¹¹ unabhängig voneinander jeweils für Wasserstoff, (C₁-C₆)-Alkyl oder (C₃-C₈)-Cycloalkyl stehen,
   worin (C₁-C₆)-Alkyl seinerseits mit 1 oder 2 Substituenten unabhängig voneinander ausgewählt aus der Gruppe Fluor, Difluormethyl, Trifluormethyl, Hydroxy, (C₁-C₆)-Alkoxy, Difluormethoxy, Trifluormethoxy, (C₁-C₆)-Alkoxycarbonyl, Amino, Mono-(C₁-C₆)-alkylamino, Di-(C₁-C₆)-alkylamino und 4- bis 7-gliedriges Heterocyclyl substituiert sein kann, oder
   R⁹ und R¹⁰ bilden zusammen mit dem/den Atom/-en, an die sie jeweils gebunden sind, einen 4- bis 7-gliedrigen Heterocyclus, oder worin der 4- bis 7-gliedrigen Heterocyclus seinerseits mit 1 oder 2 Substituenten unabhängig voneinander ausgewählt aus der Gruppe Fluor, Trifluormethyl, (C₁-C₆)-Alkyl, Hydroxy, Oxo, (C₁-C₆)-Alkoxy, Trifluormethoxy, (C₁-C₆)-Alkoxycarbonyl, Amino, Mono-(C₁-C₆)-alkylamino und Di-(C₁-C₆)-alkylamino substituiert sein kann,
   R¹⁰ und R¹¹ bilden zusammen mit dem/den Atom/-en, an die sie jeweils gebunden sind, einen 4- bis 7-gliedrigen Heterocyclus, worin der 4- bis 7-gliedrigen Heterocyclus seinerseits mit 1 oder 2 Substituenten unabhängig voneinander ausgewählt aus der Gruppe Fluor, Trifluormethyl, (C₁-C₆)-Alkyl, Hydroxy, Oxo, (C₁-C₆)-Alkoxy, Trifluormethoxy, (C₁-C₆)-Alkoxycarbonyl, Amino, Mono-(C₁-C₆)-alkylamino und Di-(C₁-C₆)-alkylamino substituiert sein kann,
   und worin
   R¹² für (C₁-C₆)-Alkyl oder (C₃-C₇)-Cycloalkyl steht,
und
worin die zuvor genannten (C₁-C₄)-Alkyl-, (C₁-C₆)-Alkyl-, (C₃-C₈)-Cycloalkyl-, (C₃-C₇)-Cycloalkyl- und 4- bis 7-gliedriges Heterocyclyl-Gruppen sofern nicht anders angegeben jeweils unabhängig voneinander weiterhin mit 1 bis 3 Substituenten unabhängig voneinander ausgewählt aus der Gruppe Fluor, Difluormethyl, Trifluormethyl, (C₁-C₄)-Alkyl, (C₃-C₇)-Cycloalkyl, Hydroxy, Difluormethoxy, Trifluormethoxy, (C₁-C₄)-Alkoxy, Hydroxycarbonyl, (C₁-C₄)-Alkoxycarbonyl, Amino, Phenyl, 4- bis 7-gliedriges Heterocyclyl und 5- oder 6-gliedriges Heteroaryl substituiert sein können,
- R⁵: für Wasserstoff oder (C₁-C₄)-Alkyl steht,
- R⁶: für (C₁-C₆)-Alkyl, (C₃-C₇)-Cycloalkyl, 4- bis 7-gliedriges Heterocyclyl, Phenyl oder 5- oder 6-gliedriges Heteroaryl steht,
worin (C₁-C₆)-Alkyl (C₃-C₇)-Cycloalkyl, 4- bis 7-gliedriges Heterocyclyl, Phenyl und 5- oder 6-gliedriges Heteroaryl mit 1 bis 3 Substituenten unabhängig voneinander ausgewählt aus der Gruppe Fluor, Difluormethyl, Trifluormethyl, (C₁-C₆)-Alkyl, (C₃-C₇)-Cycloalkyl, Difluormethoxy, Trifluormethoxy, Oxo,-(C=O)ₚ-OR⁹, -(C=O)ₚ-NR⁹R¹⁰, -NR⁹-(C=O)-R¹⁰, -NR⁹-(C=O)-OR¹⁰, -NR⁹-(C=O)-NR¹⁰R¹¹, -NR⁹-SO₂-R¹⁰, -S(O)_{q}-R¹², -SO₂-NR⁹R¹⁰, Phenyl, 4-bis 7-gliedriges Heterocyclyl und 5- oder 6-gliedriges Heteroaryl substituiert sein können,
worin
p die Zahl 0 oder 1 bedeutet,
q die Zahl 0, 1 oder 2 bedeutet,
R⁹, R¹⁰ und R¹¹ unabhängig voneinander jeweils für Wasserstoff, (C₁-C₆)-Alkyl oder (C₃-C₈)-Cycloalkyl stehen,
worin (C₁-C₆)-Alkyl seinerseits mit 1 bis 3 Substituenten unabhängig voneinander ausgewählt aus der Gruppe Fluor, Difluormethyl, Trifluormethyl, (C₃-C₇)-Cycloalkyl, Difluormethoxy, Trifluormethoxy und (C₁-C₄)-Alkoxy substituiert sein kann,
oder
R⁹ und R¹⁰ bilden zusammen mit dem/den Atom/-en, an die sie jeweils gebunden sind, einen 4- bis 7-gliedrigen Heterocyclus,
worin der 4- bis 7-gliedrigen Heterocyclus seinerseits mit 1 oder 2 Substituenten unabhängig voneinander ausgewählt aus der Gruppe Fluor, Cyano, Trifluormethyl, (C₁-C₆)-Alkyl, Hydroxy, Oxo, (C₁-C₆)-Alkoxy, Trifluormethoxy, (C₁-C₆)-Alkoxycarbonyl, Amino, Mono-(C₁-C₆)-alkylamino und Di-(C₁-C₆)-alkylamino substituiert sein kann,
oder
R¹⁰ und R¹¹ bilden zusammen mit dem/den Atom/-en, an die sie jeweils gebunden sind, einen 4- bis 7-gliedrigen Heterocyclus,
worin der 4- bis 7-gliedrigen Heterocyclus seinerseits mit 1 oder 2 Substituenten unabhängig voneinander ausgewählt aus der Gruppe Cyano, Trifluormethyl, (C₁-C₆)-Alkyl, Hydroxy, Oxo, (C₁-C₆)-Alkoxy, Trifluormethoxy, (C₁-C₆)-Alkoxycarbonyl, Amino, Mono-(C₁-C₆)-alkylamino und Di-(C₁-C₆)-alkylamino substituiert sein kann,
worin
R¹² für (C₁-C₆)-Alkyl oder (C₃-C₇)-Cycloalkyl steht,
und
worin Phenyl, 4- bis 7-gliedriges Heterocyclyl und 5- oder 6-gliedriges Heteroaryl ihrerseits mit 1 bis Substituenten unabhängig voneinander ausgewählt aus der Gruppe Halogen, Cyano, Difluormethyl, Trifluormethyl, (C₁-C₄)-Alkyl, (C₃-C₇)-Cycloalkyl, Hydroxy, Oxo, Difluormethoxy, Trifluormethoxy und (C₁-C₄)-Alkoxy substituiert sein können, oder
- R⁵ und R⁶: bilden zusammen mit dem Stickstoffatom, an das sie gebunden sind, einen 4- bis 7-gliedrigen Heterocyclus oder ein 5- oder 6-gliedriges Heteroaryl,
worin der 4- bis 7-gliedrigen Heterocyclus und das 5- oder 6-gliedriges Heteroaryl mit 1 bis 3 Substituenten unabhängig voneinander ausgewählt aus der Gruppe Fluor, Cyano, Difluormethyl, Trifluormethyl, (C₁-C₆)-Alkyl, (C₃-C₇)-Cycloalkyl, Hydroxy, Oxo, (C₁-C₆)-Alkoxy, Difluormethoxy, Trifluormethoxy, (C₁-C₆)-Alkoxycarbonyl, (C₁-C₆)-Alkylcarbonylamino, Amino, Mono-(C₁-C₆)-alkylamino, Di-(C₁-C₆)-alkylamino und 4- bis 7-gliedriges Heterocyclyl substituiert sein können,
und
worin die zuvor genannten (C₁-C₄)-Alkyl-, (C₁-C₆)-Alkyl-, (C₃-C₈)-Cycloalkyl-, (C₃-C₇)-Cycloalkyl- und 4- bis 7-gliedriges Heterocyclyl-Gruppen sofern nicht anders angegeben jeweils unabhängig voneinander weiterhin mit 1 bis 3 Substituenten unabhängig voneinander ausgewählt aus der Gruppe Fluor, Difluormethyl, Trifluormethyl, (C₁-C₄)-Alkyl, (C₃-C₇)-Cycloalkyl, Hydroxy, Difluormethoxy, Trifluormethoxy, (C₁-C₄)-Alkoxy, Hydroxycarbonyl, (C₁-C₄)-Alkoxycarbonyl, Amino, Phenyl, 4- bis 7-gliedriges Heterocyclyl und 5- oder 6-gliedriges Heteroaryl substituiert sein können,
- R¹: für Fluor, Chlor, Cyano, Difluormethyl, Trifluormethyl, (C₁-C₄)-Alkyl, (C₃-C₇)-Cycloalkyl oder (C₁-C₄)-Alkoxy steht,
- n: für eine Zahl 0, 1 oder 2 steht,
- R²: für Trifluormethyl, (C₁-C₆)-Alkyl, (C₃-C₈)-Cycloalkyl, Phenyl oder 5- oder 6-gliedriges Heteroaryl steht,
wobei (C₁-C₆)-Alkyl mit einem Substituenten ausgewählt aus der Gruppe Difluormethyl und Trifluormethyl substituiert ist,
wobei (C₁-C₆)-Alkyl mit 1 bis 3 Substituenten Fluor substituiert sein kann,
wobei (C₃-C₈)-Cycloalkyl mit 1 oder 2 Substituenten unabhängig voneinander ausgewählt aus der Gruppe Fluor, Methyl und Methoxy substituiert sein kann,
wobei Phenyl mit 1 bis 3 Substituenten Fluor substituiert ist,
wobei Phenyl mit 1 oder 2 Substituenten unabhängig voneinander ausgewählt aus der Gruppe Methyl und Methoxy substituiert sein kann,
und
wobei 5- und 6-gliedriges Heteroaryl mit 1 oder 2 Substituenten unabhängig voneinander ausgewählt aus der Gruppe Fluor, Trifluormethyl und Methyl substituiert sein können,
sowie ihre *N*-Oxide, Salze, Solvate, Salze der *N*-Oxide und Solvate der *N*-Oxide und Salze.

Offenbarungsgegenstand sind Verbindungen gemäß Formel (I), in welcher
- L: für eine Gruppe #¹-CR^{7A}R^{7B}-(CR^{8A}R^{8B})ₘ-#² steht,
wobei
#¹ für die Anknüpfstelle an die Carbonylgruppe steht,
#² für die Anknüpfstelle an den Pyrimidinring steht,
m für eine Zahl 0 oder 1 steht,
R^{7A} für Wasserstoff, Fluor, Methyl, Ethyl, Hydroxy oder Amino steht,
R^{7B} für Wasserstoff, Fluor, Difluormethyl, Trifluormethyl, (C₁-C₄)-Alkyl, Methoxycarbonylamino, Cyano, Cyclopropyl, Cyclobutyl, Cyclopentyl, Phenyl oder eine Gruppe der Formel -M-R¹³ steht, worin (C₁-C₄)-Alkyl mit 1 bis 3 Substituenten unabhängig voneinander ausgewählt aus der Gruppe Fluor, Cyano, Trifluormethyl, Cyclopropyl, Cyclobutyl, Cyclopentyl, Hydroxy, Difluormethoxy, Trifluormethoxy, Methoxy, Ethoxy, Hydroxycarbonyl, Methoxycarbonyl, Ethoxycarbonyl und Amino substituiert sein kann,
und worin
M für eine Bindung oder Methylen steht,
R¹³ für -(C=O)ᵣ-NR¹⁴R¹⁵, -C(=S)-NR¹⁴R¹⁵, Oxadiazolonyl, Oxadiazolthionyl, Phenyl, Oxazolyl, Thiazolyl, Pyrazolyl, Triazolyl, Oxadiazolyl, Thiadiazolyl, Pyridyl, Pyrimidinyl oder Pyrazinyl steht,
worin
r die Zahl 0 oder 1 bedeutet,
R¹⁴ und R¹⁵ unabhängig voneinander jeweils für Wasserstoff, Methyl, Ethyl, iso-Propyl, Cyclopropyl, Cyclobutyl, Cyclopentyl, Oxetanyl, Azetidinyl, Tetrahydrofuranyl, Pyrrolidinyl, Tetrahydropyranyl, Piperidinyl, Piperazinyl, Morpholinyl, Phenyl, Pyrazolyl oder Pyridyl stehen,
worin Methyl, Ethyl und iso-Propyl weiterhin mit 1 oder 2 Substituenten unabhängig voneinander ausgewählt aus der Gruppe Fluor, Difluormethyl, Trifluormethyl, Cyclopropyl, Cyclobutyl, Cyclopentyl, Hydroxy, Difluormethoxy, Trifluormethoxy, Methoxy, Ethoxy, Hydroxycarbonyl, Methoxycarbonyl, Ethoxycarbonyl und Amino substituiert sein können, und
worin Oxadiazolonyl, Oxadiazolthionyl, Phenyl, Oxazolyl, Thiazolyl, Pyrazolyl, Triazolyl, Oxadiazolyl, Thiadiazolyl, Pyridyl, Pyrimidinyl und Pyrazinyl ihrerseits mit 1 oder 2 Substituenten unabhängig voneinander ausgewählt aus der Gruppe Fluor, Chlor, Cyano, Difluormethyl, Trifluormethyl, Methyl, Ethyl, iso-Propyl, 2,2,2-Trifluorethyl, 1,1,2,2,2-Pentafluorethyl, Cyclopropyl, Cyclobutyl, Cyclopropylmetyl, Cyclobutylmethyl, Hydroxy, Methoxy und Ethoxy substituiert sein können, oder
R^{7A} und R^{7B} zusammen mit dem Kohlenstoffatom, an das sie gebunden sind, einen Cyclopropyl-, Cyclobutyl-, Cyclopentyl-, Azetidinyl-, Tetrahydrofuranyl-, Pyrrolidinyl- oder Tetrahydropyranyl-Ring bilden,
worin der Cyclopropyl-, Cyclobutyl-, Cyclopentyl-, Azetidinyl-, Tetrahydrofuranyl-, Pyrrolidinyl- und Tetrahydropyranyl-Ring mit 1 oder 2 Substituenten unabhängig voneinander ausgewählt aus der Gruppe Fluor und Methyl substituiert sein können,
R^{8A} für Wasserstoff, Fluor, Methyl, Ethyl oder Hydroxy steht,
R^{8B} für Wasserstoff, Fluor, Methyl, Ethyl oder Trifluormethyl steht,
der Ring Q für eine Gruppe der Formel 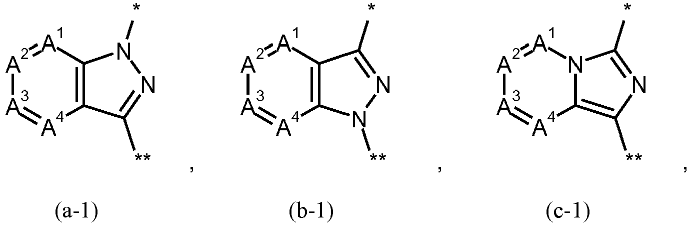 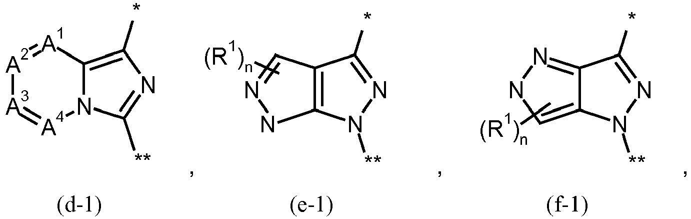 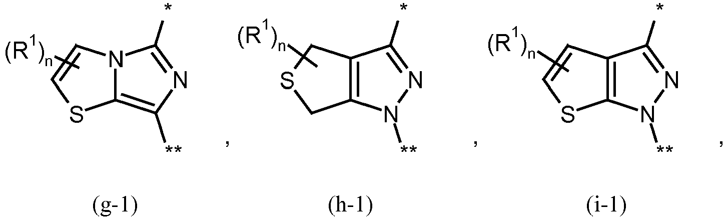 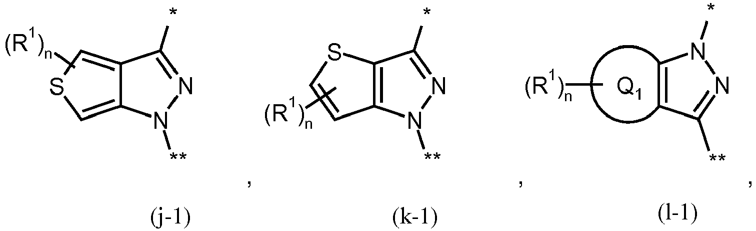 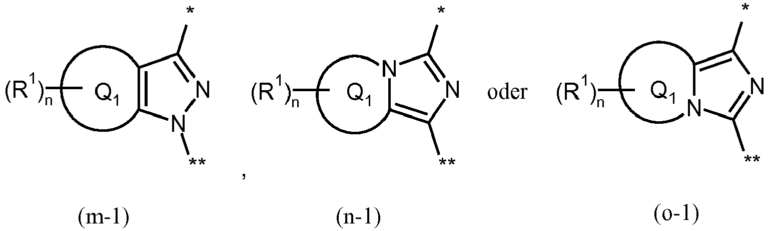 steht, wobei
- *: für die Anknüpfungsstelle an -CH₂-R² steht,
- **: für die Anknüpfungsstelle an das Pyrimidin steht,
- der Ring Q₁: zusammen mit den Atomen, an die er gebunden ist, einen 5- bis 7-gliedrigen gesättigten oder teilweise ungesättigten Carbocyclus oder einen 5- bis 7-gliedrigen gesättigten oder teilweise ungesättigten Heterocyclus bildet,
- R¹: für Fluor, Chlor oder Methyl steht,
- n: für eine Zahl 0, 1 oder 2 steht,
A¹, A², A³ und A⁴ unabhängig voneinander jeweils für N, CH oder CR¹ stehen, mit der Maßgabe, dass maximal zwei der Gruppen A¹, A², A³ und A⁴ für N stehen,
- R³: für -OR⁴ oder -NR⁵R⁶ steht,
wobei
R⁴ für (C₁-C₆)-Alkyl, Cyclopropyl, Cyclobutyl, Cyclopentyl, Oxetanyl, Azetidinyl, Tetrahydrofuranyl, Pyrrolidinyl, Tetrahydropyranyl, Piperidinyl, Piperazinyl, Morpholinyl, Phenyl, Pyrazolyl, Oxazolyl, Thiazolyl, Triazolyl, Oxadiazolyl, Thiadiazolyl, Pyridyl oder Pyrimidinyl steht, worin (C₁-C₆)-Alkyl mit 1 bis 3 Substituenten unabhängig voneinander ausgewählt aus der Gruppe Fluor, Difluormethyl, Trifluormethyl, Cyclopropyl, Cyclobutyl, Cyclopentyl, Difluormethoxy, Trifluormethoxy, -(C=O)ₚ-OR⁹, -(C=O)ₚ-NR⁹R¹⁰, und -NR⁹-(C=O)-R¹⁰ substituiert sein kann,
und
worin Cyclopropyl, Cyclobutyl, Cyclopentyl, Oxetanyl, Azetidinyl, Tetrahydrofuranyl, Pyrrolidinyl, Tetrahydropyranyl, Piperidinyl, Piperazinyl, Morpholinyl, Phenyl, Pyrazolyl, Oxazolyl, Thiazolyl, Triazolyl, Oxadiazolyl, Thiadiazolyl, Pyridyl oder Pyrimidinyl mit 1 bis 3 Substituenten unabhängig voneinander ausgewählt aus der Gruppe Fluor, Difluormethyl, Trifluormethyl, 2,2,2-Trifluorethyl, 1,1,2,2,2-Pentafluorethyl, Methyl, Ethyl, Isopropyl, Cyclopropyl, Cyclobutyl, Cyclopentyl, Difluormethoxy, Trifluormethoxy, Oxo, -(C=O)ₚ-OR⁹ und -(C=O)ₚ-NR⁹R¹⁰ substituiert sein können,
worin
   p die Zahl 0 oder 1 bedeutet,
   R⁹ und R¹⁰ unabhängig voneinander jeweils für Wasserstoff, Methyl, Ethyl, Isopropyl, Trifluormethyl, 2,2,2-Trifluorethyl, 1,1,2,2,2-Pentafluorethyl, Cyclopropyl, Cyclobutyl oder Cyclopentyl stehen,
   oder
   R⁹ und R¹⁰ bilden zusammen mit dem/den Atom/-en, an die sie jeweils gebunden sind, einen Azetidinyl-, Pyrrolidinyl-, Piperidinyl-, Piperazinyl- oder Morpholinyl-Ring,
   worin der Azetidinyl-, Pyrrolidinyl-, Piperidinyl-, Piperazinyl- und Morpholinyl-Ring seinerseits mit 1 oder 2 Substituenten unabhängig voneinander ausgewählt aus der Gruppe Fluor, Trifluormethyl, Methyl, Ethyl, Hydroxy, Oxo, Methoxy, Ethoxy, Amino, Methylamino, Ethylamino, Dimethylamino und Diethylamino substituiert sein kann,

R⁵ für Wasserstoff, Methyl oder Ethyl steht,
R⁶ für (C₁-C₆)-Alkyl, Cyclopropyl, (C₃-C₆)-Cycloalkyl, Oxetanyl, Azetidinyl, Tetrahydrofuranyl, Pyrrolidinyl, Tetrahydropyranyl, Piperidinyl, Piperazinyl, Morpholinyl, Phenyl, Pyrazolyl, Oxazolyl, Thiazolyl, Triazolyl, Oxadiazolyl, Thiadiazolyl, Pyridyl oder Pyrimidinyl steht,
worin (C₁-C₆)-Alkyl und (C₃-C₆)-Cycloalkyl mit 1 bis 3 Substituenten unabhängig voneinander ausgewählt aus der Gruppe Fluor, Difluormethyl, Trifluormethyl Methyl, Ethyl, Cyclopropyl, Cyclobutyl, Cyclopentyl, Difluormethoxy, Trifluormethoxy, -(C=O)ₚ-OR⁹, -(C=O)ₚ-NR⁹R¹⁰, -NR⁹-(C=O)-R¹⁰, Oxetanyl, Azetidinyl, Tetrahydrofuranyl, Pyrrolidinyl, Tetrahydropyranyl, Piperidinyl, Piperazinyl, Morpholinyl, Phenyl, Furanyl, Pyrazolyl, Imidazolyl, Oxazolyl, Thiazolyl, Triazolyl, Oxadiazolyl, Thiadiazolyl, Pyridyl und Pyrimidinyl substituiert sind,
worin Cyclopropyl, Cyclobutyl, Cyclopentyl, Oxetanyl, Azetidinyl, Tetrahydrofuranyl, Pyrrolidinyl, Tetrahydropyranyl, Piperidinyl, Piperazinyl, Morpholinyl, Phenyl, Furanyl, Pyrazolyl, Imidazolyl, Oxazolyl, Thiazolyl, Triazolyl, Oxadiazolyl, Thiadiazolyl, Pyridyl und Pyrimidinyl ihrerseits mit 1 oder 2 Substituenten unabhängig voneinander ausgewählt aus der Gruppe Fluor, Chlor, Cyano, Difluormethyl, Trifluormethyl, Methyl, Ethyl, Cyclopropyl, Cyclobutyl, Cyclopentyl, Hydroxy, Oxo, Difluormethoxy, Trifluormethoxy, Methoxy und Ethoxy substituiert sein können,
worin Oxetanyl, Azetidinyl, Tetrahydrofuranyl, Pyrrolidinyl, Tetrahydropyranyl, Piperidinyl, Piperazinyl, Morpholinyl, Phenyl, Pyrazolyl, Oxazolyl, Thiazolyl, Triazolyl, Oxadiazolyl, Thiadiazolyl, Pyridyl und Pyrimidinyl mit 1 bis 3 Substituenten unabhängig voneinander ausgewählt aus der Gruppe Fluor, Difluormethyl, Trifluormethyl, Methyl, Ethyl, Cyclopropyl, Cyclobutyl, Cyclopentyl, Difluormethoxy, Trifluormethoxy, Oxo, -(C=O)ₚ-OR⁹ und -(C=O)ₚ-NR⁹R¹⁰ substituiert sein können,
worin
   p die Zahl 0 oder 1 bedeutet,
   R⁹ und R¹⁰ unabhängig voneinander jeweils für Wasserstoff, Methyl, Ethyl, Isopropyl, Trifluormethyl, 2,2,2-Trifluorethyl, 1,1,2,2,2-Pentafluorethyl, Cyclopropyl, Cyclobutyl oder Cyclopentyl stehen,
   oder
   R⁹ und R¹⁰ bilden zusammen mit dem/den Atom/-en, an die sie jeweils gebunden sind, einen Azetidinyl-, Pyrrolidinyl-, Piperidinyl-, Piperazinyl- oder Morpholinyl-Ring,
   worin der Azetidinyl-, Pyrrolidinyl-, Piperidinyl-, Piperazinyl- und Morpholinyl-Ring seinerseits mit 1 oder 2 Substituenten unabhängig voneinander ausgewählt aus der Gruppe Fluor, Trifluormethyl, Methyl, Ethyl, Hydroxy, Oxo, Methoxy, Ethoxy, Amino, Methylamino, Ethylamino, Dimethylamino und Diethylamino substituiert sein kann,
oder
R⁵ und R⁶ bilden zusammen mit dem Stickstoffatom, an das sie gebunden sind, einen Azetidinyl-, Pyrrolidinyl-, Imidazolidinyl-, Piperidinyl-, Dihydropiperdinyl-, Piperazinyl-, Morpholinyl-, Pyrazolyl-, Imidazolyl- oder Triazolyl-Ring,
worin der Azetidinyl-, Pyrrolidinyl-, Imidazolidinyl-, Piperidinyl-, Dihydropiperdinyl-, Piperazinyl-, Morpholinyl-, Pyrazolyl-, Imidazolyl- und Triazolyl-Ring mit 1 oder 2 Substituenten unabhängig voneinander ausgewählt aus der Gruppe Fluor, Cyano, Difluormethyl, Trifluormethyl, 2,2,2-Trifluorethyl, 1,1,2,2,2-Pentafluorethyl, Methyl, Ethyl, 1-Hydroxyethyl, Cyclopropyl, Cyclobutyl, Cyclopentyl, Hydroxy, Oxo, Methoxy, Ethoxy, Difluormethoxy, Trifluormethoxy, Oxetanyl, Azetidinyl, Tetrahydrofuranyl, Pyrrolidinyl, Tetrahydropyranyl, Piperidinyl, Piperazinyl und Morpholinyl substituiert sein können,
- R²: für Trifluormethyl, 2,2,2-Trifluorethyl, 3,3,3-Trifluorprop-1-yl, 2,2,3,3,3-Pentafluorprop-1-yl, Cyclopropyl, Cyclobutyl, Cyclopentyl, Cyclohexyl, Phenyl, Pyridyl, Pyrimidinyl, Pyrazinyl oder Pyridazinyl steht,
wobei Phenyl mit 1 bis 3 Substituenten Fluor substituiert ist,
und
wobei Cyclopropyl, Cyclobutyl, Cyclopentyl, Cyclohexyl, Pyridyl, Pyrimidinyl, Pyrazinyl und Pyridazinyl mit 1 oder 2 Substituenten Fluor substituiert sein können,
sowie ihre Salze, Solvate und Solvate der Salze.

Offenbarungsgegenstand sind Verbindungen gemäß Formel (I), in welcher
- L: für eine Gruppe #¹-CR^{7A}R^{7B}-(CR^{8A}R^{8B})ₘ-#² steht,
wobei
#¹ für die Anknüpfstelle an die Carbonylgruppe steht,
#² für die Anknüpfstelle an den Pyrimidinring steht,
m für eine Zahl 0 steht,
R^{7A} für Wasserstoff, Fluor, Methyl oder Hydroxy steht,
R^{7B} für Wasserstoff, Fluor, Trifluormethyl, Methyl oder 2,2,2-Trifluorethyl steht,
oder
R^{7A} und R^{7B} zusammen mit dem Kohlenstoffatom, an das sie gebunden sind, einen Tetrahydrofuranyl-Ring bilden,
der Ring Q für eine Gruppe der Formel 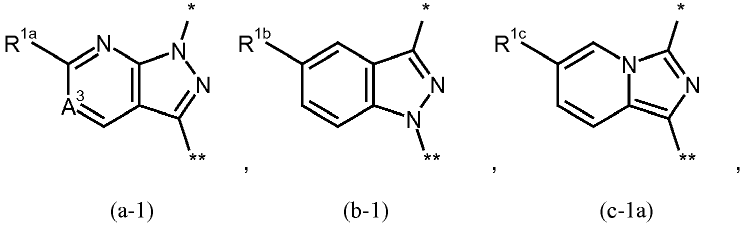 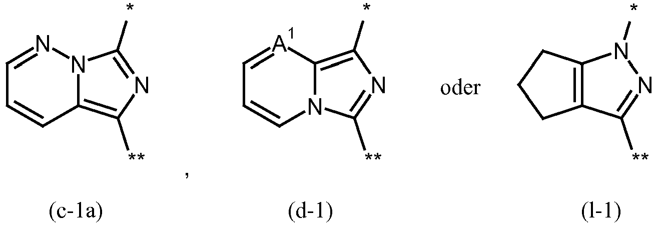 steht, wobei
- *: für die Anknüpfungsstelle an -CH₂-R² steht,
- **: für die Anknüpfungsstelle an das Pyrimidin steht,
- R^{1a}: für Wasserstoff oder Methyl steht,
- R^{1b}: für Wasserstoff oder Fluor steht,
- R^{1c}: für Wasserstoff oder Chlor steht,
- A¹: für N oder CH steht,
- A³: für N, CH oder C-F steht,
- R³: für -NR⁵R⁶ steht,
wobei
R⁵ für Wasserstoff steht,
R⁶ für (C₁-C₆)-Alkyl steht,
worin (C₁-C₆)-Alkyl mit 1 oder 2 Substituenten unabhängig voneinander ausgewählt aus der Gruppe Fluor, Difluormethyl, Trifluormethyl oder -(C=O)ₚ-OR⁹ substituiert ist,
worin
   p die Zahl 0 bedeutet,
   R⁹ für Wasserstoff steht,
- R²: für 2-Fluorphenyl, 2,3-Difluorphenyl oder 3-Fluorpyrid-2-yl steht,
sowie ihre Salze, Solvate und Solvate der Salze.

Offenbarungsgegenstand sind Verbindungen gemäß Formel (I), in welcher
- L: für eine Gruppe #¹-CR^{7A}R^{7B}-(CR^{8A}R^{8B})ₘ-#² steht,
wobei
#¹ für die Anknüpfstelle an die Carbonylgruppe steht,
#² für die Anknüpfstelle an den Pyrimidinring steht,
m für eine Zahl 0 steht,
R^{7A} für Methyl steht,
R^{7B} für Methyl steht,
der Ring Q für eine Gruppe der Formel 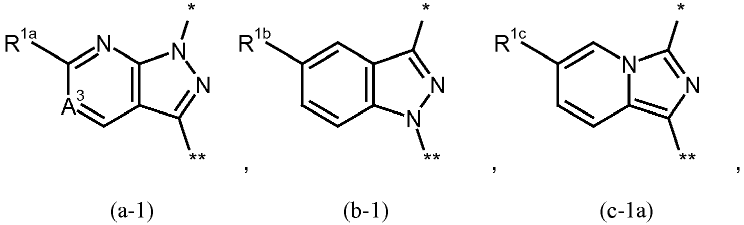 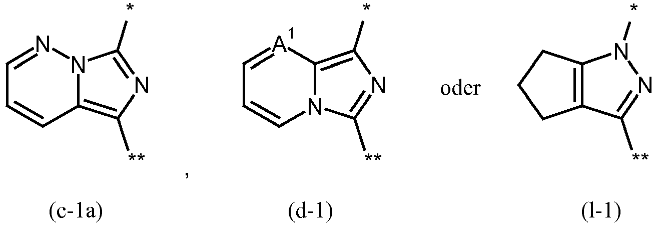 steht, wobei
- *: für die Anknüpfungsstelle an -CH₂-R² steht,
- **: für die Anknüpfungsstelle an das Pyrimidin steht,
- R^{1a}: für Wasserstoff oder Methyl steht,
- R^{1b}: für Wasserstoff oder Fluor steht,
- R^{1c}: für Wasserstoff oder Chlor steht,
- A¹: für N oder CH steht,
- A³: für N, CH oder C-F steht,
- R³: für -NR⁵R⁶ steht,
wobei
R⁵ für Wasserstoff, Methyl oder Ethyl steht,
R⁶ für (C₁-C₆)-Alkyl, Oxetanyl, Azetidinyl, Tetrahydrofuranyl, Pyrrolidinyl, Tetrahydropyranyl, Piperidinyl, Piperazinyl, Morpholinyl, Phenyl, Pyrazolyl, Oxazolyl, Thiazolyl, Triazolyl, Oxadiazolyl, Thiadiazolyl, Pyridyl oder Pyrimidinyl steht,
worin (C₁-C₆)-Alkyl mit 1 oder 2 Substituenten unabhängig voneinander ausgewählt aus der Gruppe Fluor, Difluormethyl, Trifluormethyl Cyclopropyl, Cyclobutyl, Cyclopentyl, Trifluormethoxy, -(C=O)ₚ-OR⁹, -(C=O)ₚ-NR⁹R¹⁰, Tetrahydrofuranyl, Pyrrolidinyl, Tetrahydropyranyl, Phenyl, Furanyl, Pyrazolyl, Imidazolyl, Triazolyl und Pyridyl substituiert ist,
worin
   p die Zahl 0 oder 1 bedeutet,
   R⁹ und R¹⁰ unabhängig voneinander jeweils für Wasserstoff, Methyl, Ethyl, Trifluormethyl, 2,2,2-Trifluorethyl, 1,1,2,2,2-Pentafluorethyl, Cyclopropyl oder Cyclobutyl stehen, und
worin Tetrahydrofuranyl, Pyrrolidinyl, Tetrahydropyranyl, Phenyl, Furanyl, Pyrazolyl, Imidazolyl, Triazolyl und Pyridyl ihrerseits mit 1 oder 2 Substituenten unabhängig voneinander ausgewählt aus der Gruppe Fluor, Chlor, Cyano, Difluormethyl, Trifluormethyl, Methyl, Ethyl und Oxo sein können,
worin Oxetanyl, Azetidinyl, Tetrahydrofuranyl, Pyrrolidinyl, Tetrahydropyranyl, Piperidinyl, Piperazinyl, Morpholinyl, Phenyl, Pyrazolyl, Oxazolyl, Thiazolyl, Triazolyl, Oxadiazolyl, Thiadiazolyl, Pyridyl und Pyrimidinylmit 1 oder 2 Substituenten unabhängig voneinander ausgewählt aus der Gruppe Fluor, Difluormethyl, Trifluormethyl, Methyl, Ethyl und Oxo substituiert sein können, oder
- R⁵ und R⁶: bilden zusammen mit dem Stickstoffatom, an das sie gebunden sind, einen Azetidinyl-, Pyrrolidinyl-, Imidazolidinyl-, Piperidinyl-, Dihydropiperdinyl-, Piperazinyl-, Morpholinyl-, Pyrazolyl- oder Imidazolyl-Ring,
worin der Azetidinyl-, Pyrrolidinyl-, Imidazolidinyl-, Piperidinyl-, Dihydropiperdinyl-, Piperazinyl-, Morpholinyl-, Pyrazolyl- und Imidazolyl-Ring mit 1 oder 2 Substituenten unabhängig voneinander ausgewählt aus der Gruppe Fluor, Cyano, Difluormethyl, Trifluormethyl, 2,2,2-Trifluorethyl, 1,1,2,2,2-Pentafluorethyl, Methyl, Ethyl, 1-Hydroxyethyl, Cyclopropyl, Cyclobutyl, Cyclopentyl, Hydroxy, Oxo, Methoxy, Ethoxy, Difluormethoxy und Trifluormethoxy substituiert sein können,
- R²: für 3,3,3-Trifluoreth-1-yl, 2,2,3,3,3-Pentafluorprop-1-yl, Phenyl oder Pyridyl steht,
wobei Phenyl mit 1 bis 3 Substituenten Fluor substituiert ist,
und
wobei Pyridyl mit 1 Substituenten Fluor substituiert sein kann,
sowie ihre Salze, Solvate und Solvate der Salze.

Offenbarungsgegenstand sind Verbindungen gemäß Formel (I), in welcher
- L: für eine Gruppe #¹-CR^{7A}R^{7B}-(CR^{8A}R^{8B})ₘ-#² steht,
wobei
#¹ für die Anknüpfstelle an die Carbonylgruppe steht,
#² für die Anknüpfstelle an den Pyrimidinring steht,
m für eine Zahl 0 steht,
R^{7A} für Wasserstoff, Fluor, Methyl, Hydroxy steht,
R^{7B} für Wasserstoff, Fluor, Methyl oder Trifluormethyl steht, oder
R^{7A} und R^{7B} zusammen mit dem Kohlenstoffatom, an das sie gebunden sind, einen Tetrahydrofuranyl-Ring bilden,
der Ring Q für eine Gruppe der Formel 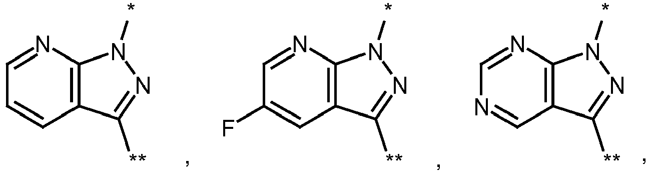 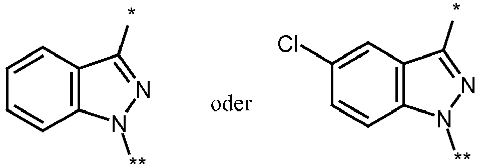 steht, wobei
- *: für die Anknüpfungsstelle an -CH₂-R² steht,
- **: für die Anknüpfungsstelle an das Pyrimidin steht,
- R³: für -OR⁴ oder -NR⁵R⁶ steht,
wobei
R⁴ für (C₁-C₆)-Alkyl oder Pyrazolyl steht,
worin (C₁-C₆)-Alkyl mit 1 bis 3 Substituenten unabhängig voneinander ausgewählt aus der Gruppe Fluor, Trifluormethyl, -(C=O)ₚ-OR⁹ und -(C=O)ₚ-NR⁹R¹⁰ substituiert sein kann,
worin
   p die Zahl 0 oder 1 bedeutet,
   R⁹ und R¹⁰ unabhängig voneinander jeweils für Wasserstoff oder Methyl stehen,
und
worin Pyrazolyl mit 1 oder 2 Substituenten unabhängig voneinander ausgewählt aus der Gruppe Fluor, Difluormethyl, Trifluormethyl, 2,2,2-Trifluorethyl, 1,1,2,2,2-Pentafluorethyl, Methyl, Cyclopropyl, Cyclobutyl, Cyclopentyl substituiert sein kann,
R⁵ für Wasserstoff, Methyl oder Ethyl steht,
R⁶ für (C₁-C₆)-Alkyl, Oxetanyl, Azetidinyl, Tetrahydrofuranyl, Pyrrolidinyl, Tetrahydropyranyl, Piperidinyl, Piperazinyl, Morpholinyl, Phenyl, Pyrazolyl, Oxazolyl, Thiazolyl, Triazolyl, Oxadiazolyl, Thiadiazolyl, Pyridyl oder Pyrimidinyl steht,
worin (C₁-C₆)-Alkyl mit 1 oder 2 Substituenten unabhängig voneinander ausgewählt aus der Gruppe Fluor, Difluormethyl, Trifluormethyl Cyclopropyl, Cyclobutyl, Cyclopentyl, Trifluormethoxy, -(C=O)ₚ-OR⁹, -(C=O)ₚ-NR⁹R¹⁰, Tetrahydrofuranyl, Pyrrolidinyl, Tetrahydropyranyl, Phenyl, Furanyl, Pyrazolyl, Imidazolyl, Triazolyl und Pyridyl substituiert ist,
worin
   p die Zahl 0 oder 1 bedeutet,
   R⁹ und R¹⁰ unabhängig voneinander jeweils für Wasserstoff, Methyl, Ethyl, Trifluormethyl, 2,2,2-Trifluorethyl, 1,1,2,2,2-Pentafluorethyl, Cyclopropyl oder Cyclobutyl stehen, und
worin Cyclopropyl, Cyclobutyl, Cyclopentyl, Tetrahydrofuranyl, Pyrrolidinyl, Tetrahydropyranyl, Phenyl, Furanyl, Pyrazolyl, Imidazolyl, Triazolyl und Pyridyl ihrerseits mit 1 oder 2 Substituenten unabhängig voneinander ausgewählt aus der Gruppe Fluor, Chlor, Cyano, Difluormethyl, Trifluormethyl, Methyl, Ethyl, Oxo und Hydroxy sein können,
worin Oxetanyl, Azetidinyl, Tetrahydrofuranyl, Pyrrolidinyl, Tetrahydropyranyl, Piperidinyl, Piperazinyl, Morpholinyl, Phenyl, Pyrazolyl, Oxazolyl, Thiazolyl, Triazolyl, Oxadiazolyl, Thiadiazolyl, Pyridyl und Pyrimidinyl mit 1 oder 2 Substituenten unabhängig voneinander ausgewählt aus der Gruppe Fluor, Difluormethyl, Trifluormethyl, Methyl, Ethyl, Oxo, Azetidinyl und Pyrrolidinyl substituiert sein können,
oder
R⁵ und R⁶ bilden zusammen mit dem Stickstoffatom, an das sie gebunden sind, einen Azetidinyl-, Pyrrolidinyl-, Imidazolidinyl-, Piperidinyl-, Dihydropiperdinyl-, Piperazinyl-, Morpholinyl-, Pyrazolyl- oder Imidazolyl-Ring, worin der Azetidinyl-, Pyrrolidinyl-, Imidazolidinyl-, Piperidinyl-, Dihydropiperdinyl-, Piperazinyl-, Morpholinyl-, Pyrazolyl- und Imidazolyl-Ring mit 1 oder 2 Substituenten unabhängig voneinander ausgewählt aus der Gruppe Fluor, Cyano, Difluormethyl, Trifluormethyl, 2,2,2-Trifluorethyl, 1,1,2,2,2-Pentafluorethyl, Methyl, Ethyl, 1-Hydroxyethyl, Cyclopropyl, Cyclobutyl, Cyclopentyl, Hydroxy, Oxo, Methoxy, Ethoxy, Difluormethoxy und Trifluormethoxy substituiert sein können,
R² für 2-Fluorphenyl, 2,3-Difluorphenyl oder 3-Fluorpyrid-2-yl steht,
sowie ihre Salze, Solvate und Solvate der Salze.

Ebenfalls bevorzugt sind Verbindungen der vorliegenden Erfindung gemäß Formel (I) in welcher
- L: für eine Gruppe #¹-CR^{7A}R^{7B}-(CR^{8A}R^{8B})ₘ-#² steht,
wobei
#¹ für die Anknüpfstelle an die Carbonylgruppe steht,
#² für die Anknüpfstelle an den Pyrimidinring steht,
m für eine Zahl 0 steht,
R^{7A} für Methyl steht,
R^{7B} für Methyl steht,
oder
R^{7A} und R^{7B} zusammen mit dem Kohlenstoffatom, an das sie gebunden sind, einen Tetrahydrofuranyl-Ring bilden,
der Ring Q für eine Gruppe der Formel 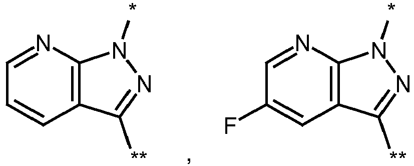 steht, wobei
- *: für die Anknüpfungsstelle an -CH₂-R² steht,
- **: für die Anknüpfungsstelle an das Pyrimidin steht,
- R³: für -OR⁴ oder -NR⁵R⁶ steht,
wobei
R⁴ für (C₁-C₆)-Alkyl oder Pyrazolyl steht,
worin (C₁-C₆)-Alkyl mit 1 bis 3 Substituenten unabhängig voneinander ausgewählt aus der Gruppe Fluor, Trifluormethyl, -(C=O)ₚ-OR⁹ und -(C=O)ₚ-NR⁹R¹⁰ substituiert sein kann,
worin
   p die Zahl 0 oder 1 bedeutet,
   R⁹ und R¹⁰ unabhängig voneinander jeweils für Wasserstoff oder Methyl stehen,
und
worin Pyrazolyl mit 1 oder 2 Substituenten unabhängig voneinander ausgewählt aus der Gruppe Fluor, Difluormethyl, Trifluormethyl, 2,2,2-Trifluorethyl, 1,1,2,2,2-Pentafluorethyl, Methyl, Cyclopropyl, Cyclobutyl, Cyclopentyl substituiert sein kann,
R⁵ für Wasserstoff, Methyl oder Ethyl steht,
R⁶ für (C₁-C₆)-Alkyl, Oxetanyl, Azetidinyl, Tetrahydrofuranyl, Pyrrolidinyl, Tetrahydropyranyl, Piperidinyl, Piperazinyl, Morpholinyl, Phenyl, Pyrazolyl, Oxazolyl, Thiazolyl, Triazolyl, Oxadiazolyl, Thiadiazolyl, Pyridyl oder Pyrimidinyl steht,
worin (C₁-C₆)-Alkyl mit 1 oder 2 Substituenten unabhängig voneinander ausgewählt aus der Gruppe Fluor, Difluormethyl, Trifluormethyl Cyclopropyl, Cyclobutyl, Cyclopentyl, Trifluormethoxy, -(C=O)ₚ-OR⁹, -(C=O)ₚ-NR⁹R¹⁰, Tetrahydrofuranyl, Pyrrolidinyl, Tetrahydropyranyl, Phenyl, Furanyl, Pyrazolyl, Imidazolyl, Triazolyl und Pyridyl substituiert ist,
worin
p die Zahl 0 oder 1 bedeutet,
R⁹ und R¹⁰ unabhängig voneinander jeweils für Wasserstoff, Methyl, Ethyl, Trifluormethyl, 2,2,2-Trifluorethyl, 1,1,2,2,2-Pentafluorethyl, Cyclopropyl oder Cyclobutyl stehen, und
worin Cyclopropyl, Cyclobutyl, Cyclopentyl, Tetrahydrofuranyl, Pyrrolidinyl, Tetrahydropyranyl, Phenyl, Furanyl, Pyrazolyl, Imidazolyl, Triazolyl und Pyridyl ihrerseits mit 1 oder 2 Substituenten unabhängig voneinander ausgewählt aus der Gruppe Fluor, Chlor, Cyano, Difluormethyl, Trifluormethyl, Methyl, Ethyl, Oxo und Hydroxy sein können,
worin Oxetanyl, Azetidinyl, Tetrahydrofuranyl, Pyrrolidinyl, Tetrahydropyranyl, Piperidinyl, Piperazinyl, Morpholinyl, Phenyl, Pyrazolyl, Oxazolyl, Thiazolyl, Triazolyl, Oxadiazolyl, Thiadiazolyl, Pyridyl und Pyrimidinyl mit 1 oder 2 Substituenten unabhängig voneinander ausgewählt aus der Gruppe Fluor, Difluormethyl, Trifluormethyl, Methyl, Ethyl, Oxo, Azetidinyl und Pyrrolidinyl substituiert sein können,
oder
R⁵ und R⁶ bilden zusammen mit dem Stickstoffatom, an das sie gebunden sind, einen Azetidinyl-, Pyrrolidinyl-, Imidazolidinyl-, Piperidinyl-, Dihydropiperdinyl-, Piperazinyl-, Morpholinyl-, Pyrazolyl- oder Imidazolyl-Ring,
worin der Azetidinyl-, Pyrrolidinyl-, Imidazolidinyl-, Piperidinyl-, Dihydropiperdinyl-, Piperazinyl-, Morpholinyl-, Pyrazolyl- und Imidazolyl-Ring mit 1 oder 2 Substituenten unabhängig voneinander ausgewählt aus der Gruppe Fluor, Cyano, Difluormethyl, Trifluormethyl, 2,2,2-Trifluorethyl, 1,1,2,2,2-Pentafluorethyl, Methyl, Ethyl, 1-Hydroxyethyl, Cyclopropyl, Cyclobutyl, Cyclopentyl, Hydroxy, Oxo, Methoxy, Ethoxy, Difluormethoxy und Trifluormethoxy substituiert sein können,
- R²: für 2-Fluorphenyl, 2,3-Difluorphenyl oder 3-Fluorpyrid-2-yl steht,
sowie ihre Salze, Solvate und Solvate der Salze.

Ebenfalls bevorzugt sind Verbindungen der vorliegenden Erfindung gemäß Formel (I), die eine Wirkung an rekombinanter Guanylatcyclase-Reporterzellinien gemäß der Untersuchung unter B-2 als minimal effective concentration (MEC) von ≤ 3 µm zeigen und eine Inhibition der humanen Phosphodiesterase 5 (PDE5) gemäß der Untersuchung unter B-6 als IC50 < 100 nm zeigen,
sowie ihre Salze, Solvate und Solvate der Salze.

Besonders bevorzugt sind Verbindungen der vorliegenden Erfindung gemäß Anspruch 1 und der Beispiele 1-117, die eine Wirkung an rekombinanter Guanylatcyclase-Reporterzellinien gemäß der Untersuchung unter B-2 als minimal effective concentration (MEC) von < 3 µm zeigen und eine Inhibition der humanen Phosphodiesterase 5 (PDE5) gemäß der Untersuchung unter B-6 als IC50 < 100 nm zeigen,
sowie ihre Salze, Solvate und Solvate der Salze.

Die in den jeweiligen Kombinationen bzw. bevorzugten Kombinationen von Resten im einzelnen angegebenen Reste-Definitionen werden unabhängig von den jeweiligen angegebenen Kombinationen der Reste beliebig auch durch Reste-Definitionen anderer Kombinationen ersetzt.

Besonders bevorzugt sind Kombinationen von zwei oder mehreren der oben genannten Vorzugsbereiche.

Die erfindungsgemäßen Verbindungen der Formel (I), in welcher R³ für Hydroxy steht (I-A), können auch in der tautomeren Keto-Form (I'-A) vorliegen (siehe nachfolgendes Schema 7); beide tautomeren Formen werden von der vorliegenden Erfindung ausdrücklich umfasst.

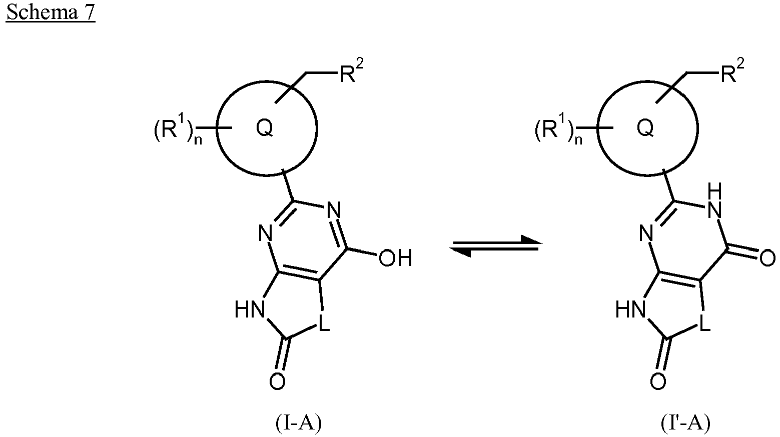

Weiterer Gegenstand der Erfindung ist ein Verfahren zur Herstellung der erfindungsgemäßen Verbindungen der Formel (I) dadurch gekennzeichnet, dass man eine Verbindung der Formel (II) 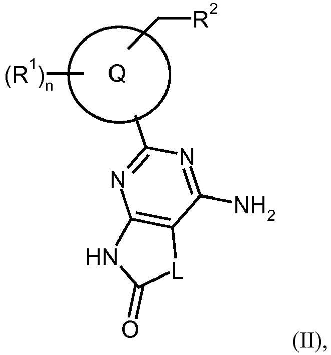 in welcher n, L, Q, R¹ und R² jeweils die oben genannten Bedeutungen haben,
in einem inerten Lösungsmittel mit iso-Pentylnitrit und einem Halogen-Äquivalent in eine Verbindung der Formel (III) 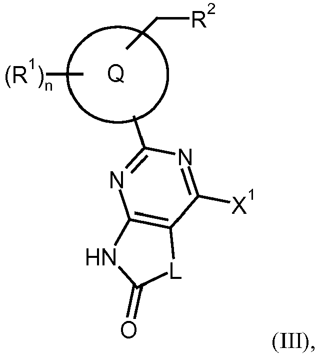 in welcher n, L, Q, R¹ und R² jeweils die oben angegebenen Bedeutungen haben und X¹ für Chlor, Brom oder Iod steht
überführt, und diese im Anschluss in einen inerten Lösungsmittel gegebenenfalls in Gegenwart einer geeigneten Base mit einer Verbindung der Formel (IV)

R³-H (IV),

in welcher R³ die oben angegebene Bedeutung hat,
zu einer Verbindung der Formel (I) 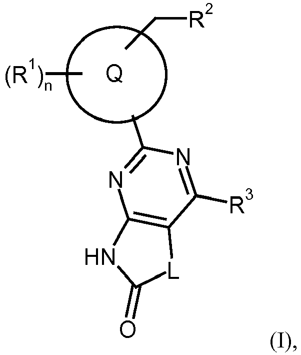 in welcher n, L, Q, R¹, R² und R³ jeweils die oben angegebenen Bedeutungen haben,
umsetzt,
und gegebenenfalls die resultierenden Verbindungen der Formel (I) gegebenenfalls mit den entsprechenden (i) Lösungsmitteln und/oder (ii) Säuren oder Basen in ihre Solvate, Salze und/oder Solvate der Salze überführt.

Der Verfahrensschritt (II) → (III) erfolgt mit oder ohne Lösungsmittel. Als Lösungsmittel eignen sich alle organischen Lösungsmittel, die unter den Reaktionsbedingungen inert sind. Bevorzugtes Lösungsmittel ist Dimethoxyethan.

Die Reaktion (II) → (III) erfolgt im Allgemeinen in einem Temperaturbereich von +20°C bis +100°C, bevorzugt im Bereich von +50°C bis +100°C, gegebenenfalls in einer Mikrowelle. Die Umsetzung kann bei normalem, erhöhtem oder erniedrigtem Druck durchgeführt werden (z.B. im Bereich von 0.5 bis 5 bar). Im Allgemeinen arbeitet man bei Normaldruck.

Als Halogen-Quelle bei der Umsetzung (II) → (III) eignen sich beispielsweise Diiodmethan, eine Mischung aus Cäsiumiodid, Iod und Kupfer-(I)-iodid oder Kupfer-(II)-bromid.

Inerte Lösungsmittel für den Verfahrensschritt (III) + (IV) → (I) sind beispielsweise Ether wie Diethylether, Dioxan, Dimethoxyethan, Tetrahydrofuran, Glykoldimethylether oder Diethylenglykoldimethylether, Kohlenwasserstoffe wie Benzol, Xylol, Toluol, Hexan, Cyclohexan oder Erdölfraktionen, oder andere Lösungsmittel wie Dimethylformamid (DMF), Dimethylsulfoxid (DMSO), *N,N*'-Dimethylpropylenharnstoff (DMPU), *N-*Methylpyrrolidon (NMP), Pyridin, Acetonitril oder Sulfolan. Ebenso ist es möglich, Gemische der genannten Lösungsmittel einzusetzen. Bevorzugt ist NMP.

Im Fall von R³ = -OR⁴ erfolgt die Reaktion (III) + (IV) → (I) bevorzugt ohne Lösungsmittel.

Im Fall von R³ = -OR⁴ erfolgt die Umsetzung (III) + (IV) → (I) in Gegenwart eines geeigneten Kupfer-Katalysators wie beispielsweise Kupfer-(I)-iodid, unter Zusatz von 3,4,7,8-Tetramethyl-1,10-Phenanthrolin, sowie einer geeigneten Base wie beispielsweise Erdalkalicarbonate wie Lithium-, Natrium-, Kalium-, Calcium- oder Cäsiumcarbonat, bevorzugt Cäsiumcarbonat.

Alternativ kann im Fall von R³ = -OR⁴ die Darstellung der Verbindungen der Formel (I) auch unter Mitsunobu-Bedingungen [siehe: a) Hughes, D. L. "The Mitsunobu Reaction," Organic Reactions; John Wiley & Sons, Ltd, 1992, vol. 42, p. 335. b) Hughes, D. L. Org. Prep. Proceed. Int. 1996, 28, 127.] ausgehend von einer Verbindung der Formel (I-A) 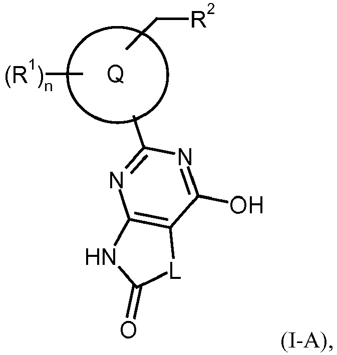 in welcher n, L, Q, R¹ und R² jeweils die oben angegebenen Bedeutungen haben,
erfolgen.

Hierbei erfolgt die Mitsunobu-Reaktion unter Verwendung von Triphenylphosphin, oder Tri-n-butylphosphin, 1,2-Bis(diphenylphosphino)ethan (DPPE), Diphenyl(2-pyridyl)phosphin (Ph2P-Py), (p-Dimethylaminophenyl)diphenylphosphin (DAP-DP), tris(4-Dimethylaminophenyl)-phosphin (tris-DAP) und eines geeigneten Dialkylazodicarboxylats, wie beispielsweise Diethylazodicarboxylat (DEAD), Diisopropylazodicarboxylat (DIAD), Di-tert-butylazodicarboxylat, N,N,N'N'-Tetramethylazodicarboxamid (TMAD), 1,1'-(Azodicarbonyl)-dipiperidin (ADDP) oder 4,7-Dimethyl-3,5,7-hexahydro-1,2,4,7-tetrazocin-3,8-dion (DHTD). Bervorzugt werden Triphenylphosphin und Diisopropylazodicarboxylat (DIAD) verwendet, oder eines geeigneten Azodicarbonamides, wie beispielsweise N,N,N',N'-Tetramethyldiazen-1,2-dicarboxamid.

Inerte Lösungsmittel für die Mitsunobu-Reaktion (I-A) + (IV) → (I) sind beispielsweise Ether wie Tetrahydrofuran, Diethylether, Kohlenwasserstoffe wie Benzol, Toluol, Xylol, Halogenkohlenwasserstoffe wie Dichlormethan, Dichlorethan oder andere Lösungsmittel wie Acetonitril, DMF oder NMP. Ebenso ist es möglich, Gemische der genannten Lösungsmittel einzusetzen. Bevorzugt wird THF verwendet.

Die Mitsunobu-Reaktion (III) + (IV) → (I) erfolgt im Allgemeinen in einem Temperaturbereich von -78°C bis +180°C, bevorzugt bei 0°C bis +50°C, gegebenenfalls in einer Mikrowelle. Die Umsetzungen können bei normalem, erhöhtem oder bei erniedrigtem Druck durchgeführt werden (z.B. von 0.5 bis 5 bar).

Alternativ kann im Fall von R³ = -OR⁴ die Darstellung der Verbindungen der Formel (I) auch unter Alkylierungsbedingungen ausgehend von einer Verbindung der Formel (I-A) hergestellt werden. Hierzu wird ein Alkylhalogenid, bevorzugt Alkyliodid unter Basenzusatz mit (I-A) in einem inerten Lösungsmittel umgesetzt.

Als Basen für den Verfahrensschritt (I-A) → (I) eignen sich die üblichen anorganischen oder organischen Basen. Hierzu gehören bevorzugt Alkalihydroxide wie beispielsweise Lithium-, Natrium- oder Kaliumhydroxid, Alkali- oder Erdalkalicarbonate wie Lithium-, Natrium-, Kalium-, Calcium- oder Cäsiumcarbonat, Alkali-Alkoholate wie Natrium- oder Kaliummethanolat, Natrium- oder Kaliumethanolat oder Natrium- oder Kalium-tert.-butylat, Alkalihydride wie Natrium- oder Kaliumhydrid oder Amide wie Natriumamid, Lithium- oder Kalium-bis(trimethylsilyl)amid oder Lithiumdiisopropylamid. Bevorzugt wird Cäsiumcarbonat verwendet.

Inerte Lösungsmittel sind beispielsweise Ether wie Tetrahydrofuran, Diethylether, Kohlenwasserstoffe wie Benzol, Toluol, Xylol, sowie andere Lösungsmittel wie DMF oder NMP. Ebenso ist es möglich, Gemische der genannten Lösungsmittel einzusetzen. Bevorzugt wird DMF verwendet.

Die Alkylierungsreaktion erfolgt im Allgemeinen in einem Temperaturbereich von -78°C bis +180°C, bevorzugt bei 0°C bis +130°C, gegebenenfalls in einer Mikrowelle. Die Umsetzungen können bei normalem, erhöhtem oder bei erniedrigtem Druck durchgeführt werden (z.B. von 0.5 bis 5 bar).

Im Fall von R³ = -NR⁵R⁶, wenn R⁵ und R⁶ zusammen mit dem Stickstoffatom, an das sie gebunden sind, ein 5- oder 6-gliedriges Heteroaryl bilden, das im oben angegebenen Bedeutungsumfang substituiert sein kann, erfolgt die Umsetzung (III) + (IV) → (I) in Gegenwart eines geeigneten Kupfer-Katalysators wie beispielsweise Kupfer-(I)-oxid, unter Zusatz von 2-Hydroxybenzaldehydoxim, sowie einer geeigneten Base wie beispielsweise Erdalkalicarbonate wie Lithium-, Natrium-, Kalium-, Calcium- oder Cäsiumcarbonat, bevorzugt Cäsiumcarbonat.

Die Reaktion (III) + (IV) → (I) wird im Allgemeinen in einem Temperaturbereich von +20°C bis +200°C, bevorzugt bei +150°C bis +200°C, bevorzugt in einer Mikrowelle, durchgeführt. Die Umsetzung kann bei normalem, erhöhtem oder bei erniedrigtem Druck erfolgen (z.B. von 0.5 bis 5 bar).

Das beschriebene Herstellverfahren kann durch das folgende Syntheseschema (Schema 1) beispielhaft verdeutlicht werden: 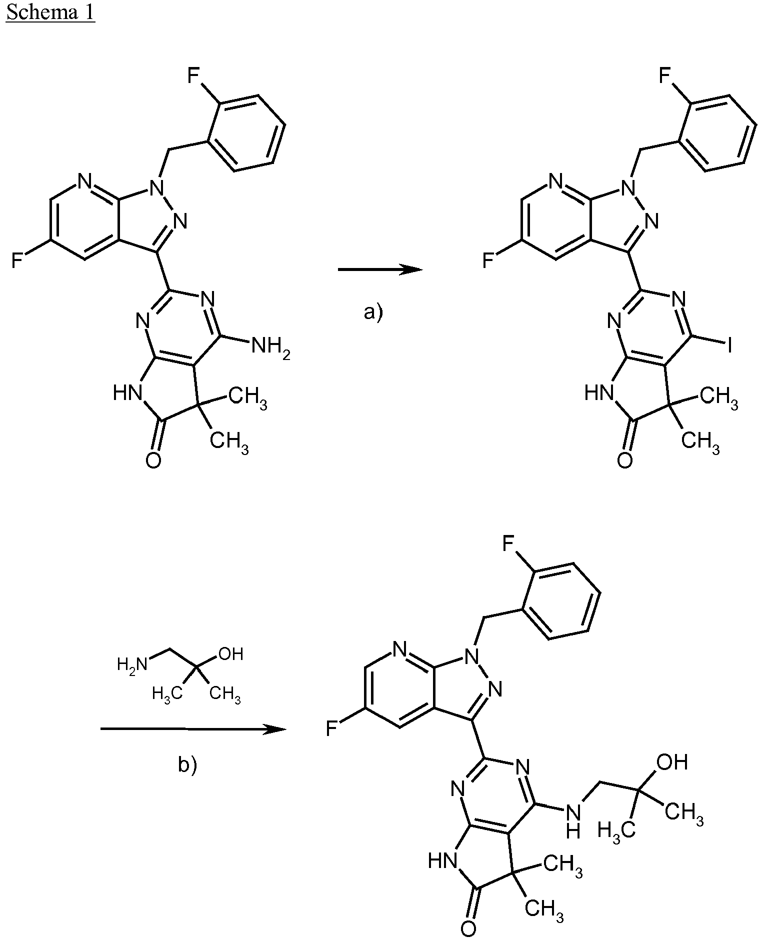 [a): Diiodmethan, iso-Pentylnitrit; b): NMP, Mikrowelle, 150°C].

Verbindungen der Formel (I-A) werden als Nebenprodukte bei der Herstellung der Verbindungen der Formel (III) erhalten.

Verbindungen der Formel (I-A) können alternativ auch ausgehend von Verbindungen der Formel (V) durch Umsetzung mit Verbindungen der Formel (VIII) 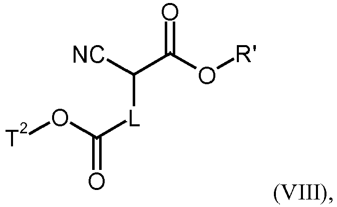 in welcher L die oben angegebene Bedeutung hat,
- T²: für (C₁-C₄)-Alkyl steht und
- R': für (C₁-C₄)-Alkyl steht,
erhalten verden, wie z.B. bei Foeldi, et al., Chemische Berichte, 1942 , vol. 75, p. 760 beschrieben.

Verbindungen der Formel (I-A) können in einem weiteren Alternativverfahren auch ausgehend von Verbindungen der Formel (II) durch Umsetzung mit Nitriten in Säuren, ggf unter Zusatz von Wasser hergestellt werden. Bevorzugt ist Natriumnitrit in einer Mischung aus Trifluoressigsäure und Wasser.

Die Reaktion (II)) → (I-A) wird im Allgemeinen in einem Temperaturbereich von -15°C bis +70°C, bevorzugt bei 0°C bis +40°C, unter portionsweiser Zugabe des Nitrites durchgeführt. Die Umsetzung kann bei normalem, erhöhtem oder bei erniedrigtem Druck erfolgen (z.B. von 0.5 bis 5 bar).

Das zuvor beschriebene Herstellverfahren kann durch das nachfolgende Syntheseschema (Schema 2) beispielhaft verdeutlicht werden: 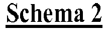 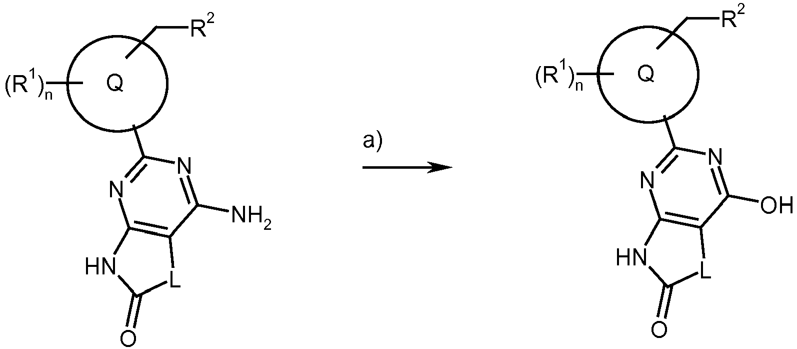 [a): Natriumnitrit, Trifluoressigsäure, Wasser, 0°C - RT].

Die Verbindungen der Formel (II) sind literaturbekannt (siehe z.B. WO 2010/065275, WO 2011/ 115804 und WO 2011/149921) oder können in Analogie zu literaturbekannten Verfahen hergestellt werden.

Die Verbindungen der Formel (II) können hergestellt werden, indem man eine Verbindung der Formel (V) 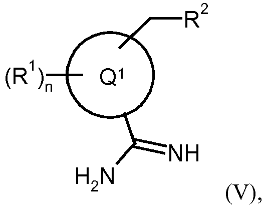 in welcher n, Q¹, R¹ und R² jeweils die zuvor genannten Bedeutungen haben,
in einem inerten Lösungsmittel in Gegenwart einer geeigneten Base mit einer Verbindung der Formel (VI) 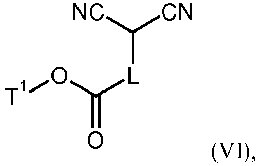 in welcher L die oben genannte Bedeutung hat und
- T¹: für (C₁-C₄)-Alkyl steht,
zu einer Verbindung der Formel (II) 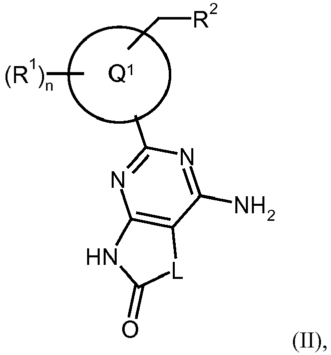 in welcher n, L, Q¹, R¹ und R² jeweils die oben genannten Bedeutungen haben,
umsetzt.

Inerte Lösungsmittel für den Verfahrensschritt (V) + (VI) → (II) sind beispielsweise Alkohole wie Methanol, Ethanol, n-Propanol, Isopropanol, n-Butanol oder tert.-Butanol, Ether wie Diethylether, Dioxan, Dimethoxyethan, Tetrahydrofuran, Glykoldimethylether oder Diethylenglykoldimethylether, Kohlenwasserstoffe wie Benzol, Xylol, Toluol, Hexan, Cyclohexan oder Erdölfraktionen, oder andere Lösungsmittel wie Dimethylformamid (DMF), Dimethylsulfoxid (DMSO), *N,N'-*Dimethylpropylenharnstoff (DMPU), *N-*Methylpyrrolidon (NMP), Pyridin, Acetonitril, Sulfolan oder auch Wasser. Ebenso ist es möglich, Gemische der genannten Lösungsmittel einzusetzen. Bevorzugt ist tert.-Butanol oder Methanol.

Geeignete Basen für den Verfahrensschritt (V) + (VI) → (II) sind Alkalihydroxide wie beispielsweise Lithium-, Natrium- oder Kaliumhydroxid, Alkalicarbonate wie Lithium-, Natrium-, Kalium- oder Cäsiumcarbonat, Alkalihydrogencarbonate wie Natrium- oder Kaliumhydrogencarbonat, Alkalialkoholate wie Natrium- oder Kaliummethanolat, Natrium- oder Kaliumethanolat oder Kalium-tert.-butylat, oder organische Amine wie Triethylamin, Diisopropylethylamin, Pyridin, 1,8-Diazabicyclo[5.4.0]undec-7-en (DBU) oder 1,5-Diazabicyclo[4.3.0]non-5-en (DBN). Bevorzugt ist Kalium-tert.-butylat oder Natriummethanolat.

Die Reaktion (V) + (VI) → (II) wird im Allgemeinen in einem Temperaturbereich von +20°C bis +150°C, bevorzugt bei +75°C bis +100°C, gegebenenfalls in einer Mikrowelle, durchgeführt. Die Umsetzung kann bei normalem, erhöhtem oder bei erniedrigtem Druck erfolgen (z.B. von 0.5 bis 5 bar). Im Allgemeinen arbeitet man bei Normaldruck.

Das zuvor beschriebene Herstellverfahren kann durch das nachfolgende Syntheseschema (Schema 3) beispielhaft verdeutlicht werden: 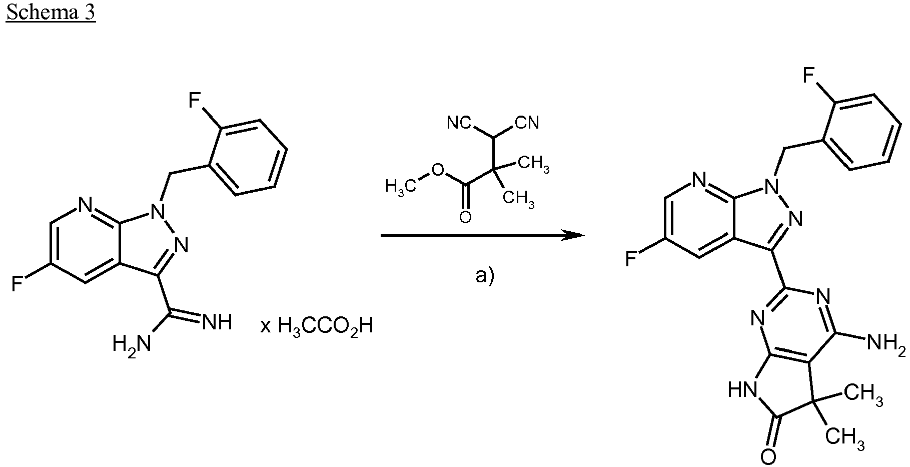 [a): KOt-Bu, tert.-Butanol;].

Die Verbindungen der Formel (V) sind literaturbekannt (siehe z.B. WO 03/095451, Beispiel 6A) oder können wie in den nachfolgenden Syntheseschemata (Schema 4 bis 6) hergestellt werden 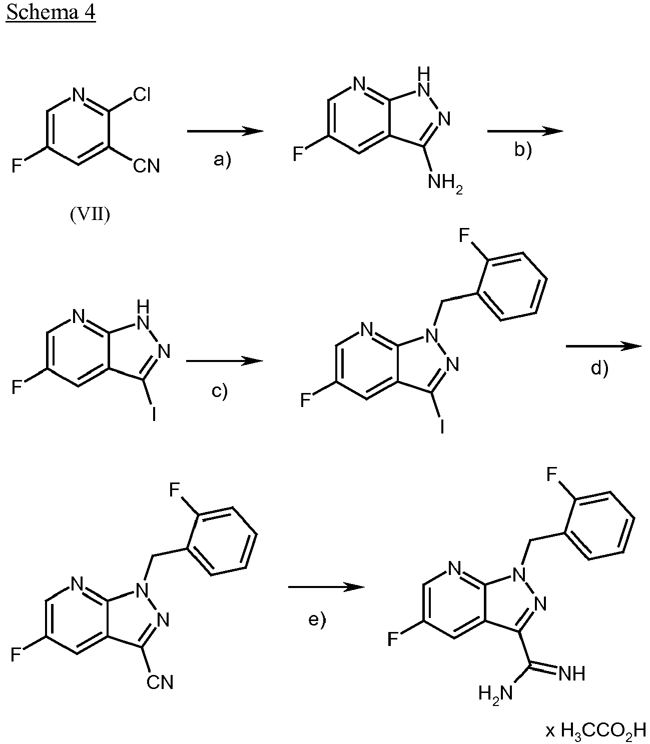 [a): Hydrazinhydrat, 1,2-Ethandiol; b): iso-Pentylnitrit, NaI, THF; b): 2-Fluorbenzylbromid, Cs₂CO₃, DMF; d): CuCN, DMSO, e): 1. NaOMe, MeOH, 2. NH₄Cl, Essigsäure]. 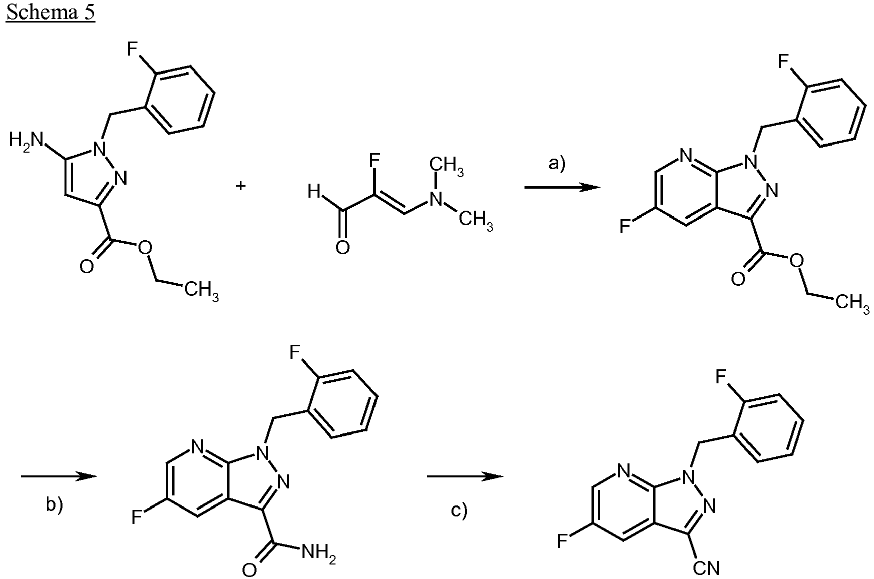 [a): TFA, Dioxan; b) NH₃; c) Trifluoressigsäureanhydrid]. 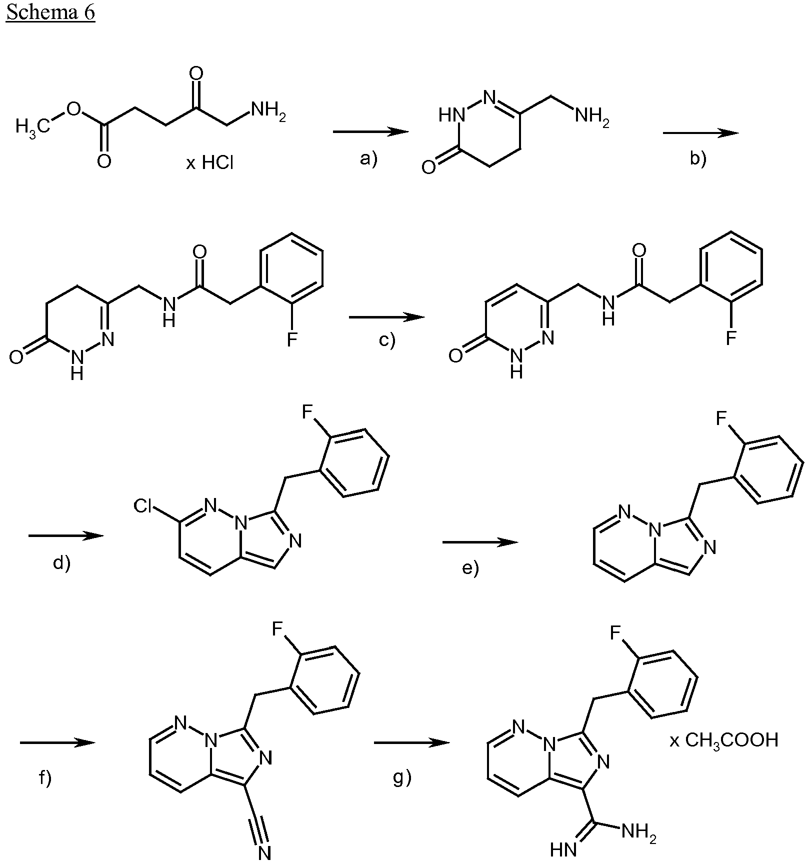 [a): Hydrazinhydrat, Triethylamin, Ethanol, Rückfluss; b): (2-Fluorphenyl)acetylchlorid, Triethylamin, Acetonitril; c): Brom, Essigsäure, 50°C; d): Phosphoroxychlorid, Sulfolan, 100°C; e): Palladium auf Kohle (5%), Triethylamin, Wasserstoff, Essigsäureethylester; f): 1. N-Bromsuccinimid, Dichlormethan; 2. Kupfer-(I)-cyanid, DMSO, 170°C; g): 1. Natriummethanolat, Methanol, 2. NH₄Cl, Essigsäure, Rückfluss].

Die Verbindung der Formel (VII) ist literaturbekannt [vgl. z.B. Winn M., J. Med. Chem. 1993, 36, 2676-7688; EP 634 413-A1; CN 1613849-A; EP 1626045-A1; WO 2009/018415], kann in Analogie zu literaturbekannten Verfahren oder wie im nachstehenden Syntheseschema gezeigt (Schema 7) hergestellt werden: 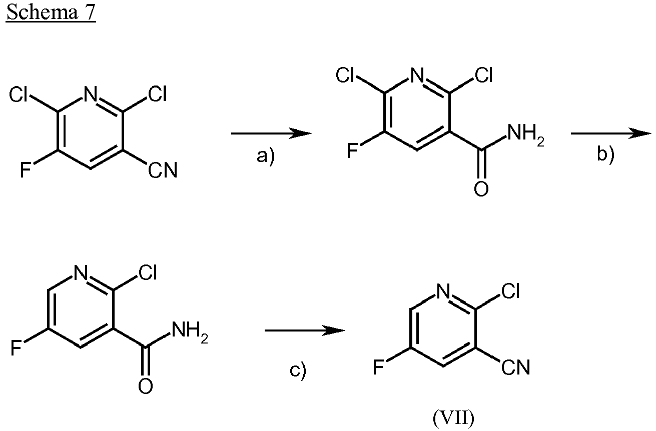 [a): Schwefelsäure; b): Zink, Methanol, Eisessig; c): Trifluoressigsäureanhydrid, Dichlormethan].

Die Verbindungen der Formel (IV) und (VI) sind kommerziell erhältlich, literaturbekannt oder können in Analogie zu literaturbekannten Verfahren hergestellt werden.

Die erfindungsgemäßen Verbindungen wirken als potente Stimulatoren der löslichen Guanylatcyclase und Inhibitoren von Phosphodiesterase-5, besitzen wertvolle pharmakologische Eigenschaften, und weisen ein verbessertes therapeutisches Profil auf, wie beispielsweise hinsichtlich ihrer *in-vivo* Eigenschaften und/oder ihres pharmakokinetischen Verhaltens und/oder metabolischen Profils. Sie eignen sich daher zur Behandlung und/ oder Prophylaxe von Erkrankungen bei Menschen und Tieren.

Die erfindungsgemäßen Verbindungen bewirken eine Gefäßrelaxation und eine Hemmung der Thrombozytenaggregation und führen zu einer Blutdrucksenkung sowie zu einer Steigerung des koronaren Blutflusses. Diese Wirkungen sind über eine direkte Stimulation der löslichen Guanylatcyclase und einen intrazellulären cGMP-Anstieg vermittelt. Außerdem verstärken die erfindungsgemäßen Verbindungen die Wirkung von Substanzen, die den cGMP-Spiegel steigern, wie beispielsweise EDRF (endothelium-derived relaxing factor), NO-Donatoren, Protoporphyrin IX, Arachidonsäure oder Phenylhydrazin-Derivate.

Die erfindungsgemäßen Verbindungen eignen sich zur Behandlung und/oder Prophylaxe von kardiovaskulären, pulmonalen, thromboembolischen und fibrotischen Erkrankungen.

Die erfindungsgemäßen Verbindungen können daher in Arzneimitteln zur Behandlung und/oder Prophylaxe von kardiovaskulären Erkrankungen wie beispielsweise Bluthochdruck (Hypertonie), resistente Hypertonie, akute und chronische Herzinsuffizienz, koronare Herzerkrankung, stabile und instabile Angina pectoris, periphere und kardiale Gefäßerkrankungen, Arrhythmien, Rhythmusstörungen der Vorhöfe und der Kammern sowie Überleitungsstörungen wie beispielsweise atrio-ventrikuläre Blockaden Grad I-III (AB-Block I-III), supraventrikuläre Tachyarrhythmie, Vorhofflimmern, Vorhoffflattern, Kammerflimmern, Kammerflattern, ventrikuläre Tachyarrhytmie, Torsade de pointes-Tachykardie, Extrasystolen des Vorhoffs und des Ventrikels, AV-junktionale Extrasystolen, Sick-Sinus Syndrom, Synkopen, AV-Knoten-Reentrytachykardie, Wolff-Parkinson-White-Syndrom, von akutem Koronarsyndrom (ACS), autoimmune Herzerkrankungen (Perikarditis, Endokarditis, Valvolitis, Aortitis, Kardiomyopathien), Schock wie kardiogenem Schock, septischem Schock und anaphylaktischem Schock, Aneurysmen, Boxerkardiomyopathie (premature ventricular contraction (PVC)), zur Behandlung und/oder Prophylaxe von thromboembolischen Erkrankungen und Ischämien wie myokardiale Ischämie, Myokardinfarkt, Hirnschlag, Herzhypertrophie, transistorischen und ischämischen Attacken, Präeklampsie, entzündliche kardiovaskuläre Erkrankungen, Spasmen der Koronararterien und peripherer Arterien, Ödembildung wie beispielsweise pulmonales Ödem, Hirnödem, renales Ödem oder Herzinsuffizienz-bedingtes Ödem, peripheren Durchblutungsstörungen, Reperfusionsschäden, arterielle und venöse Thrombosen, Mikroalbuminurie, Herzmuskelschwäche, endotheliale Dysfunktion, zur Verhinderung von Restenosen wie nach Thrombolysetherapien, percutan-transluminalen Angioplastien (PTA), transluminalen Koronarangioplastien (PTCA), Herztransplantationen und Bypass-Operationen, sowie mikro- und makrovaskuläre Schädigungen (Vasculitis), erhöhte Spiegel von Fibrinogen und von LDL geringer Dichte sowie erhöhte Konzentrationen von Plasminogenaktivator-Inhibitor 1 (PAI-1), sowie zur Behandlung und/oder Prophylaxe von erektiler Dysfunktion und weiblicher sexueller Dysfunktion eingesetzt werden.

Im Sinne der vorliegenden Erfindung umfasst der Begriff Herzinsuffizienz sowohl akute als auch chronische Erscheinungsformen der Herzinsuffizienz, wie auch spezifischere oder verwandte Krankheitsformen wie akut dekompensierte Herzinsuffizienz, Rechtsherzinsuffizienz, Linksherzinsuffizienz, Globalinsuffizienz, ischämische Kardiomyopathie, dilatative Kardiomyopathie, hypertrophe Kardiomyopathie, idiopathische Kardiomyopathie, angeborene Herzfehler, Herzinsuffizienz bei Herzklappenfehlern, Mitralklappenstenose, Mitralklappeninsuffizienz, Aortenklappenstenose, Aortenklappeninsuffizienz, Trikuspidalstenose, Trikuspidalinsuffizienz, Pulmonalklappenstenose, Pulmonalklappeninsuffizienz, kombinierte Herzklappenfehler, Herzmuskelentzündung (Myokarditis), chronische Myokarditis, akute Myokarditis, virale Myokarditis, diabetische Herzinsuffizienz, alkoholtoxische Kardiomyopathie, kardiale Speichererkrankungen, diastolische Herzinsuffizienz sowie systolische Herzinsuffizienz und akute Phasen der Verschlechterung einer bestehenden chronischen Herzinsuffizienz (worsening heart failure).

Darüber hinaus können die erfindungsgemäßen Verbindungen auch zur Behandlung und/oder Prophylaxe von Arteriosklerose, Lipidstoffwechselstörungen, Hypolipoproteinämien, Dyslipidämien, Hypertriglyceridämien, Hyperlipidämien, Hypercholesterolämien, Abetelipoproteinämie, Sitosterolämie, Xanthomatose, Tangier Krankheit, Fettsucht (Adipositas), Fettleibigkeit (Obesitas) und von kombinierten Hyperlipidämien sowie des Metabolischen Syndroms eingesetzt werden.

Außerdem können die erfindungsgemäßen Verbindungen zur Behandlung und/oder Prophylaxe von primärem und sekundärem Raynaud-Phänomen, von Mikrozirkulationsstörungen, Claudicatio, peripheren und autonomen Neuropathien, diabetischen Mikroangiopathien, diabetischer Retinopathie, diabetischen Geschwüren an den Extremitäten, Gangren, CREST-Syndrom, Erythematose, Onychomykose, rheumatischen Erkrankungen sowie zur Förderung der Wundheilung verwendet werden.

Weiterhin eignen sich die erfindungsgemäßen Verbindungen zur Behandlung urologischer Erkrankungen wie beispielsweise benignes Prostata-Syndrom (BPS), benigne Prostata-Hyperplasie (BPH), benigne Prostata Vergrösserung (BPE), Blasenentleerungsstörung (BOO), untere Harnwegssyndrome (LUTS, einschließlich Felines Urologisches Syndrom (FUS)), Erkrankungen des Urogenital-Systems einschliesslich neurogene überaktive Blase (OAB) und (IC), Inkontinenz (UI) wie beispielsweise Misch-, Drang-, Stress-, oder Überlauf-Inkontinenz (MUI, UUI, SUI, OUI), Beckenschmerzen, benigne und maligne Erkrankungen der Organe des männlichen und weiblichen Urogenital-Systems.

Weiterhin eignen sich die erfindungsgemäßen Verbindungen zur Behandlung und/oder Prophylaxe von Nierenerkrankungen, insbesondere von aktuer und chronischer Niereninsuffizienz, sowie von akutem und chronischem Nierenversagen. Im Sinne der vorliegenden Erfindung umfasst der Begriff Niereninsuffizienz sowohl akute als auch chronische Erscheinungsformen der Niereninsuffizienz, wie auch zugrundeliegende oder verwandte Nierenerkrankungen wie renale Hypoperfusion, intradialytische Hypotonie, obstruktive Uropathie, Glomerulopathien, Glomerulonephritis, akute Glomerulonephritis, Glomerulosklerose, tubulointerstitielle Erkrankungen, nephropathische Erkrankungen wie primäre und angeborene Nierenerkrankung, Nierenentzündung, immunologische Nierenerkrankungen wie Nierentransplantatabstoßung, Immunkomplex-induzierte Nierenerkrankungen, durch toxische Substanzen induzierte Nephropathie, Kontrastmittel-induzierte Nephropathie, diabetische und nicht-diabetische Nephropathie, Pyelonephritis, Nierenzysten, Nephrosklerose, hypertensive Nephrosklerose und nephrotisches Syndrom, welche diagnostisch beispielsweise durch abnorm verminderte Kreatinin- und/oder Wasser-Ausscheidung, abnorm erhöhte Blutkonzentrationen von Harnstoff, Stickstoff, Kalium und/oder Kreatinin, veränderte Aktivität von Nierenenzymen wie z.B. Glutamylsynthetase, veränderte Urinosmolarität oder Urinmenge, erhöhte Mikroalbuminurie, Makroalbuminurie, Läsionen an Glomerula und Arteriolen, tubuläre Dilatation, Hyperphosphatämie und/oder die Notwendigkeit zur Dialyse charakterisiert werden können. Die vorliegende Erfindung umfasst auch die Verwendung der erfindungsgemäßen Verbindungen zur Behandlung und/oder Prophylaxe von Folgeerscheinungen einer Niereninsuffizienz, wie beispielsweise Lungenödem, Herzinsuffizienz, Urämie, Anämie, Elektrolytstörungen (z.B. Hyperkalämie, Hyponaträmie) und Störungen im Knochen- und Kohlenhydrat-Metabolismus.

Weiterhin eignen sich die erfindungsgemäßen Verbindungen auch zur Behandlung und/oder Prophylaxe von asthmatischen Erkrankungen, pulmonaler arterieller Hypertonie (PAH) und anderen Formen der pulmonalen Hypertonie (PH), umfassend mit Linksherzerkrankung, HIV, Sichelzellanämie, Thromboembolien (CTEPH), Sarkoidose, COPD oder Lungenfibrose assoziierte pulmonale Hypertonie, der chronisch-obstruktive Lungenerkrankung (COPD), des akuten Atemwegssyndrom (ARDS), der akuten Lungenschädigung (ALI), der alpha-1-Antitrypsin-Defizienz (AATD), der Lungenfibrose, des Lungenemphysem (z.B. durch Zigarettenrauch induziertes Lungenemphysem) und der zystischen Fibrose (CF).

Die in der vorliegenden Erfindung beschriebenen Verbindungen stellen auch Wirkstoffe zur Bekämpfung von Krankheiten im Zentralnervensystem dar, die durch Störungen des NO/cGMP-Systems gekennzeichnet sind. Insbesondere sind sie geeignet zur Verbesserung der Wahrnehmung, Konzentrationsleistung, Lernleistung oder Gedächtnisleistung nach kognitiven Störungen, wie sie insbesondere bei Situationen/Krankheiten/Syndromen auftreten wie "Mild cognitive impairment", altersassoziierten Lern- und Gedächtnisstörungen, altersassoziierten Gedächtnisverlusten, vaskulärer Demenz, Schädel-Hirn-Trauma, Schlaganfall, Demenz, die nach Schlaganfällen auftritt ("post stroke dementia"), post-traumatischem Schädel-Hirn-Trauma, allgemeinen Konzentrationsstörungen, Konzentrationsstörungen bei Kindern mit Lern- und Gedächtnisproblemen, Alzheimer'scher Krankheit, Demenz mit Lewy-Körperchen, Demenz mit Degeneration der Frontallappen einschliesslich des Pick's-Syndroms, Parkinson'scher Krankheit, progressiver nuclear palsy, Demenz mit corticobasaler Degeneration, Amyolateralsklerose (ALS), Huntington'scher Krankheit, Demyelinisation, Multipler Sklerose, Thalamischer Degeneration, Creutzfeld-Jacob-Demenz, HIV-Demenz, Schizophrenie mit Demenz oder Korsakoff-Psychose. Sie eignen sich auch zur Behandlung und/oder Prophylaxe von Erkrankungen des Zentralnervensystems wie Angst-, Spannungs- und Depressionszuständen, zentral-nervös bedingten Sexualdysfunktionen und Schlafstörungen sowie zur Regulierung krankhafter Störungen der Nahrungs-, Genuss- und Suchtmittelaufnahme.

Weiterhin eignen sich die erfindungsgemäßen Verbindungen auch zur Regulation der cerebralen Durchblutung und stellen wirkungsvolle Mittel zur Bekämpfung von Migräne dar. Auch eignen sie sich zur Prophylaxe und Bekämpfung der Folgen cerebraler Infarktgeschehen (Apoplexia cerebri) wie Schlaganfall, cerebraler Ischämien und des Schädel-Hirn-Traumas. Ebenso können die erfindungsgemäßen Verbindungen zur Bekämpfung von Schmerzzuständen und Tinnitus eingesetzt werden.

Zudem besitzen die erfindungsgemäßen Verbindungen antiinflammatorische Wirkung und können daher als entzündungshemmende Mittel zur Behandlung und/oder Prophylaxe von Sepsis (SIRS), multiplem Organversagen (MODS, MOF), entzündlichen Erkrankungen der Niere, chronischen Darmentzündungen (IBD, Crohn's Disease, UC), Pankreatitis, Peritonitis, rheumatoiden Erkrankungen, entzündlichen Hauterkrankungen sowie entzündlichen Augenerkrankungen eingesetzt werden.

Des weiteren können die erfindungsgemäßen Verbindungen ebenfalls zur Behandlung und/ oder Prophylaxe von Autoimmunerkrankungen eingesetzt werden.

Weiterhin sind die erfindungsgemäßen Verbindungen zur Behandlung und/oder Prophylaxe fibrotischer Erkrankungen der inneren Organe, wie beispielsweise der Lunge, des Herzens, der Niere, des Knochenmarks und insbesondere der Leber, sowie dermatologischer Fibrosen und fibrotischer Erkrankungen des Auges, geeignet. Im Sinne der vorliegenden Erfindungen umfasst der Begriff fibrotischer Erkrankungen insbesondere die folgenden Begriffe Leberfibrose, Leberzirrhose, Lungenfibrose, Endomyocardfibrose, Nephropathie, Glomerulonephritis, interstitielle Nierenfibrose, fibrotische Schäden in Folge von Diabetes, Knochenmarksfibrose und ähnliche fibrotische Erkrankungen, Sklerodermie, Morphaea, Keloide, hypertrophe Narbenbildung (auch nach chirurgischen Eingriffen), Naevi, diabetische Retinopathie, proliferative Vitroretinopathie und Erkrankungen des Bindegewebes (z.B. Sarkoidose).

Weiterhin eignen sich die erfindungsgemäßen Verbindungen zur Bekämpfung postoperativer Narbenbildung, z.B. in Folge von Glaukom-Operationen.

Die erfindungsgemäßen Verbindungen können ebenfalls kosmetisch bei alternder und verhornender Haut eingesetzt werden.

Außerdem sind die erfindungsgemäßen Verbindungen zur Behandlung und/ oder Prophylaxe von Hepatitis, Neoplasma, Osteoporose, Glaukom und Gastroparese geeignet.

Weiterer Gegenstand der vorliegenden Erfindung ist die Verwendung der erfindungsgemäßen Verbindungen zur Behandlung und/oder Prophylaxe von Erkrankungen, insbesondere der zuvor genannten Erkrankungen.

Weiterer Gegenstand der vorliegenden Erfindung ist die Verwendung der erfindungsgemäßen Verbindungen zur Behandlung und/oder Prophylaxe von Herzinsuffizienz, Angina pectoris, Hypertonie, pulmonaler Hypertonie, Ischämien, Gefäßerkrankungen, Niereninsuffizienz, thromboembolischen Erkrankungen, fibrotischen Erkrankungen und Arteriosklerose.

Weiterer Gegenstand der vorliegenden Erfindung sind die erfindungsgemäßen Verbindungen zur Verwendung in einem Verfahren zur Behandlung und/ oder Prophylaxe von Herzinsuffizienz, Angina pectoris, Hypertonie, pulmonaler Hypertonie, Ischämien, Gefäßerkrankungen, Niereninsuffizienz, thromboembolischen Erkrankungen, fibrotischen Erkrankungen und Arteriosklerose.

Weiterer Gegenstand der vorliegenden Erfindung ist die Verwendung der erfindungsgemäßen Verbindungen zur Herstellung eines Arzneimittels zur Behandlung und/oder Prophylaxe von Erkrankungen, insbesondere der zuvor genannten Erkrankungen.

Weiterer Gegenstand der vorliegenden Erfindung ist die Verwendung der erfindungsgemäßen Verbindungen zur Herstellung eines Arzneimittels zur Behandlung und/oder Prophylaxe von Herzinsuffizienz, Angina pectoris, Hypertonie, pulmonaler Hypertonie, Ischämien, Gefäßerkrankungen, Niereninsuffizienz, thromboembolischen Erkrankungen, fibrotischen Erkrankungen und Arteriosklerose.

Die erfindungsgemäßen Verbindungen können allein oder bei Bedarf in Kombination mit anderen Wirkstoffen eingesetzt werden. Weiterer Gegenstand der vorliegenden Erfindung sind Arzneimittel, enthaltend mindestens eine der erfindungsgemäßen Verbindungen und einen oder mehrere weitere Wirkstoffe, insbesondere zur Behandlung und/oder Prophylaxe der zuvor genannten Erkrankungen. Als geeignete Kombinationswirkstoffe seien beispielhaft und vorzugsweise genannt:
- organische Nitrate und NO-Donatoren, wie beispielsweise Natriumnitroprussid, Nitroglycerin, Isosorbidmononitrat, Isosorbiddinitrat, Molsidomin oder SIN-1, sowie inhalatives NO;
- Verbindungen, die den Abbau von cyclischem Guanosinmonophosphat (cGMP) inhibieren, wie beispielsweise Inhibitoren der Phosphodiesterasen (PDE) 1, 2 und/oder 5, insbesondere PDE 5-Inhibitoren wie Sildenafil, Vardenafil und Tadalafil;
- antithrombotisch wirkende Mittel, beispielhaft und vorzugsweise aus der Gruppe der Thrombozytenaggregationshemmer, der Antikoagulantien oder der profibrinolytischen Substanzen;
- den Blutdruck senkende Wirkstoffe, beispielhaft und vorzugsweise aus der Gruppe der Calcium-Antagonisten, Angiotensin AII-Antagonisten, ACE-Hemmer, Endothelin-Antagonisten, Renin-Inhibitoren, alpha-Rezeptoren-Blocker, beta-Rezeptoren-Blocker, Mineralocorticoid-Rezeptor-Antagonisten sowie der Diuretika; und/oder
- den Fettstoffwechsel verändernde Wirkstoffe, beispielhaft und vorzugsweise aus der Gruppe der Thyroidrezeptor-Agonisten, Cholesterinsynthese-Inhibitoren wie beispielhaft und vorzugsweise HMG-CoA-Reduktase- oder Squalensynthese-Inhibitoren, der ACAT-Inhibitoren, CETP-Inhibitoren, MTP-Inhibitoren, PPAR-alpha-, PPAR-gamma- und/oder PPAR-delta-Agonisten, Cholesterin-Absorptionshemmer, Lipase-Inhibitoren, polymeren Gallensäureadsorber, Gallensäure-Reabsorptionshemmer und Lipoprotein(a)-Antagonisten.

Unter antithrombotisch wirkenden Mittel werden vorzugsweise Verbindungen aus der Gruppe der Thrombozytenaggregationshemmer, der Antikoagulantien oder der profibrinolytischen Substanzen verstanden.

Bei einer bevorzugten Ausführungsform der Erfindung werden die erfindungsgemäßen Verbindungen in Kombination mit einem Thrombozytenaggregationshemmer, wie beispielhaft und vorzugsweise Aspirin, Clopidogrel, Ticlopidin oder Dipyridamol, verabreicht.

Bei einer bevorzugten Ausführungsform der Erfindung werden die erfindungsgemäßen Verbindungen in Kombination mit einem Thrombin-Inhibitor, wie beispielhaft und vorzugsweise Ximelagatran, Dabigatran, Melagatran, Bivalirudin oder Clexane, verabreicht.

Bei einer bevorzugten Ausführungsform der Erfindung werden die erfindungsgemäßen Verbindungen in Kombination mit einem GPIIb/IIIa-Antagonisten, wie beispielhaft und vorzugsweise Tirofiban oder Abciximab, verabreicht.

Bei einer bevorzugten Ausführungsform der Erfindung werden die erfindungsgemäßen Verbindungen in Kombination mit einem Faktor Xa-Inhibitor, wie beispielhaft und vorzugsweise Rivaroxaban (BAY 59-7939), DU-176b, Apixaban, Otamixaban, Fidexaban, Razaxaban, Fondaparinux, Idraparinux, PMD-3112, YM-150, KFA-1982, EMD-503982, MCM-17, MLN-1021, DX 9065a, DPC 906, JTV 803, SSR-126512 oder SSR-128428, verabreicht.

Bei einer bevorzugten Ausführungsform der Erfindung werden die erfindungsgemäßen Verbindungen in Kombination mit Heparin oder einem low molecular weight (LMW)-Heparin-Derivat verabreicht.

Bei einer bevorzugten Ausführungsform der Erfindung werden die erfindungsgemäßen Verbindungen in Kombination mit einem Vitamin K-Antagonisten, wie beispielhaft und vorzugsweise Coumarin, verabreicht.

Unter den Blutdruck senkenden Mitteln werden vorzugsweise Verbindungen aus der Gruppe der Calcium-Antagonisten, Angiotensin AII-Antagonisten, ACE-Hemmer, Endothelin-Antagonisten, Renin-Inhibitoren, alpha-Rezeptoren-Blocker, beta-Rezeptoren-Blocker, Mineralocorticoid-Rezeptor-Antagonisten sowie der Diuretika verstanden.

Bei einer bevorzugten Ausführungsform der Erfindung werden die erfindungsgemäßen Verbindungen in Kombination mit einem Calcium-Antagonisten, wie beispielhaft und vorzugsweise Nifedipin, Amlodipin, Verapamil oder Diltiazem, verabreicht.

Bei einer bevorzugten Ausführungsform der Erfindung werden die erfindungsgemäßen Verbindungen in Kombination mit einem alpha-1-Rezeptoren-Blocker, wie beispielhaft und vorzugsweise Prazosin, verabreicht.

Bei einer bevorzugten Ausführungsform der Erfindung werden die erfindungsgemäßen Verbindungen in Kombination mit einem beta-Rezeptoren-Blocker, wie beispielhaft und vorzugsweise Propranolol, Atenolol, Timolol, Pindolol, Alprenolol, Oxprenolol, Penbutolol, Bupranolol, Metipranolol, Nadolol, Mepindolol, Carazalol, Sotalol, Metoprolol, Betaxolol, Celiprolol, Bisoprolol, Carteolol, Esmolol, Labetalol, Carvedilol, Adaprolol, Landiolol, Nebivolol, Epanolol oder Bucindolol, verabreicht.

Bei einer bevorzugten Ausführungsform der Erfindung werden die erfindungsgemäßen Verbindungen in Kombination mit einem Angiotensin AII-Antagonisten, wie beispielhaft und vorzugsweise Losartan, Candesartan, Valsartan, Telmisartan oder Embursatan, verabreicht.

Bei einer bevorzugten Ausführungsform der Erfindung werden die erfindungsgemäßen Verbindungen in Kombination mit einem ACE-Hemmer, wie beispielhaft und vorzugsweise Enalapril, Captopril, Lisinopril, Ramipril, Delapril, Fosinopril, Quinopril, Perindopril oder Trandopril, verabreicht.

Bei einer bevorzugten Ausführungsform der Erfindung werden die erfindungsgemäßen Verbindungen in Kombination mit einem Endothelin-Antagonisten, wie beispielhaft und vorzugsweise Bosentan, Darusentan, Ambrisentan oder Sitaxsentan, verabreicht.

Bei einer bevorzugten Ausführungsform der Erfindung werden die erfindungsgemäßen Verbindungen in Kombination mit einem Renin-Inhibitor, wie beispielhaft und vorzugsweise Aliskiren, SPP-600 oder SPP-800, verabreicht.

Bei einer bevorzugten Ausführungsform der Erfindung werden die erfindungsgemäßen Verbindungen in Kombination mit einem Mineralocorticoid-Rezeptor-Antagonisten, wie beispielhaft und vorzugsweise Spironolacton oder Eplerenon, verabreicht.

Bei einer bevorzugten Ausführungsform der Erfindung werden die erfindungsgemäßen Verbindungen in Kombination mit einem Schleifendiuretikum, wie beispielsweise Furosemid, Torasemid, Bumetanid und Piretanid, mit kaliumsparenden Diuretika wie beispielsweise Amilorid und Triamteren, mit Aldosteronantagonisten, wie beispielsweise Spironolacton, Kaliumcanrenoat und Eplerenon sowie Thiaziddiuretika, wie beispielsweise Hydrochlorothiazid, Chlorthalidon, Xipamid, und Indapamid, verabreicht.

Unter den Fettstoffwechsel verändernden Mitteln werden vorzugsweise Verbindungen aus der Gruppe der CETP-Inhibitoren, Thyroidrezeptor-Agonisten, Cholesterinsynthese-Inhibitoren wie HMG-CoA-Reduktase- oder Squalensynthese-Inhibitoren, der ACAT-Inhibitoren, MTP-Inhibitoren, PPAR-alpha-, PPAR-gamma- und/oder PPAR-delta-Agonisten, Cholesterin-Absorptionshemmer, polymeren Gallensäureadsorber, Gallensäure-Reabsorptionshemmer, Lipase-Inhibitoren sowie der Lipoprotein(a)-Antagonisten verstanden.

Bei einer bevorzugten Ausführungsform der Erfindung werden die erfindungsgemäßen Verbindungen in Kombination mit einem CETP-Inhibitor, wie beispielhaft und vorzugsweise Dalcetrapib, BAY 60-5521, Anacetrapib oder CETP-vaccine (CETi-1), verabreicht.

Bei einer bevorzugten Ausführungsform der Erfindung werden die erfindungsgemäßen Verbindungen in Kombination mit einem Thyroidrezeptor-Agonisten, wie beispielhaft und vorzugsweise D-Thyroxin, 3,5,3'-Trüodothyronin (T3), CGS 23425 oder Axitirome (CGS 26214), verabreicht.

Bei einer bevorzugten Ausführungsform der Erfindung werden die erfindungsgemäßen Verbindungen in Kombination mit einem HMG-CoA-Reduktase-Inhibitor aus der Klasse der Statine, wie beispielhaft und vorzugsweise Lovastatin, Simvastatin, Pravastatin, Fluvastatin, Atorvastatin, Rosuvastatin oder Pitavastatin, verabreicht.

Bei einer bevorzugten Ausführungsform der Erfindung werden die erfindungsgemäßen Verbindungen in Kombination mit einem Squalensynthese-Inhibitor, wie beispielhaft und vorzugsweise BMS-188494 oder TAK-475, verabreicht.

Bei einer bevorzugten Ausführungsform der Erfindung werden die erfindungsgemäßen Verbindungen in Kombination mit einem ACAT-Inhibitor, wie beispielhaft und vorzugsweise Avasimibe, Melinamide, Pactimibe, Eflucimibe oder SMP-797, verabreicht.

Bei einer bevorzugten Ausführungsform der Erfindung werden die erfindungsgemäßen Verbindungen in Kombination mit einem MTP-Inhibitor, wie beispielhaft und vorzugsweise Implitapide, BMS-201038, R-103757 oder JTT-130, verabreicht.

Bei einer bevorzugten Ausführungsform der Erfindung werden die erfindungsgemäßen Verbindungen in Kombination mit einem PPAR-gamma-Agonisten, wie beispielhaft und vorzugsweise Pioglitazone oder Rosiglitazone, verabreicht.

Bei einer bevorzugten Ausführungsform der Erfindung werden die erfindungsgemäßen Verbindungen in Kombination mit einem PPAR-delta-Agonisten, wie beispielhaft und vorzugsweise GW 501516 oder BAY 68-5042, verabreicht.

Bei einer bevorzugten Ausführungsform der Erfindung werden die erfindungsgemäßen Verbindungen in Kombination mit einem Cholesterin-Absorptionshemmer, wie beispielhaft und vorzugsweise Ezetimibe, Tiqueside oder Pamaqueside, verabreicht.

Bei einer bevorzugten Ausführungsform der Erfindung werden die erfindungsgemäßen Verbindungen in Kombination mit einem Lipase-Inhibitor, wie beispielhaft und vorzugsweise Orlistat, verabreicht.

Bei einer bevorzugten Ausführungsform der Erfindung werden die erfindungsgemäßen Verbindungen in Kombination mit einem polymeren Gallensäureadsorber, wie beispielhaft und vorzugsweise Cholestyramin, Colestipol, Colesolvam, CholestaGel oder Colestimid, verabreicht.

Bei einer bevorzugten Ausführungsform der Erfindung werden die erfindungsgemäßen Verbindungen in Kombination mit einem Gallensäure-Reabsorptionshemmer, wie beispielhaft und vorzugsweise ASBT (= IBAT)-Inhibitoren wie z.B. AZD-7806, S-8921, AK-105, BARI-1741, SC-435 oder SC-635, verabreicht.

Bei einer bevorzugten Ausführungsform der Erfindung werden die erfindungsgemäßen Verbindungen in Kombination mit einem Lipoprotein(a)-Antagonisten, wie beispielhaft und vorzugsweise Gemcabene calcium (CI-1027) oder Nicotinsäure, verabreicht.

Weiterer Gegenstand der vorliegenden Erfindung sind Arzneimittel, die mindestens eine erfindungsgemäße Verbindung, üblicherweise zusammen mit einem oder mehreren inerten, nichttoxischen, pharmazeutisch geeigneten Hilfsstoffen enthalten, sowie deren Verwendung zu den zuvor genannten Zwecken.

Die erfindungsgemäßen Verbindungen können systemisch und/oder lokal wirken. Zu diesem Zweck können sie auf geeignete Weise appliziert werden, wie z.B. oral, parenteral, pulmonal, nasal, sublingual, lingual, buccal, rectal, dermal, transdermal, conjunctival, otisch oder als Implantat bzw. Stent.

Für diese Applikationswege können die erfindungsgemäßen Verbindungen in geeigneten Applikationsformen verabreicht werden.

Für die orale Applikation eignen sich nach dem Stand der Technik funktionierende, die erfindungsgemäßen Verbindungen schnell und/oder modifiziert abgebende Applikationsformen, die die erfindungsgemäßen Verbindungen in kristalliner und/oder amorphisierter und/oder gelöster Form enthalten, wie z.B. Tabletten (nicht-überzogene oder überzogene Tabletten, beispielsweise mit magensaftresistenten oder sich verzögert auflösenden oder unlöslichen Überzügen, die die Freisetzung der erfindungsgemäßen Verbindung kontrollieren), in der Mundhöhle schnell zerfallende Tabletten oder Filme/Oblaten, Filme/Lyophylisate, Kapseln (beispielsweise Hart- oder Weichgelatinekapseln), Dragees, Granulate, Pellets, Pulver, Emulsionen, Suspensionen, Aerosole oder Lösungen.

Die parenterale Applikation kann unter Umgehung eines Resorptionsschrittes geschehen (z.B. intravenös, intraarteriell, intrakardial, intraspinal oder intralumbal) oder unter Einschaltung einer Resorption (z.B. intramuskulär, subcutan, intracutan, percutan oder intraperitoneal). Für die parenterale Applikation eignen sich als Applikationsformen u.a. Injektions- und Infusionszubereitungen in Form von Lösungen, Suspensionen, Emulsionen, Lyophilisaten oder sterilen Pulvern.

Für die sonstigen Applikationswege eignen sich z.B. Inhalationsarzneiformen (u.a. Pulverinhalatoren, Nebulizer), Nasentropfen, -lösungen oder -sprays, lingual, sublingual oder buccal zu applizierende Tabletten, Filme/Oblaten oder Kapseln, Suppositorien, Ohren- oder Augenpräparationen, Vaginalkapseln, wäßrige Suspensionen (Lotionen, Schüttelmixturen), lipophile Suspensionen, Salben, Cremes, transdermale therapeutische Systeme (z.B. Pflaster), Milch, Pasten, Schäume, Streupuder, Implantate oder Stents.

Bevorzugt sind die orale oder parenterale Applikation, insbesondere die orale Applikation.

Die erfindungsgemäßen Verbindungen können in die angeführten Applikationsformen überführt werden. Dies kann in an sich bekannter Weise durch Mischen mit inerten, nichttoxischen, pharmazeutisch geeigneten Hilfsstoffen geschehen. Zu diesen Hilfsstoffen zählen u.a. Trägerstoffe (beispielsweise mikrokristalline Cellulose, Lactose, Mannitol), Lösungsmittel (z.B. flüssige Polyethylenglycole), Emulgatoren und Dispergier- oder Netzmittel (beispielsweise Natriumdodecylsulfat, Polyoxysorbitanoleat), Bindemittel (beispielsweise Polyvinylpyrrolidon), synthetische und natürliche Polymere (beispielsweise Albumin), Stabilisatoren (z.B. Antioxidantien wie beispielsweise Ascorbinsäure), Farbstoffe (z.B. anorganische Pigmente wie beispielsweise Eisenoxide) und Geschmacks- und/oder Geruchskorrigentien.

Im Allgemeinen hat es sich als vorteilhaft erwiesen, bei parenteraler Applikation Mengen von etwa 0.001 bis 1 mg/kg, vorzugsweise etwa 0.01 bis 0.5 mg/kg Körpergewicht zur Erzielung wirksamer Ergebnisse zu verabreichen. Bei oraler Applikation beträgt die Dosierung etwa 0.001 bis 2 mg/kg, vorzugsweise etwa 0.001 bis 1 mg/kg Körpergewicht.

Trotzdem kann es gegebenenfalls erforderlich sein, von den genannten Mengen abzuweichen, und zwar in Abhängigkeit von Körpergewicht, Applikationsweg, individuellem Verhalten gegenüber dem Wirkstoff, Art der Zubereitung und Zeitpunkt bzw. Intervall, zu welchem die Applikation erfolgt. So kann es in einigen Fällen ausreichend sein, mit weniger als der vorgenannten Mindestmenge auszukommen, während in anderen Fällen die genannte obere Grenze überschritten werden muss. Im Falle der Applikation größerer Mengen kann es empfehlenswert sein, diese in mehreren Einzelgaben über den Tag zu verteilen.

Die nachfolgenden Ausführungsbeispiele erläutern die Erfindung. Die Erfindung ist nicht auf die Beispiele beschränkt.

Die Prozentangaben in den folgenden Tests und Beispielen sind, sofern nicht anders angegeben, Gewichtsprozente; Teile sind Gewichtsteile. Lösungsmittelverhältnisse, Verdünnungsverhältnisse und Konzentrationsangaben von flüssig/flüssig-Lösungen beziehen sich jeweils auf das Volumen.

### A. Beispiele

### Abkürzungen und Akronyme:

- aq.: wässrige Lösung
- ber.: Berechnet
- DCI: direkte chemische Ionisation (bei MS)
- DMF: Dimethylformamid
- DMSO: Dimethylsulfoxid
- d. Th.: der Theorie (bei Ausbeute)
- eq.: Äquivalent(e)
- ESI: Elektrospray-Ionisation (bei MS)
- Et: Ethyl
- gef.: Gefunden
- h: Stunde(n)
- HPLC: Hochdruck-, Hochleistungsflüssigchromatographie
- HRMS: hochaufgelöste Massenspektrometrie
- konz.: konzentriert
- LC/MS: Flüssigchromatographie-gekoppelte Massenspektrometrie
- LiHMDS: Lithiumhexamethyldisilazid
- Me: Methyl
- min: Minute(n)
- MS: Massenspektrometrie
- NMR: Kernresonanzspektrometrie
- Pd/C: Palladium auf Aktivkohle (10%-ig)
- Ph: Phenyl
- qt: Quartett vom Triplett (NMR)
- RT: Raumtemperatur
- Rₜ: Retentionszeit (bei HPLC)
- t-Bu: tert.-Butyl
- TFA: Trifluoressigsäure
- THF: Tetrahydrofuran
- UV: Ultraviolett-Spektrometrie
- v/v: Volumen zu Volumen-Verhältnis (einer Lösung)
- XPHOS: Dicyclohexyl-(2',4',6'-trüsopropylbiphenyl-2-yl)-phosphin

### HPLC- und LC/MS-Methoden:

### Methode 1 (LC-MS):

Instrument: Waters ACQUITY SQD UPLC System; Säule: Waters Acquity UPLC HSS T3 1.8 µ 50 x 1 mm; Eluent A: 1 l Wasser + 0.25 ml 99%ige Ameisensäure , Eluent B: 1 l Acetonitril + 0.25 ml 99%ige Ameisensäure; Gradient: 0.0 min 90% A → 1.2 min 5% A → 2.0 min 5% A Ofen: 50 °C; Fluss: 0.40 ml/min; UV-Detektion: 210 - 400 nm.

### Methode 2 (LC-MS):

Gerätetyp MS: Waters ZQ; Gerätetyp HPLC: Agilent 1100 Series; UV DAD; Säule: Thermo Hypersil GOLD 3 µ 20 mm x 4 mm; Eluent A: 1 l Wasser + 0.5 ml 50%ige Ameisensäure, Eluent B: 1 l Acetonitril + 0.5 ml 50%ige Ameisensäure; Gradient: 0.0 min 100% A → 3.0 min 10% A → 4.0 min 10% A, Ofen: 55°C; Fluss 2ml/min; UV-Detektion: 210 nm.

### Methode 3 (LC-MS):

Instrument: Waters ACQUITY SQD UPLC System; Säule: Waters Acquity UPLC HSS T3 1.8 µ 30 x 2 mm; Eluent A: 1 l Wasser + 0.25 ml 99%ige Ameisensäure , Eluent B: 1 l Acetonitril + 0.25 ml 99%ige Ameisensäure; Gradient: 0.0 min 90% A → 1.2 min 5% A → 2.0 min 5% A Ofen: 50°C; Fluss: 0.60 ml/min; UV-Detektion: 208 - 400 nm.

### Methode 4 (LC-MS):

Instrument: Micromass Quattro Premier mit Waters UPLC Acquity; Säule: Thermo Hypersil GOLD 1.9 µ 50 x 1 mm; Eluent A: 1 l Wasser + 0.5 ml 50%ige Ameisensäure, Eluent B: 1 l Acetonitril + 0.5 ml 50%ige Ameisensäure; Gradient: 0.0 min 97% A → 0.5 min 97% A → 3.2 min 5% A → 4.0 min 5% A Ofen: 50°C; Fluss: 0.3 ml/min; UV-Detektion: 210 nm.

### Methode 5 (LC-MS):

Instrument MS: Waters SQD; Instrument HPLC: Waters UPLC; Säule: Zorbax SB-Aq (Agilent), 50 mm x 2.1 mm, 1.8 µm; Eluent A: Wasser + 0.025% Ameisensäure, Eluent B: Acetonitril (ULC) + 0.025% Ameisensäure; Gradient: 0.0 min 98%A - 0.9 min 25%A - 1.0 min 5%A - 1.4 min 5%A - 1.41 min 98%A - 1.5 min 98%A; Ofen: 40°C; Fluss: 0.600 ml/min; UV-Detektion: DAD; 210 nm

### Methode 6 (präp. HPLC):

Instrument MS: Waters, Instrument HPLC: Waters (Säule Phenomenex Luna 5µ C18(2) 100A, AXIA Tech. 50 x 21.2 mm, Eluent A: Wasser + 0.05% Ameisensäure, Eluent B: Methanol (ULC) + 0.05% Ameisensäure, mit Gradient, Fluss: 40 ml/min; UV-Detektion: DAD; 210 - 400 nm).

### Methode 7 (LC-MS):

Instrument MS: Waters (Micromass) Quattro Micro; Instrument HPLC: Agilent 1100 Serie; Säule : YMC-Triart C18 3 µ 50 x 3 mm; Eluent A: 1 l Wasser + 0.01 mol Ammoniumcarbonat, Eluent B: 1 l Acetonitril; Gradient: 0.0 min 100% A → 2.75 min 5% A → 4.5 min 5% A; Ofen: 40°C; Fluss: 1.25 ml/min; UV-Detektion: 210 nm.

### Methode 8 (LC-MS): Methode: MCW_SQ-HSST3_long

Instrument: Waters ACQUITY SQD UPLC System; Säule: Waters Acquity UPLC HSS T3 1.8 µ 50 x 1 mm; Eluent A: 1 l Wasser + 0.25 ml 99%ige Ameisensäure , Eluent B: 1 l Acetonitril + 0.25 ml 99%ige Ameisensäure; Gradient: 0.0 min 95% A → 6.0 min 5% A → 7.5 min 5% A Ofen: 50°C; Fluss: 0.35 ml/min; UV-Detektion: 210 - 400 nm.

### Ausgangsverbindungen und Intermediate:

### Beispiel 1A

### 1-(2-Fluorbenzyl)-1H-pyrazolo[3,4-b]pyridin-3-carboximidamid-Hydrochlorid

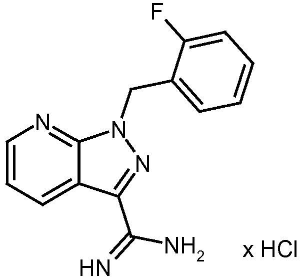

Die Synthese dieser Verbindung ist beschrieben in WO 03/095451, Beispiel 6A.

### Beispiel 2A

### 2,6-Dichlor-5-fluornicotinamid

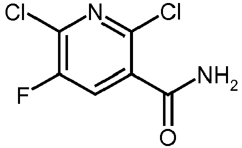

Eine Suspension aus 25 g (130.90 mmol) 2,6-Dichlor-5-fluor-3-cyanopyridin in konz. Schwefelsäure (125 ml) wurde 1 h bei 60-65°C gerührt. Nach Abkühlen auf RT wurde der Kolbeninhalt auf Eiswasser gegossen und dreimal mit Essigsäureethylester (je 100 ml) extrahiert. Die vereinigten organischen Phasen wurden mit Wasser (100 ml) und anschliessend mit gesättigter wässriger Natriumhydrogencarbonat-Lösung (100 ml) gewaschen, getrocknet und am Rotationsverdampfer eingeengt. Das erhaltene Material wurde am Hochvakuum getrocknet.
Ausbeute: 24.5 g (90% d. Theorie)
¹H-NMR (400 MHz, DMSO-d₆): δ = 7.95 (br s, 1H), 8.11 (br s, 1H), 8.24 (d, 1H).

### Beispiel 3A

### 2-Chlor-5-fluornicotinamid

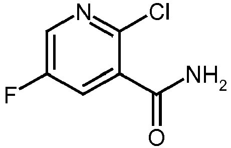

Zu einer Suspension von 21.9 g (335.35 mmol) Zink in Methanol (207 ml) wurden bei RT 44 g (210.58 mmol) 2,6-Dichlor-5-fluornicotinamid gegeben. Danach wurde mit Essigsäure (18.5 ml) versetzt und unter Rühren für 24 h zum Rückfluss erhitzt. Danach wurde der Kolbeninhalt vom Zink dekantiert und Essigsäureethylester (414 ml) sowie gesättigte wässrige Natriumhydrogencarbonat-Lösung(414 ml) zugegeben und intensiv ausgerührt. Anschliessend wurde über Kieselgur abgesaugt und dreimal mit Essigsäureethylester (je 517 ml) nachgewaschen. Die organische Phase wurde abgetrennt und die wässrige Phase mit Essigsäureethylester (258 ml) gewaschen. Die vereinigten organischen Phasen wurden einmal mit gesättigter wässriger Natriumhydrogencarbonat-Lösung (414 ml) gewaschen, getrocknet und im Vakuum eingeengt. Die so erhaltenen Kristalle wurden mit Dichlormethan (388 ml) versetzt und 20 min ausgerührt. Es wurde erneut abgesaugt und mit Diethylether nachgewaschen und trockengesaugt.
Ausbeute: 20.2 g (53% d. Theorie)
¹H-NMR (400 MHz, DMSO-d₆): δ = 7.87 (br s, 1H), 7.99 (dd, 1H), 8.10 (br s, 1H), 8.52 (d, 1H).

### Beispiel 4A

### 2-Chlor-5-fluornicotinonitril

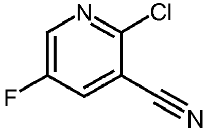

Eine Suspension von 46.2 g (264.66 mmol) 2-Chlor-5-fluornicotinamid in Dichlormethan (783 ml) wurde mit 81.2 ml (582.25 mmol) Triethylamin versetzt und auf 0°C gekühlt. Unter Rühren wurden dann 41.12 ml (291.13 mmol) Trifluoressigsäureanhydrid langsam zugetropft und 1.5 h bei 0°C nachgerührt. Die Reaktionslösung wurde danach zweimal mit gesättigter wässriger Natriumhydrogencarbonat-Lösung (je 391 ml) gewaschen, getrocknet und im Vakuum eingeengt.
Ausbeute: 42.1 g (90% d. Theorie).
¹H-NMR (400 MHz, DMSO-d₆): δ = 8.66 (dd, 1H), 8.82 (d, 1H).

### Beispiel 5A

### 5-Fluor-1H-pyrazolo[3,4-b]pyridin-3-amin

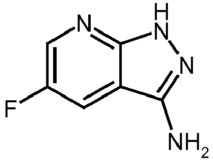

Eine Suspension aus 38.5 g (245.93 mmol) 2-Chlor-5-fluornicotinonitril wurde in 1,2-Ethandiol (380 ml) vorgelegt und danach mit Hydrazinhydrat (119.6 ml) versetzt. Es wurde unter Rühren für 4 h zum Rückfluss erhitzt. Beim Abkühlen fiel das Produkt aus. Die Kristalle wurden mit Wasser (380 ml) versetzt und 10 min bei RT ausgerührt. Anschliessend wurde die Suspension über eine Fritte abgesaugt, mit Wasser (200 ml) und mit -10°C-kaltem THF (200 ml) nachgewaschen. Trocknung im Hochvakuum über Phosphorpentoxid.
Ausbeute: 22.8 g (61 % d. Theorie)
¹H-NMR (400 MHz, DMSO-d₆): δ = 5.54 (s, 2H), 7.96 (dd, 1H), 8.38 (m, 1H), 12.07(m, 1H).

### Beispiel 6A

### 5-Fluor-3-iod-1H-pyrazolo[3,4-b]pyridin

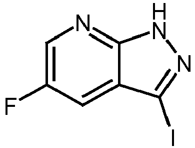

In THF (329 ml) wurden 10 g (65.75 mmol) 5-Fluor-1H-pyrazolo[3,4-b]pyridin-3-amin vorgelegt und auf 0°C abgekühlt. Anschliessend wurden 16.65 ml (131.46 mmol) Bortrifluorid-Diethylether-Komplex langsam zugesetzt. Die Reaktionsmischung wurde weiter auf -10°C abgekühlt. Danach wurde eine Lösung von 10.01 g (85.45 mmol) Isopentylnitrit in THF (24.39 ml) langsam zugefügt und weitere 30 min nachgerührt. Die Mischung wurde mit kaltem Diethylether (329 ml) verdünnt und der entstandene Feststoff abfiltriert. Das so hergestellte Diazoniumsalz wurde portionsweise in eine 0°C kalte Lösung von 12.81 g (85.45 mmol) Natriumiodid in Aceton (329 ml) gegeben und die Mischung 30 min bei RT nachgerührt. Die Reaktionsmischung wurde auf Eiswasser (1.8 l) gegeben und zweimal mit Essigsäureethylester (je 487 ml) extrahiert. Die vereinigten organischen Phasen wurden mit gesättigter wässriger Natriumchlorid-Lösung (244 ml) gewaschen, getrocknet, filtriert und eingeengt. Man erhielt 12.1 g (86%-ige Reinheit, 60 % d. Th.) der Titelverbindung als Feststoff. Das Rohprodukt wurde ohne weitere Reinigung umgesetzt.
LC-MS (Methode 2): Rₜ = 1.68 min
MS (ESIpos): m/z = 264 [M+H]⁺

### Beispiel 7A

### 5-Fluor-1-(2-fluorbenzyl)-3-iod-1H-pyrazolo[3,4-b]pyridin

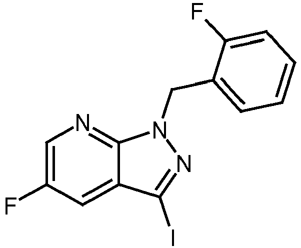

In DMF (217 ml) wurden 12.1 g (ca. 39.65 mmol) der Verbindung aus Beispiel 6A vorgelegt und anschließend 8.25 g (43.62 mmol) 2-Fluorbenzylbromid sowie 14.21 g (43.62 mmol) Cäsiumcarbonat zugefügt. Die Mischung wurde zwei Stunden bei RT gerührt. Anschließend wurde die Reaktionsmischung auf Wasser (1.17 l) gegeben und zweimal mit Essigsäureethylester (502 ml) extrahiert. Die vereinigten organischen Phasen wurden mit gesättigter wässriger Natriumchlorid-Lösung (335 ml) gewaschen, getrocknet, filtriert und eingeengt. Der Rückstand wurde an Kieselgel (Laufmittel: Petrolether/ Essigsäureethylester 97:3) chromatographiert und die Produktfraktionen eingeengt. Man erhielt 9.0 g (61 % d. Th.) der Titelverbindung als Feststoff. Der Feststoff wurde in Essigsäureethylester aufgenommen und mit 10%-iger wässriger Natriumthiosulfatlösung und danach mit gesättigter wässriger Natriumchlorid-Lösung gewaschen, getrocknet und eingeengt.
LC-MS (Methode 2): Rₜ = 2.57 min
MS (ESIpos): m/z = 372 [M+H]⁺
1H-NMR (400 MHz, DMSO-d6): δ = 5.73 (s, 2H), 7.13 - 7.26 (m, 3H), 7.33 - 7.41 (m, 1H), 7.94 (dd, 1H), 8.69 - 8.73 (m, 1H).

### Beispiel 8A

### Ethyl-5-fluor-1-(2-fluorbenzyl)-1H-pyrazolo[3,4-b]pyridin-3-carboxylat

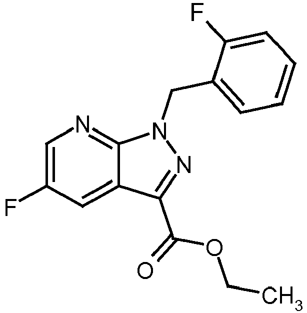

13.487 g (51.228 mmol) Ethyl-5-amino-1-(2-fluorbenzyl)-1H-pyrazol-3-carboxylat (Darstellung beschrieben für Beispiel 20A in WO 00/06569) wurden in 300 ml Dioxan vorgelegt und bei RT mit 6 g (51.228 mmol) 3-(Dimethylamino)-2-fluoracrylaldehyd (Darstellung beschrieben in Justus Liebigs Annalen der Chemie 1970; 99 - 107) versetzt. Anschliessend wurden 4.736 ml (61.473 mmol) Trifluoressigsäure zugegeben und der Ansatz wurde für 3 Tage unter Rühren zum Rückfluss erhitzt. Nach Abkühlen wurde im Vakuum eingeengt und der Rückstand mit Wasser und Essigsäureethylester versetzt. Die Phasen wurden getrennt und die organische Phase wurde zweimal mit Wasser gewaschen. Die vereinigten wässrigen Phasen wurden anschliessend zweimal mit Essigsäureethylester extrahiert. Die vereinigten organischen Phasen wurden über Natriumsulfat getrocknet, filtriert und im Vakuum eingeengt. Der Rückstand (22 g) wurde anschliessend durch Chromatographie an Kieselgel (Laufmittel: Dichlormethan) gereinigt. Man erhielt 5.67 g (35 % d. Th.) der Titelverbindung.
LC-MS (Methode 1): Rₜ = 1.17 min
MS (ESIpos): m/z = 318 [M+H]⁺
¹H-NMR (400 MHz, DMSO-d₆): δ = 1.37 (t, 3H), 4.40 (q, 2H), 5.86 (s, 2H), 7.15 - 7.27 (m, 3H), 7.36 - 7.41 (m, 1H), 8.25 (d, 1H), 8.78 (s br., 1H).

### Beispiel 9A

### 5-Fluor-1-(2-fluorbenzyl)-1H-pyrazolo[3,4-b]pyridin-3-carboxamid

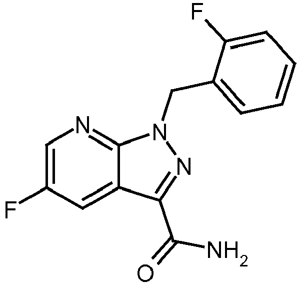

1.00 g (3.152 mmol) der unter Beispiel 8A erhaltenen Verbindung wurden in 10 ml einer 7N Lösung von Ammoniak in Methanol bei RT für drei Tage gerührt. Anschliessend wurde im Vakuum eingeengt. Man erhielt 908 mg (99 %d. Th.) der Titelverbindung.
LC-MS (Methode 1): Rₜ = 0.85 min
MS (ESIpos): m/z = 289 [M+H]⁺
¹H-NMR (400 MHz, DMSO-d₆): δ = 5.87 (s, 2H), 7.12 - 7.26 (m, 3H), 7.34 - 7.40 (m, 1H), 7.60 (s br., 1H), 7.87 (s br., 1H), 8.28 (dd, 1H), 8.72 (dd, 1H).

### Beispiel 10A

### 5-Fluor-1-(2-fluorbenzyl)-1H-pyrazolo[3,4-b]pyridin-3-carbonitril

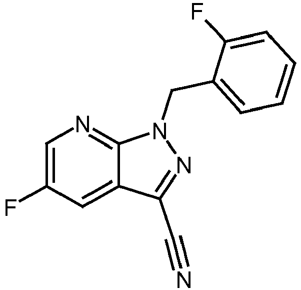

### Variante A:

Eine Suspension aus 16.03 g (43.19 mmol) 5-Fluor-1-(2-fluorbenzyl)-3-iod-1H-pyrazolo[3,4-b]pyridin (Beispiel 7A) und 4.25 g (47.51 mmol) Kupfer-(I)-cyanid wurden in DMSO (120 ml) vorgelegt und 2 h bei 150°C gerührt. Nach Abkühlen wurde der Kolbeninhalt auf ca. 40°C abgekühlt, auf eine Lösung aus konz. Ammoniakwasser (90 ml) und Wasser (500 ml) gegossen, mit Essigsäureethylester (200 ml) versetzt und kurz ausgerührt. Die wässrige Phase wurde abgetrennt und noch zweimal mit Essigsäureethylester (je 200 ml) extrahiert. Die vereinigten organischen Phasen wurden zweimal mit 10%-iger wässriger Natriumchlorid-Lösung (je 100 ml) gewaschen, getrocknet und im Vakuum eingeengt. Das Rohprodukt wurde ohne weitere Reinigung umgesetzt.
Ausbeute: 11.1 g (91 %d. Theorie)

### Variante B:

900 mg (3.122 mmol) der unter Beispiel 9A erhaltenen Verbindung wurden in THF (14 ml) gelöst und mit 0.646 ml (7.993 mmol) Pyridin versetzt. Danach wurden unter Rühren 1.129 ml (7.993 mmol) Trifluoressigsäureanhydrid langsam zugetropft und anschliessend über Nacht bei RT gerührt. Danach wurde das Reaktionsgemisch auf Wasser gegossen und dreimal mit Essigsäureethylester extrahiert. Die vereinigten organischen Phasen wurden mit gesättigter wässriger Natriumhydrogencarbonat-Lösung und 1N Salzsäure extrahiert und danach mit gesättigter wässriger Natriumchlorid-Lösung gewaschen. Die organische Phase wurde über Natriumsulfat getrocknet, filtriert und eingeengt. Man erhielt 850 mg (99 % d. Th.) der Titelverbindung.
LC-MS (Methode 1): Rₜ = 1.06 min
MS (ESIpos): m/z = 271 [M+H]⁺
¹H-NMR (400 MHz, DMSO-d₆): δ = 5.87 (s, 2H), 7.17 - 7.42 (m, 4H), 8.52 (dd, 1H), 8.87 (dd, 1H).

### Beispiel 11A

### 5-Fluor-1-(2-fluorbenzyl)-1H-pyrazolo[3,4-b]pyridin-3-carboximidamid-Acetat

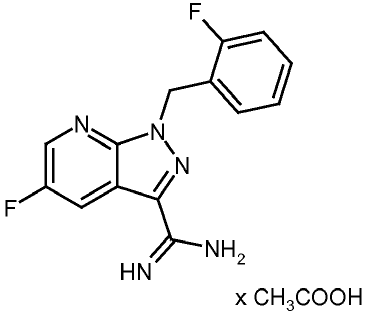

Zu 2.22 g (41.07 mmol) Natriummethanolat in Methanol (270 ml) wurden 11.1 g (41.07 mmol) 5-Fluor-1-(2-fluorbenzyl)-1H-pyrazolo[3,4-b]pyridin-3-carbonitril (Beispiel 10A) gegeben und 2 h bei RT gerührt. Danach wurden 2.64 g (49.29 mmol) Ammoniumchlorid und Essigsäure (9.17 ml) zugegeben und über Nacht zum Rückfluss erhitzt. Danach wurde das Reaktionsgemisch bis zur Trockene eingeengt und der Rückstand in Wasser (100 ml) und Essigsäureethylester (100 ml) aufgenommen und mit 2N Natronlauge auf pH 10 gestellt. Es wurde für ca. 1 h bei RT intensiv gerührt. Die erhaltene Suspension wurde abgesaugt und mit Essigsäureethylester (100 ml), Wasser (100 ml) und nochmals Essigsäureethylester (100 ml) nachgewaschen. Der Rückstand wird über Phosphorpentoxid im Hochvakuum getrocknet.
Ausbeute: 9.6 g (78 %d. Theorie)
MS (ESIpos): m/z = 288 [M+H]⁺
¹H-NMR (400 MHz, DMSO-d₆): δ = 1.85 (s, 3H), 5.80 (s, 2H), 7.14 - 7.25 (m, 3H), 7.36 (m, 1H), 8.42 (dd, 1H), 8.72 (dd, 1H).

### Beispiel 12A

### Methyl-3,3-dicyan-2,2-dimethylpropanoat

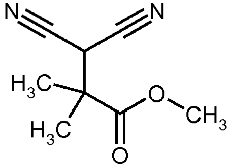

In THF (91 ml) wurden 1.816 g (45.411 mmol) Natriumhydrid (60% in Mineralöl) langsam mit 3 g (45.411 mmol) Malonsäuredinitril versetzt. Anschliessend wurden 5.876 ml (45.411 mmol) Methyl-2-brom-2-methylpropanoat zugegeben und es wurde über Nacht bei RT gerührt. Danach wurden nochmals 5.876 ml (45.411 mmol) Methyl-2-brom-2-methylpropanoat zugegeben und es wurde über Nacht auf 50°C erhitzt. Dann wurden abermals 1.762 ml (13.623) mmol) Methyl-2-brom-2-methylpropanoat zugegeben und es wurde weitere 4h auf 50°C erhitzt. Der Ansatz wurde dann mit gesättigter wässriger Natriumhydrogencarbonat-Lösung versetzt und dreimal mit Essigsäureethylester extrahiert. Die vereinigten organischen Phasen wurden mit gesättigter wässriger Natriumchlorid-Lösung gewaschen, mit Natriumsulfat getrocknet, filtriert und bis zur Trockene eingeengt. Man erhielt 8.9 g Rohprodukt, welches per Chromatographie an Kieselgel (Cyclohexan-Essigsäureethylester 4:1) gereinigt wurde.
Ausbeute: 6.47 g (85%d. Th.)
¹H-NMR (400 MHz, DMSO-d₆): δ [ppm] = 1.40 (s, 6H), 3.74 (s, 3H), 5.27 (s, 1H).

### Beispiel 13A

### 4-Amino-2-[1-(2-fluorbenzyl)-1H-pyrazolo[3,4-b]pyridin-3-yl]-5,5-dimethyl-5,7-dihydro-6H-pyrrolo[2,3-d]pyrimidin-6-on

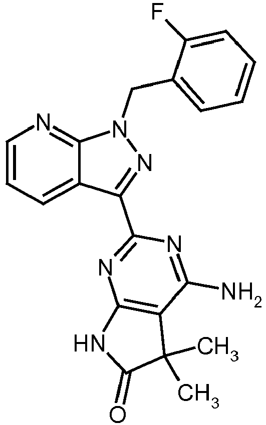

5.887 g (19.256 mmol) Beispiel 1A wurden in tert.-Butanol (50 ml) vorgelegt und mit 2.593 g (23.107 mmol) Kalium-tert.-butylat versetzt. Anschliessend wurden 3.2 g (19.256 mmol) Beispiel 12A in tert.-Butanol (25 ml) zugetropft und die Mischung über Nacht zum Rückfluss erhitzt. Am nächsten Tag wurden nochmal 0.64 g (3.851 mmol) Beispiel 12A zugegeben und es wurde für einen weiteren Tag zum Rückfluss erhitzt. Nach Abkühlen wurde ein Niederschlag abfiltriert, welcher mit Diethylether nachgewaschen wurde. Anschliessend wurde in Wasser aufgeschlämmt und ein weiteres Mal abfiltriert und mit Diethylether nachgewaschen. Nach Trocknung im Hochvakuum konnten 6.65 g der Titelverbindung erhalten (85 %d. Th.) werden.
LC-MS (Methode 1): Rₜ = 0.90 min; MS (ESIpos): m/z = 404 [M+H]⁺
¹H-NMR (400 MHz, DMSO-d₆): δ [ppm] = 1.35 (s, 6H), 5.82 (s, 2H), 6.82 (br s, 2H), 7.14-7.25 (m, 3H), 7.33-7.40 (m, 2H), 8.63 (dd, 1H), 9.03 (dd, 1H), 10.98 (s br, 1H).

### Beispiel 14A

### 4-Amino-2-[5-fluor-1-(2-fluorbenzyl)-1H-pyrazolo[3,4-b]pyridin-3-yl]-5,5-dimethyl-5,7-dihydro-6H-pyrrolo[2,3-d]pyrimidin-6-on

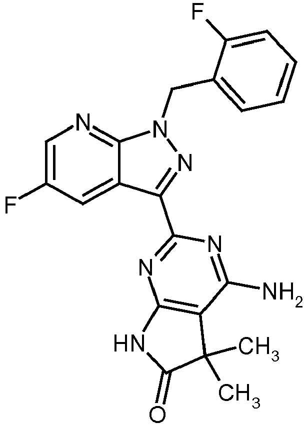

In Analogie zur Darstellung von Beispiel 13A wurden 4.18 g (12.035 mmol) Beispiel 11A mit 2.20 g (13.239 mmol) Beispiel 12A umgesetzt. Es wurden 3.72 g der Titelverbindung erhalten (73 %d. Th.).
LC-MS (Methode 1): Rₜ = 0.98 min; MS (ESIpos): m/z = 422 [M+H]⁺
¹H-NMR (400 MHz, DMSO-d₆): δ [ppm] = 1.34 (s, 6H), 5.81 (s, 2H), 6.85 (br s, 2H), 7.13-7.25 (m, 3H), 7.36 (m, 1H), 8.69 (dd, 1H), 8.84 (dd, 1H), 10.96 (s br, 1H).

Alternativ kann zur Herstellung auch Beispiel 73A anstelle von Beispiel 11A verwendet werden.

### Beispiel 15A

### 2-[1-(2-Fluorbenzyl)-1H-pyrazolo[3,4-b]pyridin-3-yl]-4-iod-5,5-dimethyl-5,7-dihydro-6H-pyrrolo[2,3-d]pyrimidin-6-on

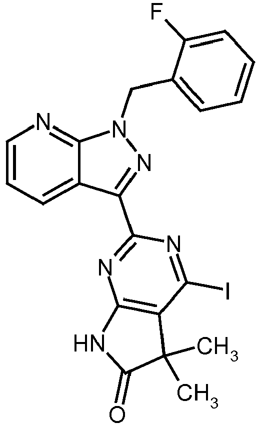

5.00 g (12.394 mmol) von Beispiel 13A wurden in iso-Pentylnitrit (35.87 ml) und Diiodmethan (1.16 mol, 93.71 ml) vorgelegt und und für 12 h auf 85°C erhitzt. Nach Abkühlen wurde von Feststoffen filtriert, das Filtrat eingeengt und der Rückstand anschliessend durch Chromatographie an Kieselgel gereinigt (Laufmittel: erst Cyclohexan-Dichlormethan Gradient, dann Dichlormethan-Methanol Gradient). Es wurden 5.50 g der Titelverbindung erhalten (67%d. Th.).
LC-MS (Methode 1): Rₜ = 1.19 min; MS (ESIpos): m/z = 515 [M+H]⁺
¹H-NMR (400 MHz, DMSO-d₆): δ [ppm] = 1.42 (s, 6H), 5.88 (s, 2H), 7.13-7.26 (m, 3H), 7.34-7.38 (m, 1H), 7.48 (dd, 1H), 8.69 (dd, 1H), 8.79 (dd, 1H), 11.78 (s br, 1H).

### Beispiel 16A

### 2-[5-Fluor-1-(2-fluorbenzyl)-1H-pyrazolo[3,4-b]pyridin-3-yl]-4-iod-5,5-dimethyl-5,7-dihydro-6H-pyrrolo[2,3-d]pyrimidin-6-on

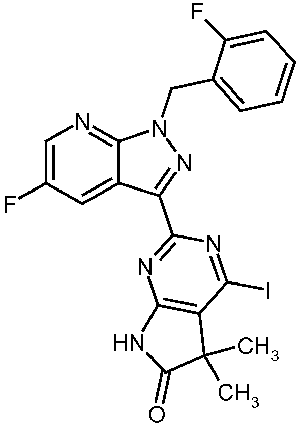

3.325 g (7.890 mmol) von Beispiel 14A wurden in Analogie zu Beispiel 15A umgesetzt. Es wurden 3.65 g der Titelverbindung erhalten (87 %d. Th., ca. 61 % Reinheit laut LC/MS).
LC-MS (Methode 1): Rₜ = 1.26 min; MS (ESIpos): m/z = 533 [M+H]⁺
¹H-NMR (400 MHz, DMSO-d₆): δ [ppm] = 1.42 (s, 6H), 5.87 (s, 2H), 7.14-7.26 (m, 3H), 7.37 (m, 1H), 8.48 (dd, 1H), 8.77 (dd, 1H), 11.76 (s br, 1H).

### Verbessertes Protokoll für grössere Ansätze:

52.6 g (113.585 mmol, 91 %ige Reinheit) Beispiel 14A wurden in Dioxan (239 ml) mit 91.26 g (340.75 mmol) Diiodmethan und 39.91 g (340.75 mmol) Isopentylnitrit für 2h bei 85°C gerührt.

Danach wurde eingeengt und der Rückstand an Kieselgel mit Dichlormethan:Aceton (95:5) als Eluent chromatographiert.. Es wurden 29.90 g der Titelverbindung erhalten (49 %d. Th.).

### Beispiel 17A

### 5-Fluor-1-[(3-fluorpyridin-2-yl)methyl]-3-iod-1H-pyrazolo[3,4-b]pyridin

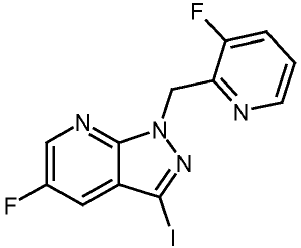

In DMF (10 ml) wurden 6.291 g (23.921 mmol) 5-Fluor-3-iod-1H-pyrazolo[3,4-b]pyridin und 8.573 g (26.313 mmol) Cäsiumcarbonat vorgelegt und anschließend 5.00 g (26.313 mmol) 2-(Brommethyl)-3-fluorpyridin in DMF (20 ml) gelöst zugetropft. Die Mischung wurde über Nacht bei RT gerührt. Anschließend wurde abgekühlt und auf 200 ml Wasser gegossen. Es wurde von einem Niederschlag abgesaugt, mit Wasser nachgewaschen und über Nacht im Hochvakuum getrocknet. Es wurden 6.28 g (70 %d. Th.) der Titelverbindung erhalten.
LC-MS (Methode 4): Rₜ = 2.17 min
MS (ESIpos): m/z = 373 [M+H]⁺
¹H-NMR (400 MHz, DMSO-d₆): δ = 5.88 (s, 2H), 7.42-7.46 (m, 1H), 7.77 (dd, 1H), 7.93 (dd, 1H), 8.27 (d, 1H), 8.67 (t, 1H).

### Beispiel 18A

### 5-Fluor-1-[(3-fluorpyridin-2-yl)methyl]-1H-pyrazolo[3,4-b]pyridin-3-carbonitril

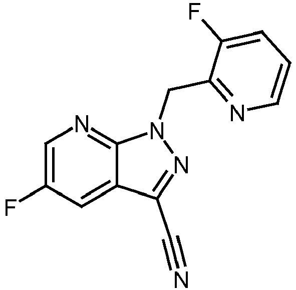

6.280 g (16.876 mmol) Beispiel 17A und 1.663 g (18.564 mmol) Kupfer-(I)-cyanid wurden in DMSO (100 ml) vorgelegt und 3 h bei 150°C gerührt. Nach Abkühlen wurde das Reaktionsgemisch über Celite filtriert und es wurde mit Essigsäureethylester nachgewaschen. Das Filtrat wurde viermal mit gesättigter wässriger Ammoniumchloridlösung und konz. Ammoniakwasser (3:1 v/v) extrahiert und die organische Phase abgetrennt. Diese wurde danach mit gesättigter wässriger Natriumchlorid-Lösung gewaschen, über Natriumsulfat getrocknet, filtriert und im Vakuum eingeengt. Es wurden 3.97 g (86 %d. Th.) der Titelverbindung erhalten.
LC-MS (Methode 1): Rₜ = 0.92 min
MS (ESIpos): m/z = 272 [M+H]⁺
¹H-NMR (400 MHz, DMSO-d₆): δ = 6.04 (s, 2H), 7.44-7.48 (m, 1H), 7.61 (t, 1H), 8.26 (d, 1H), 8.52 (dd, 1H), 8.83 (dd, 1H).

### Beispiel 19A

### 5-Fluor-1-[(3-fluorpyridin-2-yl)methyl]-1H-pyrazolo[3,4-b]pyridin-3-carboximidamid-Acetat

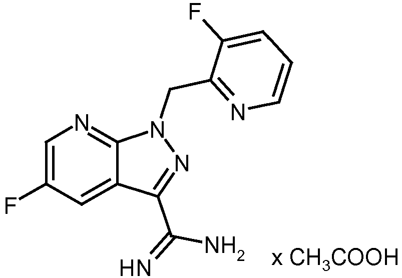

Zu 777 mg (14.379 mmol) Natriummethanolat in Methanol (20 ml) wurden 3.900 g (14.379 mmol) Beispiel 18A in Methanol (40 ml) gegeben und 2 h bei RT gerührt. Danach wurden 932 mg (17.255 mmol) Ammoniumchlorid und Essigsäure (3.210 ml) zugegeben und über Nacht zum Rückfluss erhitzt. Danach wurde das Reaktionsgemisch bis zur Trockene eingeengt und der Rückstand mit Essigsäureethylester und mit 1N Natronlauge versetzt und für 2h bei RT gerührt. Es wurde dann von einem Feststoff abfiltriert, welcher mit Essigsäureethylester und Wasser nachgewaschen wurde. Der Feststoff wurde über Nacht am Hochvakuum getrocknet. Es wurden 0.56 g (11 %d. Th.) der Titelverbindung erhalten. Die Phasen des Filtrats wurden getrennt, und die wässrige Phase wurde zweimal mit Essigsäureethylester extrahiert. Die vereinigten organischen Phasen wurden über Natriumsulfat getrocknet und eingeengt. Es wurden weitere 1.86 g (14 %d. Th., 39%ige Reinheit) der Titelverbindung erhalten. Die wässrige Phase wurde ebenfalls eingeengt, der Rückstand mit DMF versetzt und 30 min bei RT gerührt. Es wurde von einem Niederschlag abgesaugt, mit DMF nachgewaschen, das Filtrat eingeengt und über Nacht im Hochvakuum getrocknet. Es wurden weitere 1.77 g (35 %d. Th.) der Titelverbindung erhalten werden.
LC-MS (Methode 4): Rₜ = 1.25 min
MS (ESIpos): m/z = 289 [M+H]⁺

### Beispiel 20A

### 4-Amino-2-{5-fluor-1-[(3-fluorpyridin-2-yl)methyl]-1H-pyrazolo[3,4-b]pyridin-3-yl}-5,5-dimethyl-5,7-dihydro-6H-pyrrolo[2,3-d]pyrimidin-6-on

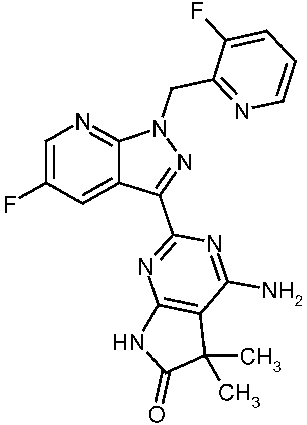

567 mg (1.628 mmol) Beispiel 19A wurden in tert.-Butanol (10 ml) vorgelegt und mit 274 mg (2.442 mmol) Kalium-tert.-butylat versetzt. Anschliessend wurden 324 mg (1.953 mmol) Beispiel 12A in tert.-Butanol (5 ml) zugegeben und die Mischung über Nacht zum Rückfluss erhitzt. Nach Abkühlen wurde das Reaktionsgemisch mit Wasser und Ethanol versetzt, und für 1 h gerührt. Der entstandene Niederschlag wurde abgesaugt und mit wenig Ethanol nachgewaschen. Der Feststoff wurde am Hochvakuum getrocknet. Es wurden 568 mg der Titelverbindung erhalten (80 %d. Th.).
LC-MS (Methode 3): Rₜ = 0.82 min; MS (ESIpos): m/z = 423 [M+H]⁺
¹H-NMR (400 MHz, DMSO-d₆): δ [ppm] = 1.34 (s, 6H), 5.94 (s, 2H), 6.87 (br s, 2H), 7.42-7.46 (m, 1H), 7.75-7.80 (m, 1H), 8.27 (d, 1H), 8.67 (dd, 1H), 8.83 (dd, 1H), 10.95 (br s, 1H).

### Beispiel 21A

### 2-{5-Fluor-1-[(3-fluorpyridin-2-yl)methyl]-1H-pyrazolo[3,4-b]pyridin-3-yl}-4-iod-5,5-dimethyl-5,7-dihydro-6H-pyrrolo[2,3-d]pyrimidin-6-on

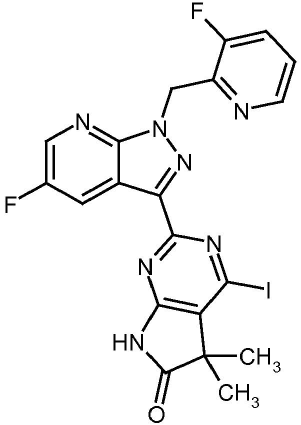

2.040 g (4.830 mmol) von Beispiel 20A wurden in iso-Pentylnitrit (14 ml) und Diiodmethan (37 ml) vorgelegt und für 1h auf 85°C erhitzt. Nach Abkühlen wurde ein Feststoff abfiltriert, welcher mit wenig Acetonitril gewaschen wurde. Anschliessend wurde der Feststoff über Nacht im Hochvakuum getrocknet. Es wurden 1.83 g der Titelverbindung erhalten (39% d. Th., 55%ige Reinheit). Die Rohverbindung wurde ohne weitere Reinigung in den nächsten Schritten eingesetzt.
LC-MS (Methode 1): Rₜ = 1.12 min; MS (ESIpos): m/z = 534 [M+H]⁺

### Beispiel 22A

### 2-[6-Chlor-3-(2,3,6-trifluorbenzyl)imidazo[1,5-a]pyridin-1-yl]-4-iod-5,5-dimethyl-5,7-dihydro-6H-pyrrolo[2,3-d]pyrimidin-6-on

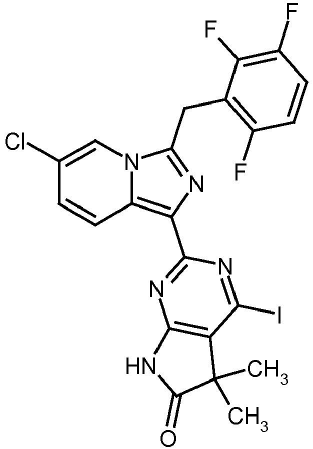

556 mg (1.176 mmol) 4-Amino-2-[6-chlor-3-(2,3,6-trifluorbenzyl)imidazo[1,5-a]pyridin-1-yl]-5,5-dimethyl-5,7-dihydro-6H-pyrrolo[2,3-d]pyrimidin-6-on (beschrieben in WO 2010/065275) wurden in 1,2-Dimethoxyethan (14 ml) vorgelegt und bei Raumtemperatur mit 305 mg (1.176 mmol) Cäsiumiodid, 149 mg (0.588 mmol) Iod und 67 mg (0.353 mmol) Kupfer-(I)-iodid versetzt. Anschliessend wurde iso-Pentylnitrit (0.933 ml) zugegeben und es wurde über Nacht auf 60°C erhitzt. Am nächsten Tag wurden nochmals 305 mg (1.176 mmol) Cäsiumiodid, 149 mg (0.588 mmol) Iod und 67 mg (0.353 mmol) Kupfer-(I)-iodid, sowie iso-Pentylnitrit (0.933 ml) zugegeben und es wurde für 3 Tage auf 60°C erhitzt. Nach Abkühlen wurde mit einem kleineren Ansatz vereinigt (ausgehend von 50 mg 4-Amino-2-[6-chlor-3-(2,3,6-trifluorbenzyl)imidazo[1,5-a]pyridin-1-yl]-5,5-dimethyl-5,7-dihydro-6H-pyrrolo[2,3-d]pyrimidin-6-on). Es wurde mit Essigsäureethylester und gesättigter wässriger Natriumthiosulfat extrahiert und die Phasen getrennt. Die organische Phase wurde noch zweimal mit gesättigter wässriger Natriumthiosulfat extrahiert. Anschliessend wurde die organische Phase mit gesättigter wässriger Natriumchloridlösung gewaschen, über Natriumsulfat getrocknet, filtriert, eingeengt und der Rückstand mittels präparativer HPLC gereinigt (Acetonitril:Wasser (+0.05 % Ameisensäure) - Gradient). Es wurden 236 mg der Titelverbindung erhalten (31% d. Th.).
LC-MS (Methode 1): Rₜ = 1.28 min; MS (ESIpos): m/z = 584 [M+H]⁺
¹H-NMR (400 MHz, DMSO-d₆): δ [ppm] = 1.37 (s, 6H), 4.57 (s, 2H), 7.19-7.25 (m, 1H), 7.30 (dd, 1H), 7.48-7.56 (m, 1H), 8.43 (d, 1H), 8.87 (s, 1H), 11.58 (s, 1H).

Neben der Titelverbindung wurden ausserdem 27 mg (5% d. Th., 90%ige Reinheit) 2-[6-Chlor-3-(2,3,6-trifluorbenzyl)imidazo[1,5-a]pyridin-1-yl]-4-hydroxy-5,5-dimethyl-5,7-dihydro-6H-pyrrolo[2,3-d]pyrimidin-6-on erhalten.

### Beispiel 23A

### 4-(Chlormethyl)-3-fluorpyridin-Hydrochlorid

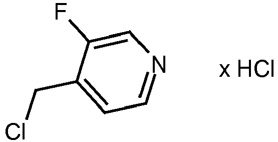

6.710 g (52.785 mmol) (3-Fluorpyridin-4-yl)methanol wurden in 29 ml Acetonitril vorgelegt und auf 50°C erhitzt. Danach wurde eine Lösung von 7.701 ml Thionylchlorid in 14.5 ml Acetonitril zugetropft und die Reaktionsmischung wurde 4 h bei 50 °C gerührt. Danach wurde der Ansatz eingeengt und dreimal mit Dichlormethan kodestilliert. Nach Trocknung im Hochvakuum erhielt man 10.27 g der Titelverbindung, die ohne weitere Reinigung in der nächsten Stufe eingesetzt wurde.

### Beispiel 24A

### 5-Fluor-1-[(3-fluorpyridin-4-yl)methyl]-3-iod-1H-pyrazolo[3,4-b]pyridin

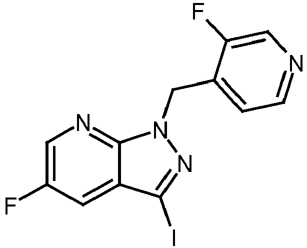

12.225 g (46.482 mmol) 5-Fluor-3-iod-1H-pyrazolo[3,4-b]pyridin wurden in Analogie zur Vorschrift unter Beispiel 7A mit Beispiel 23A umgesetzt. Es wurden 11.34 g (65 %d. Th.) der Titelverbindung erhalten.
LC-MS (Methode 3): Rₜ = 1.01 min
MS (ESIpos): m/z = 373 [M+H]⁺

### Beispiel 25A

### 5-Fluor-1-[(3-fluorpyridin-4-yl)methyl]-1H-pyrazolo[3,4-b]pyridin-3-carbonitril

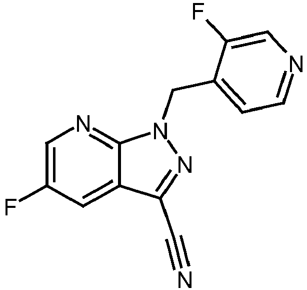

11.340 g (30.474 mmol) Beispiel 24A wurden in Analogie zur Vorschrift unter Beispiel 10A, Variante A umgesetzt. Es wurden 6.31 g (76 %d. Th.) der Titelverbindung erhalten.
LC-MS (Methode 3): Rₜ = 0.89 min
MS (ESIpos): m/z = 272 [M+H]⁺

### Beispiel 26A

### 5-Fluor-1-[(3-fluorpyridin-4-yl)methyl]-1H-pyrazolo[3,4-b]pyridin-3-carboximidamid-Acetat

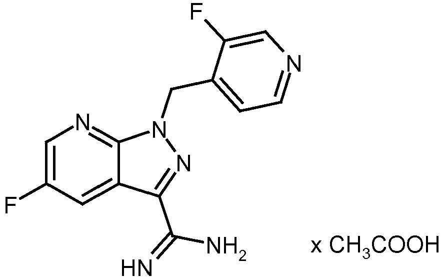

6.310 g (23.264 mmol) Beispiel 25A wurden in Analogie zur Vorschrift unter Beispiel 11A umgesetzt. Es wurden 6.12 g (75 %d. Th.) der Titelverbindung erhalten.
LC-MS (Methode 1): Rₜ = 0.45 min
MS (ESIpos): m/z = 289 [M+H]⁺

### Beispiel 27A

### 4-Amino-2-{5-fluor-1-[(3-fluorpyridin-4-yl)methyl]-1H-pyrazolo[3,4-b]pyridin-3-yl}-5,5-dimethyl-5,7-dihydro-6H-pyrrolo[2,3-d]pyrimidin-6-on

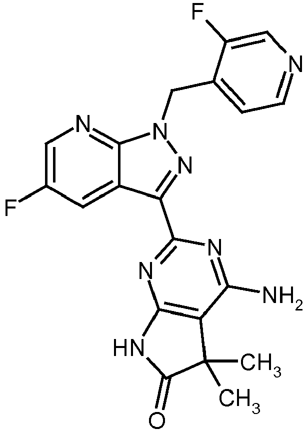

3.050 g (8.756 mmol) Beispiel 26A wurden in Analogie zur Vorschrift unter Beispiel 13A umgesetzt. Reinigung mittels präparativer Chromatographie an Kieselgel (Dichlormethan:Methanol - Gradient). Es wurden 528 mg der Titelverbindung erhalten (14 %d. Th,).
LC-MS (Methode 1): Rₜ = 0.80 min; MS (ESIpos): m/z = 423 [M+H]⁺
¹H-NMR (400 MHz, DMSO-d₆): δ [ppm] = 1.35 (s, 6H), 5.90 (s, 2H), 6.89 (br s, 2H), 7.11 (t, 1H), 8.35 (d, 1H), 8.59 (d, 1H), 8.70 (dd, 1H), 8.87 (dd, 1H), 10.99 (br s, 1H).

### Beispiel 28A

### 2-{5-Fluor-1-[(3-fluorpyridin-4-yl)methyl]-1H-pyrazolo[3,4-b]pyridin-3-yl}-4-iod-5,5-dimethyl-5,7-dihydro-6H-pyrrolo[2,3-d]pyrimidin-6-on

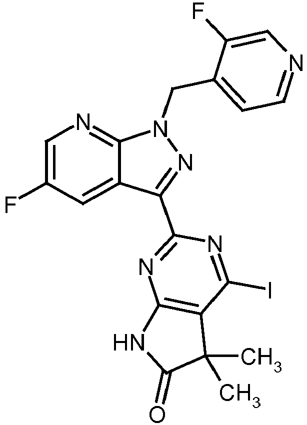

527 mg (1.248 mmol) Beispiel 27A wurden in Analogie zur Vorschrift unter Beispiel 21A umgesetzt. Es wurden 395 mg der Titelverbindung erhalten (39% d. Th., 66 % Reinheit). Die Rohverbindung wurde ohne weitere Reinigung in den nächsten Schritten eingesetzt.
LC-MS (Methode 3): Rₜ = 1.09 min; MS (ESIpos): m/z = 534 [M+H]⁺

### Beispiel 29A

### 1,4,5,6-Tetrahydrocyclopenta[c]pyrazol-3-carbonitril

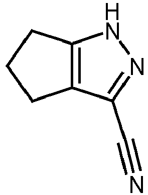

Die Darstellung der Verbindung ist beschrieben in: Org. Process Res. Dev. 2009, 13, 543.

### Beispiel 30A

### 1-(2-Fluorbenzyl)-1,4,5,6-tetrahydrocyclopenta[c]pyrazol-3-carbonitril

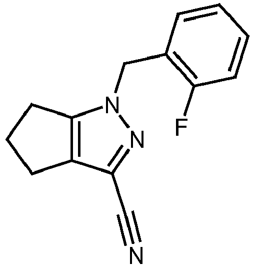

10.320 g (77.50 mmol) 1,4,5,6-Tetrahydrocyclopenta[c]pyrazol-3-carbonitril wurden in 100 ml DMF gelöst, mit 30.304 g (93.01 mmol) Cäsiumcarbonat und 16.116 g (85.26 mmol) 2-Fluorbenzylbromid versetzt und bei RT über Nacht gerührt. Der Ansatz wurde eingeengt und in Dichlormethan aufgenommen und mit Wasser versetzt. Die organische Phase wurde abgetrennt und die wässrige Phase zwei mal mit Dichlormethan extrahiert. Die vereinigten organischen Phasen wurden mit gesättigter wässriger Natriumchlorid-Lösung gewaschen, über einen Siliconfilter filtriert und eingeengt. Der Rückstand wurde durch Flashchromatographie an Kieselgel (Eluent: Hexan/Essigsäureethylester, Gradient) gereinigt. Es wurden 11.37 g (60% d. Th.) der Zielverbindung erhalten.
¹H-NMR (400MHz, DMSO-d₆): δ [ppm]= 2.59-2.64 (m, 4H), 5.33 (s, 2H), 7.15-7.23 (m, 2H), 7.27-7.33 (m, 1H), 7.36-7.43 (m, 1H).

### Beispiel 31A

### 1-(2-Fluorbenzyl)-1,4,5,6-tetrahydrocyclopenta[c]pyrazol-3-carboximidamid

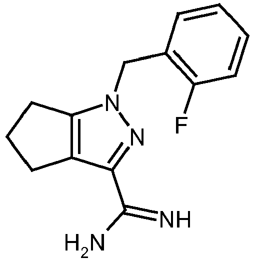

Unter Stickstoffatmosphäre wurden 3.600 g (14.92 mmol) 1-(2-Fluorbenzyl)-1,4,5,6-tetrahydrocyclopenta[c]pyrazol-3-carbonitril in 37 ml absolutem Methanol gelöst. Es wurden 1.306 (24.17 mmol) Natriummethylat zugegeben und 4 h bei RT gerührt. Es wurden 1.452 g (24.17 mmol) Essigsäure sowie 1.197 g (22.38 mmol) Ammoniumchlorid addiert und die Suspension über Nacht bei 50°C gerührt. Das Reaktionsgemisch wurde eingeengt und der Rückstand in 100 ml Wasser und 25 ml 1N Salzsäure suspendiert. Das Gemisch wurde mit Dichlormethan extrahiert.

Die wässrige Phase wurde mit 2N Natronlauge basisch gestellt (pH = 12) und dreimal mit einem Gemisch aus Dichlormethan/Methanol (v/v = 8:2) extrahiert. Die vereinigten organischen Phasen wurden mit Natriumsulfat getrocknet, eingeengt, mit Toluol versetzt und erneut zur Trockne eingeengt. Es wurden 1.94 g (50% d. Th.) der Zielverbindung erhalten.
LC-MS (Methode 7): Rₜ = 2.52 min; MS (ESIpos): m/z = 259 [M+H]⁺

### Beispiel 32A

### 4-Amino-2-[1-(2-fluorbenzyl)-1,4,5,6-tetrahydrocyclopenta[c]pyrazol-3-yl]-5,5-dimethyl-5,7-dihydro-6H-pyrrolo[2,3-d]pyrimidin-6-on

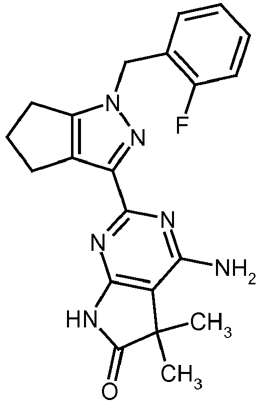

300 mg (1.15 mmol) 1-(2-Fluorbenzyl)-1,4,5,6-tetrahydrocyclopenta[c]pyrazol-3-carboximidamid wurden mit 2 ml *tert*.-Butanol, 287 mg (1.38 mmol) Methyl-3,3-dicyan-2,2-dimethylpropanoat gelöst in 2 ml *tert*.-Butanol sowie 181 mg (1.61 mmol) Kalium-*tert*.-butylat versetzt und 72 h unter Rückfluss erhitzt. Es wurde zur Trockne eingeengt und der Rückstand mit Wasser/Isopropanol (v/v = 3:1) verrührt. Der Feststoff wurde abfiltriert und im Hochvakuum getrocknet. Es wurden 385 mg (80% d. Th.) der Zielverbindung erhalten.
LC-MS (Methode 1): Rₜ = 0.83 min; MS (ESIpos): m/z = 393 [M+H]⁺

### Beispiel 33A

### 2-[1-(2-Fluorbenzyl)-1,4,5,6-tetrahydrocyclopenta[c]pyrazol-3-yl]-4-iod-5,5-dimethyl-5,7-dihydro-6H-pyrrolo[2,3-d]pyrimidin-6-on

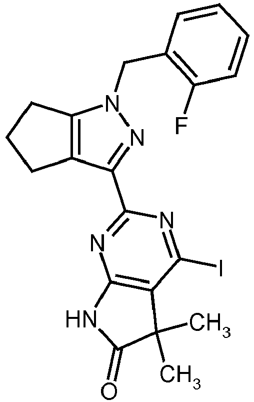

285 mg (0.68 mmol) 4-Amino-2-[1-(2-fluorbenzyl)-1,4,5,6-tetrahydrocyclopenta[c]pyrazol-3-yl]-5,5-dimethyl-5,7-dihydro-6H-pyrrolo[2,3-d]pyrimidin-6-on wurden in absolutem Dimethoxyethan vorgelegt und mit 800 mg (6.83 mmol) Isopentylnitrit, 87 mg (0.34 mmol) Iod, 39 mg (0.21 mmol) Kupfer(I)iodid sowie 177 mg (0.68 mmol) Cäsiumiodid versetzt. Das Gemisch wurde 40 min bei 100 °C gerührt. Das Gemisch wurde am Rotationsverdampfer eingeengt, der Rückstand in Dichlormethan aufgenommen und mit 5%-iger wässriger Natriumthiosulfat-Lösung und gesättigter wässriger Natriumchlorid-Lösung gewaschen. Die organische Phase wurde über Natriumsulfat getrocknet, eingeengt und mittels päparativer HPLC gereinigt (Eluent: Acetonitril/Wasser mit 0.1% Ameisensäure, Gradient 20:80 → 100:0). Es wurden 148 mg (40% d. Th.) der Zielverbindung erhalten.
LC-MS (Methode 1): Rₜ = 1.23 min; MS (ESIpos): m/z = 504 [M+H]⁺

### Beispiel 34A

### 1-(2-Bromphenyl)-2-(2-fluorphenyl)ethanon

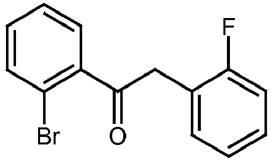

15.0 g (69.8 mmol) 2-Brombenzoesäuremethylester und 11.8 g (76.7 mmol) 2-Fluorphenylessigsäure wurden unter Argonatmosphäre in THF (278 ml) bei -70°C vorgelegt und 174 ml einer 1M Lösung von Natriumhexamethyldisilazan in THF über 20 min zugetropft. Das Reaktionsgemisch wurde auf 0°C erwärmt, 30 min bei dieser Temperatur gerührt und 1N Salzsäure (278 ml) hinzugefügt. Nach 1 h kräftigem Rühren unter Gasentwicklung (CO₂-Abspaltung) wurde das Reaktionsgemisch mit Essigsäureethylester (500 ml) extrahiert. Die organische Phase wurde zweimal mit gesättigter wässriger Natriumhydrogencarbonat-Lösung, einmal mit Wasser und einmal mit gesättigter, wässriger Natriumchloridlösung gewaschen. Nach dem Trocknen und dem Entfernen des Lösungsmittels am Rotationsverdampfer wurden 16.8 g Rückstand (55%ige Reinheit) erhalten. Der Rückstand wurde in THF (140 ml) gelöst, mit 1N Natronlauge (70 ml) versetzt und 4 h bei RT gerührt, um überschüssigen Ester zu verseifen. Das THF wurde am Rotationsverdampfer entfernt, die wässrige Phase mit Diethylether extrahiert und die organische Phase mit gesättigter wässriger Natriumhydrogencarbonat-Lösung sowie gesättigter, wässriger Natriumchloridlösung gewaschen. Nach der Trocknung und dem Entfernen des Lösungsmittels wurden 12.2 g Rückstand erhalten (ca 80%ige Reinheit). Der Rückstand wurde in THF (100 ml) gelöst, mit 1N Natronlauge (40 ml) versetzt und über Nacht bei RT gerührt. Das THF wurde am Rotationsverdampfer entfernt, die wässrige Phase mit Diethylether extrahiert und die organische Phase mit gesättigter wässriger Natriumhydrogencarbonat-Lösung sowie gesättigter, wässriger Natriumchloridlösung gewaschen. Nach der Trocknung und dem Entfernen des Lösungsmittels wurden 7.90 g (37 % d. Th.) der Titelverbindung isoliert.
¹H-NMR (400MHz, DMSO-d₆): δ [ppm]= 4.35 (s, 2H), 7.14 - 7.22 (m, 2H), 7.30 - 7.39 (m, 2H), 7.41 - 7.47 (m, 1H), 7.49 - 7.55 (m, 1H), 7.70 - 7.78 (m, 2H).

### Beispiel 35A

### 2-[1-(2-Bromphenyl)-2-(2-fluorphenyl)ethyliden]hydrazincarboximidamid

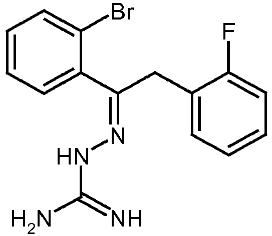

7.80 g (26.6 mmol) Beispiel 34A und 5.88 g (53.2 mmol) Aminoguanidin-Hydrochlorid wurden in Ethylenglykol (193 ml) vorgelegt und 8.50 g (59.9 mmol) Bortrifluorid-Diethylether-Komplex hinzugegeben. Das Reaktionsgemisch wurde 2 h bei 120°C an einer Destillationsbrücke erhitzt. Nach Abkühlung wurden nochmals 5.88 g (53.2 mmol) Aminoguanidin-Hydrochlorid sowie 8.50 g (59.9 mmol) Bortrifluorid-Diethylether-Komplex hinzugegeben und 3h bei 120°C gerührt. Nach dem Abkühlen wurde mit Wasser (750 ml) versetzt und mit 1N Natronlauge ein pH Wert von 11-12 eingestellt. Nach beginnender Kristallbildung wurden 300 g Eis hinzugegeben, 5 min gerührt und der Feststoff dann abfiltriert. Der Rückstand wurde zuerst mit Wasser, dann mit Pentan gewaschen und im Vakuum getrocknet. Es wurden 8.30 g (87 % d. Th.) der Titelverbindung erhalten.
LC-MS (Methode 1): Rₜ = 0.78 min; MS (ESIpos): m/z (Br-Isotop 1 + 2) = 349 + 351 [M+H]⁺

### Beispiel 36A

### 3-(2-Fluorbenzyl)-1H-indazol-1-carboximidamid

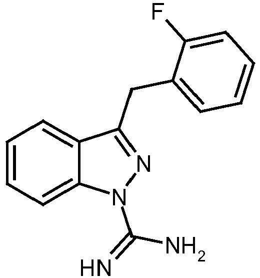

320 ml N-Methylpyrrolidon wurden auf 140°C erhitzt, 8.20 g (23.5 mmol) Beispiel 35A und 4.47 g (23.5 mmol) Kupfer-(I)-iodid hinzugegeben und 14 min bei 170° Badtemperatur gerührt. Das Reaktionsgemisch wurde anschließend langsam auf 1 L Eiswasser gegeben und konzentrierte wäßrige Ammoniak-Lösung (350 mL) hinzugegeben. Nach 5 minütigem Rühren wurde 1 L Essigsäureethylester hinzugegeben und das Gemisch 10 min gerührt. Die wässrige Phase wurde einmal mit Essigsäureethylester extrahiert und die vereinigten organischen Phasen dreimal mit Wasser gewaschen. Nach dem Trocknen und dem Entfernen des Lösungsmittels am Rotationsverdampfer wurden 7.10 g (74 % d. Th., 66%ige Reinheit) der Titelverbindung erhalten. Das Rohprodukt wurde ohne Reinigung weiter umgesetzt.
LC-MS (Methode 1): Rₜ = 0.68 min; MS (ESIpos): m/z = 269 [M+H]⁺

### Beispiel 37A

### 4-Amino-2-[3-(2-fluorbenzyl)-1H-indazol-1-yl]-5,5-dimethyl-5,7-dihydro-6H-pyrrolo[2,3-d]pyrimidin-6-on

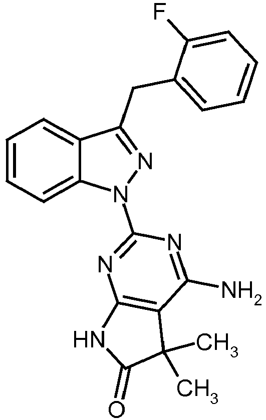

7.00 g (ca. 17.2 mmol, 66 %-ige Reinheit) des Rohprodukts von Beispiel 36A und 5.72 g (34.4 mmol) Beispiel 12A wurden in tert.-Butanol (77.0 ml) vorgelegt und 3.29 g (29.3 mmol) Kalium-tert.-Butylat hinzugegeben. Das Reaktionsgemisch wurde 18 h zum Rückfluss erhitzt. Nach dem Abkühlen wurde das Reaktionsgemisch mit Essigsäureethylester verdünnt und mit ca. 7%-iger wässriger Ammoniumchlorid-Lösung gewaschen. Die organische Phase wurde mit gesättigter, wässriger Natriumchloridlösung gewaschen, getrocknet und das Lösungsmittel am Rotationsverdampfer entfernt. Der Rückstand wurde an 600 ml Kieselgel mit Cyclohexan / Essigsäureethylester 2:3 chromatographisch gereinigt. Es wurden 2.20 g (29 % d. Th.) der Titelverbindung als Feststoff erhalten.
LC-MS (Methode 4): Rₜ = 2.19 min; MS (ESIpos): m/z = 403 [M+H]⁺
¹H-NMR (400MHz, DMSO-d₆): δ [ppm]= 1.35 (s, 6H), 4.39 (s, 2H), 6.97 (br. s., 2H), 7.11 - 7.18 (m, 1H), 7.21 (d, 1H), 7.24 - 7.33 (m, 2H), 7.36 (t, 1H), 7.50 (t, 1H), 7.70 (d, 1H), 8.82 (d, 1H), 11.10 (s, 1H).

### Beispiel 38A

### 2-[3-(2-Fluorbenzyl)-1H-indazol-1-yl]-4-iod-5,5-dimethyl-5,7-dihydro-6H-pyrrolo [2,3-d]pyrimidin-6-on

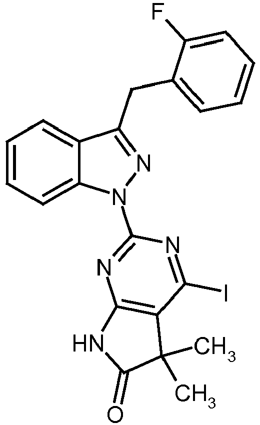

500 mg (1.242 mmol) von Beispiel 37A wurden in iso-Pentylnitrit (3.552 ml) und Diiodmethan (9.430 ml) vorgelegt und über Nacht auf 85°C erhitzt. Nach Abkühlen wurde das Reaktionsgemisch über Kieselgel filtriert (Dichlormethan:Methanol - Gradient) und eingeengt. Der Rückstand wurde mit Dichlormethan und Methanol versetzt und für 10 min bei Raumtemperatur gerührt. Der gebildete Feststoff wurde abfiltriert und mit Dichlormethan und Methanol nachgewaschen. Das Filtrat wurde eingeengt. Dieser Rückstand wurde dann mit Methanol und Acetonitril versetzt. Es bildete sich erneut ein Niederschlag, welcher abgesaugt wurde und mit Acetontril nachgewaschen wurde. Nach Trocknung im Hochvakuum wurden 127 mg der Titelverbindung erhalten (18% d. Th.). Das Filtrat wurde eingeengt, so wurden weitere 334 mg der Titelverbindung in 57%iger Reinheit (30% d. Th.) erhalten werden.
LC-MS (Methode 1): Rₜ = 1.31 min; MS (ESIpos): m/z = 514 [M+H]⁺
¹H-NMR (400 MHz, DMSO-d₆): δ [ppm] = 1.42 (s, 6H), 4.43 (s, 2H), 7.13-7.23 (m, 2H), 7.29-7.41 (m, 3H), 7.62 (t, 1H), 7.74 (d, 1H), 8.58 (d, 1H), 11.89 (s, 1H).

Neben der Titelverbindung wurden 57 mg (9 % d. Th., 86%ige Reinheit) 2-[3-(2-Fluorbenzyl)-1H-indazol-1-yl]-4-hydroxy-5,5-dimethyl-5,7-dihydro-6H-pyrrolo[2,3-d]pyrimidin-6-on (Beispiel 22) erhalten.

### Beispiel 39A

### 1-(2-Brom-5-fluorphenyl)-2-(2-fluorphenyl)ethanon

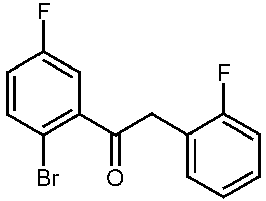

15.0 g (63.1 mmol) 2-Brom-5-fluorbenzoesäuremethylester und 11.7 g (75.7 mmol) 2-Fluorphenylessigsäure wurden unter Argonatmosphäre in THF (278 ml) bei -70°C vorgelegt und eine 1M Lösung von Natriumhexamethyldisilazan in THF (158 ml) über 20 min zugetropft. Das Reaktionsgemisch wurde 30 min bei dieser Temperatur gerührt, auf 0°C erwärmt, weitere 30 min bei 0°C gerührt und dann 1N Salzsäure (251 ml) hinzugefügt. Nach 1 h kräftigem Rühren unter Gasentwicklung (CO₂-Abspaltung) wurde das Reaktionsgemisch mit Essigsäureethylester (700 ml) extrahiert. Die organische Phase wurde zweimal mit gesättigter wässriger Natriumhydrogencarbonat-Lösung, einmal mit Wasser und einmal mit gesättigter, wässriger Natriumchloridlösung gewaschen. Nach dem Trocknen und dem Entfernen des Lösungsmittels am Rotationsverdampfer wurden 16.9 g Rückstand erhalten (50%ige Reinheit). Der Rückstand wurde in THF (200 ml) gelöst, mit 1N Natronlauge (100 ml) versetzt und über Nacht bei RT gerührt. Das THF wurde am Rotationsverdampfer entfernt, die wässrige Phase mit Diethylether extrahiert und die organische Phase mit gesättigter, wässriger Natriumhydrogencarbonat-Lösung sowie gesättigter, wässriger Natriumchloridlösung gewaschen. Nach dem Trocknen und dem Entfernen des Lösungsmittels am Rotationsverdampfer wurden 9.10 g (42 % d.Th.) der Titelverbindung isoliert.
¹H-NMR (400MHz, DMSO-d₆): δ [ppm]= 4.36 (s, 2H), 7.14 - 7.24 (m, 2H), 7.30 - 7.39 (m, 3H), 7.71 - 7.80 (m, 2H).

### Beispiel 40A

### 2-[1-(2-Brom-5-fluorphenyl)-2-(2-fluorphenyl)ethyliden]hydrazincarboximidamid

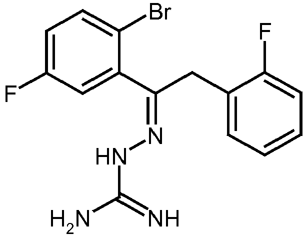

9.00 g (28.9 mmol) Beispiel 39A und 6.40 g (58.9 mmol) Aminoguanidin-Hydrochlorid wurden in Ethylenglykol (207 ml) vorgelegt und 9.24 g (65.1 mmol) Bortrifluorid-Diethylether-Komplex hinzugegeben. Das Reaktionsgemisch wurde 2 h bei 120°C an einer Destillationsbrücke erhitzt. Nach Abkühlung wurden nochmals 6.40 g (58.9 mmol) Aminoguanidin-Hydrochlorid sowie 9.24 g (65.1 mmol) Bortrifluorid-Diethylether-Komplex hinzugegeben und 3h bei 120°C gerührt. Nach dem Abkühlen wurde das Reaktionsgemisch langsam auf Wasser (800 ml) gegeben und mit 1N Natronlauge ein pH Wert von 11-12 eingestellt. Nach beginnender Niederschlagsbildung wurden 300 g Eis hinzugegeben und 15 min gerührt. Wegen der klebrigen Beschaffenheit des Niederschlags wurde das Wasser abdekantiert und der Rückstand noch zweimal mit je 200 ml Wasser ausgerührt. Der klebrige Niederschlag wurde in Diethylether gelöst, mit Wasser gewaschen, die organische Phase getrocknet, das Lösungsmittel am Rotationsverdampfer entfernt und 6.00 g (54 % d. Th.) der Titelverbindung als Schaums isoliert.
LC-MS (Methode 1): Rₜ = 0.80 min; MS (ESIpos): m/z = 367 + 369 [M+H]⁺

### Beispiel 41A

### 5-Fluor-3-(2-fluorbenzyl)-1H-indazol-1-carboximidamid

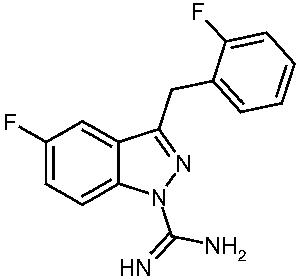

222 ml N-Methylpyrrolidon wurden auf 140°C erhitzt, 6.00 g (16.3 mmol) Beispiel 40A und 3.11 g (16.3 mmol) Kupfer-(I)-iodid hinzugegeben und 14 min bei 170°C Badtemperatur gerührt. Das Reaktionsgemisch wurde anschließend langsam auf 700 ml Eiswasser gegeben und konzentrierte, wäßrige Ammoniak-Lösung (230 mL) hinzugegeben. Nach 5 minütigem Rühren wurde 700 ml Essigsäureethylester hinzugegeben und 10 min gerührt. Die wässrige Phase wurde noch einmal mit Essigsäureethylester extrahiert und die vereinten organischen Phasen dreimal mit Wasser gewaschen. Nach dem Trocknen und dem Entfernen des Lösungsmittels am Rotationsverdampfer wurden 6.00 g (64 % d. Th., 50%ige Reinheit) Produkt erhalten. Das Rohprodukt wurde ohne Reinigung weiter umgesetzt.
LC-MS (Methode 3): Rₜ = 1.60 min; MS (ESIpos): m/z = 287 [M+H]⁺

### Beispiel 42A

### 4-Amino-2-[5-fluor-3-(2-fluorbenzyl)-1H-indazol-1-yl]-5,5-dimethyl-5,7-dihydro-6H-pyrrolo[2,3-d]pyrimidin-6-on

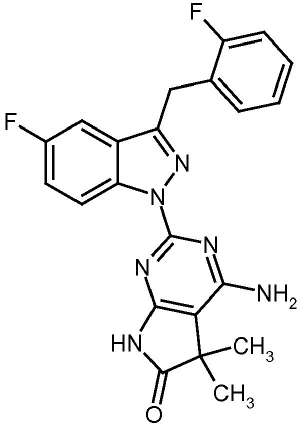

6.00 g (ca. 10.5 mmol, 50%-ige Reinheit) des Rohprodukts von Beispiel 41A und 5.22 g (31.4 mmol) Beispiel 12A wurden in tert.-Butanol (46.0 ml) vorgelegt und 2.00 g (17.8 mmol) Kalium-tert.-Butylat hinzugegeben. Das Reaktionsgemisch wurde 18 h zum Rückfluss erhitzt. Nach dem Abkühlen wurde mit Essigsäureethylester verdünnt und mit ca. 7%-iger wässriger Ammoniumchlorid-Lösung extrahiert. Die organische Phase wurde mit gesättigter, wässriger Natriumchloridlösung gewaschen, getrocknet und das Lösungsmittel am Rotationsverdampfer entfernt. Der Rückstand wurde an 600 ml Kieselgel mit Cyclohexan / Essigsäureethylester 2:3 chromatographisch gereinigt. Die produkthaltigen Fraktionen wurden eingeengt und mit ca. 20 ml Diethylether verrührt, abgesaugt und mit Diethylether gewaschen. Es wurden 1.80 g (37 % d. Th.) der Titelverbindung als Feststoff erhalten.
LC-MS (Methode 1): Rₜ = 1.00 min; MS (ESIpos): m/z = 421 [M+H]⁺

### Beispiel 43A

### 2-[5-Fluor-3-(2-fluorbenzyl)-1H-indazol-1-yl]-4-iod-5,5-dimethyl-5,7-dihydro-6H-pyrrolo[2,3-d]pyrimidin-6-on

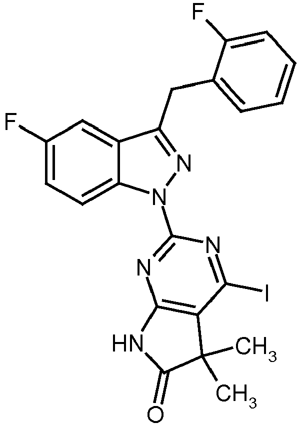

500 mg (1.189 mmol) von Beispiel 42A wurden in iso-Pentylnitrit (3.40 ml) und Diiodmethan (9.027 ml) vorgelegt und über Nacht auf 85°C erhitzt. Nach Abkühlen wurde über Kieselgel filtriert (Dichlormethan:Methanol - Gradient) und eingeengt. Der Rückstand wurde wurde mittels präparativer HPLC gereinigt (Acetonitril:Wasser (+0.05 % Ameisensäure) - Gradient). Es wurden 274 mg der Titelverbindung erhalten (43% d. Th.).
LC-MS (Methode 1): Rₜ = 1.33 min; MS (ESIpos): m/z = 532 [M+H]⁺
¹H-NMR (400 MHz, DMSO-d₆): δ [ppm] = 1.41 (s, 6H), 4.41 (s, 2H), 7.14-7.23 (m, 2H), 7.29-7.35 (m, 1H), 7.39-7.43 (ddd, 1H), 7.52-7.61 (m, 2H), 8.58 (d, 1H), 11.91 (s, 1H).

Neben der Titelverbindung wurden 72 mg (14 % d. Th., 83%ige Reinheit) 2-[5-Fluor-3-(2-fluorbenzyl)-1H-indazol-1-yl]-4-hydroxy-5,5-dimethyl-5,7-dihydro-6H-pyrrolo[2,3-d]pyrimidin-6-on (Beispiel 31) erhalten.

### Beispiel 44A

### Ethyl-8-(2-fluorbenzyl)imidazo[1,5-a]pyrimidin-6-carboxylat

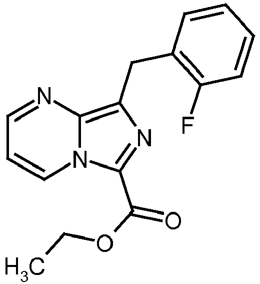

Die Darstellung der Verbindung ist beschrieben in: US 2010/29653, Seite 19, Beispiel 10A.

### Beispiel 45A

### 8-(2-Fluorbenzyl)imidazo[1,5-a]pyrimidin-6-carboxamid

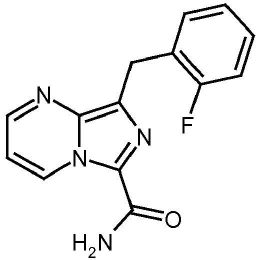

8.200 g (27.40 mmol) Ethyl-8-(2-fluorbenzyl)imidazo[1,5-a]pyrimidin-6-carboxylat wurden auf 8 Mikrowellengefäße verteilt. Jedes Gefäß wurde mit 10 ml einer 7N Lösung von Ammoniak in Methanol beschickt und 80 min bei 150°C in der Mikrowelle gerührt. Nach dem Abkühlen wurde der Inhalt der Gefäße vereinigt, der entstandene Niederschlag abgesaugt, mit wenig Methanol gewaschen und im Hochvakuum getrocknet. Es wurden 8.42 g (quant.) der Zielverbindung erhalten.
LC-MS (Methode 1) Rₜ = 0.76 min; MS (ESIpos): m/z = 271 [M+H]⁺

### Beispiel 46A

### 8-(2-Fluorbenzyl)imidazo[1,5-a]pyrimidin-6-carbonitril

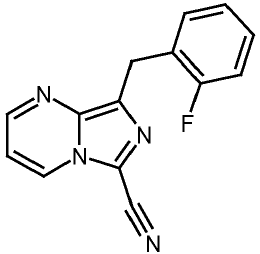

9.100 g (33.67 mmol) 8-(2-Fluorbenzyl)imidazo[1,5-a]pyrimidin-6-carboxamid wurden mit 150 ml Phosphorylchlorid versetzt und 2h bei 120°C gerührt. Das Reaktionsgemisch wurde am Rotationsverdampfer eingeengt und der Rückstand mit Wasser verrührt. Der Feststoff wurde abgesaugt, mit wenig Wasser gewaschen und im Hochvakuum getrocknet. Es wurden 8.02 g (92% d. Th.) der Zielverbindung erhalten.
LC-MS (Methode 1) Rₜ = 0.92 min; MS (ESIpos): m/z = 253 [M+H]⁺

### Beispiel 47A

### 8-(2-Fluorbenzyl)imidazo[1,5-a]pyrimidin-6-carboximidamid

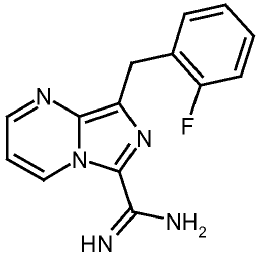

Unter Argonatmosphäre wurden 6.98 g (32.30 mmol) Natriummethylat (25%ige Lösung in Methanol) in 50 ml Methanol vorgelegt und mit 8.000 g (30.76 mmol) 8-(2-Fluorbenzyl)imidazo[1,5-a]pyrimidin-6-carbonitril gelöst in 40 ml absolutem Methanol versetzt. Das Reaktionsgemisch wurde 1 h bei RT gerührt. Es wurden 7.205 g (119.98 mmol) Essigsäure sowie 1.975 g (36.92 mmol) Ammoniumchlorid addiert und die Mischung 2h bei 50°C gerührt. Das Reaktionsgemisch wurde eingeengt und der Rückstand zwischen 150 ml Wasser und 100 ml Essigsäureethylester verteilt. Die wässrige Phase wurde mit 2N Natronlauge basisch gestellt (pH = 10) und die Phasen 1h bei RT gerührt. Es wurde Wasser addiert und dreimal mit Essigsäureethylester extrahiert. Die vereinigten organischen Phasen wurden mit Natriumsulfat getrocknet, eingeengt und der Rückstand im Hochvakuum getrocknet. Es wurden 7.53 g (Reinheit 73%, 66% d. Th.) der Zielverbindung erhalten.
LC-MS (Methode 1) Rₜ = 0.56 min; MS (ESIpos): m/z = 270 [M+H]⁺

### Beispiel 48A

### 4-Amino-2-[8-(2-fluorbenzyl)imidazo[1,5-a]pyrimidin-6-yl]-5,5-dimethyl-5,7-dihydro-6H-pyrrolo[2,3-d]pyrimidin-6-on

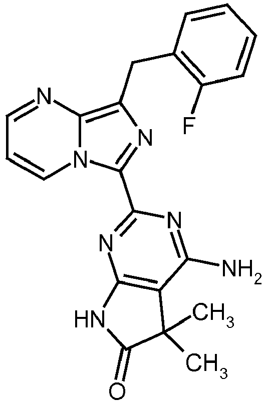

4.000 g (Reinheit 73%, 10.84 mmol) 8-(2-Fluorbenzyl)imidazo[1,5-a]pyrimidin-6-carboximidamid wurden in 25 ml *tert*.-Butanol vorgelegt, mit 2.162 g (13.01 mmol) Methyl-3,3-dicyan-2,2-dimethylpropanoat gelöst in 25 ml *tert*.-Butanol sowie 1.703 g (15.18 mmol) Kalium-*tert*.-butylat versetzt und 18 h unter Rückfluss erhitzt. Es wurden weitere 1.802 g (10.84 mmol) Methyl-3,3-dicyan-2,2-dimethylpropanoat zugegeben und für 5h unter Rückfluss gekocht. Es wurde zur Trockne eingeengt und der Rückstand mit Wasser/Isopropanol (v/v = 4:1) verrührt. Der Feststoff wurde abfiltriert und mit Methanol sowie Diethylether verrührt. Es wurde abgesaugt und der Rückstand im Hochvakuum getrocknet. Es wurden 1.90 g (Reinheit 90%, 39% d. Th.) der Zielverbindung erhalten.
LC-MS (Methode 1): Rₜ = 0.84 min; MS (ESIpos): m/z = 404 [M+H]⁺

### Beispiel 49A

### 2-[8-(2-Fluorbenzyl)imidazo[1,5-a]pyrimidin-6-yl]-4-iod-5,5-dimethyl-5,7-dihydro-6H-pyrrolo[2,3-d]pyrimidin-6-on

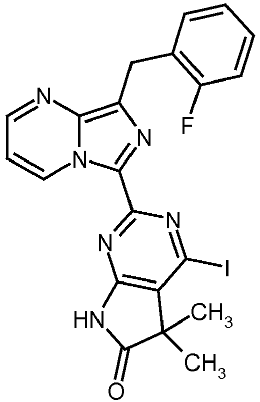

1.500 g (3.35 mmol) 4-Amino-2-[8-(2-fluorbenzyl)imidazo[1,5-a]pyrimidin-6-yl]-5,5-dimethyl-5,7-dihydro-6H-pyrrolo[2,3-d]pyrimidin-6-on wurden in 6.00 ml (44.56 mmol) Isopentylnitrit sowie 4.00 ml (49.66 mmol) Diiodmethan suspendiert und 2d bei 85°C gerührt. Es wurden 4.00 ml Isopentylnitrit sowie 5 ml NMP addiert und die Lösung 4h bei 85°C gerührt. Das Gemisch wurde am Rotationsverdampfer bis auf das NMP eingeengt und mittels päparativer HPLC gereinigt (Eluent: Acetonitril/Wasser mit 0.1% Ameisensäure, Gradient 20:80 → 100:0). Es wurden 928 mg (Reinheit 69%, 37% d. Th.) der Zielverbindung erhalten.
LC-MS (Methode 1 ): Rₜ = 1.11 min; MS (ESIpos): m/z = 515 [M+H]⁺

### Beispiel 50A

### 5-Fluor-6-methyl-1H-pyrazolo[3,4-b]pyridin-3-amin

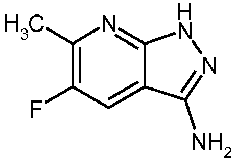

58 g (340.027 mmol) 2-Chlor-5-fluor-6-methylnicotinonitril (Darstellung beschrieben in WO2007/41052, Beispiel U-2, Seite 80) wurden in 1,2-Ethandiol (580 ml) vorgelegt und danach mit Hydrazinhydrat (24.813 ml) und 56.091 ml (340.027 mmol) Diisopropylethylamin versetzt. Das Reaktionsgemisch wurde unter Rühren für 16 h auf 80°C und danach für 66 h auf 120°C erhitzt. Nach Abkühlen wurde mit Wasser (2.5 1) und Essigsäureethylester (2.5 1) versetzt und abgesaugt. Der erhaltene Feststoff wurde getrocknet. Man erhielt 28.4 g (47% d. Th.) der Zielverbindung.
LC-MS (Methode 7): Rₜ = 1.77 min; MS (ESIpos): m/z = 167 [M+H]⁺

### Beispiel 51A

### 5-Fluor-3-iod-6-methyl-1H-pyrazolo[3,4-b]pyridin

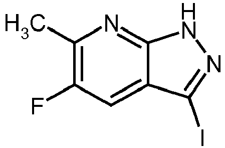

28 g (168.513 mmol) Beispiel 50A wurden in Analogie zu Beispiel 6A umgesetzt. Man erhielt nach Chromatographie an Kieselgel (Cyclohexan:Essigsäurethylester 9:1) 14.9 g (31 % d. Th.) der Titelverbindung.
LC-MS (Methode 1): Rₜ = 0.84 min; MS (ESIpos): m/z = 278 [M+H] ⁺

### Beispiel 52A

### 5-Fluor-1-(2-fluorbenzyl)-3-iod-6-methyl-1H-pyrazolo[3,4-b]pyridin

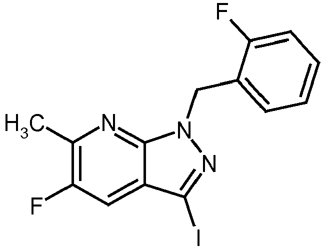

13 g (46.925 mmol) Beispiel 51A wurden in Analogie zu Beispiel 7A umgesetzt. Man erhielt nach Chromatographie an Kieselgel (Cyclohexan:Essigsäurethylester Gradient) 8.4 g (43 % d. Th.) der Titelverbindung.
LC-MS (Methode 1): Rₜ = 1.32 min; MS (ESIpos): m/z = 386 [M+H]⁺

### Beispiel 53A

### 5-Fluor-1-(2-fluorbenzyl)-6-methyl-1H-pyrazolo[3,4-b]pyridin-3-carbonitril

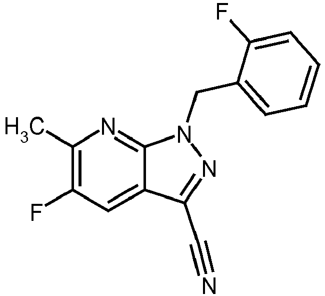

9.3 g (24.146 mmol) Beispiel 52A wurden in Analogie zu Beispiel 10A, Variante A, umgesetzt. Man erhielt nach Chromatographie an Kieselgel (Cyclohexan:Essigsäurethylester Gradient) 5.7 g (80 % d. Th.) der Titelverbindung.
LC-MS (Methode 1): Rₜ = 1.20 min; MS (ESIpos): m/z = 285 [M+H]⁺

### Beispiel 54A

### 5-Fluor-1-(2-fluorbenzyl)-6-methyl-1H-pyrazolo[3,4-b]pyridin-3-carboximidamid-Acetat

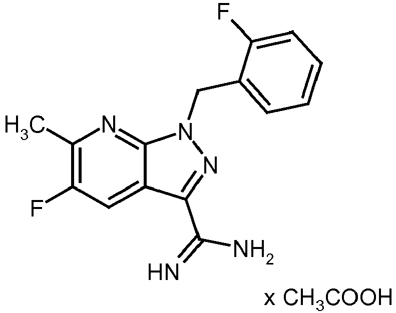

5.7 g (18.908 mmol, ca 95%ig) Beispiel 53A wurden in Analogie zu Beispiel 11A umgesetzt. Man erhielt 6.6 g (96 % d. Th.) der Titelverbindung.
LC-MS (Methode 1): Rₜ = 0.66 min; MS (ESIpos): m/z = 302 [M+H]⁺

### Beispiel 55A

### 4-Amino-2-[5-fluor-1-(2-fluorbenzyl)-6-methyl-1H-pyrazolo[3,4-b]pyridin-3-yl]-5,5-dimethyl-5,7-dihydro-6H-pyrrolo[2,3-d]pyrimidin-6-on

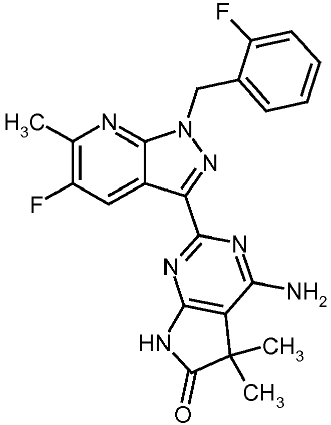

1 g (2.767 mmol) Beispiel 54A wurden in Analogie zu Beispiel 13A umgesetzt. Man erhielt 971 mg (80 % d. Th.) der Titelverbindung.
LC-MS (Methode 1): Rₜ = 1.05 min; MS (ESIpos): m/z = 436 [M+H]⁺

### Beispiel 56A

### 2-[5-Fluor-1-(2-fluorbenzyl)-6-methyl-1 H-pyrazolo [3,4-b]pyridin-3-yl]-4-iod-5,5-dimethyl-5,7-dihydro-6H-pyrrolo[2,3-d]pyrimidin-6-on

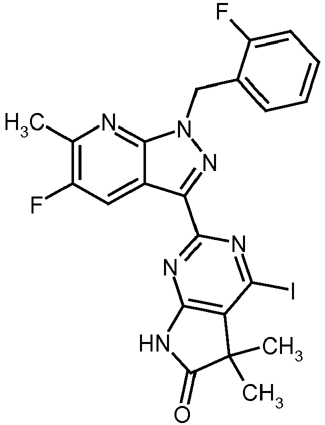

960 mg (2.205 mmol) Beispiel 55A wurden in Analogie zu Beispiel 15A umgesetzt. Man erhielt 749 mg (62 % d. Th., 84%-ige Reinheit) der Titelverbindung.
LC-MS (Methode 1): Rₜ = 1.35 min; MS (ESIpos): m/z = 547 [M+H]⁺.

Neben der Titelverbindung wurden ausserdem 99 mg (10 % d.Th.) von 2-[5-Fluor-1-(2-fluorbenzyl)-6-methyl-1H-pyrazolo[3,4-b]pyridin-3-yl]-4-hydroxy-5,5-dimethyl-5,7-dihydro-6H-pyrrolo[2,3-d]pyrimidin-6-on in diesem Ansatz. erhalten.

### Beispiel 57A

### 4'-Amino-2'-[5-fluor-1-(2-fluorbenzyl)-1H-pyrazolo[3,4-b]pyridin-3-yl]-4,5-dihydrospiro[furan-3,5'-pyrrolo[2,3-d]pyrimidin]-6'(7'H)-on

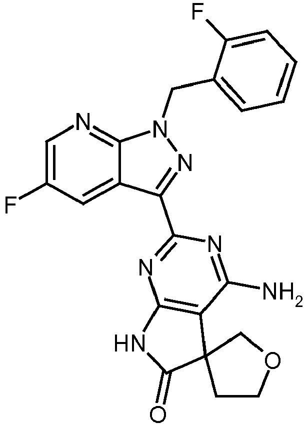

1.505 g (4.650 mmol) Beispiel 73A wurden in Analogie zu Beispiel 13A mit 0.903 g (4.650 mmol) Methyl-3-(dicyanmethyl)tetrahydrofuran-3-carboxylat (beschrieben in WO 2012/004259, Beispiel 12A, Seite 42) umgesetzt. Man erhielt 178 mg (8 % d. Th.) der Titelverbindung.
LC-MS (Methode 1): Rₜ = 0.99 min; MS (ESIpos): m/z = 450 [M+H]⁺

### Beispiel 58A

### 2'-[5-Fluor-1-(2-fluorbenzyl)-1H-pyrazolo[3,4-b]pyridin-3-yl]-4'-iod-4,5-dihydrospiro[furan-3,5'-pyrrolo[2,3-d]pyrimidin]-6'(7'H)-on

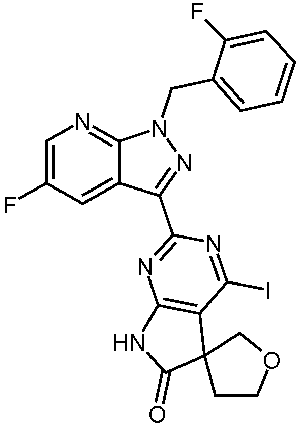

155 mg (0.345 mmol) Beispiel 57A wurden in Analogie zu WO 2012/004258, Beispiel 57A, Seite 97-98 umgesetzt. Man erhielt 86 mg (44 % d. Th.) der Titelverbindung.
LC-MS (Methode 1): Rₜ = 1.16 min; MS (ESIpos): m/z = 561 [M+H]⁺

### Beispiel 59A

### Ethyl-4-amino-2-[1-(2-fluorbenzyl)-1H-pyrazolo[3,4-b]pyridin-3-yl]-5-methyl-6-oxo-6,7-dihydro-5H-pyrrolo[2,3-d]pyrimidin-5-carboxylat

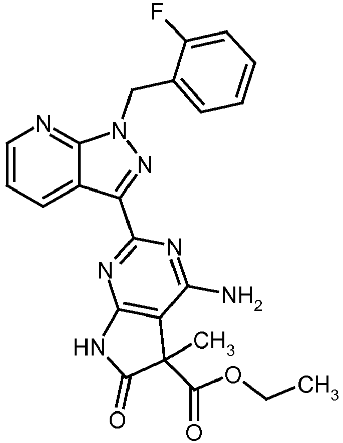

4.687 g (15.329 mmol) Beispiel 1A wurden in tert.-Butanol (120 ml) vorgelegt und mit 3.069 g (30.659 mmol) Kaliumhydrogencarbonat versetzt. Anschliessend wurden 4.2 g (17.629 mmol) Diethyl-(dicyanmethyl)(methyl)malonat zugegeben und die Mischung 5 h auf 85°C erhitzt. Anschliessend wurde mit Wasser versetzt, 30 min bei Raumtemperatur gerührt und danach ein Feststoff abgesaugt. Dieser wurde mit wenig Diethylether nachgewaschen. Nach Trocknung im Hochvakuum konnten 6.20 g der Titelverbindung erhalten (87 % d. Th.) werden.
LC-MS (Methode 1): Rₜ = 0.95 min; MS (ESIpos): m/z = 462 [M+H]⁺

### Beispiel 60A

### Ethyl-2-[1-(2-fluorbenzyl)-1H-pyrazolo[3,4-b]pyridin-3-yl]-4-iod-5-methyl-6-oxo-6,7-dihydro-5H-pyrrolo[2,3-d]pyrimidin-5-carboxylat

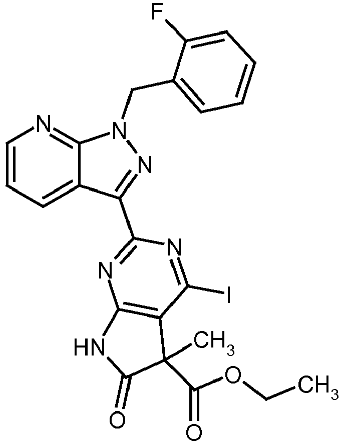

1.00 g (2.167 mmol) Beispiel 59A wurden in Analogie zur Vorschrift unter Beispiel 15A umgesetzt. Es wurden 0.887 g der Titelverbindung erhalten (71 % d. Th.).
LC-MS (Methode 1): Rₜ = 1.22 min; MS (ESIpos): m/z = 573 [M+H]⁺

Neben der Titelverbindung wurden 173 mg (17 % d. Th.) Ethyl-2-[1-(2-fluorbenzyl)-1H-pyrazolo[3,4-b]pyridin-3-yl]-4-hydroxy-5-methyl-6-oxo-6,7-dihydro-5H-pyrrolo[2,3-d]pyrimidin-5-carboxylat (Beispiel 101) erhalten.

### Beispiel 61A

### tert.-Butyl-(1-{2-[1-(2-fluorbenzyl)-1H-pyrazolo[3,4-b]pyridin-3-yl]-5,5-dimethyl-6-oxo-6,7-dihydro-5H-pyrrolo[2,3-d]pyrimidin-4-yl}pyrrolidin-3-yl)carbamat (Racemat)

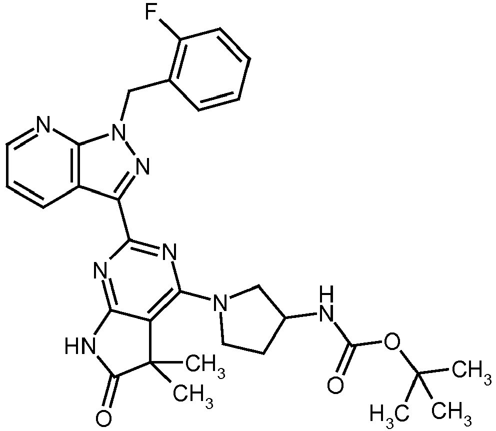

150 mg (0.21 mmol, Reinheit ca. 71%) 2-[1-(2-Fluorbenzyl)-1H-pyrazolo[3,4-b]pyridin-3-yl]-4-iod-5,5-dimethyl-5,7-dihydro-6H-pyrrolo[2,3-d]pyrimidin-6-on (Beispiel 15A) wurden in einem mikrowellengeeigneten Reaktionsgefäß in 1-Methyl-2-pyrrolidon (3.1 ml) gelöst, mit 0.22 ml (1.24 mmol) N,N-Diisopropylethylamin und mit 154 mg (0.83 mmol) tert.-Butyl-pyrrolidin-3-ylcarbamat versetzt. Anschließend wurde das Reaktionsgefäß mit einem Septum verschlossen und für 6 h bei 150°C in der Mikrowelle erhitzt. Nach Abkühlen wurde das Reaktionsgemisch mit Wasser versetzt, und Trifluoressigsäuredreimal mit Dichlormethan extrahiert. Die vereinigten organischen Phasen wurden über Natriumsulfat getrocknet, fitlriert und eingeengt. Der Rückstand wurde mittels präparativer HPLC gereinigt (Acetonitril:Wasser (+ 0.1 % Trifluoressigsäure) - Gradient). Es wurden 69 mg der Titelverbindung erhalten (59 % d. Th.).
LC-MS (Methode 1): Rₜ = 1.17 min; MS (EIpos): m/z = 573 [M+H]⁺.

### Beispiel 62A

### tert.-Butyl-(1-{2-[1-(2-fluorbenzyl)-1H-pyrazolo[3,4-b]pyridin-3-yl]-5,5-dimethyl-6-oxo-6,7-dihydro-5H-pyrrolo[2,3-d]pyrimidin-4-yl}azetidin-3-yl)carbamat

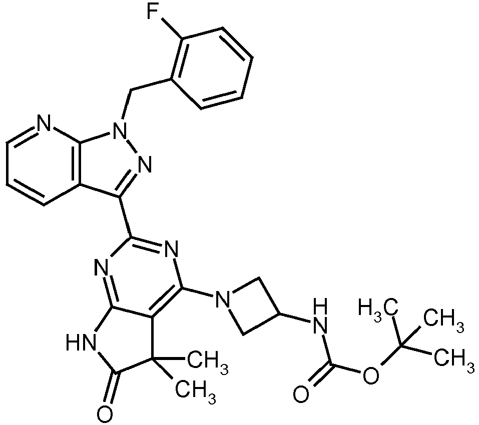

150 mg (0.21 mmol, Reinheit ca. 71%) 2-[1-(2-Fluorbenzyl)-1H-pyrazolo[3,4-b]pyridin-3-yl]-4-iod-5,5-dimethyl-5,7-dihydro-6H-pyrrolo[2,3-d]pyrimidin-6-on (Beispiel 15A) wurden in einem mikrowellengeeigneten Reaktionsgefäß in 1-Methyl-2-pyrrolidon (3.1 ml) gelöst, mit 0.22 ml (1.24 mmol) N,N-Diisopropylethylamin und mit 143 mg (0.83 mmol) tert.-Butyl-azetidin-3-ylcarbamat versetzt. Anschließend wurde das Reaktionsgefäß mit einem Septum verschlossen und für 8 h bei 150°C in der Mikrowelle erhitzt. Anschließend wurden nochmals 0.15 ml (0.82 mmol) N,N-Diisopropylethylamin und 107 mg (0.62 mmol) tert.-Butyl-azetidin-3-ylcarbamat hinzugegeben und die Reaktionsmischung wurde für 3 h bei 150°C in der Mikrowelle erhitzt. Nach Abkühlen wurde das Reaktionsgemisch mit Wasser versetzt und mittels präparativer HPLC gereinigt (Acetonitril:Wasser (+ 0.1 % Trifluoressigsäure) - Gradient). Es wurden 81 mg der Titelverbindung erhalten (64 % d. Th.; Reinheit 91%).
LC-MS (Methode 1): Rₜ = 1.15 min; MS (EIpos): m/z = 559 [M+H]⁺.

### Beispiel 63A

### 4-Chlor-6-methyl-1H-pyrazolo[3,4-d]pyrimidin

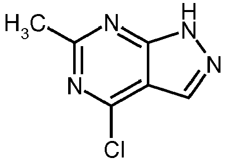

Die Darstellung der Verbindung erfolgte nach einer modifizierten Vorschrift aus : C.C. Cheng, R.K. Robins, J. Org. Chem. 1958, 23, 191.

4.878 g (33.2 mmol) 6-Methyl-1H-pyrazolo[3,4-d]pyrimidin-4-ol (J. Org. Chem. 1958, 23, 191) wurden in 50 ml Toluol vorgelegt, mit 15.5 ml (165.8 mmol) Phosphorylchlorid und 12.7 ml (72.9 mmol) Diisopropylethylamin versetzt und 1h bei 80°C gerührt. Es wurde eingeengt und zwischen Essigsäureethylester und 1 M Salzsäure verteilt. Die Organische Phase wurde über Natriumsulfat getrocknet und eingengt. Der Rückstand (4.464 g, 92% Reinheit, 73% d.Th.) wurde roh weiterverarbeitet.
LC-MS (Methode 1): Rₜ = 0.53 min; MS (ESIpos): m/z = 169 (M+H)⁺

### Beispiel 64A

### 6-Methyl-1H-pyrazolo[3,4-d]pyrimidin

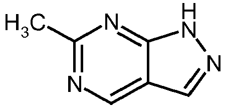

4.464 g (ca. 24.28 mmol, Reinheit 92%) 4-Chlor-6-methyl-1H-pyrazolo[3,4-d]pyrimidin wurden in 180 ml Dioxan gelöst und mit 2.948 g (29.14 mmol) Triethylamin sowie 5.629 g 20%-iges Palladiumhydroxid auf Kohle versetzt und bei 3 bar Wasserstoff-Druck und RT für 2 Tage hydriert. Es wurden 100 ml Essigsäureethylester, 2.948 g (29.14 mmol Triethylamin sowie 2.000 g 20%-iges Palladiumhydroxid auf Kohle addiert. Das Gemisch wurde mit Wasserstoff bei 3 bar Wasserstoff-Druck und RT für für 3 h hydriert. Das Reaktionsgemisch wurde über Celite filtriert mit wenig Dioxan/Essigsäureethylester gewaschen und das Filtrat am Rotationsverdampfer eingeengt. Es wurden 2.180 g (Reinheit 73%, 49% d. Th.) der Zielverbindung erhalten.
LC-MS (Methode 4): Rₜ = 0.40 min; MS (ESIpos): m/z = 135 (M+H)⁺

### Beispiel 65A

### 3-Iod-6-methyl-1H-pyrazolo[3,4-d]pyrimidin

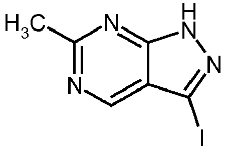

Es wurden 2.180 g (Reinheit 73%, ca. 11.82 mmol) 6-Methyl-1H-pyrazolo[3,4-d]pyrimidin und 3.987 g (17.72 mmol) N-Iodsuccinimid in 30 ml DMF gelöst und 2 h bei 80°C erhitzt. Nach dem Abkühlen wurde das Gemisch am Rotationsverdampfer eingeengt und der Rückstand mit Dichlormethan verrührt, abgesaugt und im Hochvakuum getrocknet. Es wurden 7.950 g (ca. 38%-ige Reinheit) der Zielverbindung erhalten.
LC-MS (Methode 1): Rₜ = 0.52 min; MS (ESIpos): m/z = 261 (M+H)⁺

### Beispiel 66A

### 1-(2-Fluorbenzyl)-3-iod-6-methyl-1H-pyrazolo[3,4-d]pyrimidin

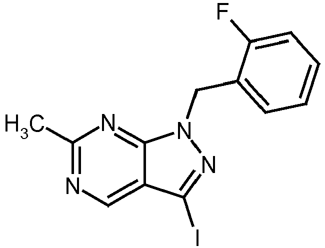

Es wurden 7.950 g (13.76 mmol) 3-Iod-6-methyl-1H-pyrazolo[3,4-d]pyrimidin und 4.930 g (15.13 mmol) Cäsiumcarbonat in 20 ml DMF vorgelegt und mit 2.860 g (15.13 mmol) 2-Fluorbenzylbromid gelöst in 5 ml DMF versetzt. Das Reaktionsgemisch wurde über Nacht bei RT gerührt, mit 100 ml Wasser verdünnt und mit Essigsäureethylester extrahiert. Die organische Phase wurde über Natriumsulfat getrocknet und am Rotationsverdampfer eingeengt. Der Rückstand wurde mittels präparativer HPLC (Eluent: Acetonitril/Wasser, Gradient 30:70 → 95:5) gereinigt. Es wurden 1.030 g der Zielverbindung erhalten (20% d. Th.).
LC-MS (Methode 4): Rₜ = 2.27 min; MS (ESIpos): m/z = 369 (M+H)⁺

### Beispiel 67A

### 1-(2-Fluorbenzyl)-6-methyl-1H-pyrazolo[3,4-d]pyrimidin-3-carbonitril

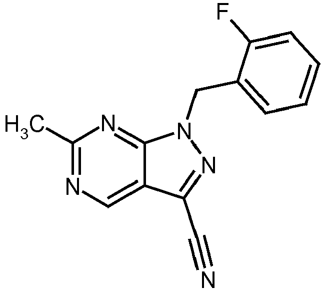

Es wurden 1.485 g (4.03 mmol) 1-(2-Fluorbenzyl)-3-iod-6-methyl-1H-pyrazolo[3,4-d]pyrimidin und 397 mg (4.44 mmol) Kupfer(I)cyanid in 11 ml absolutem DMSO vorgelegt und 2 h bei 150°C erhitzt. Nach dem Abkühlen wurde das Reaktionsgemisch über Celite filtriert und mit Essigsäureethylester sowie THF nachgewaschen. Die organische Phase wurde mit 25%-iger wässriger Ammoniak-Lösung, gesättigter wässriger Ammoniumchlorid-Lösung sowie gesättigter wässriger Natriumchlorid-Lösung gewaschen, über Natriumsulfat getrocknet und am Rotationsverdampfer eingeengt. Es wurden 994 mg (Reinheit 81%, 75 % d. Th.) der Zielverbindung erhalten.
LC-MS (Methode 1): Rₜ = 0.96 min; MS (ESIpos): m/z = 268 (M+H)⁺

### Beispiel 68A

### 1-(2-Fluorbenzyl)-6-methyl-1H-pyrazolo[3,4-d]pyrimidin-3-carboximidamid

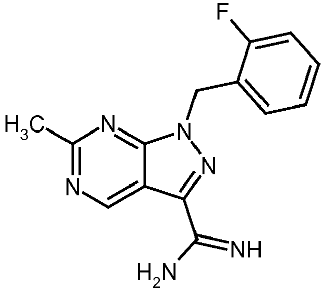

Unter einer Argonatmosphäre wurden 994 mg (Reinheit 81%, ca. 3.01 mmol) 1-(2-Fluorbenzyl)-6-methyl-1H-pyrazolo[3,4-d]pyrimidin-3-carbonitril in 15 ml absolutem Methanol gelöst. Es wurden 209 mg (3.72 mmol) Natriummethylat zugegeben und 1 h bei RT gerührt. Anschließend wurden weitere 31 mg (0.56 mmol) Natriummethylat zugegeben und 15 min bei RT gerührt. Es wurden 871 mg (14.50 mmol) Essigsäure sowie 489 mg (4.46 mmol) Ammoniumchlorid addiert und die Mischung für 45 min bei 45°C gerührt. Das Reaktionsgemisch wurde eingeengt, der Rückstand mit 1N Natronlauge verrührt, der Niederschlag abgesaugt und im Hochvakuum getrocknet. Es wurden 918 mg (Reinheit 91%, 97% d. Th.) der Zielverbindung erhalten.
LC-MS (Methode 2) Rₜ = 0.53 min; MS (ESIpos): m/z = 285 (M+H)⁺
¹H-NMR (400MHz, DMSO-d₆): δ [ppm]= 2.75 (s, 3H), 5.65 - 5.73 (m, 1H), 5.71 (s, 2H), 7.10 - 7.18 (m, 2H), 7.19 - 7.29 (m, 1H), 7.33 - 7.43 (m, 1H), 9.51 (s, 1H).

### Beispiel 69A

### 4-Amino-2-[1-(2-fluorbenzyl)-6-methyl-1H-pyrazolo[3,4-d]pyrimidin-3-yl]-5,5-dimethyl-5,7-dihydro-6H-pyrrolo[2,3-d]pyrimidin-6-on

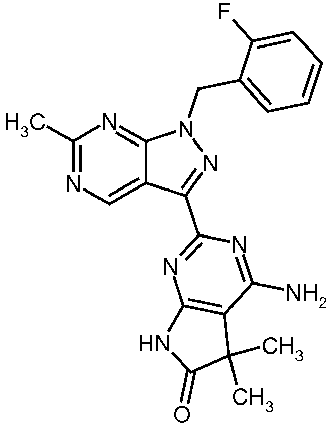

200 mg (0.70 mmol) 1-(2-Fluorbenzyl)-6-methyl-1H-pyrazolo[3,4-d]pyrimidin-3-carboximidamid wurden mit 3 ml *tert*.-Butanol, eine Lösung von 146 mg (0.70 mmol) Methyl-3,3-dicyan-2,2-dimethylpropanoat in 1.5 ml *tert*.-Butanol sowie 94 mg (0.84 mmol) Kalium-*tert*.-butylat versetzt und 48 h zum Rückfluss erhitzt. Es wurde Wasser addiert und der Niederschlag abfiltriert. Das Filtrat wurde mit Dichlormethan extrahiert, die organische Phase über Natriumsulfat getrocknet und am Rotationsverdampfer eingeengt. Der Rückstand wurde mit Wasser/Ethanol verrührt. Der Feststoff wurde abfiltriert und im Hochvakuum getrocknet. Es wurden 102 mg (34% d. Th.) der Zielverbindung erhalten.
LC-MS (Methode 1): Rₜ = 0.81 min; MS (ESIpos): m/z = 419 (M+H)⁺

### Beispiel 70A

### 2-[1-(2-Fluorbenzyl)-6-methyl-1H-pyrazolo[3,4-d]pyrimidin-3-yl]-4-iod-5,5-dimethyl-5,7-dihydro-6H-pyrrolo[2,3-d]pyrimidin-6-on

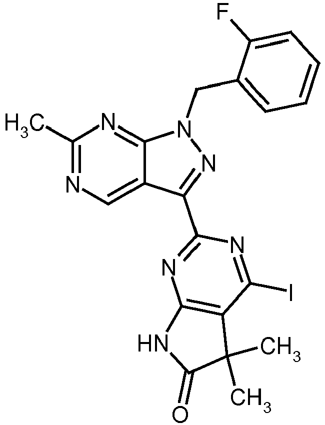

70 mg (0.17 mmol) 4-Amino-2-[1-(2-fluorbenzyl)-6-methyl-1H-pyrazolo[3,4-d]pyrimidin-3-yl]-5,5-dimethyl-5,7-dihydro-6H-pyrrolo[2,3-d]pyrimidin-6-on wurden mit 3.770 g (14.08 mmol) Diiodmethan sowie 411 mg (3.51 mmol) Isopentylnitrit versetzt. Das Gemisch wurde 8 h bei 85 °C gerührt. Nach dem Abkühlen wurde mit Acetonitril verdünnt und das Gemisch mittels päparativer HPLC gereinigt (Eluent: Acetonitril/Wasser mit, Gradient 30:70 → 95:5). Es wurden 35 mg (24% d. Th.) der Zielverbindung erhalten.
LC-MS (Methode 4): Rₜ = 2.37 min; MS (ESIpos): m/z = 530 (M+H)⁺

Ausserdem wurden 10 mg (14% d. Th.) 2-[1-(2-Fluorbenzyl)-6-methyl-1H-pyrazolo[3,4-d]pyrimidin-3-yl]-4-hydroxy-5,5-dimethyl-5,7-dihydro-6H-pyrrolo[2,3-d]pyrimidin-6-on erhalten.

### Beispiel 71A

### 4-Amino-2-[1-(2,3-difluorbenzyl)-5-fluor-1H-pyrazolo[3,4-b]pyridin-3-yl]-5,5-dimethyl-5,7-dihydro-6H-pyrrolo[2,3-d]pyrimidin-6-on

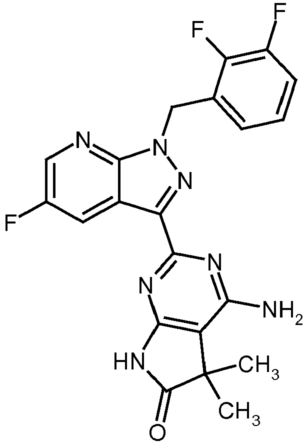

In Analogie zur Darstellung von Beispiel 13A wurden 5.00 g (13.687 mmol) 1-(2,3-Difluorbenzyl)-5-fluor-1H-pyrazolo[3,4-b]pyridin-3-carboximidamid-Acetat (Beispiel 64A aus WO 2012/004258, Seite 102-103) umgesetzt. Es wurden 5.13 g der Titelverbindung erhalten (85 % d. Th.).
LC-MS (Methode 1) Rₜ = 0.97 min; MS (ESIpos): m/z = 440 [M+H]⁺

### Beispiel 72A

### 2-[1-(2,3-Difluorbenzyl)-5-fluor-1H-pyrazolo[3,4-b]pyridin-3-yl]-4-iod-5,5-dimethyl-5,7-dihydro-6H-pyrrolo[2,3-d]pyrimidin-6-on

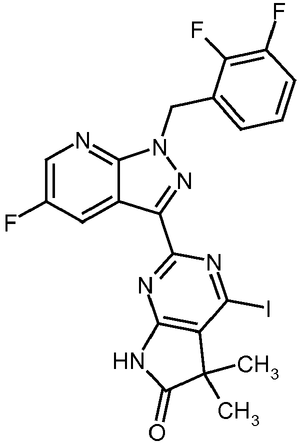

In Analogie zur Darstellung von Beispiel 16A wurden 5.11 g (11.629 mmol) Beispiel 71A umgesetzt. Es wurden 2.39 g der Titelverbindung erhalten (85 % d. Th.).
LC-MS (Methode 1) Rₜ = 1.25 min; MS (ESIpos): m/z = 551 [M+H]⁺

Neben der Titelverbindung wurden 660 mg (12 % d. Th.) 2-[1-(2,3-Difluorbenzyl)-5-fluor-1H-pyrazolo[3,4-b]pyridin-3-yl]-4-hydroxy-5,5-dimethyl-5,7-dihydro-6H-pyrrolo[2,3-d]pyrimidin-6-on (Beispiel 116) erhalten.

### Beispiel 73A

### 5-Fluor-1-(2-fluorbenzyl)-1H-pyrazolo[3,4-b]pyridin-3-carboximidamid-Hydrochlorid

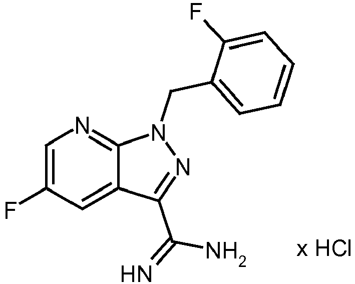

406.0 g (1.50 mol) der Verbindung aus Beispiel 10A wurden in 2.08 L Ethanol suspendiert. Anschließend wurden 54.1 g (0.30 mol) Natriummethanolat in Methanol (30%ig) zugegeben und über Nacht bei Raumtemperatur gerührt. Es wurden 88.4 g (1.65 mol) Ammoniumchlorid zugegeben, auf 65°C erhitzt und 3.5 h bei 65°C gerührt. Die Lösemittel wurden abdestilliert und der Rückstand mit 1.6 L Ethylacetat über Nacht gerührt. Der ausgefallene Feststoff wurde abgesaugt, zweimal mit je 140 ml Ethylacetat gewaschen und im Vakuumtrockenschrank bei 50°C unter leichtem Stickstoffstrom getrocknet. Man erhielt 441.4 g (90.7 % d. Th.) der Titelverbindung.
MS (ESIpos): m/z = 288 (M+H)⁺
¹H-NMR (400 MHz, DMSO-d₆): δ = 5.90 (s, 2H), 7.15 - 7.20 (m, 1H), 7.22 - 7.28 (m, 1H), 7.29 - 7.35 (m, 1H), 7.36 - 7.43 (m, 1H), 8.48 (dd, 1H), 8.86 (dd, 1H), 9.35 (br. s, 3H) ppm.

### Ausführungsbeispiele:

### Beispiel 1

### 2-[1-(2-Fluorbenzyl)-1H-pyrazolo[3,4-b]pyridin-3-yl]-4-methoxy-5,5-dimethyl-5,7-dihydro-6H-pyrrolo[2,3-d]pyrimidin-6-on

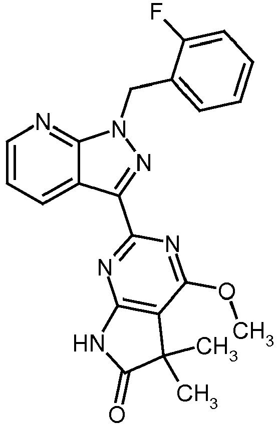

100 mg (0.194 mmol) Beispiel 15A wurden in einem mikrowellengeeigneten Reaktionsgefäß mit Methanol (3 ml), 126 mg (0.389 mmol) Cäsiumcarbonat, 3.7 mg (0.019 mmol) Kupfer-(I)-iodid und 9 mg (0.039 mmol) 3,4,7,8-Tetramethyl-1,10-Phenanthrolin versetzt. Es wurde unter Ultraschallbehandlung für 5 min mit Argon gespült und anschliessend mit einem entsprechenden Septum verschlossen. Danach wurde in 3 Zyklen für jeweils 2 h bei 140 °C in der Mikrowelle erhitzt. Nach Abkühlen wurde das Reaktionsgemisch filtriert, eingeengt und der Rückstand wurde mittels präparativer HPLC gereinigt (Acetonitril:Wasser (+0.05 % Ameisensäure) - Gradient). Es wurden 32 mg der Titelverbindung erhalten (39 % d. Th.).
LC-MS (Methode 1): Rₜ = 1.08 min; MS (EIpos): m/z = 419 [M+H]⁺.
¹H-NMR (400 MHz, DMSO-d₆): δ [ppm] = 1.35 (s, 6H), 4.15 (s, 3H), 5.87 (s, 2H), 7.13-7.26 (m, 3H), 7.34-7.38 (m, 1H), 7.45 (dd, 1H), 8.68 (dd, 1H), 8.91 (dd, 1H), 11.44 (s br, 1H).

### Beispiel 2

### 2-[5-Fluor-1-(2-fluorbenzyl)-1H-pyrazolo[3,4-b]pyridin-3-yl]-4-[(2-hydroxyethyl)amino]-5,5-dimethyl-5,7-dihydro-6H-pyrrolo[2,3-d]pyrimidin-6-on

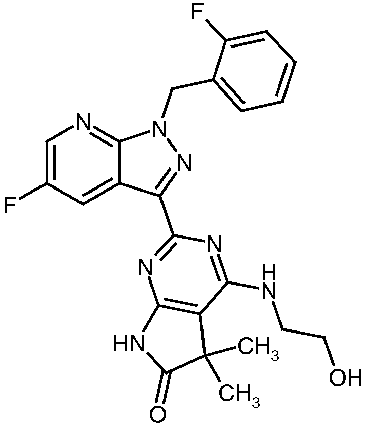

100 mg (0.115 mmol, ca. 61% Reinheit) Beispiel 16A wurden in einem mikrowellengeeigneten Reaktionsgefäß in 1-Methyl-2-pyrrolidon (2 ml) gelöst und mit 0.75 ml Aminoethanol versetzt. Anschliessend wurde mit einem entsprechenden Septum verschlossen und für 5 h bei 150 °C in der Mikrowelle erhitzt. Nach Abkühlen wurde das Reaktionsgemisch mittels präparativer HPLC gereinigt (Acetonitril:Wasser (+0.05 % Ameisensäure) - Gradient). Es wurden 68 mg der Titelverbindung erhalten (100 % d. Th., 94 %-ige Reinheit laut LC/MS).
LC-MS (Methode 3): Rₜ = 0.94 min; MS (EIpos): m/z = 466 [M+H]⁺.
¹H-NMR (400 MHz, DMSO-d₆): δ [ppm] = 1.36 (s, 6H), 3.29 (s, Signal überlagert von Wasser-Signal, 2H), 3.64 (s, 2H), 4.82 (t, 1H), 5.83 (s, 2H), 6.65 (t br, 1H), 7.13-7.25 (m, 3H), 7.33-7.39 (m, 1H), 8.55 (dd, 1H), 8.71 (dd, 1H), 11.00 (s br, 1H).

### Beispiel 3

### 4-[(2-Amino-2-methylpropyl)amino]-2-[5-fluor-1-(2-fluorbenzyl)-1H-pyrazolo[3,4-b]pyridin-3-yl]-5,5-dimethyl-5,7-dihydro-6H-pyrrolo[2,3-d]pyrimidin-6-on

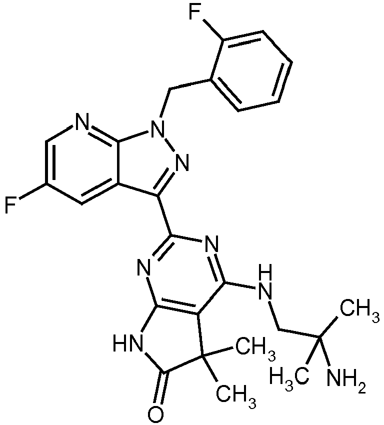

100 mg (0.115 mmol, ca. 61% Reinheit) Beispiel 16A wurden in einem mikrowellengeeigneten Reaktionsgefäß in 1-Methyl-2-pyrrolidon (2 ml) gelöst und mit 0.75 ml 2-Methylpropan-1,2-diamin versetzt. Anschliessend wurde mit einem entsprechenden Septum verschlossen und für 3 h bei 150 °C in der Mikrowelle erhitzt. Nach Abkühlen wurde das Reaktionsgemisch mittels präparativer HPLC gereinigt (Acetonitril:Wasser (+0.05 % Ameisensäure) - Gradient). Es wurden 57 mg der Titelverbindung erhalten (100 % d. Th.).
LC-MS (Methode 3): Rₜ = 0.82 min; MS (EIpos): m/z = 493 [M+H]⁺.
¹H-NMR (400 MHz, DMSO-d₆): δ [ppm] = 1.21 (s, 6H), 1.41 (s, 6H), 3.67 (Signal überlagert mit Wasser-Signal wahrscheinlich 2H), 5.84 (s, 2H), 6.98 (m br, 1H), 7.15 (t, 1H), 7.20-7.26 (m, 2H), 7.34-7.39 (m, 1H), 8.56 (dd, 1H), 8.73 (dd, 1H), Signale für -NH-C=O und -NH₂ nicht beobachtet.

### Beispiel 4

### 2-[5-Fluor-1-(2-fluorbenzyl)-1H-pyrazolo[3,4-b]pyridin-3-yl]-4-[(2-hydroxy-2-methylpropyl)amino]-5,5-dimethyl-5,7-dihydro-6H-pyrrolo[2,3-d]pyrimidin-6-on

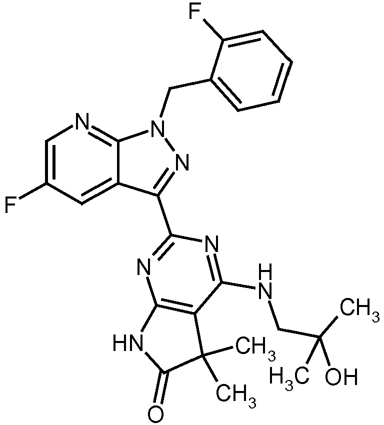

100 mg (0.115 mmol, ca. 61% Reinheit) Beispiel 16A wurden in einem mikrowellengeeigneten Reaktionsgefäß in 1-Methyl-2-pyrrolidon (2 ml) gelöst und mit 0.5 ml 1-Amino-2-methylpropan-2-ol versetzt. Anschliessend wurde mit einem entsprechenden Septum verschlossen und für 3 h bei 150 °C in der Mikrowelle erhitzt. Nach Abkühlen wurde das Reaktionsgemisch mittels präparativer HPLC gereinigt (Acetonitril:Wasser (+0.05 % Ameisensäure) - Gradient). Es wurden 56 mg der Titelverbindung erhalten (100 % d. Th.).
LC-MS (Methode 1): Rₜ = 1.06 min; MS (EIpos): m/z = 494 [M+H]⁺.
¹H-NMR (400 MHz, DMSO-d₆): δ [ppm] = 1.15 (s, 6H), 1.39 (s, 6H), 3.60 (d, 2H), 4.76 (s, 1H), 5.82 (s, 2H), 6.41 (t, 1H), 7.15 (t, 1H), 7.20-7.26 (m, 2H), 7.34-7.39 (m, 1H), 8.64 (dd, 1H), 8.72 (dd, 1H), 11.06 (s br, 1H).

### Beispiel 5

### 2-[5-Fluor-1-(2-fluorbenzyl)-1H-pyrazolo[3,4-b]pyridin-3-yl]-5,5-dimethyl-4-[(3,3,3-trifluorpropyl)amino]-5,7-dihydro-6H-pyrrolo[2,3-d]pyrimidin-6-on

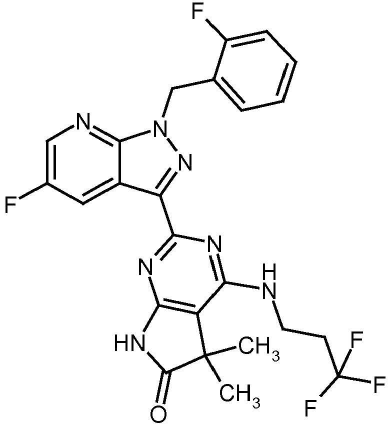

100 mg (0.115 mmol, ca. 61% Reinheit) Beispiel 16A wurden in einem mikrowellengeeigneten Reaktionsgefäß in 1-Methyl-2-pyrrolidon (2 ml) gelöst und mit 0.5 ml 3,3,3-Trifluorpropyl-1-amin versetzt. Anschliessend wurde mit einem entsprechenden Septum verschlossen und für 3 h bei 150 °C in der Mikrowelle erhitzt. Nach Abkühlen wurde das Reaktionsgemisch mittels präparativer HPLC gereinigt (Acetonitril:Wasser (+0.05 % Ameisensäure) - Gradient). Es wurden 60 mg der Titelverbindung erhalten (100 % d. Th.).
LC-MS (Methode 1): Rₜ = 1.21 min; MS (EIpos): m/z = 518 [M+H]⁺.
¹H-NMR (400 MHz, DMSO-d₆): δ [ppm] = 1.35 (s, 6H), 2.64-2.71 (m, 2H), 3.82 (q, 2H), 5.83 (s, 2H), 6.88 (t, 1H), 7.15 (t, 1H), 7.20-7.25 (m, 2H), 7.34-7.40 (m, 1H), 8.48 (dd, 1H), 8.72 (dd, 1H), 11.10 (s, 1H).

### Beispiel 6

### 4-[(2-Amino-3,3,3-trifluorpropyl)amino]-2-[5-fluor-1-(2-fluorbenzyl)-1H-pyrazolo[3,4-b]pyridin-3-yl]-5,5-dimethyl-5,7-dihydro-6H-pyrrolo[2,3-d]pyrimidin-6-on

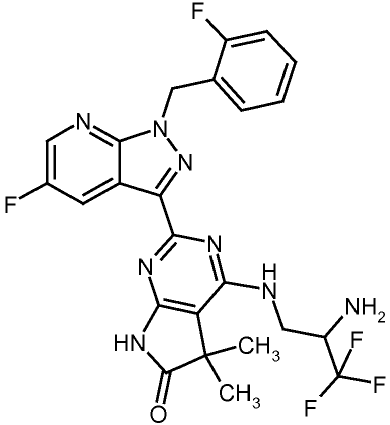

100 mg (0.115 mmol, ca. 61% Reinheit) Beispiel 16A wurden in einem mikrowellengeeigneten Reaktionsgefäß in 1-Methyl-2-pyrrolidon (2 ml) suspendiert, mit 1 ml Diisopropylethylamin und anschliessend mit 300 mg (1.492 mmol) 1-(Trifluormethyl)ethylen-1,2-diamin-Dihydrochlorid versetzt. Anschliessend wurde mit einem entsprechenden Septum verschlossen und für 3 h bei 150 °C in der Mikrowelle erhitzt. Nach Abkühlen wurde das Reaktionsgemisch mittels präparativer HPLC gereinigt (Acetonitril:Wasser (+0.05 % Ameisensäure) - Gradient). Es wurden 5.7 mg der Titelverbindung erhalten (9 % d. Th.).
LC-MS (Methode 1): Rₜ = 0.98 min; MS (EIpos): m/z = 534 [M+H]⁺.
H-NMR (400 MHz, DMSO-d₆): δ [ppm] = 1.37 (d, 6H), 3.47-3.45 (m, 1H), 3.62-3.73 (m, 1H), 3.94-4.00 (m, 1H), 5.83 (s, 2H), 6.82 (t, 1H), 7.15 (t, 1H), 7.20-7.26 (m, 2H), 7.34-7.39 (m, 1H), 8.56 (dd, 1H), 8.72 (br d, 1H), 11.11 (br s, 1H), -NH₂ nicht beobachtet

### Beispiel 7

### 4-[(2,2-Difluorethyl)amino]-2-[5-fluor-1-(2-fluorbenzyl)-1H-pyrazolo[3,4-b]pyridin-3-yl]-5,5-dimethyl-5,7-dihydro-6H-pyrrolo[2,3-d]pyrimidin-6-on

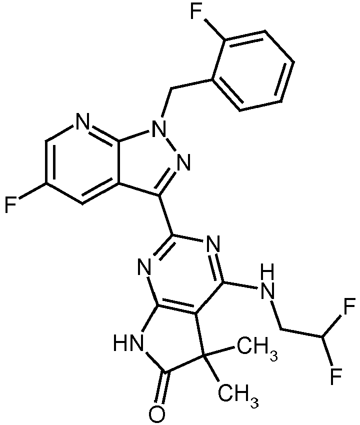

100 mg (0.115 mmol, ca. 61% Reinheit) Beispiel 16A wurden in einem mikrowellengeeigneten Reaktionsgefäß in 1-Methyl-2-pyrrolidon (2 ml) gelöst und mit 0.5 ml 2,2-Difluorethylamin versetzt. Anschliessend wurde mit einem entsprechenden Septum verschlossen und für 3 h bei 150 °C in der Mikrowelle erhitzt. Nach Abkühlen wurde das Reaktionsgemisch mittels präparativer HPLC gereinigt (Acetonitril:Wasser (+0.05 % Ameisensäure) - Gradient). Es wurden 30 mg der Titelverbindung erhalten (55 % d. Th., 100 %).
LC-MS (Methode 1): Rₜ = 1.15 min; MS (EIpos): m/z = 486 [M+H]⁺.
¹H-NMR (400 MHz, DMSO-d₆): δ [ppm] = 1.37(s, 6H), 3.91-4.00 (m, 2H), 5.83 (s, 2H), 6.13-6.43 (m, 1H), 7.04 (t, 1H), 7.15 (dd, 1H), 7.20-7.25 (m, 2H), 7.34-7.39 (m, 1H), 8.46 (dd, 1H), 8.72 (dd, 1H), 11.13 (s, 1H).

### Beispiel 8

### 4-(3-Fluorazetidin-1-yl)-2-[5-fluor-1-(2-fluorbenzyl)-1H-pyrazolo[3,4-b]pyridin-3-yl]-5,5-dimethyl-5,7-dihydro-6H-pyrrolo[2,3-d]pyrimidin-6-on

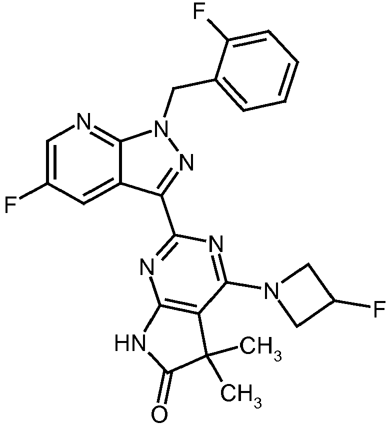

100 mg (0.115 mmol, ca. 61% Reinheit) Beispiel 16A wurden in einem mikrowellengeeigneten Reaktionsgefäß in 1-Methyl-2-pyrrolidon (2 ml) gelöst, mit 0.314 ml (1.800 mmol) N,N-Diisopropylethylamin und mit 200 mg (1.793 mmol) 3-Fluorazetidin-Hydrochlorid versetzt. Anschliessend wurde mit einem entsprechenden Septum verschlossen und für 3 h bei 150 °C in der Mikrowelle erhitzt. Nach Abkühlen wurde das Reaktionsgemisch mittels präparativer HPLC gereinigt (Acetonitril:Wasser (+0.05 % Ameisensäure) - Gradient). Es wurden 22 mg der Titelverbindung erhalten (41 % d. Th.).
LC-MS (Methode 1): Rₜ = 1.19 min; MS (EIpos): m/z = 480 [M+H]⁺.
¹H-NMR (400 MHz, DMSO-d₆): δ [ppm] = 1.35 (s, 6H), 4.34-4.42 (m, 2H), 4.63-4.73 (m, 2H), 5.48-5.66 (m, 1H), 5.84 (s, 2H), 7.13-7.25 (m, 3H), 7.34-7.40 (m, 1H), 8.52 (dd, 1H), 8.73 (dd, 1H), 11.27 (s, 1H).

### Beispiel 9

### 4-[(Dicyclopropylmethyl)amino]-2-[5-fluor-1-(2-fluorbenzyl)-1H-pyrazolo[3,4-b]pyridin-3-yl]-5,5-dimethyl-5,7-dihydro-6H-pyrrolo[2,3-d]pyrimidin-6-on

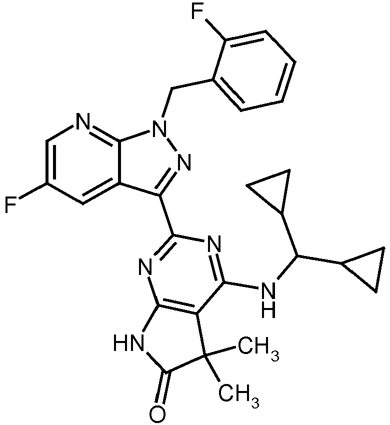

100 mg (0.115 mmol, ca. 61% Reinheit) Beispiel 16A wurden in einem mikrowellengeeigneten Reaktionsgefäß in 1-Methyl-2-pyrrolidon (2 ml) gelöst und mit 0.5 ml Dicyclopropylmethylamin versetzt. Anschliessend wurde mit einem entsprechenden Septum verschlossen und für 3 h bei 150 °C in der Mikrowelle erhitzt. Nach Abkühlen wurde das Reaktionsgemisch mittels präparativer HPLC gereinigt (Acetonitril:Wasser (+0.05 % Ameisensäure) - Gradient). Es wurden 25 mg der Titelverbindung erhalten (42 % d. Th.,).
LC-MS (Methode 1): Rₜ = 1.34 min; MS (EIpos): m/z = 516 [M+H]⁺.
¹H-NMR (400 MHz, DMSO-d₆): δ [ppm] = 0.28-0.41 (m, 6H), 0.49-0.58 (m, 2H), 1.25-1.34 (m, 2H), 1.40 (s, 6H), 3.49 (dd, 1H), 5.81 (s, 2H), 6.40 (d, 1H), 7.14 (t, 1H), 7.20-7.24(m, 2H), 7.34-7.39 (m, 1H), 8.37 (dd, 1H), 8.72 (m, 1H), 11.02 (s br, 1H).

### Beispiel 10

### 4-[(Cyclopropylmethyl)amino]-2-[5-fluor-1-(2-fluorbenzyl)-1H-pyrazolo[3,4-b]pyridin-3-yl]-5,5-dimethyl-5,7-dihydro-6H-pyrrolo[2,3-d]pyrimidin-6-on

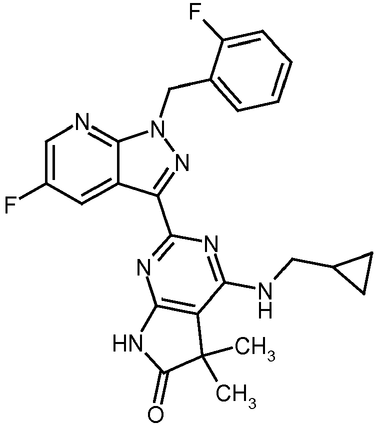

100 mg (0.115 mmol, ca. 61% Reinheit) Beispiel 16A wurden in einem mikrowellengeeigneten Reaktionsgefäß in 1-Methyl-2-pyrrolidon (2 ml) gelöst und mit 0.5 ml Aminomethylcyclopropan versetzt. Anschliessend wurde mit einem entsprechenden Septum verschlossen und für 3 h bei 150 °C in der Mikrowelle erhitzt. Nach Abkühlen wurde das Reaktionsgemisch mittels präparativer HPLC gereinigt (Acetonitril:Wasser (+0.05 % Ameisensäure) - Gradient). Es wurden 45 mg der Titelverbindung erhalten 82 % d. Th.).
LC-MS (Methode 1): Rₜ = 1.22 min; MS (EIpos): m/z = 476 [M+H]⁺.
¹H-NMR (400 MHz, DMSO-d₆): δ [ppm] = 0.31-0.34 (m, 2H), 0.42-0.46 (m, 2H), 1.21-1.26 (m, 1H), 1.37 (s, 6H), 3.45 (t, 2H), 5.83 (s, 2H), 6.88 (t, 1H), 7.15 (t, 1H), 7.21-7.25 (m, 2H), 7.34-7.39 (m, 1H), 8.57 (dd, 1H), 8.72 (m, 1H), 11.02 (s br, 1H).

### Beispiel 11

### 2-[5-Fluor-1-(2-fluorbenzyl)-1H-pyrazolo[3,4-b]pyridin-3-yl]-5,5-dimethyl-4-[(2,2,2-trifluorethyl)amino]-5,7-dihydro-6H-pyrrolo[2,3-d]pyrimidin-6-on

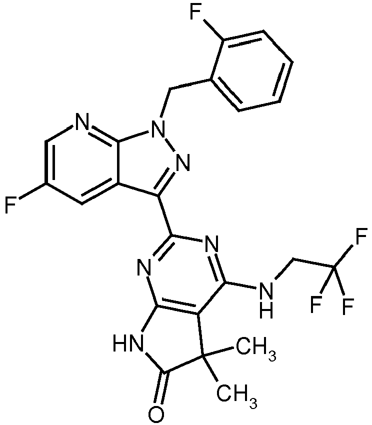

100 mg (0.115 mmol, ca. 61% Reinheit) Beispiel 16A wurden in einem mikrowellengeeigneten Reaktionsgefäß in 1-Methyl-2-pyrrolidon (2 ml) gelöst und mit 1 ml 2,2,2-Trifluorethylamin versetzt. Anschliessend wurde mit einem entsprechenden Septum verschlossen und für 20 h bei 150 °C in der Mikrowelle erhitzt. Nach Abkühlen wurde das Reaktionsgemisch mittels präparativer HPLC gereinigt (Acetonitril:Wasser (+0.05 % Ameisensäure) - Gradient). Es wurden 35 mg der Titelverbindung erhalten 61 % d. Th.).
LC-MS (Methode 1): Rₜ = 1.12 min; MS (EIpos): m/z = 504 [M+H]⁺.
¹H-NMR (400 MHz, DMSO-d₆): δ [ppm] = 1.39 (s, 6H), 4.35-4.43 (m, 2H), 5.84 (s, 2H), 7.15 (t, 1H), 7.20-7.25 (m, 3H), 7.34-7.40 (m, 1H), 8.46 (dd, 1H), 8.73 (m, 1H), 11.22 (s br, 1H).

### Beispiel 12

### 2-{5-Fluor-1-[(3-fluorpyridin-2-yl)methyl]-1H-pyrazolo[3,4-b]pyridin-3-yl}-5,5-dimethyl-4-[(3,3,3-trifluorpropyl)amino]-5,7-dihydro-6H-pyrrolo[2,3-d]pyrimidin-6-on

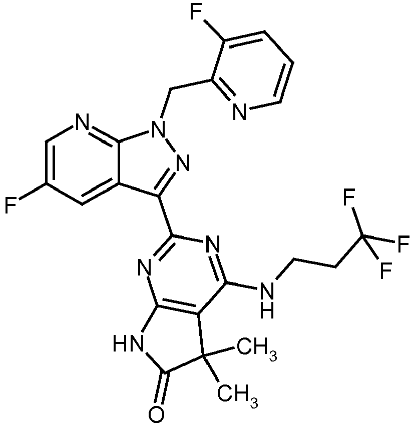

350 mg (0.361 mmol, ca. 55% Reinheit) Beispiel 21A wurden in einem mikrowellengeeigneten Reaktionsgefäß in 1-Methyl-2-pyrrolidon (4 ml) gelöst und mit 1.2 ml 3,3,3-Trifluorpropyl-1-amin versetzt. Anschliessend wurde mit einem entsprechenden Septum verschlossen und für 3 h bei 150 °C in der Mikrowelle erhitzt. Nach Abkühlen wurde das Reaktionsgemisch mittels präparativer HPLC gereinigt (Acetonitril:Wasser (+0.05 % Ameisensäure) - Gradient). Es wurden 108 mg der Titelverbindung erhalten (57 % d. Th.).
LC-MS (Methode 1): Rₜ = 1.08 min; MS (EIpos): m/z = 519 [M+H]⁺.
¹H-NMR (400 MHz, DMSO-d₆): δ [ppm] = 1.35 (s, 6H), 2.62-2.72 (m, 2H), 3.82 (q, 2H), 5.96 (s, 2H), 6.82 (t, 1H), 7.42-7.45 (m, 1H), 7.76 (t, 1H), 8.27 (d, 1H), 8.48 (dd, 1H), 8.67 (br s, 1H), 11.03 (s, 1H).

### Beispiel 13

### 2-[6-Chlor-3-(2,3,6-trifluorbenzyl)imidazo[1,5-a]pyridin-1-yl]-5,5-dimethyl-4-[(3,3,3-trifluorpropyl)amino]-5,7-dihydro-6H-pyrrolo[2,3-d]pyrimidin-6-on

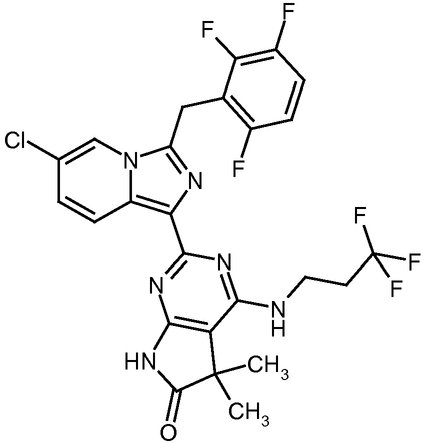

128 mg (0.219 mmol) Beispiel 22A wurden in einem mikrowellengeeigneten Reaktionsgefäß in 1-Methyl-2-pyrrolidon (2.334 ml) gelöst und mit 0.584 ml 3,3,3-Trifluorpropyl-1-amin versetzt. Anschliessend wurde mit einem entsprechenden Septum verschlossen und für 3 h bei 150 °C in der Mikrowelle erhitzt. Nach Abkühlen wurde das Reaktionsgemisch mittels präparativer HPLC gereinigt (Acetonitril:Wasser (+0.05 % Ameisensäure) - Gradient). Es wurden 57 mg der Titelverbindung erhalten (46 % d. Th.).
LC-MS (Methode 1): Rₜ = 1.21 min; MS (EIpos): m/z = 569 [M+H]⁺.
¹H-NMR (400 MHz, DMSO-d₆): δ [ppm] = 1.31 (s, 6H), 2.62-2.71 (m, 2H), 3.73 (q, 2H), 4.53 (s, 2H), 6.69 (t, 1H), 7.05 (dd, 1H), 7.17-7.24 (m, 1H), 7.46-7.55 (m, 1H), 8.42 (d, 1H), 8.80 (s, 1H), 10.91 (s, 1H).

### Beispiel 14

### 2-{5-Fluor-1-[(3-fluorpyridin-2-yl)methyl]-1H-pyrazolo[3,4-b]pyridin-3-yl}-5,5-dimethyl-4-[(3,3,3-trifluorpropyl)amino]-5,7-dihydro-6H-pyrrolo[2,3-d]pyrimidin-6-on

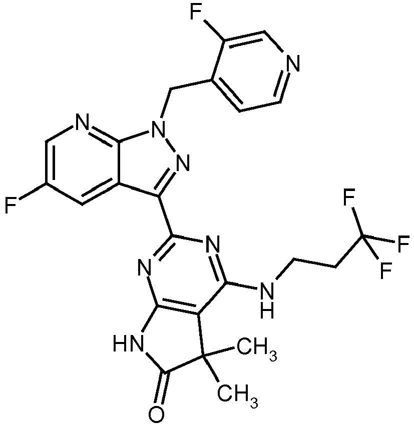

150 mg (0.186 mmol, ca. 66% Reinheit) der unter Beispiel 28A erhaltenen Verbindung wurden in einem mikrowellengeeigneten Reaktionsgefäß in 1-Methyl-2-pyrrolidon (3.6 ml) gelöst und mit 0.9 ml 3,3,3-Trifluorpropyl-1-amin versetzt. Anschliessend wurde mit einem entsprechenden Septum verschlossen und für 3 h bei 150 °C in der Mikrowelle erhitzt. Nach Abkühlen wurde das Reaktionsgemisch mittels präparativer HPLC gereinigt (Acetonitril:Wasser (+0.05 % Ameisensäure) - Gradient). Es wurden 33 mg der Titelverbindung erhalten (34 % d. Th.).
LC-MS (Methode 1): Rₜ = 1.00 min; MS (EIpos): m/z = 519 [M+H]⁺.
¹H-NMR (400 MHz, DMSO-d₆): δ [ppm] = 1.36 (s, 6H), 2.61-2.74 (m, 2H), 3.82 (q, 2H), 5.92 (s, 2H), 6.89 (t, 1H), 7.13 (t, 1H), 8.35 (d, 1H), 8.50 (dd, 1H), 8.59 (d, 1H), 8.73 (dd, 1H), 11.10 (s, 1H).

### Beispiel 15

### 4-(3-Fluorazetidin-1-yl)-2-{5-fluor-1-[(3-fluorpyridin-2-yl)methyl]-1H-pyrazolo[3,4-b]pyridin-3-yl}-5,5-dimethyl-5,7-dihydro-6H-pyrrolo[2,3-d]pyrimidin-6-on

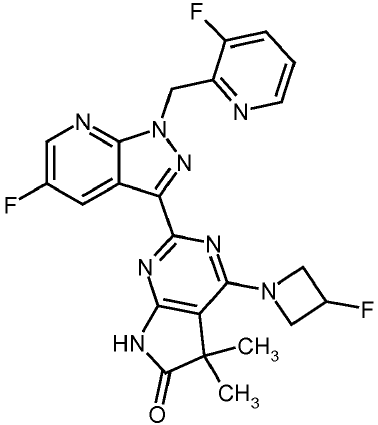

150 mg (0.155 mmol, ca. 55% Reinheit) der unter Beispiel 21A erhaltenen Verbindung wurden in einem mikrowellengeeigneten Reaktionsgefäß in 1-Methyl-2-pyrrolidon (2.7 ml) gelöst, mit 0.423 ml (2.430 mmol) N,N-Diisopropylethylamin und mit 270 mg (2.420 mmol) 3-Fluorazetidin Hydrochlorid versetzt. Anschliessend wurde mit einem entsprechenden Septum verschlossen und für 3 h bei 150 °C in der Mikrowelle erhitzt. Nach Abkühlen wurde das Reaktionsgemisch mittels präparativer HPLC gereinigt (Acetonitril:Wasser (+0.05 % Ameisensäure) - Gradient). Es wurden 30 mg der Titelverbindung erhalten (40 % d. Th.).
LC-MS (Methode 1): Rₜ = 0.97 min; MS (EIpos): m/z = 481 [M+H]⁺.
¹H-NMR (400 MHz, DMSO-d₆): δ [ppm] = 1.35 (s, 6H), 4.34-4.42 (m, 2H), 4.63-4.73 (m, 2H), 5.48-5.65 (m, 1H), 5.98 (s, 2H), 7.41-7.45 (m, 1H), 7.74-7.79 (m, 1H), 8.24-8.28 (m, 1H), 8.52 (dd, 1H), 8.68 (dd, 1H), 11.22 (s, 1H).

### Beispiel 16

### 2-5-Fluor-1-[(3-fluorpyridin-2-yl)methyl]-1H-pyrazolo[3,4-b]pyridin-3-yl}-5,5-dimethyl-4-[(2,2,2-trifluorethyl)amino]-5,7-dihydro-6H-pyrrolo[2,3-d]pyrimidin-6-on

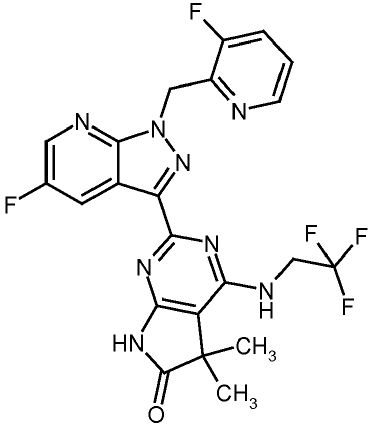

150 mg (0.155 mmol, ca. 55% Reinheit) der unter Beispiel 21A erhaltenen Verbindung wurden in einem mikrowellengeeigneten Reaktionsgefäß in 1-Methyl-2-pyrrolidon (2.7 ml) gelöst und mit 0.675 ml 2,2,2-Trifluorethylamin versetzt. Anschliessend wurde mit einem entsprechenden Septum verschlossen und für 21 h bei 150 °C in der Mikrowelle erhitzt. Nach Abkühlen wurde das Reaktionsgemisch mittels präparativer HPLC gereinigt (Acetonitril:Wasser (+0.05 % Ameisensäure) - Gradient). Es wurden 31 mg der Titelverbindung erhalten (39 % d. Th.).
LC-MS (Methode 1): Rₜ = 0.99 min; MS (EIpos): m/z = 505 [M+H]⁺.
¹H-NMR (400 MHz, DMSO-d₆): δ [ppm] = 1.39 (s, 6H), 4.35-4.44 (m, 2H), 5.98 (s, 2H), 7.19 (t, 1H), 7.41-7.45 (m, 1H), 7.74-7.79 (m, 1H), 8.24-8.28 (m, 1H), 8.46 (dd, 1H), 8.68 (dd, 1H), 11.16 (s br, 1H).

### Beispiel 17

### 4-[(Cyclopropylmethyl)amino]-2-{5-fluor-1-[(3-fluorpyridin-2-yl)methyl]-1H-pyrazolo[3,4-b]pyridin-3-yl}-5,5-dimethyl-5,7-dihydro-6H-pyrrolo[2,3-d]pyrimidin-6-on

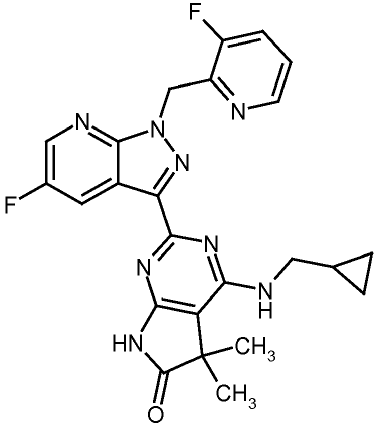

200 mg (0.206 mmol, ca. 55% Reinheit) der unter Beispiel 21A erhaltenen Verbindung wurden in einem mikrowellengeeigneten Reaktionsgefäß in 1-Methyl-2-pyrrolidon (4 ml) gelöst und mit 1 ml Aminomethylcyclopropan versetzt. Anschliessend wurde mit einem entsprechenden Septum verschlossen und für 3 h bei 150 °C in der Mikrowelle erhitzt. Nach Abkühlen wurde das Reaktionsgemisch mittels präparativer HPLC gereinigt (Acetonitril:Wasser (+0.05 % Ameisensäure) - Gradient). Es wurden 64 mg der Titelverbindung erhalten (65 % d. Th.).
LC-MS (Methode 1): Rₜ = 1.09 min; MS (EIpos): m/z = 477 [M+H]⁺.
¹H-NMR (400 MHz, DMSO-d₆): δ [ppm] = 0.30-0.33 (m, 2H), 0.42-0.46 (m, 2H), 1.21-1.27 (m, 1H), 1.37 (s, 6H), 3.46 (t, 2H), 5.96 (s, 2H), 6.78-6.85 (m, 1H), 7.41-7.45 (m, 1H), 7.76 (t, 1H), 8.24-8.28 (m, 1H), 8.57 (dd, 1H), 8.67 (dd, 1H), 10.95 (s br, 1H).

### Beispiel 18

### 2-[5-Fluor-1-(2-fluorbenzyl)-1H-pyrazolo[3,4-b]pyridin-3-yl]-5,5-dimethyl-4-[(4,4,4-trifluorbutyl)amino]-5,7-dihydro-6H-pyrrolo[2,3-d]pyrimidin-6-on

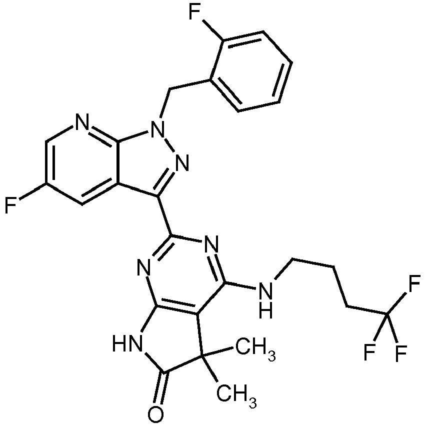

150 mg (0.186 mmol, ca. 66% Reinheit) der unter Beispiel 16A erhaltenen Verbindung wurden in einem mikrowellengeeigneten Reaktionsgefäß in 1-Methyl-2-pyrrolidon (3 ml) gelöst und mit 0.4 ml 4,4,4-Trifluorbutylamin versetzt. Anschliessend wurde mit einem entsprechenden Septum verschlossen und für 3 h bei 150 °C in der Mikrowelle erhitzt. Nach Abkühlen wurde das Reaktionsgemisch mittels präparativer HPLC gereinigt (Acetonitril:Wasser (+0.05 % Ameisensäure) - Gradient). Es wurden 47 mg der Titelverbindung erhalten (48 % d. Th.).
LC-MS (Methode 1): Rₜ = 1.19 min; MS (EIpos): m/z = 532 [M+H]⁺.
¹H-NMR (400 MHz, DMSO-d₆): δ [ppm] = 1.37 (s, 6H), 1.87-1.94 (m, 2H), 2.30-2.43 (m, 2H), 3.63 (q, 2H), 5.83 (s, 2H), 6.81 (t, 1H), 7.12-7.17 (m, 1H), 7.21-7.25 (m, 2H), 7.34-7.39 (m, 1H), 8.47 (dd, 1H), 8.72 (dd, 1H), 11.04 (s, 1H).

### Beispiel 19

### 2-[1-(2-Fluorbenzyl)-1,4,5,6-tetrahydrocyclopenta[c]pyrazol-3-yl]-5,5-dimethyl-4-[(2,2,2-trifluorethyl)amino]-5,7-dihydro-6H-pyrrolo[2,3-d]pyrimidin-6-on

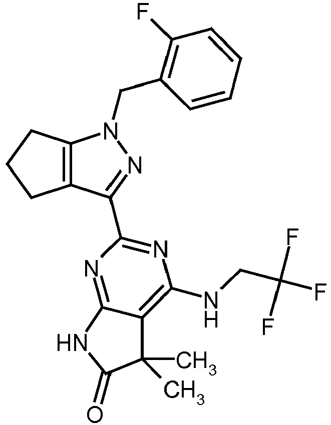

Es wurden 50 mg (0.09 mmol) 2-[1-(2-Fluorbenzyl)-1,4,5,6-tetrahydrocyclopenta[c]pyrazol-3-yl]-4-iod-5,5-dimethyl-5,7-dihydro-6H-pyrrolo[2,3-d]pyrimidin-6-on (Beispiel 33A) in 1 ml absolutem NMP gelöst und mit 336 mg (3.70 mmol) 2,2,2-Trifluorethanamin versetzt. Das Gemisch wurde 2h bei 150°C, 18h bei 150°C, 2h bei 160°C, 2h bei 170°C und 5h bei 170°C in der Mikrowelle erhitzt. Die Reaktionslösung wurde filtriert und mittels päparativer HPLC gereinigt (Eluent: Acetonitril/Wasser mit 0.1% Ameisensäure, Gradient 20:80 → 100:0). Es wurden 18 mg (Reinheit 88%, 36% d. Th.) der Zielverbindung erhalten.
LC-MS (Methode 1): Rₜ = 1.09 min; MS (ESIpos): m/z = 475 [M+H]⁺
¹H-NMR (400MHz, DMSO-d₆): δ [ppm]= 1.34 (s, 6H), 2.64 (t, 2H), 2.79 (t, 2H), 4.24-4.32 (m, 2H), 5.30 (s, 2H), 7.01 (t, 1H), 7.18-7.28 (m, 3H), 7.36-7.42 (m, 1H), 11.01 (s, 1H).

### Beispiel 20

### 2-[1-(2-Fluorbenzyl)-1,4,5,6-tetrahydrocyclopenta[c]pyrazol-3-yl]-5,5-dimethyl-4-[(3,3,3-trifluorpropyl)amino]-5,7-dihydro-6H-pyrrolo[2,3-d]pyrimidin-6-on

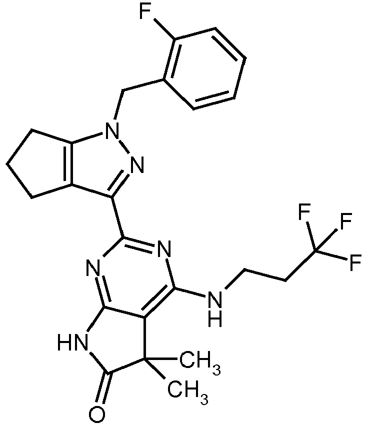

Es wurden 40 mg (0.07 mmol) 2-[1-(2-Fluorbenzyl)-1,4,5,6-tetrahydrocyclopenta[c]pyrazol-3-yl]-4-iod-5,5-dimethyl-5,7-dihydro-6H-pyrrolo[2,3-d]pyrimidin-6-on (Beispiel 33A) in 1 ml absolutem NMP gelöst und mit 334 mg (2.96 mmol) 3,3,3-Trifluorpropan-1-amin versetzt. Das Gemisch wurde 2h bei 150°C und 1h bei 150°C in der Mikrowelle erhitzt. Die Reaktionslösung wurde filtriert und mittels päparativer HPLC gereinigt (Eluent: Acetonitril/Wasser mit 0.1% Salzsäure, Gradient 20:80 → 100:0). Es wurden 27 mg (Reinheit 93%, 70% d. Th.) der Zielverbindung erhalten.
LC-MS (Methode 1): Rₜ = 1.17 min; MS (ESIpos): m/z = 489 [M+H]⁺
¹H-NMR (400MHz, DMSO-d₆): δ [ppm]= 1.32 (s, 6H), 2.57-2.67 (m, 4H), 2.80 (t, 2H), 3.68-3.73 (m, 2H), 5.32 (s, 2H), 6.85 (s br, 1H), 7.19-7.29 (m, 3H), 7.37-7.42 (m, 1H), 10.93 (s, 1H).

### Beispiel 21

### 4-(3-Ethyl-2-oxoimidazolidin-1-yl)-2-[1-(2-fluorbenzyl)-1H-pyrazolo[3,4-b]pyridin-3-yl]-5,5-dimethyl-5,7-dihydro-6H-pyrrolo[2,3-d]pyrimidin-6-on

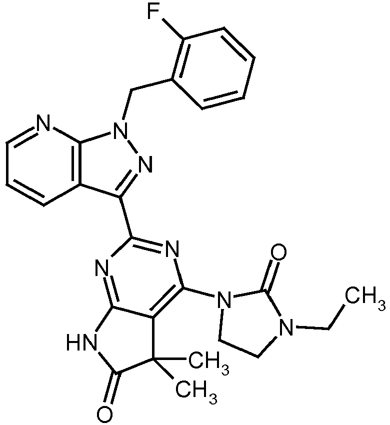

Unter Argonatmosphäre wurden 150 mg (Reinheit 62%, 0.18 mmol) 2-[1-(2-Fluorbenzyl)-1H-pyrazolo[3,4-b]pyridin-3-yl]-4-iod-5,5-dimethyl-5,7-dihydro-6H-pyrrolo[2,3-d]pyrimidin-6-on (Beispiel 15A) in 2 ml absolutem Acetonitril suspendiert und mit 413 mg (3.62 mmol) 1-Ethylimidazolidin-2-on, 118 mg (0.36 mmol) Cäsiumcarbonat, 5 mg (0.04 mmol) Kupfer(I)oxid sowie 20 mg (0.15 mmol) 2-Hydroxybenzaldehydoxim versetzt. Das Gemisch wurde 1h bei 200°C in der Mikrowelle erhitzt. Die Reaktionslösung wurde filtriert und mittels päparativer HPLC gereinigt (Eluent: Acetonitril/Wasser, Gradient 20:80 → 100:0). Es wurden 35 mg (Reinheit 91%, 35% d. Th.) der Zielverbindung erhalten.
LC-MS (Methode 1): Rₜ = 1.03 min; MS (ESIpos): m/z = 501 [M+H]⁺
¹H-NMR (400MHz, DMSO-d₆): δ [ppm]= 1.13 (t, 3H), 1.41 (s, 6H), 3.28 (q, 2H), 3.57 (t, 2H), 3.97 (t, 2H), 5.87 (s, 2H), 7.12-7.25 (m, 3H), 7.34-7.38 (m, 1H), 7.44 (dd, 1H), 8.67 (dd, 1H), 8.83 (dd, 1H), 11.63 (s, 1H).

### Beispiel 22

### 2-[3-(2-Fluorbenzyl)-1H-indazol-1-yl]-4-hydroxy-5,5-dimethyl-5,7-dihydro-6H-pyrrolo[2,3-d]pyrimidin-6-on

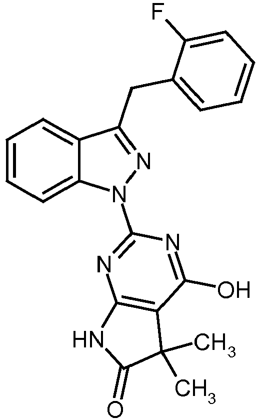

Die Titelverbindung wurde als Nebenkomponente bei der Versuchsdurchführung zu Beispiel 38A erhalten. Ausbeute: 57 mg (9 % d. Th., 86%-ige Reinheit)
LC-MS (Methode 1): Rₜ = 1.03 min; MS (ESIpos): m/z = 404 [M+H]⁺
¹H-NMR (400 MHz, DMSO-d₆): δ [ppm] = 1.33 (s, 6H), 4.42 (s, 2H), 7.13-7.23 (m, 2H), 7.28-7.44 (m, 3H), 7.57-7.61 (m, 1H), 7.73 (d, 1H), 3H nicht eindeutig zuzuordnen.

### Beispiel 23

### 4-(3,5-Dimethyl-1H-pyrazol-1-yl)-2-[1-(2-fluorbenzyl)-1H-pyrazolo[3,4-b]pyridin-3-yl]-5,5-dimethyl-5,7-dihydro-6H-pyrrolo[2,3-d]pyrimidin-6-on

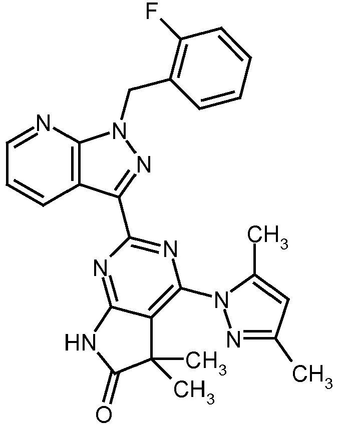

Unter Argonatmosphäre wurden 150 mg (Reinheit 62%, 0.18 mmol) 2-[1-(2-Fluorbenzyl)-1H-pyrazolo[3,4-b]pyridin-3-yl]-4-iod-5,5-dimethyl-5,7-dihydro-6H-pyrrolo[2,3-d]pyrimidin-6-on (Beispiel 15A) in 2 ml absolutem Acetonitril suspendiert und mit 26 mg (0.27 mmol) 3,5-Dimethyl-1H-pyrazol, 118 mg (0.36 mmol) Cäsiumcarbonat, 5 mg (0.04 mmol) Kupfer(I)oxid sowie 20 mg (0.15 mmol) 2-Hydroxybenzaldehydoxim versetzt. Das Gemisch wurde 1h bei 150°C in der Mikrowelle erhitzt. Anschließend wurden 346 mg (3.62 mmol) 3,5-Dimethyl-1H-pyrazol addiert und das Gemisch für 45 min bei 200°C in der Mikrowelle erhitzt. Die Reaktionslösung wurde filtriert und mittels päparativer HPLC gereinigt (Eluent: Acetonitril/Wasser, Gradient 20:80 → 100:0). Es wurden 20 mg (23% d. Th.) der Zielverbindung erhalten.
LC-MS (Methode 1): Rₜ = 1.23 min; MS (ESIpos): m/z = 483 [M+H]⁺
¹H-NMR (400MHz, DMSO-d₆): δ [ppm]= 1.43 (s, 6H), 2.27 (s, 3H), 2.53 (s, 3H), 5.87 (s, 2H), 6.22 (s, 1H), 7.16 (t, 1H), 7.21-7.30 (m, 2H), 7.35-7.39 (m, 1H), 7.46 (dd, 1H), 8.69 (dd, 1H), 8.79 (dd, 1H), 11.76 (s, 1H).

### Beispiel 24

### 2-[1-(2-Fluorbenzyl)-1H-pyrazolo[3,4-b]pyridin-3-yl]-4-(3-fluor-2-oxopyridin-1(2H)-yl)-5,5-dimethyl-5,7-dihydro-6H-pyrrolo[2,3-d]pyrimidin-6-on

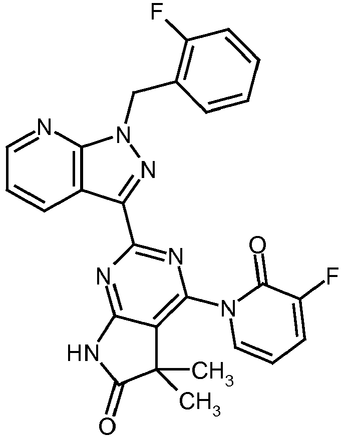

Unter Argonatmosphäre wurden 200 mg (Reinheit 62%, 0.24 mmol) 2-[1-(2-Fluorbenzyl)-1H-pyrazolo[3,4-b]pyridin-3-yl]-4-iod-5,5-dimethyl-5,7-dihydro-6H-pyrrolo[2,3-d]pyrimidin-6-on (Beispiel 15A) in 2.5 ml absolutem Acetonitril suspendiert und mit 545 mg (4.82 mmol) 3-Fluorpyridin-2-ol, 157 mg (0.48 mmol) Cäsiumcarbonat, 7 mg (0.05 mmol) Kupfer(I)oxid sowie 20 mg (0.15 mmol) 2-Hydroxybenzaldehydoxim versetzt. Das Gemisch wurde 1h bei 200°C in der Mikrowelle erhitzt. Die Reaktionslösung wurde filtriert und mittels päparativer HPLC gereinigt (Eluent: Acetonitril/Wasser, Gradient 20:80 → 100:0). Es wurden 20 mg (23% d. Th.) der Zielverbindung erhalten.
LC-MS (Methode 1): Rₜ = 1.11 min; MS (ESIpos): m/z = 500 [M+H]⁺
¹H-NMR (400MHz, DMSO-d₆): δ [ppm]= 1.49 (s, 6H), 5.81 (s, 2H), 7.10-7.23 (m, 4H), 7.32-7.38 (m, 1H), 7.61-7.65 (m, 2H), 8.07-8.12 (m, 1H), 8.36 (dd, 1H), 8.59 (dd, 1H), 11.75 (s, 1H).

### Beispiel 25

### 2-[1-(2-Fluorbenzyl)-1H-pyrazolo[3,4-b]pyridin-3-yl]-5,5-dimethyl-4-[2-oxo-4-(trifluormethyl)pyrrolidin-1-yl]-5,7-dihydro-6H-pyrrolo[2,3-d]pyrimidin-6-on

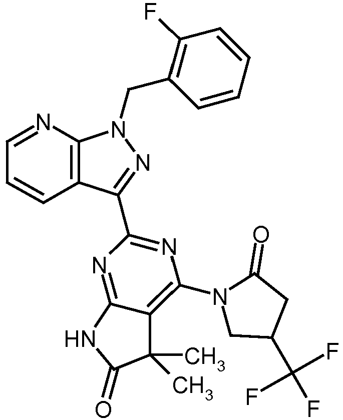

Unter Argonatmosphäre wurden 200 mg (Reinheit 62%, 0.24 mmol) 2-[1-(2-Fluorbenzyl)-1H-pyrazolo[3,4-b]pyridin-3-yl]-4-iod-5,5-dimethyl-5,7-dihydro-6H-pyrrolo[2,3-d]pyrimidin-6-on (Beispiel 15A) in 2.5 ml absolutem Acetonitril suspendiert und mit 738 mg (4.82 mmol) 4-(Trifluormethyl)pyrrolidin-2-on, 157 mg (0.48 mmol) Cäsiumcarbonat, 7 mg (0.05 mmol) Kupfer(I)oxid sowie 26 mg (0.19 mmol) 2-Hydroxybenzaldehydoxim versetzt. Das Gemisch wurde 1h bei 200°C in der Mikrowelle erhitzt. Die Reaktionslösung wurde filtriert und mittels päparativer HPLC gereinigt (Eluent: Acetonitril/Wasser mit 0.1% Ameisensäure, Gradient 20:80 → 100:0). Es wurden 26 mg (19% d. Th.) der Zielverbindung erhalten.
LC-MS (Methode 1): Rₜ = 1.02 min; MS (ESIpos): m/z = 540 [M+H]⁺
¹H-NMR (400MHz, DMSO-d₆): δ [ppm]= 1.32 (s, 3H), 1.36 (s, 3H), 2.70 (dd, 1H), 3.08 (dd, 1H), 3.67-3.75 (m, 1H), 3.87(dd, 1H), 4.32 (dd, 1H), 5.88 (s, 2H), 7.14 (t, 1H), 7.16-7.25 (m, 2H), 7.34-7.38 (m, 1H), 7.45 (dd, 1H), 8.69 (dd, 1H), 8.84 (dd, 1H), 11.83 (s, 1H).

### Beispiel 26

### 2-[1-(2-Fluorbenzyl)-1H-pyrazolo[3,4-b]pyridin-3-yl]-4-(4-hydroxy-1H-pyrazol-1-yl)-5,5-dimethyl-5,7-dihydro-6H-pyrrolo[2,3-d]pyrimidin-6-on

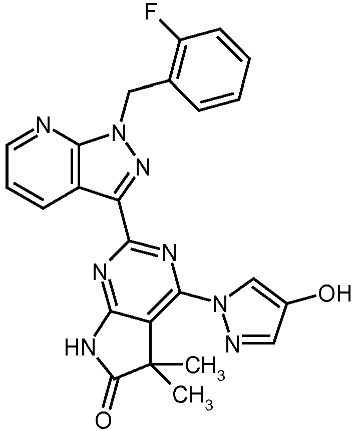

Unter einer Argonatmosphäre wurden 150 mg (Reinheit 62%, 0.18 mmol) 2-[1-(2-Fluorbenzyl)-1H-pyrazolo[3,4-b]pyridin-3-yl]-4-iod-5,5-dimethyl-5,7-dihydro-6H-pyrrolo[2,3-d]pyrimidin-6-on (Beispiel 15A) in 2 ml absolutem Acetonitril suspendiert und mit 304 mg (3.62 mmol) 1H-Pyrazol-4-ol, 118 mg (0.36 mmol) Cäsiumcarbonat, 5 mg (0.04 mmol) Kupfer(I)oxid sowie 20 mg (0.15 mmol) 2-Hydroxybenzaldehydoxim versetzt. Das Gemisch wurde 1h bei 200°C in der Mikrowelle erhitzt. Die Reaktionslösung wurde filtriert und mittels päparativer HPLC gereinigt (Eluent: Acetonitril/Wasser mit 0.1% Ameisensäure, Gradient 20:80 → 100:0). Es wurden 20 mg (24% d. Th.) der Zielverbindung erhalten.
LC-MS (Methode 1): Rₜ = 1.03 min; MS (ESIpos): m/z = 471 [M+H]⁺
¹H-NMR (400MHz, DMSO-d₆): δ [ppm]= 1.59 (s, 6H), 5.89 (s, 2H), 7.15 (t, 1H), 7.22-7.25 (m, 2H), 7.35-7.39 (m, 1H), 7.51 (dd, 1H), 7.72 (s, 1H), 8.25 (s, 1H), 8.71 (dd, 1H), 8.87 (dd, 1H), 9.37 (s, 1H), 11.75 (s br, 1H).

Ausserdem wurde 2-[1-(2-Fluorbenzyl)-1H-pyrazolo[3,4-b]pyridin-3-yl]-5,5-dimethyl-4-(1H-pyrazol-4-yloxy)-5,7-dihydro-6H-pyrrolo[2,3-d]pyrimidin-6-on bei diesem Ansatz erhalten (s. Beispiel 27)

### Beispiel 27

### 2-[1-(2-Fluorbenzyl)-1H-pyrazolo[3,4-b]pyridin-3-yl]-5,5-dimethyl-4-(1H-pyrazol-4-yloxy)-5,7-dihydro-6H-pyrrolo[2,3-d]pyrimidin-6-on

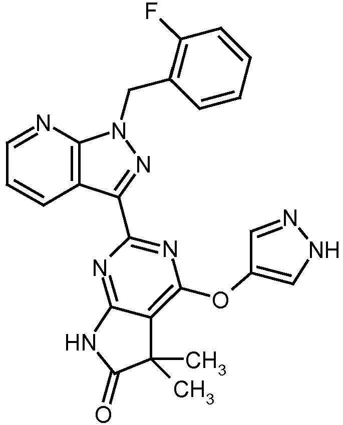

Enstanden bei der Herstellung von Beispiel 26 (s. Beispiel 26). Es wurden 20 mg (23% d. Th.) der Zielverbindung erhalten.
LC-MS (Methode 1): Rₜ = 0.94 min; MS (ESIpos): m/z = 471 [M+H]⁺
¹H-NMR (400MHz, DMSO-d₆): δ [ppm]= 1.45 (s, 6H), 5.83 (s, 2H), 7.14 (t, 1H), 7.19-7.25 (m, 3H), 7.33-7.38 (m, 1H), 7.67 (s br, 1H), 8.00 (s br, 1H), 8.22 (d, 1H), 8.62 (dd, 1H), 11.57 (s, 1H), 12.92 (s br, 1H).

### Beispiel 28

### 2-[1-(2-Fluorbenzyl)-1H-pyrazolo[3,4-b]pyridin-3-yl]-4-[3-(1-hydroxyethyl)-1H-pyrazol-1-yl]-5,5-dimethyl-5,7-dihydro-6H-pyrrolo[2,3-d]pyrimidin-6-on

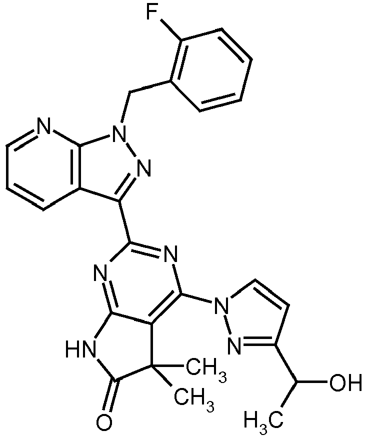

UnterArgonatmosphäre wurden 150 mg (Reinheit 62%, 0.18 mmol) 2-[1-(2-Fluorbenzyl)-1H-pyrazolo[3,4-b]pyridin-3-yl]-4-iod-5,5-dimethyl-5,7-dihydro-6H-pyrrolo[2,3-d]pyrimidin-6-on (Beispiel 15A) in 2 ml absolutem Acetonitril suspendiert und mit 405 mg (3.62 mmol) 1-(1H-Pyrazol-3-yl)ethanol, 118 mg (0.36 mmol) Cäsiumcarbonat, 5 mg (0.04 mmol) Kupfer(I)oxid sowie 20 mg (0.15 mmol) 2-Hydroxybenzaldehydoxim versetzt. Das Gemisch wurde 1h bei 200°C in der Mikrowelle erhitzt. Die Reaktionslösung wurde filtriert und mittels päparativer HPLC gereinigt (Eluent: Acetonitril/Wasser mit 0.1% Ameisensäure, Gradient 20:80 → 100:0). Es wurden 20 mg (24% d. Th.) der Zielverbindung erhalten.
LC-MS (Methode 1): Rₜ = 0.96 min; MS (ESIpos): m/z = 499 [M+H]⁺
¹H-NMR (400MHz, DMSO-d₆): δ [ppm]= 1.38 (s, 3H), 1.40 (s, 3H), 1.86 (d, 3H), 5.66 (q, 1H), 5.84 (s, 2H), 6.20 (d, 1H), 7.13 (t, 1H), 7.20-7.38 (m, 3H), 7.41 (dd, 1H), 7.63 (s, 1H), 8.28 (d, 1H), 8.69 (dd, 1H), 12.63 (s br, 2H).

### Beispiel 29

### 2-[1-(2-Fluorbenzyl)-1H-pyrazolo[3,4-b]pyridin-3-yl]-5,5-dimethyl-4-[4-(trifluormethyl)-1H-pyrazol-1-yl]-5,7-dihydro-6H-pyrrolo[2,3-d]pyrimidin-6-on

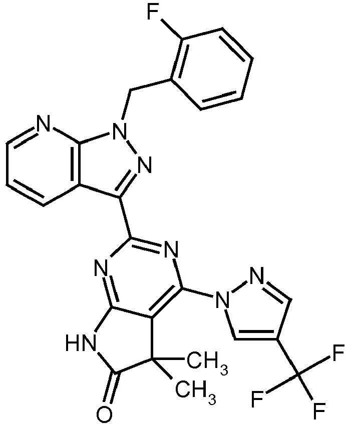

Unter Argonatmosphäre wurden 200 mg (Reinheit 62%, 0.24 mmol) 2-[1-(2-Fluorbenzyl)-1H-pyrazolo[3,4-b]pyridin-3-yl]-4-iod-5,5-dimethyl-5,7-dihydro-6H-pyrrolo[2,3-d]pyrimidin-6-on (Beispiel 15A) in 2.5 ml absolutem Acetonitril suspendiert und mit 656 mg (4.82 mmol) 4-(Trifluormethyl)-1H-pyrazol, 157 mg (0.48 mmol) Cäsiumcarbonat, 7 mg (0.05 mmol) Kupfer(I)oxid sowie 26 mg (0.19 mmol) 2-Hydroxybenzaldehydoxim versetzt. Das Gemisch wurde 1h bei 200°C in der Mikrowelle erhitzt. Die Reaktionslösung wurde filtriert und mittels päparativer HPLC gereinigt (Eluent: Acetonitril/Wasser mit 0.1% Ameisensäure, Gradient 20:80 → 100:0). Es wurden 85 mg (63% d. Th.) der Zielverbindung erhalten.
LC-MS (Methode 1): Rₜ = 1.33 min; MS (ESIpos): m/z = 523 [M+H]⁺
¹H-NMR (400MHz, DMSO-d₆): δ [ppm]= 1.56 (s, 6H), 5.91 (s, 2H), 7.15 (t, 1H), 7.20-7.25 (m, 2H), 7.34-7.39 (m, 1H), 7.52 (dd, 1H), 8.51 (s, 1H), 8.71 (dd, 1H), 8.92 (dd, 1H), 9.30 (s, 1H), 11.99 (s, 1H).

### Beispiel 30

### 2-[1-(2-Fluorbenzyl)-1H-pyrazolo[3,4-b]pyridin-3-yl]-5,5-dimethyl-4-[3-(trifluormethyl)-1H-pyrazol-1-yl]-5,7-dihydro-6H-pyrrolo[2,3-d]pyrimidin-6-on

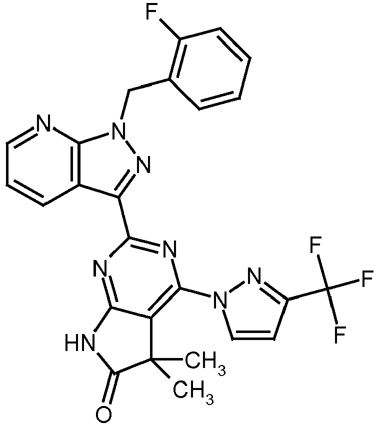

Unter Argonatmosphäre wurden 200 mg (Reinheit 62%, 0.24 mmol) 2-[1-(2-Fluorbenzyl)-1H-pyrazolo[3,4-b]pyridin-3-yl]-4-iod-5,5-dimethyl-5,7-dihydro-6H-pyrrolo[2,3-d]pyrimidin-6-on (Beispiel 15A) in 2.5 ml absolutem Acetonitril suspendiert und mit 656 mg (4.82 mmol) 3-(Trifluormethyl)-1H-pyrazol, 157 mg (0.48 mmol) Cäsiumcarbonat, 7 mg (0.05 mmol) Kupfer(I)oxid sowie 26 mg (0.19 mmol) 2-Hydroxybenzaldehydoxim versetzt. Das Gemisch wurde 1h bei 200°C in der Mikrowelle erhitzt. Die Reaktionslösung wurde filtriert und mittels päparativer HPLC gereinigt (Eluent: Acetonitril/Wasser mit 0.1% Ameisensäure, Gradient 20:80 → 100:0). Es wurden 41 mg (33% d. Th.) der Zielverbindung erhalten.
LC-MS (Methode 1): Rₜ = 1.32 min; MS (ESIpos): m/z = 523 [M+H]⁺
¹H-NMR (400MHz, DMSO-d₆): δ [ppm]= 1.55 (s, 6H), 5.90 (s, 2H), 7.15 (t, 1H), 7.21-7.25 (m, 3H), 7.35-7.39 (m, 1H), 7.50 (dd, 1H), 8.72 (dd, 1H), 8.93 (dd, 1H), 9.05 (s, 1H), 12.02 (s, 1H).

### Beispiel 31

### 2-[5-Fluor-3-(2-fluorbenzyl)-1H-indazol-1-yl]-4-hydroxy-5,5-dimethyl-5,7-dihydro-6H-pyrrolo[2,3-d]pyrimidin-6-on

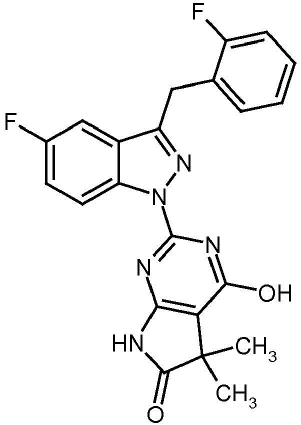

Die Titelverbindung wurde als Nebenkomponente bei der Versuchsdurchführung zu Beispiel 43A erhalten. Ausbeute: 72 mg (14 % d. Th., 83%-ige Reinheit)
LC-MS (Methode 1): Rₜ = 1.33 min; MS (ESIpos): m/z = 422 [M+H]⁺
¹H-NMR (400 MHz, DMSO-d₆): δ [ppm] = 1.33 (s, 6H), 4.40 (s, 2H), 7.14-7.23 (m, 2H), 7.27-7.34 (m, 1H), 7.42 (t, 1H), 7.49-7.58 (m, 2H), 8.75 (s br, 1H), 11.31 (s br, 1H), 12.37 (s br, 1H).

### Beispiel 32

### 2-[1-(2-Fluorbenzyl)-1H-pyrazolo[3,4-b]pyridin-3-yl]-5,5-dimethyl-4-[2-(trifluormethyl)morpholin-4-yl]-5,7-dihydro-6H-pyrrolo[2,3-d]pyrimidin-6-on

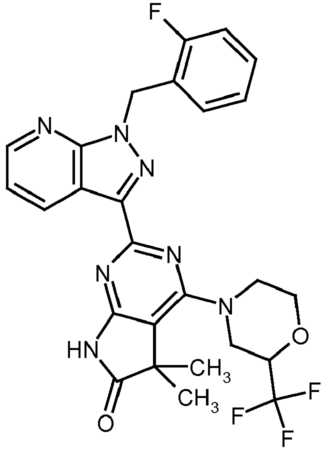

Unter Argonatmosphäre wurden 200 mg (Reinheit 62%, 0.24 mmol) 2-[1-(2-Fluorbenzyl)-1H-pyrazolo[3,4-b]pyridin-3-yl]-4-iod-5,5-dimethyl-5,7-dihydro-6H-pyrrolo[2,3-d]pyrimidin-6-on (Beispiel 15A) in 4 ml absolutem NMP vorgelegt und mit 924 mg (4.82 mmol) 2-(Trifluormethyl)morpholin sowie mit 623 mg (4.82 mmol) N,N-Diisopropylethylamin versetzt. Das Gemisch wurde 5h bei 150°C in der Mikrowelle erhitzt. Die Reaktionslösung wurde filtriert und mittels päparativer HPLC gereinigt (Eluent: Acetonitril/Wasser mit 0.1% Ameisensäure, Gradient 20:80 → 100:0). Es wurden 60 mg (46% d. Th.) der Zielverbindung erhalten.
LC-MS (Methode 1): Rₜ = 1.16 min; MS (ESIpos): m/z = 542 [M+H]⁺
¹H-NMR (400MHz, DMSO-d₆): δ [ppm]= 1.43 (s, 6H), 3.36-3.42 (m, 1H), 3.80 (dt, 1H), 4.13 (dd, 2H), 4.43-4.51 (m, 2H), 5.85 (s, 2H), 7.14 (t, 1H), 7.18-7.24 (m, 2H), 7.34-7.38 (m, 1H), 7.40 (dd, 1H), 8.66 (dd, 1H), 8.75 (dd, 1H), 11.41 (s, 1H).

### Beispiel 33

### 2-[1-(2-Fluorbenzyl)-1H-pyrazolo[3,4-b]pyridin-3-yl]-5,5-dimethyl-4-[3-(trifluormethyl)pyrrolidin-1-yl]-5,7-dihydro-6H-pyrrolo[2,3-d]pyrimidin-6-on

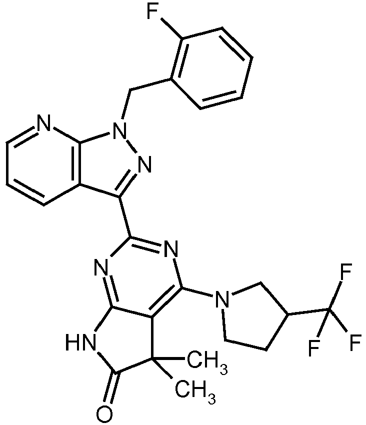

Unter einer Argonatmosphäre wurden 200 mg (Reinheit 62%, 0.24 mmol) 2-[1-(2-Fluorbenzyl)-1H-pyrazolo[3,4-b]pyridin-3-yl]-4-iod-5,5-dimethyl-5,7-dihydro-6H-pyrrolo[2,3-d]pyrimidin-6-on (Beispiel 15A) in 4 ml absolutem NMP vorgelegt und mit 671 mg (4.82 mmol) 3-(Trifluormethyl)pyrrolidin versetzt. Das Gemisch wurde 5h bei 150°C in der Mikrowelle erhitzt. Die Reaktionslösung wurde filtriert und mittels päparativer HPLC gereinigt (Eluent: Acetonitril/Wasser mit 0.1% Ameisensäure, Gradient 20:80 → 100:0). Es wurden 65 mg (49% d. Th.) der Zielverbindung erhalten.
LC-MS (Methode 1): Rₜ = 1.21 min; MS (ESIpos): m/z = 526 [M+H]⁺
¹H-NMR (400MHz, DMSO-d₆): δ [ppm]= 1.42 (s, 3H), 1.43 (s, 3H), 2.15-2.23 (m, 1H), 2.32-2.41 (m, 1H), 3.38-3.48 (m, 1H), 3.79-3.94 (m, 3H), 4.07 (dd, 1H), 5.84 (s, 2H), 7.12-7.25 (m, 3H), 7.33-7.39 (m, 1H), 7.42 (dd, 1H), 8.65 (dd, 1H), 8.81 (dd, 1H), 11.29 (s, 1H).

### Beispiel 34

### 2-[5-Fluor-3-(2-fluorbenzyl)-1H-indazol-1-yl]-5,5-dimethyl-4-(3,3,3-trifluorpropoxy)-5,7-dihydro-6H-pyrrolo[2,3-d]pyrimidin-6-on

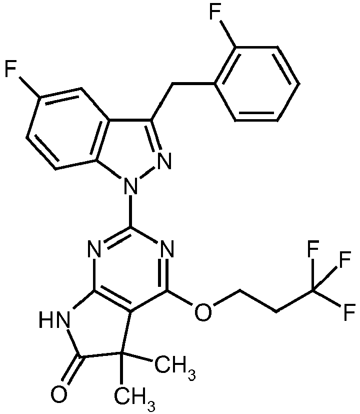

Unter Argon wurden 80 mg (0.19 mmol) der Verbindung aus Beispiel 31, in 2 ml THF weitgehend gelöst mit 32.5 mg (0.29 mmol) 3,3,3-Trifluorpropan-1-ol, 75 mg (0.29 mmol) Triphenylphosphin und 56 µl (0.29 mmol) Diisopropylazodicarboxylat versetzt und 3 d bei RT gerührt. Es wurden weitere 11 mg (0.01 mmol) 3,3,3-Trifluorpropan-1-ol, 25 mg (0.01 mmol) Triphenylphosphin und 19 µl (0.01 mmol) Diisopropylazodicarboxylat zugegeben und 1 d bei RT gerührt. Die Reaktionsmischung wurde eingeengt und der Rückstand per präparativer HPLC (Reprosil C18, Gradient aus Acetonitril/0.01% aq. Ameisensäure) gereinigt. Ausb.: 48 mg (49% d. Th.)
LC-MS (Methode 1): Rₜ = 1.32 min; MS (ESIpos): m/z = 518 [M+H]⁺
¹H-NMR (500MHz, DMSO-d₆): δ [ppm]= 1.34 (s, 6H), 2.82 - 3.00 (m, 2H), 4.40 (s, 2H), 4.75 (t, 2H), 7.11 - 7.24 (m, 2H), 7.26 - 7.32 (m, 1H), 7.37 - 7.50 (m, 2H), 7.53 - 7.62 (m, 1H), 8.60 (dd, 1H), 11.53 (br. s., 1 H).

### Beispiel 35

### 2-[3-(2-Fluorbenzyl)-1H-indazol-1-yl]-5,5-dimethyl-4-[(3,3,3-trifluorpropyl)amino]-5,7-dihydro-6H-pyrrolo[2,3-d]pyrimidin-6-on

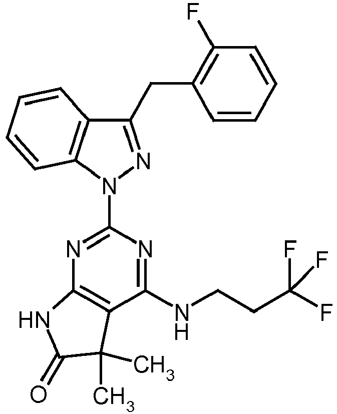

60 mg (0.12 mmol) der Verbindung aus Beispiel 38A wurden in 1.2 ml NMP gelöst und nach Zugabe von 0.3 ml 3,3,3-Trifluorpropylamin in einem verschlossenen Microwellengefäß 3h bei 150°C in der Microwelle erhitzt. Die Reaktionsmischung wurde mittels präparativer HPLC (Reprosil C18, Gradient aus Acetonitril/0.01% aq. Ameisensäure) gereinigt. Ausb.: 38 mg (65% d. Th.)
LC-MS (Methode 1): Rₜ = 1.26 min; MS (ESIpos): m/z = 499 [M+H]⁺
¹H-NMR (400MHz, DMSO-d₆): δ [ppm]= 1.36 (s, 6H), 2.58 - 2.80 (m, 2H), 3.80 (q, 2H), 4.40 (s, 2H), 6.98 (t, 1H), 7.09 - 7.24 (m, 2H), 7.25 - 7.33 (m, 2H), 7.38 (t, 1H), 7.50 (t, 1H), 7.74 (d, 1H), 8.59 (d, 1H), 11.19 (s, 1H).

In Analogie zu Beispiel 35 wurden die in Tabelle 3 aufgeführten Verbindungen hergestellt:

**Tabelle 3**

| **Beispiel Nr.** | **Struktur** | **Edukte; Ausbeute** | **Analytik** |
|---|---|---|---|
| **36** | 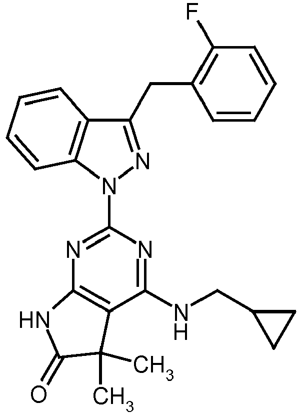 | Beispiel 38A, Cyclopropyl methylamin; 77% d. Th. | LC-MS (Methode 1): Rₜ = 1.27 min; MS (ESIpos): m/z = 457 [M+H]⁺ |
| | | | ¹H-NMR (500MHz, DMSO-d₆): δ [ppm]= 0.29 - 0.36 (m, 2H), 0.39 - 0.48 (m, 2H), 1.15 - 1.28 (m, 1H), 1.38 (s, 6H), 3.38 - 3.50 (m, 2H), 4.39 (s, 2H), 6.85 - 6.95 (m, 1H), 7.10 - 7.22 (m, 2H), 7.23 - 7.33 (m, 2H), 7.34 - 7.41 (m, 1H), 7.50 - 7.58 (m, 1H), 7.72 (dd, 1H), 8.67 (dd, 1H), 11.08 (s, 1H). |
| | 4-[(Cyclopropylmethyl)amino]-2-[3-(2-fluorbenzyl)-1H-indazol-1-yl]-5,5-dimethyl-5,7-dihydro-6H-pyrrolo[2,3-d]pyrimidin-6-on | | |
| **37** | 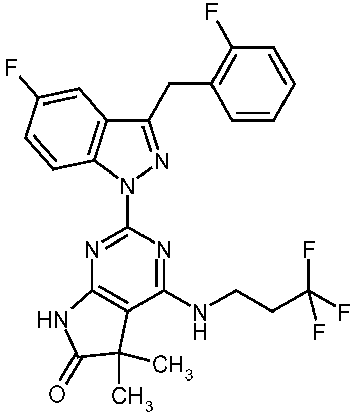 | Beispiel 43A, 3,3,3-Trifluor-propylamin, 82% d. Th. | LC-MS (Methode 1): Rₜ = 1.27 min; MS (ESIpos): m/z = 517 [M+H]⁺ |
| | | | ¹H-NMR (500MHz, DMSO-d₆): δ [ppm]= 1.36 (s, 6H), 2.60 - 2.75 (m, 2H), 3.78 (q, 2H), 4.38 (s, 2H), 6.96 (t, 1H), 7.12 - 7.22 (m, 2H), 7.27 - 7.44 (m, 3H), 7.54 (dd, 1H), 8.55 - 8.64 (m, 1H), 11.16 (s, 1H). |
| | 2-[5-Fluor-3-(2-fluorbenzyl)-1H-indazol-1-yl]-5,5-dimethyl-4-[(3,3,3-trifluorpropyl)amino]-5,7-dihydro-6H-pyrrolo[2,3-d]pyrimidin-6-on | | |
| **38** | 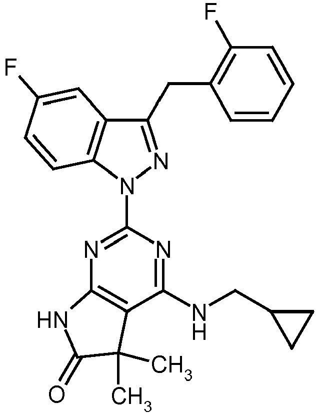 | Beispiel 43A, Cyclopentylmethylamin; 74% d. Th. | LC-MS (Methode 1): Rₜ = 1.29 min; MS (ESIpos): m/z = 475 [M+H]⁺ |
| | | | ¹H-NMR (500MHz, DMSO-d₆): δ [ppm]= 0.27 - 0.35 (m, 2H), 0.38 - 0.47 (m, 2H), 1.15 - 1.27 (m, 1H), 3.43 (t, 2H), 4.38 (s, 2H), 6.87 - 6.97 (m, 1H), 7.10-7.23 (m, 2H), 7.26 - 7.34 (m, 1H), 7.36-7.48 (m, 2H), 7.53 (d, 1H), 8.67 (dd, 1H), 11.09 (br. s., 1H). |
| | 4-[(Cyclopropylmethyl)amino]-2-[5-fluor-3-(2-fluorbenzyl)-1H-indazol-1-yl]-5,5-dimethyl-5,7-dihydro-6H-pyrrolo[2,3-d]pyrimidin-6-on | | |

### Beispiel 39

### 2-[8-(2-Fluorbenzyl)imidazo[1,5-a]pyrimidin-6-yl]-5,5-dimethyl-4-[(3,3,3-trifluorpropyl)amino]-5,7-dihydro-6H-pyrrolo[2,3-d]pyrimidin-6-on

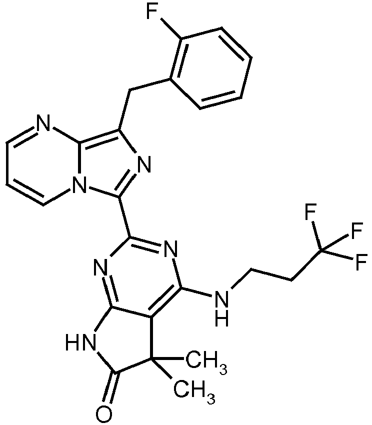

Unter einer Argonatmosphäre wurden 200 mg (Reinheit 69%, 0.27 mmol) 2-[8-(2-Fluorbenzyl)imidazo[1,5-a]pyrimidin-6-yl]-4-iod-5,5-dimethyl-5,7-dihydro-6H-pyrrolo[2,3-d]pyrimidin-6-on (Beispiel 52A) in 3.2 ml absolutem NMP suspendiert und mit 607 mg (5.37 mmol) 3,3,3-Trifluorpropan-1-amin versetzt. Das Gemisch wurde 1.5h bei 150°C in der Mikrowelle gerührt. Die Reaktionslösung wurde filtriert und mittels päparativer HPLC gereinigt (Eluent: Acetonitril/Wasser mit 0.1% Salzsäure, Gradient 20:80 → 100:0). Es wurden 41 mg (31% d. Th.) der Zielverbindung erhalten.
LC-MS (Methode 1): Rₜ = 1.09 min; MS (ESIpos): m/z = 500 [M+H]⁺
¹H-NMR (400MHz, DMSO-d₆): δ [ppm]= 1.35 (s, 6H), 2.62-2.74 (m, 2H), 3.77 (q, 2H), 4.33 (s, 2H), 6.87 (t, 1H), 6.96 (dd. 1H), 7.07-7.17 (m, 2H), 7.22-7.27 (m, 1H), 7.32 (t, 1H), 8.34 (d, 1H), 9.83 (d, 1H), 11.10 (s, 1H).

### Beispiel 40

### 2-[1-(2-Fluorbenzyl)-1H-pyrazolo[3,4-b]pyridin-3-yl]-5,5-dimethyl-4-{[(1-methyl-1H-pyrazol-5-yl)methyl]amino}-5,7-dihydro-6H-pyrrolo[2,3-d]pyrimidin-6-on

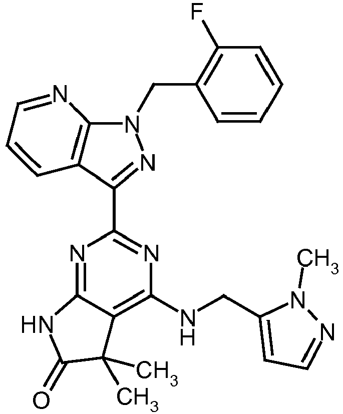

111.1 mg (1.0 mmol) 1-(1-Methyl-1H-pyrazol-5-yl)methanamin wurden in einem Vial eines Mikrowellen-Reaktorblockes vorgelegt und mit einer Lösung von 51.4 mg (100 µmol) 2-[1-(2-Fluorbenzyl)-1H-pyrazolo[3,4-b]pyridin-3-yl]-4-iod-5,5-dimethyl-5,7-dihydro-6H-pyrrolo[2,3-d]pyrimidin-6-on (Beispiel 15A) in 0.6 ml 1-Methyl-2-pyrrolidinon versetzt. Dann wurde der Reaktorblock dicht verschlossen und 6h mit Mikrowellen bestrahlt, um eine Temperatur des Gemisches von 170°C zu erreichen und zu halten. Nach dem Abkühlen wurde via präparativer LC-MS (Methode 6) aufgereinigt. Die produkthaltigen Fraktionen wurden mittels Zentrifugaltrockner im Vakuum eingeengt. Der Rückstand der einzelnen Fraktionen wurde in je 0.6 ml DMSO gelöst und vereinigt. Anschließend wurde im Zentrifugaltrockner das Lösemittel vollständig abgedampft. Es wurden 32.7 mg (61% d. Th.) Zielprodukt erhalten.
LC-MS (Methode 5): Rₜ = 1.07 min;
MS (ESIpos): m/z = 498 [M+H]⁺, Reinheit: 93%

In Analogie zu Beispiel 40 wurden die in Tabelle 4 aufgeführten Verbindungen hergestellt.

**Tabelle 4**

| **Bsp.-Nr.** | **Struktur Ausbeute (% d. Th.)** | **Name** | **Analytische Daten** |
|---|---|---|---|
| **41** | 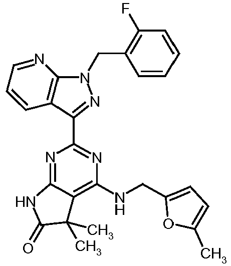 | 2-[1-(2-Fluorbenzyl)-1H-pyrazolo[3,4-b]pyridin-3-yl]-5,5-dimethyl-4-{[(5-methylfuran-2-yl)methyl]amino}-5,7-dihydro-6H-pyrrolo[2,3-d]pyrimidin-6-on | LC/MS (Methode 5): |
| | | | Rₜ = 1.19 min |
| | | | MS (ESIpos): |
| | | | m/z = 498 [M+H]⁺ |
| | 27.1 mg, (51% d. Th.) | | Reinheit: 94% |
| **42** | 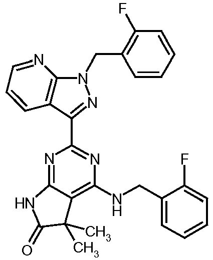 | 4-[(2-Fluorbenzyl)amino]-2-[1-(2-fluorbenzyl)-1H-pyrazolo[3,4-b]pyridin-3-yl]-5,5-dimethyl-5,7-dihydro-6H-pyrrolo[2,3-d]pyrimidin-6-on | LC/MS (Methode 5.): |
| | | | Rₜ = 1.20 min |
| | | | MS (ESIpos): |
| | 14.5 mg (28% d. Th.) | | m/z = 512 [M+H]⁺ |
| **43** | 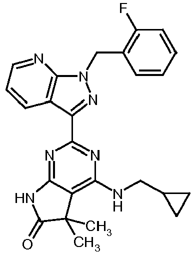 | 4-[(Cyclopropylmethyl) amino]-2-[1-(2-fluorobenzyl)-1H-pyrazolo[3,4-b]pyridin-3-yl]-5,5-dimethyl-5,7-dihydro-6H-pyrrolo[2,3-d]pyrimidin-6-on | LC/MS (Methode 5.): |
| | | | Rₜ = 1.19 min |
| | | | MS (ESIpos): |
| | 11.3 mg, (25% d. Th.) | | m/z = 458 [M+H]⁺ |
| **44** | 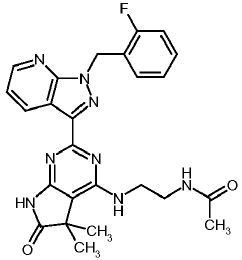 | N-[2-({2-[1-(2-Fluorbenzyl)-1 H-pyrazolo[3,4-b]pyridin-3-yl]-5,5-dimethyl-6-oxo-6,7-dihydro-5H-pyrrolo[2,3-d]pyrimidin-4-yl}amino)ethyl]acetamid | LC/MS (Methode 5.): |
| | | | Rₜ = 0.99 min |
| | | | MS (ESIpos): |
| | 10.5 mg, (21% d. Th.) | | m/z = 489 [M+H]⁺ |
| **45** | 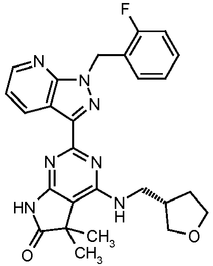 | 2-[1-(2-Fluorbenzyl)-1H-pyrazolo[3,4-b]pyridin-3-yl]-5,5-dimethyl-4-[(tetrahydrofuran-3-ylmethyl)amino]-5,7-dihydro-6H-pyrrolo[2,3-d]pyrimidin-6-on | LC/MS (Methode 5.): |
| | | | Rₜ = 1.11 min |
| | | | MS (ESIpos): |
| | 28.1 mg, (58% d. Th.) | | m/z = 488 [M+H]⁺ |
| **46** | 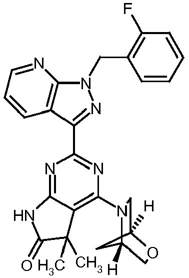 | 2-[1-(2-Fluorbenzyl)-1H-pyrazolo[3,4-b]pyridin-3-yl]-5,5-dimethyl-4-[(1S,4S)-2-oxa-5-azabicyclo[2.2.1]hept-5-yl]-5,7-dihydro-6H-pyrrolo[2,3-d]pyrimidin-6-on | LC/MS (Methode 5.): |
| | | | Rₜ = 1.10 min |
| | | | MS (ESIpos): |
| | | | m/z = 486 [M+H]⁺ |
| | 2 mg, (4% d.Th.) | | Reinheit: 92% |
| **47** | 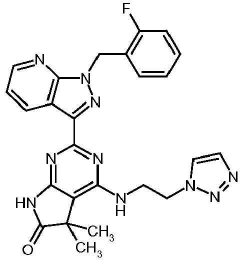 | 2-[1-(2-Fluorbenzyl)-1H-pyrazolo[3,4-b]pyridin-3-yl]-5,5-dimethyl-4-{[2-(1H-1,2,3-triazol-1-yl)ethyl]amino}-5,7-dihydro-6H-pyrrolo[2,3-d]pyrimidin-6-on | LC/MS (Methode 5.): |
| | | | Rₜ = 1.02 min |
| | | | MS (ESIpos): |
| | | | m/z = 499 [M+H]⁺ |
| | 28.2 mg, (52% d.Th.) | | Reinheit: 92% |
| **48** | 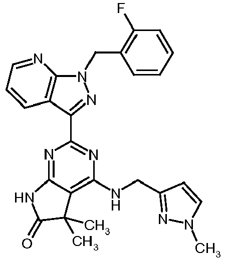 | 2-[1-(2-Fluorbenzyl)-1H-pyrazolo[3,4-b]pyridin-3-yl]-5,5-dimethyl-4-{[(1-methyl-1H-pyrazol-3-yl)methyl]amino}-5,7-dihydro-6H-pyrrolo[2,3-d]pyrimidin-6-on | LC/MS (Methode 5.): |
| | | | Rₜ = 1.05 min |
| | | | MS (ESIpos): |
| | | | m/z = 498 [M+H]⁺ |
| | 11.4 mg, (17% d. Th.) | | Reinheit: 76% |
| **49** | 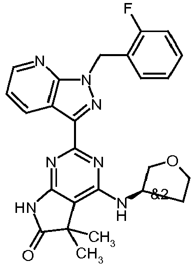 | 2-[1-(2-Fluorbenzyl)-1H-pyrazolo[3,4-b]pyridin-3-yl]-5,5-dimethyl-4-(tetrahydrofuran-3-ylamino)-5,7-dihydro-6H-pyrrolo[2,3-d]pyrimidin-6-on | LC/MS (Methode 5.): |
| | | | Rₜ = 0.79 min |
| | | | MS (ESIpos): |
| | 1.3 mg, (3% d. Th.) | | m/z = 474 [M+H]⁺ |
| **50** | 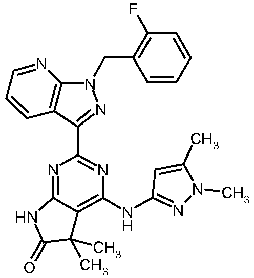 | 4-[(1,5-Dimethyl-1H-pyrazol-3-yl)amino]-2-[1-(2-fluorbenzyl)-1H-pyrazolo[3,4-b]pyridin-3-yl]-5,5-dimethyl-5,7-dihydro-6H-pyrrolo[2,3-d]pyrimidin-6-on | LC/MS (Methode 5.): |
| | | | Rₜ = 1.08 min |
| | | | MS (ESIpos): |
| | | | m/z = 498 [M+H]⁺ |
| | 9.1 mg, (17% d.Th.) | | Reinheit: 95% |
| **51** | 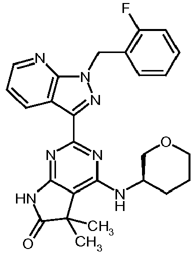 | 2-[1-(2-Fluorbenzyl)-1H-pyrazolo[3,4-b]pyridin-3-yl]-5,5-dimethyl-4-(tetrahydro-2H-pyran-3-ylamino)-5,7-dihydro-6H-pyrrolo[2,3-d]pyrimidin-6-on | LC/MS (Methode 5.): |
| | | | Rₜ = 1.14 min |
| | | | MS (ESIpos): |
| | | | m/z = 488 [M+H]⁺ |
| | 2.0 mg, (4% d. Th.) | | Reinheit: 95% |
| **52** | 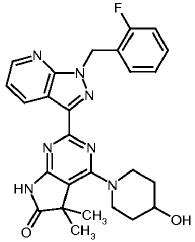 | 2-[1-(2-Fluorbenzyl)-1H-pyrazolo[3,4-b]pyridin-3-yl]-4-(4-hydroxypiperidin-1-yl)-5,5-dimethyl-5,7-dihydro-6H-pyrrolo[2,3-d]pyrimidin-6-on | LC/MS (Methode 5.): |
| | | | Rₜ = 1.04 min |
| | | | MS (ESIpos): |
| | 17.8 mg, (37% d. Th.) | | m/z = 488 [M+H]⁺ |
| **53** | 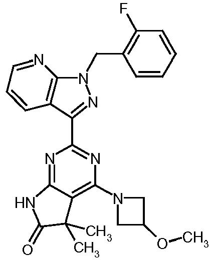 | 2-[1-(2-Fluorbenzyl)-1H-pyrazolo[3,4-b]pyridin-3-yl]-4-(3-methoxyazetidin-1-yl)-5,5-dimethyl-5,7-dihydro-6H-pyrrolo[2,3-d]pyrimidin-6-on | LC/MS (Methode 5.): |
| | | | Rₜ = 0.82 min |
| | | | MS (ESIpos): |
| | | | m/z = 474 [M+H]⁺ |
| | 6.3 mg, (11% d. Th.) | | Reinheit: 83% |
| **54** | 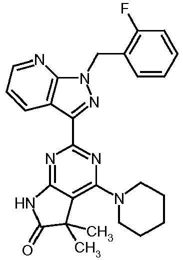 | 2-[1-(2-Fluorbenzyl)-1H-pyrazolo[3,4-b]pyridin-3-yl]-5,5-dimethyl-4-(piperidin-1-yl)-5,7-dihydro-6H-pyrrolo[2,3-d]pyrimidin-6-on | LC/MS (Methode 5.): |
| | | | Rₜ = 1.26 min |
| | | | MS (ESIpos): |
| | | | m/z = 472 [M+H]⁺ |
| | 0.5 mg, (1% d. Th.) | | Reinheit: 95% |
| **55** | 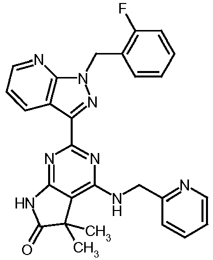 | 2-[1-(2-Fluorbenzyl)-1H-pyrazolo[3,4-b]pyridin-3-yl]-5,5-dimethyl-4-[(pyridin-2-ylmethyl)amino]-5,7-dihydro-6H-pyrrolo[2,3-d]pyrimidin-6-on | LC/MS (Methode 5.): |
| | | | Rₜ = 0.95 min |
| | | | MS (ESIpos): |
| | | | m/z = 495 [M+H]⁺ |
| | 26.7 mg, (49% d. Th.) | | Reinheit: 91% |
| **56** | 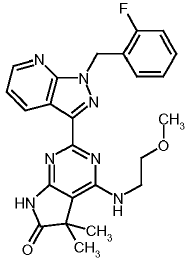 | 2-[1-(2-Fluorbenzyl)-1H-pyrazolo[3,4-b]pyridin-3-yl]-4-[(2-methoxyethyl)amino]-5,5-dimethyl-5,7-dihydro-6H-pyrrolo[2,3-d]pyrimidin-6-on | LC/MS (Methode 5.): |
| | | | Rₜ = 1.09 min |
| | | | MS (ESIpos): |
| | | | m/z = 462 [M+H]⁺ |
| | 6.0 mg, (12% d. Th.) | | Reinheit: 94% |
| **57** | 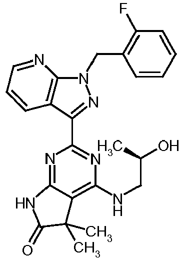 | 2-[1-(2-Fluorbenzyl)-1H-pyrazolo[3,4-b]pyridin-3-yl]-4-[(2-hydroxypropyl)amino]-5,5-dimethyl-5,7-dihydro-6H-pyrrolo[2,3-d]pyrimidin-6-on | LC/MS (Methode 5.): |
| | | | Rₜ = 1.01 min |
| | | | MS (ESIpos): |
| | | | m/z = 461 [M+H]⁺ |
| | 24.2 mg, (50% d. Th.) | | Reinheit: 95% |
| **58** | 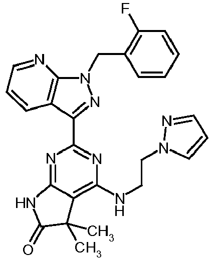 | 2-[1-(2-Fluorbenzyl)-1H-pyrazolo[3,4-b]pyridin-3-yl]-5,5-dimethyl-4-{[2-(1H-pyrazol-1-yl)ethyl]amino}-5,7-dihydro-6H-pyrrolo[2,3-d]pyrimidin-6-on | LC/MS (Methode 5.): |
| | | | Rₜ = 1.08 min |
| | | | MS (ESIpos): |
| | 25.6 mg, (51% d. Th.) | | m/z = 498 [M+H]⁺ |
| **59** | 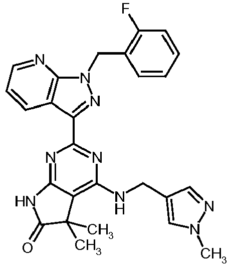 | 2-[1-(2-Fluorbenzyl)-1H-pyrazolo[3,4-b]pyridin-3-yl]-5,5-dimethyl-4-{[(1-methyl-1H-pyrazol-4-yl)methyl]amino}-5,7-dihydro-6H-pyrrolo[2,3-d]pyrimidin-6-on | LC/MS (Methode 5.): |
| | | | Rₜ = 1.07 min |
| | | | MS (ESIpos): |
| | 26.5 mg, (51% d. Th.) | | m/z = 498 [M+H]⁺ |
| **60** | 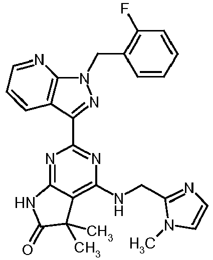 | 2-[1-(2-Fluorbenzyl)-1H-pyrazolo[3,4-b]pyridin-3-yl]-5,5-dimethyl-4-{[(1-methyl-1H-imidazol-2-yl)methyl]amino}-5,7-dihydro-6H-pyrrolo[2,3-d]pyrimidin-6-on | LC/MS (Methode 5.): |
| | | | Rₜ = 0.79 min |
| | | | MS (ESIpos): |
| | 22.6 mg, (45% d. Th.) | | m/z = 498 [M+H]⁺ |
| **61** | 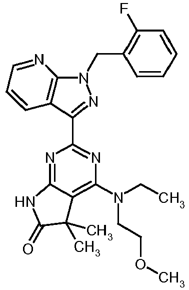 | 4-[Ethyl(2-methoxyethyl)amino]-2-[1-(2-fluorbenzyl)-1H-pyrazolo[3,4-b]pyridin-3-yl]-5,5-dimethyl-5,7-dihydro-6H-pyrrolo[2,3-d]pyrimidin-6-on | LC/MS (Methode 5.): |
| | | | Rₜ = 1.19 min |
| | | | MS (ESIpos): |
| | 1.5 mg, (3% d. Th.) | | m/z = 490 [M+H]⁺ |
| **62** | 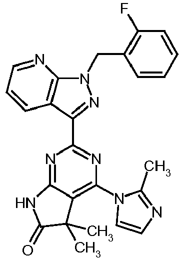 | 2-[1-(2-Fluorbenzyl)-1H-pyrazolo[3,4-b]pyridin-3-yl]-5,5-dimethyl-4-(2-methyl-1H-imidazol-1-yl)-5,7-dihydro-6H-pyrrolo[2,3-d]pyrimidin-6-on | LC/MS (Methode 5.): |
| | | | Rₜ = 0.83 min |
| | | | MS (ESIpos): |
| | 10.6 mg, (23% d. Th.) | | m/z = 469 [M+H]⁺ |
| **63** | 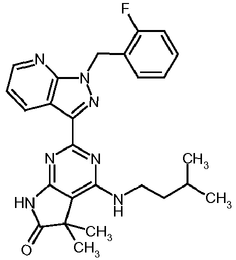 | 2-[1-(2-Fluorbenzyl)-1H-pyrazolo[3,4-b]pyridin-3-yl]-5,5-dimethyl-4-[(3-methylbutyl)amino]-5,7-dihydro-6H-pyrrolo[2,3-d]pyrimidin-6-on | LC/MS (Methode 5.): |
| | | | Rₜ = 1.25 min |
| | | | MS (ESIpos): |
| | 23.7 mg, (50% d. Th.) | | m/z = 474 [M+H]⁺ |
| **64** | 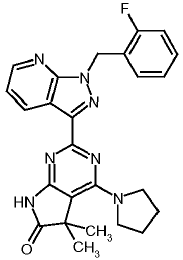 | 2-[1-(2-Fluorbenzyl)-1H-pyrazolo[3,4-b]pyridin-3-yl]-5,5-dimethyl-4-(pyrrolidin-1-yl)-5,7-dihydro-6H-pyrrolo[2,3-d]pyrimidin-6-on | LC/MS (Methode 5.): |
| | | | Rₜ = 1.32 min |
| | | | MS (ESIpos): |
| | 11.3 mg, (25% d. Th.) | | m/z = 458 [M+H]⁺ |
| **65** | 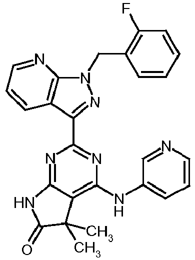 | 2-[1-(2-Fluorbenzyl)-1H-pyrazolo[3,4-b]pyridin-3-yl]-5,5-dimethyl-4-(pyridin-3-ylamino)-5,7-dihydro-6H-pyrrolo[2,3-d]pyrimidin-6-on | LC/MS (Methode 5.): |
| | | | Rₜ = 0.78 min |
| | | | MS (ESIpos): |
| | 7.6 mg, (16% d. Th.) | | m/z = 481 [M+H]⁺ |
| **66** | 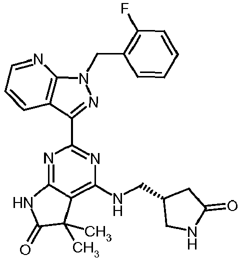 | 2-[1-(2-Fluorbenzyl)-1H-pyrazolo[3,4-b]pyridin-3-yl]-5,5-dimethyl-4-{[(5-oxopyrrolidin-3-yl)methyl]amino}-5,7-dihydro-6H-pyrrolo[2,3-d]pyrimidin-6-on | LC/MS (Methode 5.): |
| | | | Rₜ = 0.98 min |
| | | | MS (ESIpos): |
| | | | m/z = 501 [M+H]⁺ |
| | 24.0 mg, (44% d. Th.) | | Reinheit: 91% |
| **67** | 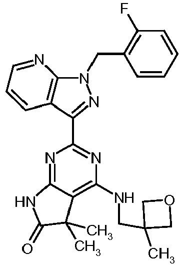 | 2-[1-(2-Fluorbenzyl)-1H-pyrazolo[3,4-b]pyridin-3-yl]-5,5-dimethyl-4-{[(3-methyloxetan-3-yl)methyl]amino}-5,7-dihydro-6H-pyrrolo[2,3-d]pyrimidin-6-on | LC/MS (Methode 5.): |
| | | | Rₜ = 1.07 min |
| | | | MS (ESIpos): |
| | | | m/z = 488 [M+H]⁺ |
| | 4.4 mg, (9% d. Th.) | | Reinheit: 87% |
| **68** | 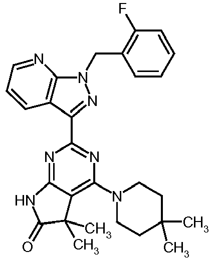 | 4-(4,4-Dimethylpiperidin-1-yl)-2-[1-(2-fluorbenzyl)-1H-pyrazolo[3,4-b]pyridin-3-yl]-5,5-dimethyl-5,7-dihydro-6H-pyrrolo[2,3-d]pyrimidin-6-on | LC/MS (Methode 5.): |
| | | | Rₜ = 1.31 min |
| | | | MS (ESIpos): |
| | | | m/z = 500 [M+H]⁺ |
| | 6.6 mg, (12% d. Th.) | | Reinheit: 93% |
| **69** | 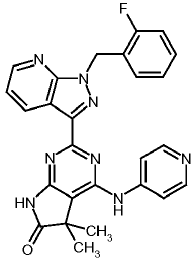 | 2-[1-(2-Fluorbenzyl)-1H-pyrazolo[3,4-b]pyridin-3-yl]-5,5-dimethyl-4-(pyridin-4-ylamino)-5,7-dihydro-6H-pyrrolo[2,3-d]pyrimidin-6-on | LC/MS (Methode 5.): |
| | | | Rₜ = 0.79 min |
| | | | MS (ESIpos): |
| | | | m/z = 481 [M+H]⁺ |
| | 3.7 mg, (7% d. Th.) | | Reinheit: 90% |
| **70** | 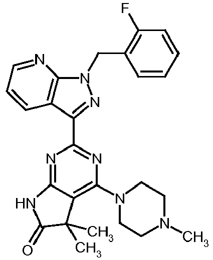 | 2-[1-(2-Fluorbenzyl)-1H-pyrazolo[3,4-b]pyridin-3-yl]-5,5-dimethyl-4-(4-methylpiperazin-1-yl)-5,7-dihydro-6H-pyrrolo[2,3-d]pyrimidin-6-on | LC/MS (Methode 5.): |
| | | | Rₜ = 0.75 min |
| | | | MS (ESIpos): |
| | 17.3 mg, (36% d. Th.) | | m/z = 487 [M+H]⁺ |
| **71** | 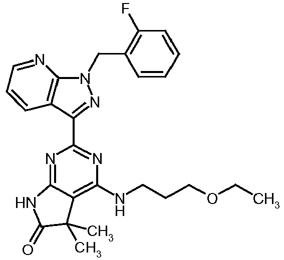 | 4-[(3-Ethoxypropyl)amino]-2-[1-(2-fluorbenzyl)-1H-pyrazolo[3,4-b]pyridin-3-yl]-5,5-dimethyl-5,7-dihydro-6H-pyrrolo[2,3-d]pyrimidin-6-on | LC/MS (Methode 5.): |
| | | | Rₜ = 1. 17 min |
| | | | MS (ESIpos): |
| | 20.3 mg, (41% d. Th.) | | m/z = 490 [M+H]⁺ |
| **72** | 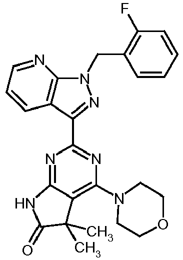 | 2-[1-(2-Fluorbenzyl)-1H-pyrazolo[3,4-b]pyridin-3-yl]-5,5-dimethyl-4-(morpholin-4-yl)-5,7-dihydro-6H-pyrrolo[2,3-d]pyrimidin-6-on | LC/MS (Methode 5.): |
| | | | Rₜ = 1.13 min |
| | | | MS (ESIpos): |
| | 17.3 mg, (37% d. Th.) | | m/z = 474 [M+H]⁺ |
| **73** | 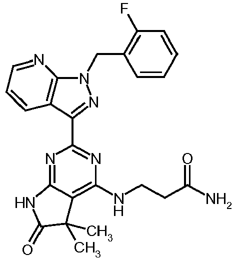 | N³-{2-[1-(2-Fluorbenzyl)-1H-pyrazolo[3,4-b]pyridin-3-yl]-5,5-dimethyl-6-oxo-6,7-dihydro-5H-pyrrolo[2,3-d]pyrimidin-4-yl} -beta-alaninamid | LC/MS (Methode 5.): |
| | | | Rₜ = 0.94 min |
| | | | MS (ESIpos): |
| | 2.5 mg, (5% d. Th.) | | m/z = 475 [M+H]⁺ |
| **74** | 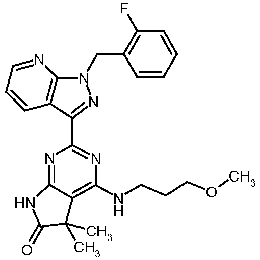 | 2-[1-(2-Fluorbenzyl)-1H-pyrazolo[3,4-b]pyridin-3-yl]-4-[(3-methoxypropyl)amino]-5,5-dimethyl-5,7-dihydro-6H-pyrrolo[2,3-d]pyrimidin-6-on | LC/MS (Methode 5.): |
| | | | Rₜ = 1.14 min |
| | | | MS (ESIpos): |
| | 27.9 mg, (59% d. Th.) | | m/z = 476 [M+H]⁺ |
| **75** | 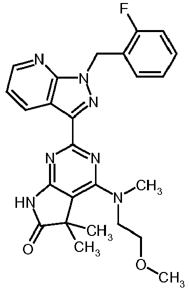 | 2-[1-(2-Fluorbenzyl)-1H-pyrazolo[3,4-b]pyridin-3-yl]-4-[(2-methoxyethyl)(methyl)ami no]-5,5-dimethyl-5,7-dihydro-6H-pyrrolo[2,3-d]pyrimidin-6-on | LC/MS (Methode 5.): |
| | | | Rₜ = 1.15 min |
| | | | MS (ESIpos): |
| | 2.1 mg, (4% d. Th.) | | m/z = 476 [M+H]⁺ |
| **76** | 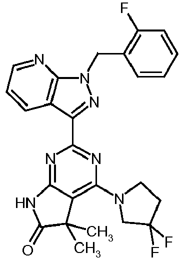 | 4-(3,3-Difluorpyrrolidin-1-yl)-2-[1-(2-fluorbenzyl)-1H-pyrazolo[3,4-b]pyridin-3-yl]-5,5-dimethyl-5,7-dihydro-6H-pyrrolo[2,3-d]pyrimidin-6-on | LC/MS (Methode 5.): |
| | | | Rₜ = 1.19 min |
| | | | MS (ESIpos): |
| | 7.7 mg, (16% d. Th.) | | m/z = 494 [M+H]⁺ |
| **77** | 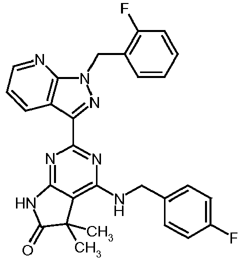 | 4-[(4-Fluorbenzyl)amino]-2-[1-(2-fluorbenzyl)-1H-pyrazolo[3,4-b]pyridin-3-yl]-5,5-dimethyl-5,7-dihydro-6H-pyrrolo[2,3-d]pyrimidin-6-on | LC/MS (Methode 5.): |
| | | | Rₜ = 1.19 min |
| | | | MS (ESIpos): |
| | 1.0 mg, (2% d. Th.) | | m/z = 512 [M+H]⁺ |
| **78** | 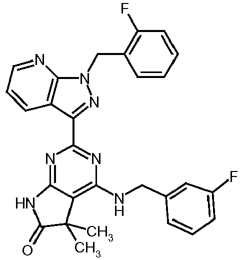 | 4-[(3-Fluorbenzyl)amino]-2-[1-(2-fluorbenzyl)-1H-pyrazolo[3,4-b]pyridin-3-yl]-5,5-dimethyl-5,7-dihydro-6H-pyrrolo[2,3-d]pyrimidin-6-on | LC/MS (Methode 5.): |
| | | | Rₜ = 1.20 min |
| | | | MS (ESIpos): |
| | 28.0 mg, (55% d. Th.) | | m/z = 512 [M+H]⁺ |
| **79** | 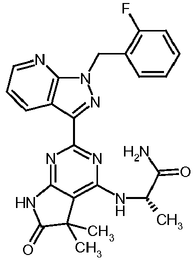 | N²-{2-[1-(2-Fluorbenzyl)-1H-pyrazolo[3,4-b]pyridin-3-yl]-5,5-dimethyl-6-oxo-6,7-dihydro-5H-pyrrolo[2,3-d]pyrimidin-4-yl}-L-alaninamid | LC/MS (Methode 5.): |
| | | | Rₜ = 0.96 min |
| | | | MS (ESIpos): |
| | | | m/z = 475 [M+H]⁺ |
| | 0.6 mg, (1% d. Th.) | | Reinheit: 94% |
| **80** | 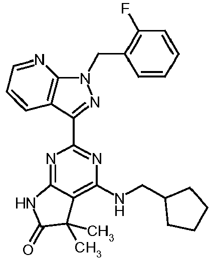 | 4-[(Cyclopentylmethyl) amino]-2-[1-(2-fluorbenzyl)-1H-pyrazolo[3,4-b]pyridin-3-yl]-5,5-dimethyl-5,7-dihydro-6H-pyrrolo[2,3-d]pyrimidin-6-on | LC/MS (Methode 5.): |
| | | | Rₜ = 1.27 min |
| | | | MS (ESIpos): |
| | 0.8 mg, (2% d. Th.) | | m/z = 486 [M+H]⁺ |
| **81** | 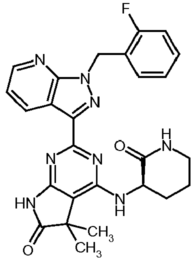 | 2-[1-(2-Fluorbenzyl)-1H-pyrazolo[3,4-b]pyridin-3-yl]-5,5-dimethyl-4-[(2-oxopiperidin-3-yl)amino]-5,7-dihydro-6H-pyrrolo[2,3-d]pyrimidin-6-on | LC/MS (Methode 5.): |
| | | | Rₜ = 1.01 min |
| | | | MS (ESIpos): |
| | | | m/z = 501 [M+H]⁺ |
| | 21.3 mg, (39% d. Th.) | | Reinheit: 92% |
| **82** | 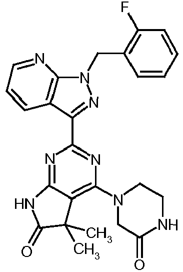 | 2-[1-(2-Fluorbenzyl)-1H-pyrazolo[3,4-b]pyridin-3-yl]-5,5-dimethyl-4-(3-oxopiperazin-1-yl)-5,7-dihydro-6H-pyrrolo[2,3-d]pyrimidin-6-on | LC/MS (Methode 5.): |
| | | | Rₜ = 0.98 min |
| | | | MS (ESIpos): |
| | 0.8 mg, (2% d. Th.) | | m/z = 487 [M+H]⁺ |
| **83** | 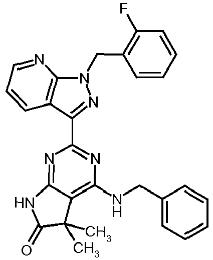 | 4-(Benzylamino)-2-[1-(2-fluorbenzyl)-1H-pyrazolo[3,4-b]pyridin-3-yl]-5,5-dimethyl-5,7-dihydro-6H-pyrrolo[2,3-d]pyrimidin-6-on | LC/MS (Methode 5.): |
| | | | Rₜ = 1.20 min |
| | | | MS (ESIpos): |
| | 24.3 mg, (49% d. Th.) | | m/z = 494 [M+H]⁺ |
| **84** | 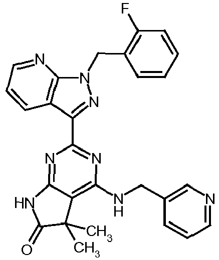 | 2-[1-(2-Fluorbenzyl)-1H-pyrazolo[3,4-b]pyridin-3-yl]-5,5-dimethyl-4-[(pyridin-3-ylmethyl)amino]-5,7-dihydro-6H-pyrrolo[2,3-d]pyrimidin-6-on | LC/MS (Methode 5.): |
| | | | Rₜ = 0.91 min |
| | | | MS (ESIpos): |
| | 22.9 mg, (46% d. Th.) | | m/z = 495 [M+H]⁺ |
| **85** | 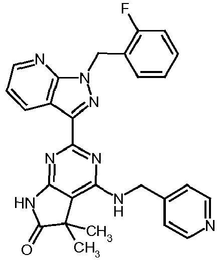 | 2-[1-(2-Fluorbenzyl)-1H-pyrazolo[3,4-b]pyridin-3-yl]-5,5-dimethyl-4-[(pyridin-4-ylmethyl)amino]-5,7-dihydro-6H-pyrrolo[2,3-d]pyrimidin-6-on | LC/MS (Methode 5.): |
| | | | Rₜ = 0.87 min |
| | | | MS (ESIpos): |
| | 16.5 mg (33% d. Th.) | | m/z = 495 [M+H]⁺ |
| **86** | 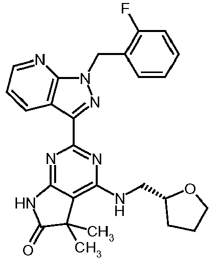 | 2-[1-(2-Fluorbenzyl)-1H-pyrazolo[3,4-b]pyridin-3-yl]-5,5-dimethyl-4-[(tetrahydrofuran-2-ylmethyl)amino]-5,7-dihydro-6H-pyrrolo[2,3-d]pyrimidin-6-on | LC/MS (Methode 5.): |
| | | | Rₜ = 1.12 min |
| | | | MS (ESIpos): |
| | 23.2 mg (48% d. Th.) | | m/z = 488 [M+H]⁺ |
| **87** | 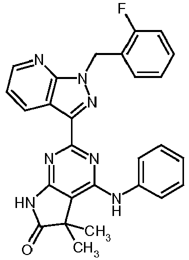 | 2-[1-(2-Fluorbenzyl)-1H-pyrazolo[3,4-b]pyridin-3-yl]-5,5-dimethyl-4-(phenylamino)-5,7-dihydro-6H-pyrrolo[2,3-d]pyrimidin-6-on | LC/MS (Methode 5.): |
| | | | Rₜ = 1.21 min |
| | | | MS (ESIpos): |
| | 17.6 mg (37% d. Th.) | | m/z = 480 [M+H]⁺ |

### Beispiel 88

### 4-{[(1R)-1-Cyclopropylethyl]amino}-2-[5-fluor-1-(2-fluorbenzyl)-1H-pyrazolo[3,4-b]pyridin-3-yl]-5,5-dimethyl-5,7-dihydro-6H-pyrrolo[2,3-d]pyrimidin-6-on

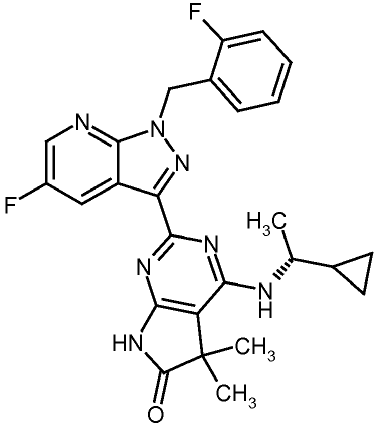

150 mg (0.186 mmol, ca. 66% Reinheit) Beispiel 16A wurden in einem mikrowellengeeigneten Reaktionsgefäß in 1-Methyl-2-pyrrolidon (3.5 ml) gelöst und mit 0.5 ml (R)-1-Cyclopropylethylamin versetzt. Anschliessend wurde mit einem entsprechenden Septum verschlossen und für 3 h bei 150°C in der Mikrowelle erhitzt. Nach Abkühlen wurde das Reaktionsgemisch mittels präparativer HPLC gereinigt (Acetonitril:Wasser: Wasser + 1 % Trifluoressigsäure - (70:24:6). Es wurden 54 mg der Titelverbindung erhalten (59 % d. Th.).
LC-MS (Methode 1): Rₜ = 1.29 min; MS (EIpos): m/z = 490 [M+H]⁺.
¹H-NMR (400 MHz, DMSO-d₆): δ [ppm] = 0.19-0.25 (m, 1H), 0.35-0.41 (m, 2H), 0.48-0.53 (m, 1H), 1.13-1.21 (m, 1H), 1.35 (d, 3H), 1.39 (2s, 6H), 4.00-4.06 (m, 1H), 5.82 (s, 2H), 6.29 (d, 1H), 7.12-7.16 (m, 1H), 7.19-7.24 (m, 2H), 7.33-7.39 (m, 1H), 8.42 (dd, 1H), 8.71 (dd, 1H), 10.99 (s, 1H).

### Beispiel 89

### 4-{[(1S)-1-Cyclopropylethyl]amino}-2-[5-fluor-1-(2-fluorbenzyl)-1H-pyrazolo[3,4-b]pyridin-3-yl]-5,5-dimethyl-5,7-dihydro-6H-pyrrolo[2,3-d]pyrimidin-6-on

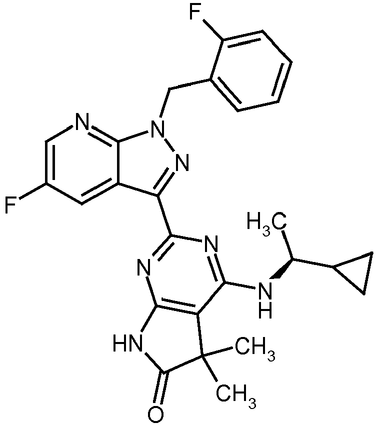

150 mg (0.186 mmol, ca. 66%-ige Reinheit) Beispiel 16A wurden in einem mikrowellengeeigneten Reaktionsgefäß in 1-Methyl-2-pyrrolidon (3.5 ml) gelöst und mit 0.5 ml (S)-1-Cyclopropylethylamin versetzt. Anschliessend wurde mit einem entsprechenden Septum verschlossen und für 3 h bei 150°C in der Mikrowelle erhitzt. Nach Abkühlen wurde das Reaktionsgemisch mittels präparativer HPLC gereinigt (Acetonitril:Wasser: Wasser + 1% Trifluoressigsäure (70:24:6). Es wurden 69 mg der Titelverbindung erhalten (75 % d. Th.).
LC-MS (Methode 1): Rₜ = 1.29 min; MS (EIpos): m/z = 490 [M+H]⁺.
¹H-NMR (400 MHz, DMSO-d₆): δ [ppm] = 0.19-0.25 (m, 1H), 0.35-0.41 (m, 2H), 0.48-0.53 (m, 1H), 1.13-1.21 (m, 1H), 1.35 (d, 3H), 1.39 (2s, 6H), 4.00-4.06 (m, 1H), 5.82 (s, 2H), 6.29 (d, 1H), 7.12-7.16 (m, 1H), 7.19-7.24 (m, 2H), 7.33-7.39 (m, 1H), 8.42 (dd, 1H), 8.71 (dd, 1H), 10.99 (s, 1H).

### Beispiel 90

### 2-[5-Fluor-1-(2-fluorbenzyl)-6-methyl-1H-pyrazolo[3,4-b]pyridin-3-yl]-5,5-dimethyl-4-[(3,3,3-trifluorpropyl)amino]-5,7-dihydro-6H-pyrrolo[2,3-d]pyrimidin-6-on

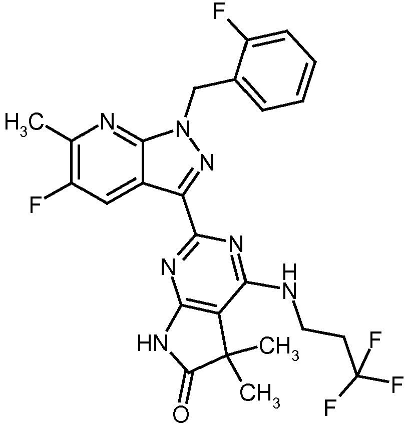

150 mg (0.231 mmol, ca. 84% Reinheit) Beispiel 53A wurden in einem mikrowellengeeigneten Reaktionsgefäß in 1-Methyl-2-pyrrolidon (3 ml) gelöst und mit 1 ml 3,3,3-Trifluorpropyl-1-amin versetzt. Anschliessend wurde mit einem entsprechenden Septum verschlossen und für 3 h bei 150°C in der Mikrowelle erhitzt. Nach Abkühlen wurde das Reaktionsgemisch mittels präparativer HPLC gereinigt (Acetonitril:Wasser (+0.05 % Ameisensäure) - Gradient). Es wurden 89 mg der Titelverbindung erhalten (73 % d. Th.).
LC-MS (Methode 1): Rₜ = 1.27 min; MS (EIpos): m/z = 532[M+H]⁺.
¹H-NMR (400 MHz, DMSO-d₆): δ [ppm] = 1.35 (s, 6H), 2.62 (d, 3H), 2.64-2.73 (m, 2H), 3.81 (q, 2H), 5.78 (s, 2H), 6.84 (t, 1H), 7.12-7.25 (m, 3H), 7.33-7.39 (m, 1H), 8.36 (d, 1H), 11.05 (s br, 1H).

### Beispiel 91

### 2-[1-(2-Fluorbenzyl)-1H-pyrazolo[3,4-b]pyridin-3-yl]-5,5-dimethyl-4-[2-oxo-5-(trifluormethyl)piperidin-1-yl]-5,7-dihydro-6H-pyrrolo[2,3-d]pyrimidin-6-on

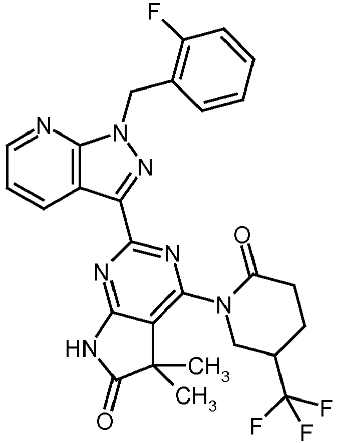

Unter Argonatmosphäre wurden 200 mg (Reinheit 62%, 0.24 mmol) 2-[1-(2-Fluorbenzyl)-1H-pyrazolo[3,4-b]pyridin-3-yl]-4-iod-5,5-dimethyl-5,7-dihydro-6H-pyrrolo[2,3-d]pyrimidin-6-on (Beispiel 15A) in 2.5 ml absolutem Acetonitril suspendiert und mit 806 mg (4.82 mmol) 5-(Trifluormethyl)piperidin-2-on, 157 mg (0.48 mmol) Cäsiumcarbonat, 7 mg (0.05 mmol) Kupfer(I)oxid sowie 26 mg (0.19 mmol) 2-Hydroxybenzaldehydoxim versetzt. Das Gemisch wurde 1h bei 200°C in der Mikrowelle erhitzt. Die Reaktionslösung wurde filtriert und mittels päparativer HPLC gereinigt (Eluent: Acetonitril/Wasser, Gradient 20:80 → 100:0). Es wurden 15 mg (11% d. Th.) der Zielverbindung erhalten.
LC-MS (Methode 1): Rₜ = 1.07 min; MS (ESIpos): m/z = 554 [M+H]⁺
¹H-NMR (400MHz, DMSO-d₆): δ [ppm]= 1.31 (d, 6H), 1.41 (s, 6H), 2.05-2.24 (m, 2H), 2.59-2.78 (m, 3H), 3.76-3.87 (m, 2H), 5.89 (s, 2H), 7.12-7.18 (m, 2H), 7.23 (t, 1H), 7.33-7.38 (m, 1H), 7.46 (dd, 1H), 8.69 (d, 1H), 8.87 (d, 1H), 11.82 (s, 1H).

### Beispiel 92

### 2-[1-(2-Fluorbenzyl)-1H-pyrazolo[3,4-b]pyridin-3-yl]-5,5-dimethyl-4-(2-methyl-3-oxopiperazin-1-yl)-5,7-dihydro-6H-pyrrolo[2,3-d]pyrimidin-6-on

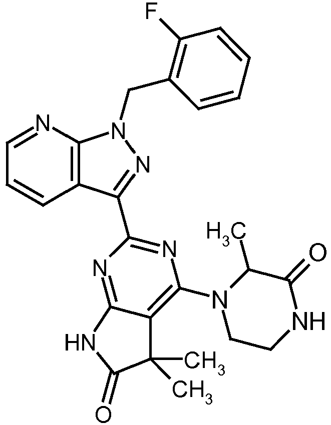

Unter Argonatmosphäre wurden 200 mg (Reinheit 62%, 0.24 mmol) 2-[1-(2-Fluorbenzyl)-1H-pyrazolo[3,4-b]pyridin-3-yl]-4-iod-5,5-dimethyl-5,7-dihydro-6H-pyrrolo[2,3-d]pyrimidin-6-on (Beispiel 15A) in 4 ml absolutem 1-Methyl-2-pyrrolidon suspendiert und mit 550 mg (4.82 mmol) 3-Methylpiperazin-2-on versetzt. Das Gemisch wurde 3h bei 150°C sowie 3h bei 220°C in der Mikrowelle erhitzt. Das Reaktionsgemisch wurde nach dem Abkühlen mittels päparativer HPLC gereinigt (Eluent: Acetonitril/Wasser, Gradient 20:80 → 100:0). Es wurden 61 mg (Reinheit 100%, 50% d. Th.) der Zielverbindung erhalten.
LC-MS (Methode 8): Rₜ = 2.55 min; MS (ESIpos): m/z = 501 [M+H]⁺
¹H-NMR (400MHz, DMSO-d₆): δ [ppm]= 1.42-1.45 (m, 9H), 3.54-3.61 (m, 1H), 4.13-4.18 (m, 1H), 4.97 (q, 1H), 5.86 (s, 2H), 7.12-7.25 (m, 3H), 7.33-7.39 (m, 1H), 7.45 (dd, 1H), 8.09 (s, 1H), 8.67 (dd, 1H), 8.76 (dd, 1H), 11.40 (s br, 1H).

### Beispiel 93

### 4-(1,1-Dioxidothiomorpholin-4-yl)-2-[1-(2-fluorbenzyl)-1H-pyrazolo[3,4-b]pyridin-3-yl]-5,5-dimethyl-5,7-dihydro-6H-pyrrolo[2,3-d]pyrimidin-6-on

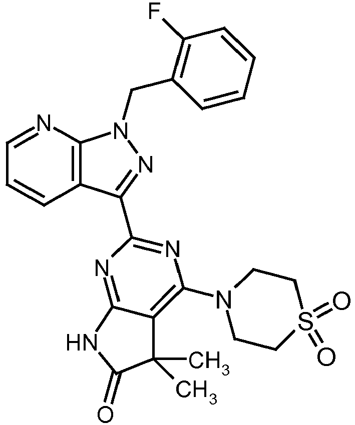

Unter Argonatmosphäre wurden 200 mg (Reinheit 62%, 0.24 mmol) 2-[1-(2-Fluorbenzyl)-1H-pyrazolo[3,4-b]pyridin-3-yl]-4-iod-5,5-dimethyl-5,7-dihydro-6H-pyrrolo[2,3-d]pyrimidin-6-on (Beispiel 15A) in 4 ml absolutem 1-Methyl-2-pyrrolidon suspendiert und mit 652 mg (4.82 mmol) Thiomorpholin-1,1-dioxid versetzt. Das Gemisch wurde 3h bei 150°C sowie 1h bei 200°C in der Mikrowelle erhitzt. Das Reaktionsgemisch wurde nach dem Abkühlen mittels päparativer HPLC gereinigt (Eluent: Acetonitril/Wasser mit 0.1% Ameisensäure, Gradient 20:80 → 100:0). Es wurden 72 mg (57% d. Th.) der Zielverbindung erhalten.
LC-MS (Methode 1): Rₜ = 0.96 min; MS (ESIpos): m/z = 522 [M+H]⁺
¹H-NMR (400MHz, DMSO-d₆): δ [ppm]= 1.43 (s, 6H), 3.36-3.39 (m, 4H), 4.14-4.17 (m, 4H), 5.85 (s, 2H), 7.11-7.25 (m, 3H), 7.33-7.39 (m, 1H), 7.44 (dd, 1H), 8.66-8.71 (m, 2H), 11.45 (s, 1H).

### Beispiel 94

### N-(1-{2-[1-(2-Fluorbenzyl)-1H-pyrazolo[3,4-b]pyridin-3-yl]-5,5-dimethyl-6-oxo-6,7-dihydro-5H-pyrrolo[2,3-d]pyrimidin-4-yl}pyrrolidin-3-yl)acetamid

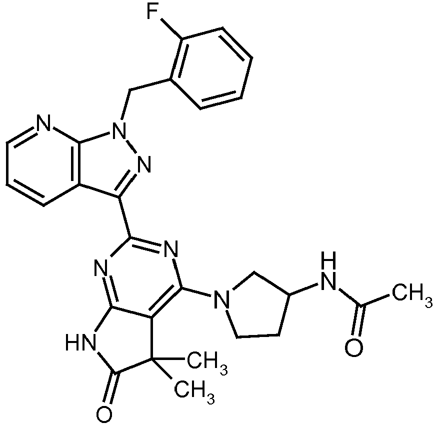

Unter Argonatmosphäre wurden 200 mg (Reinheit 62%, 0.24 mmol) 2-[1-(2-Fluorbenzyl)-1H-pyrazolo[3,4-b]pyridin-3-yl]-4-iod-5,5-dimethyl-5,7-dihydro-6H-pyrrolo[2,3-d]pyrimidin-6-on (Beispiel 15A) in 4 ml absolutem 1-Methyl-2-pyrrolidon suspendiert und mit 618 mg (4.82 mmol) N-(Pyrrolidin-3-yl)acetamid versetzt. Das Gemisch wurde 3h bei 150°C in der Mikrowelle erhitzt. Das Reaktionsgemisch wurde nach dem Abkühlen mittels päparativer HPLC gereinigt (Eluent: Acetonitril/Wasser mit 0.1% Ameisensäure, Gradient 20:80 → 100:0). Es wurden 122 mg (98% d. Th.) der Zielverbindung erhalten.
LC-MS (Methode 1): Rₜ = 0.91 min; MS (ESIpos): m/z = 515 [M+H]⁺
¹H-NMR (400MHz, DMSO-d₆): δ [ppm]= 1.42 (s, 6H), 1.83 (s, 3H), 1.90-2.01 (m, 1H), 2.17-2.25 (m, 1H), 3.65 (dd, 1H), 3.81-3.97 (m, 3H), 4.33-4.40 (m, 1H), 5.84 (s, 2H), 7.12-7.25 (m, 3H), 7.32-7.40 (m, 1H), 7.43 (dd, 1H), 8.22 (d, 1H), 8.65 (dd, 1H), 8.86 (dd, 1H), 11.21 (s, 1H).

### Beispiel 95

### 2-[1-(2-Fluorbenzyl)-1H-pyrazolo[3,4-b]pyridin-3-yl]-4-[(trans-4-hydroxycyclohexyl)amino]-5,5-dimethyl-5,7-dihydro-6H-pyrrolo[2,3-d]pyrimidin-6-on

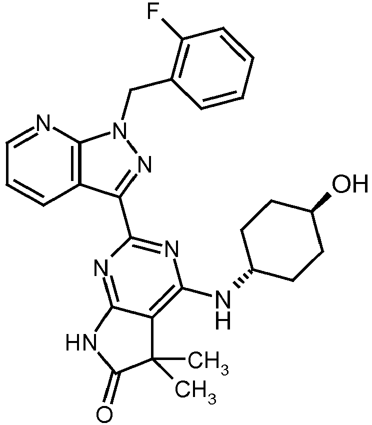

Unter Argonatmosphäre wurden 200 mg (Reinheit 62%, 0.24 mmol) 2-[1-(2-Fluorbenzyl)-1H-pyrazolo[3,4-b]pyridin-3-yl]-4-iod-5,5-dimethyl-5,7-dihydro-6H-pyrrolo[2,3-d]pyrimidin-6-on (Beispiel 15A) in 4 ml absolutem 1-Methyl-2-pyrrolidon suspendiert und mit 555 mg (4.82 mmol) *trans*-4-Aminocyclohexanol versetzt. Das Gemisch wurde 3h bei 150°C in der Mikrowelle erhitzt. Das Reaktionsgemisch wurde nach dem Abkühlen mittels päparativer HPLC gereinigt (Eluent: Acetonitril/Wasser mit 0.1% Ameisensäure, Gradient 20:80 → 100:0). Es wurden 91 mg (73% d. Th.) der Zielverbindung erhalten.
LC-MS (Methode 1): Rₜ = 0.92 min; MS (ESIpos): m/z = 502 [M+H]⁺
¹H-NMR (400MHz, DMSO-d₆): δ [ppm]= 1.31-1.41 (m, 2H), 1.36 (s, 6H), 1.49-1.58 (m, 2H), 1.91-1.98 (m, 4H), 3.42-3.51 (m, 1H), 4.12-4.22 (m, 1H), 4.61 (d, 1H), 5.83 (s, 2H), 6.15 (d, 1H), 7.12-7.25 (m, 3H), 7.33-7.38 (m, 1H), 7.41 (dd, 1H), 8.66 (dd, 1H), 8.80 (dd, 1H), 11.21 (s, 1H).

### Beispiel 96

### 2-[5-Fluor-1-(2-fluorbenzyl)-1H-pyrazolo[3,4-b]pyridin-3-yl]-5,5-dimethyl-4-[(3,3,3-trifluor-2-hydroxypropyl)amino]-5,7-dihydro-6H-pyrrolo[2,3-d]pyrimidin-6-on

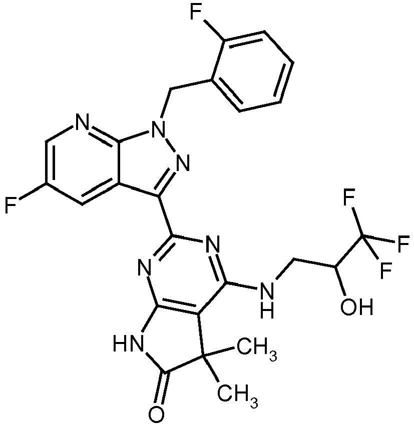

300 mg (0.372 mmol, Reinheit 66 %) Beispiel 16A wurden in einem mikrowellengeeigneten Reaktionsgefäß in 1-Methyl-2-pyrrolidon (5 ml) gelöst und mit 300 mg (2.324 mmol) 3-Amino-1,1,1-trifluor-2-propanol versetzt. Anschliessend wurde mit einem entsprechenden Septum verschlossen und zweimal für 3 h bei 150°C in der Mikrowelle erhitzt. Nach Abkühlen wurde das Reaktionsgemisch mittels präparativer HPLC gereinigt (Acetonitril:Wasser (+0.05 % Ameisensäure) - Gradient). Es wurden 164 mg der Titelverbindung erhalten (83 % d. Th.)
LC-MS (Methode 1): Rt = 1.12 min; MS (EIpos): m/z = 534 [M+H]+.

### Trennung in Enantiomere:

164 mg des erhaltenen Racemats wurden mittels präparativer HPLC (Laufmittel: ((iso-Hexan:Ethanol + 0.2 % Trifluoressigsäure + 1% Wasser) 80/20), Wellenlänge: 210 nM an chiraler Phase (Daicel Chiralpak OZ-H (HPLC), 5 µM 250 x 20 mm) in die Enantiomere aufgetrennt.

### Beispiel 96-1 (Enantiomer 1)

Ausbeute: 42 mg
ee = 99% (analytische HPLC: (Laufmittel: (iso-Hexan:Ethanol 80/20) + 0.2 % Trifluoressigsäure + 1% Wasser an chiraler Phase (Chiralcel OZ-H, 5 µM 250*4.6 mm)
Rₜ = 5.004 min.
¹H-NMR (400 MHz, DMSO-d₆): δ [ppm] = 1.36 (s, 3H), 1.37 (s, 3H), 3.61-3.68 (m, 1H), 3.91-3.97 (m, 1H), 4.32-4.42 (m, 1H), 5.82 (s, 2H), 6.56 (d, 1H), 6.90 (t, 1H), 7.13-7.26 (m, 3H), 7.34-7.39 (m, 1H), 8.53 (dd, 1H), 8.71 (dd, 1H), 11.05 (br s, 1H).

### Beispiel 96-2 (Enantiomer 2)

Ausbeute: 31 mg
ee = 96% (analytische HPLC: (Laufmittel: (iso-Hexan:Ethanol 80/20) + 0.2 % Trifluoressigsäure + 1% Wasser an chiraler Phase (Chiralcel OZ-H, 5 µM 250*4.6 mm)
Rₜ = 5.044 min.

### Beispiel 97

### 4-{[(2,2-Difluorcyclopropyl)methyl]amino}-2-[5-fluor-1-(2-fluorbenzyl)-1H-pyrazolo[3,4-b]pyridin-3-yl]-5,5-dimethyl-5,7-dihydro-6H-pyrrolo[2,3-d]pyrimidin-6-on

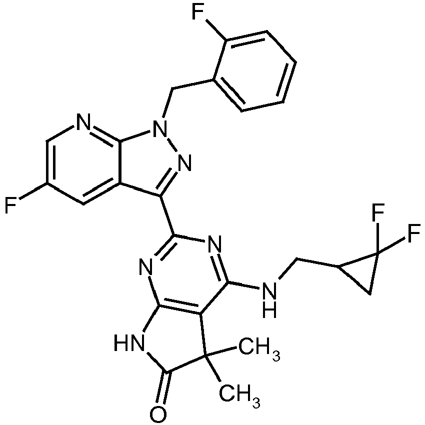

100 mg (0.115 mmol, Reinheit 61 %) Beispiel 16A wurden in einem mikrowellengeeigneten Reaktionsgefäß in 1-Methyl-2-pyrrolidon (3 ml) gelöst und mit 164 mg (1.146 mmol) 2,2-Difluorcyclopropylmethylamin-Hydrochlorid und 0.24 ml (1.375 mmol) N,N-Diisopropylethylamin versetzt. Anschliessend wurde das Reaktionsgefäß mit einem Septum verschlossen und für 12 h bei 150°C in der Mikrowelle erhitzt. Nach Abkühlen wurde das Reaktionsgemisch mittels präparativer HPLC gereinigt (Acetonitril:Wasser (+0.05 % Ameisensäure) - Gradient). Es wurden 21 mg der Titelverbindung erhalten (35 % d. Th.).
LC-MS (Methode 1): Rₜ = 1.20 min; MS (EIpos): m/z = 512 [M+H]⁺.
¹H-NMR (400 MHz, DMSO-d₆): δ [ppm] = 1.37 (s, 6H), 1.42-1.50 (m, 1H), 1.53-1.61 (m, 1H), 2.12-2.22 (m, 1H), 3.57-3.63 (m, 1H), 3.73-3.79 (m, 1H), 5.83 (s, 2H), 6.98 (t, 1H), 7.13-7.17 (m, 1H), 7.20-7.26 (m, 2H), 7.34-7.39 (m, 1H), 8.51 (dd, 1H), 8.72 (dd, 1H), 11.06 (s, 1H).

### Beispiel 98

### 2-[5-Fluor-1-(2-fluorbenzyl)-1H-pyrazolo[3,4-b]pyridin-3-yl]-4-{[(1-hydroxycyclopropyl)methyl]amino}-5,5-dimethyl-5,7-dihydro-6H-pyrrolo[2,3-d]pyrimidin-6-on

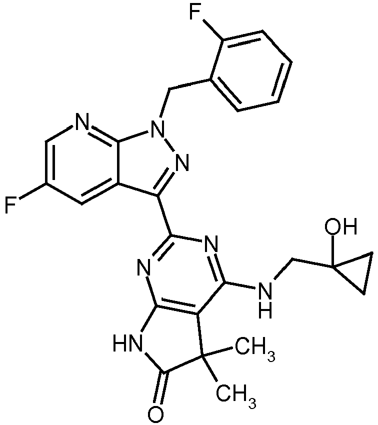

200 mg (0.376 mmol) Beispiel 16A wurden in einem mikrowellengeeigneten Reaktionsgefäß in 1-Methyl-2-pyrrolidon (3 ml) gelöst und mit 98 mg (1.127 mmol) 1-(Aminomethyl)-cyclopropanol versetzt. Anschliessend wurde das Reaktionsgefäß mit einem Septum verschlossen und für 12 h bei 150°C in der Mikrowelle erhitzt. Nach Abkühlen wurde das Reaktionsgemisch mittels präparativer HPLC gereinigt (Acetonitril:Wasser (+0.05 % Ameisensäure) - Gradient). Es wurden 56 mg der Titelverbindung erhalten (31 % d. Th.).
LC-MS (Methode 1) Rₜ = 1.07 min; MS (EIpos): m/z = 492 [M+H]⁺.
¹H-NMR (400 MHz, DMSO-d₆): δ [ppm] = 0.56-0.63 (m, 4H), 1.37 (s, 6H), 3.77-3.79 (m, 2H), 5.54 (s, 1H), 5.82 (s, 2H), 6.58 (m, 1H), 7.12-7.16 (m, 1H), 7.20-7.25 (m, 2H), 7.34-7.39 (m, 1H), 8.60 (dd, 1H), 8.71 (dd, 1H), 11.02 (s, 1H).

### Beispiel 99

### 4-{[(2,2-Dimethylcyclopropyl)methyl]amino}-2-[5-fluor-1-(2-fluorbenzyl)-1H-pyrazolo[3,4-b]pyridin-3-yl]-5,5-dimethyl-5,7-dihydro-6H-pyrrolo[2,3-d]pyrimidin-6-on

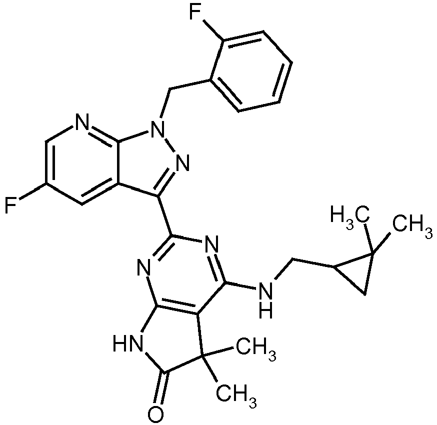

200 mg (0.376 mmol) Beispiel 16A wurden in einem mikrowellengeeigneten Reaktionsgefäß in 1-Methyl-2-pyrrolidon (3 ml) gelöst und mit 254 mg (1.879 mmol) 1-(2,2-Dimethylcyclopropyl)methanamin-Hydrochlorid und 0.393 ml (2.254 mmol) N,N-Diisopropylethylamin versetzt. Anschliessend wurde mit einem entsprechenden Septum verschlossen und für 3 h bei 150°C in der Mikrowelle erhitzt. Nach Abkühlen wurde das Reaktionsgemisch mittels präparativer HPLC gereinigt (Acetonitril:Wasser (+0.05 % Ameisensäure) - Gradient). Es wurden 64 mg der Titelverbindung erhalten (33 % d. Th.).
LC-MS (Methode 1) Rₜ = 1.38 min; MS (EIpos): m/z = 504 [M+H]⁺.
¹H-NMR (400 MHz, DMSO-d₆): δ [ppm] = 0.24 (dd, 1H), 0.43 (dd, 1H), 1.02 (s, 3H), 1.08-1.15 (m, 1H), 1.17 (s, 3H), 1.37 (s, 6H), 3.30-3.41 (m, 1H), 3.70-3.76 (m, 1H), 5.82 (s, 2H), 6.81 (t, 1H), 7.13-7.17 (m, 1H), 7.20-7.26 (m, 2H), 7.34-7.39 (m, 1H), 8.58 (dd, 1H), 8.72 (dd, 1H), 11.01 (s, 1H).

### Beispiel 100

### 2'-[5-Fluor-1-(2-fluorbenzyl)-1H-pyrazolo[3,4-b]pyridin-3-yl]-4'-[(3,3,3-trifluorpropyl)amino]-4,5-dihydrospiro[furan-3,5'-pyrrolo[2,3-d]pyrimidin]-6'(7'H)-on

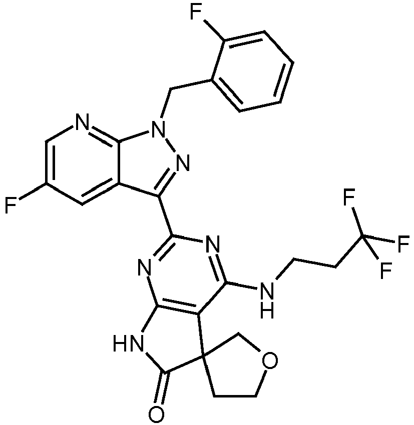

84 mg (0.150 mmol) Beispiel 58A wurden in einem mikrowellengeeigneten Reaktionsgefäß in 1-Methyl-2-pyrrolidon (3 ml) gelöst und mit 112 mg (0.750 mmol) 3,3,3-Trifluorpropyl-1-amin Hydrochlorid und 0.157 ml (0.900 mmol) N,N-Diisopropylethylamin versetzt. Anschliessend wurde das Reaktionsgefäß mit einem Septum verschlossen und für 3 h bei 150 °C in der Mikrowelle erhitzt. Nach Abkühlen wurde das Reaktionsgemisch mittels präparativer HPLC gereinigt (Acetonitril:Wasser (+0.05 % Ameisensäure) - Gradient). Es wurden 27 mg der Titelverbindung erhalten (33 % d. Th.).
LC-MS (Methode 1): Rₜ = 1.23 min; MS (EIpos): m/z = 546[M+H]⁺.
¹H-NMR (400 MHz, DMSO-d₆): δ [ppm] = 2.19-2.29 (m, 2H), 2.63-2.75 (m, 2H), 3.70 (d, 1H), 3.79-3.95 (m, 3H), 4.09 (d, 1H), 4.26-4.32 (m, 1H), 5.84 (s, 2H), 6.41(t, 1H), 7.13-7.17 (m, 1H), 7.21-7.28 (m, 2H), 7.34-7.39 (m, 1H), 8.48 (dd, 1H), 8.73 (dd, 1H), 11.24 (s, 1H).

### Beispiel 101

### Ethyl-2-[1-(2-fluorbenzyl)-1H-pyrazolo[3,4-b]pyridin-3-yl]-4-hydroxy-5-methyl-6-oxo-6,7-dihydro-5H-pyrrolo[2,3-d]pyrimidin-5-carboxylat

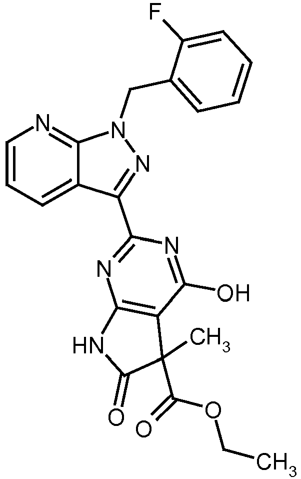

1.00 g (2.167 mmol) Beispiel 59A wurden in Analogie zur Vorschrift unter Beispiel 15A umgesetzt. Es wurden 173 mg (17 % d. Th.) der Titelverbindung sowie 0.887 g Ethyl-2-[1-(2-fluorbenzyl)-1H-pyrazolo[3,4-b]pyridin-3-yl]-4-iod-5-methyl-6-oxo-6,7-dihydro-5H-pyrrolo[2,3-d]pyrimidin-5-carboxylat (Beispiel 60A) erhalten.
LC-MS (Methode 1): Rₜ = 0.96 min; MS (EIpos): m/z = 462[M+H]⁺.
¹H-NMR (400 MHz, DMSO-d₆): δ [ppm] = 1.09 (t, 3H), 1.55 (s, 3H), 4.10 (q, 2H), 5.87 (s, 2H), 7.13-7.17 (m, 1H), 7.21-7.26 (m, 1H), 7.36-7.40 (m, 2H), 7.50 (dd, 1H), 8.73-8.78 (m, 2H), 11.48 (brs, 1H), 12.72 (br s, 1H).

### Beispiel 102

### 2-[5-Fluor-1-(2-fluorbenzyl)-1H-pyrazolo[3,4-b]pyridin-3-yl]-4-hydroxy-5,5-dimethyl-5,7-dihydro-6H-pyrrolo[2,3-d]pyrimidin-6-on

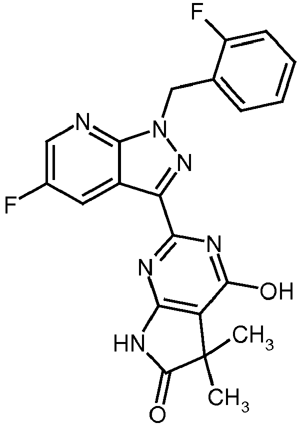

200 mg (0.376 mmol) Beispiel 16A wurden in einem mikrowellengeeigneten Reaktionsgefäß in 3 ml Wasser und 3 ml Tetrahydrofuran gelöst, das Reaktionsgefäß mit einem Septum verschlossen und für 1 h bei 140 °C in der Mikrowelle erhitzt. Danach wurden 1.127 ml (1.127 mmol) 1M Natronlauge zugegeben und für weitere 14 h bei 140 °C in der Mikrowelle erhitzt. Nach Abkühlen wurde das Reaktionsgemisch mittels präparativer HPLC gereinigt (Acetonitril:Wasser (+0.05 % Ameisensäure) - Gradient). Es wurden 24 mg der Titelverbindung erhalten (15 % d. Th.).
LC-MS (Methode 1): Rₜ = 0.96 min; MS (EIpos): m/z = 423 [M+H]⁺.
¹H-NMR (400 MHz, DMSO-d₆): δ [ppm] = 1.33 (s, 6H), 5.85 (s, 2H), 7.14-7.18 (m, 1H), 7.22-7.26 (m, 1H), 7.34-7.41 (m, 2H), 8.55 (br s, 1H), 8.78 (dd, 1H), 11.10 (s br, 1H), 12.11 und 12.61 (s br, zusammen 1H).

### Beispiel 103

### 4-(4,4-Difluorpiperidin-1-yl)-2-[5-fluor-1-(2-fluorbenzyl)-1H-pyrazolo[3,4-b]pyridin-3-yl]-5,5-dimethyl-5,7-dihydro-6H-pyrrolo[2,3-d]pyrimidin-6-on

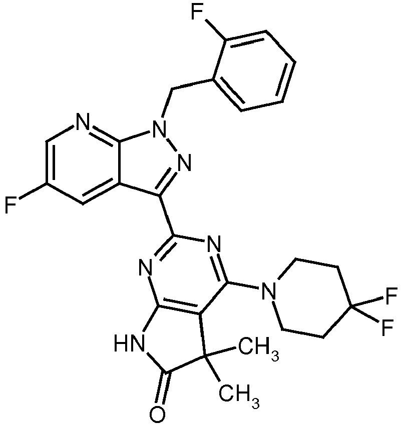

100 mg (0.18 mmol) 2-[5-Fluor-1-(2-fluorbenzyl)-1H-pyrazolo[3,4-b]pyridin-3-yl]-4-iod-5,5-dimethyl-5,7-dihydro-6H-pyrrolo[2,3-d]pyrimidin-6-on (Beispiel 16A) wurden in einem mikrowellengeeigneten Reaktionsgefäß in 1-Methyl-2-pyrrolidon (3.1 ml) gelöst, mit 0.19 ml (1.07 mmol) N,N-Diisopropylethylamin und mit 140 mg (0.89 mmol) 4,4-Difluorpiperidin-Hydrochlorid versetzt. Anschließend wurde das Reaktionsgefäß mit einem Septum verschlossen und für 5 h bei 150°C in der Mikrowelle erhitzt. Anschließend wurden nochmals dieselben Mengen von N,N-Diisopropylethylamin und 4,4-Difluorpiperidin-Hydrochlorid zu der Reaktionslösung zugegeben und die Reaktionsmischung wurde 6 h bei 150°C in der Mikrowelle gerührt. Die Reaktionsmischung wurde mit Wasser versetzt, der ausgefallene Feststoff wurde 30 min bei Raumtemperatur verrührt und anschließend abfiltriert. Der ausgefallene Feststoff wurde mit 1 ml Acetonitril verrührt, der Feststoff abfiltriert und mit 0.5 ml Acetonitril nachgewaschen. Es wurden 63 mg der Zielverbindung (64% d. Th.) erhalten.
LC-MS (Methode 1): Rₜ = 1.29 min; MS (EIpos): m/z = 526 [M+H]⁺.
¹H-NMR (400 MHz, DMSO-d₆): δ [ppm] = 1.44 (s, 6H), 2.10-2.23 (m, 4H), 3.79-3.90 (m, 4H), 5.84 (s, 2H), 7.10-7.28 (m, 3H), 7.32-7.40 (m, 1H), 8.42-8.48 (m, 1H), 8.70-8.76 (m, 1H), 11.35 (s, 1H).

### Beispiel 104

### 2-[5-Fluor-1-(2-fluorbenzyl)-1H-pyrazolo[3,4-b]pyridin-3-yl]-4-(3-hydroxyazetidin-1-yl)-5,5-dimethyl-5,7-dihydro-6H-pyrrolo[2,3-d]pyrimidin-6-on

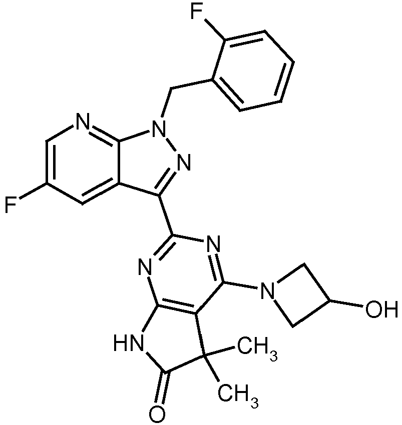

100 mg (0.18 mmol, ca. 95% Reinheit) 2-[5-Fluor-1-(2-fluorbenzyl)-1H-pyrazolo[3,4-b]pyridin-3-yl]-4-iod-5,5-dimethyl-5,7-dihydro-6H-pyrrolo[2,3-d]pyrimidin-6-on (Beispiel 16A) wurden in einem mikrowellengeeigneten Reaktionsgefäß in 1-Methyl-2-pyrrolidon (3.1 ml) gelöst, mit 0.19 ml (1.07 mmol) N,N-Diisopropylethylamin und mit 97 mg (0.89 mmol) Azetidin-3-ol-Hydrochlorid versetzt. Anschließend wurde das Reaktionsgefäß mit einem Septum verschlossen und für 3 h bei 150°C in der Mikrowelle erhitzt. Nach Abkühlen wurde das Reaktionsgemisch mit Wasser/Trifluoressigsäure versetzt und vom entstandenen Feststoff abfiltriert. Das Filtrat wurde mittels präparativer HPLC gereinigt (Acetonitril:Wasser (+ 0.1 % Trifluoressigsäure) - Gradient). Es wurden 19 mg der Titelverbindung erhalten (21 % d. Th.; Reinheit 93%).
LC-MS (Methode 1): Rₜ = 1.01 min; MS (EIpos): m/z = 478 [M+H]⁺.
¹H-NMR (400 MHz, DMSO-d₆): δ [ppm] = 1.34 (s, 6H), 4.03-4.09 (m, 2H), 4.50-4.57 (m, 2H), 4.62-4.69 (m, 1H), 5.83 (s, 2H), 7.15 (t, 1H), 7.19-7.26 (m, 2H), 7.33-7.40 (m, 1H), 8.53 (dd, 1H), 8.71-8.73 (m, 1H), 11.19 (s, 1H).

### Beispiel 105

### 2-[1-(2-Fluorbenzyl)-1H-pyrazolo[3,4-b]pyridin-3-yl]-5,5-dimethyl-4-[3-(pyrrolidin-1-yl)azetidin-1-yl]-5,7-dihydro-6H-pyrrolo[2,3-d]pyrimidin-6-on

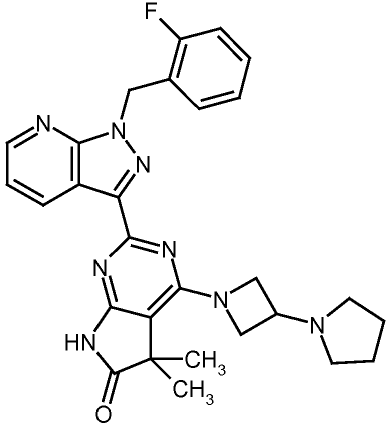

150 mg (0.21 mmol, Reinheit ca. 71%) 2-[1-(2-Fluorbenzyl)-1H-pyrazolo[3,4-b]pyridin-3-yl]-4-iod-5,5-dimethyl-5,7-dihydro-6H-pyrrolo[2,3-d]pyrimidin-6-on (Beispiel 15A) wurden in einem mikrowellengeeigneten Reaktionsgefäß in 1-Methyl-2-pyrrolidon (3.1 ml) gelöst, mit 0.29 ml (1.66 mmol) N,N-Diisopropylethylamin und mit 165 mg (0.83 mmol) 1-(Azetidin-3-yl)pyrrolidin-Dihydrochlorid versetzt. Anschließend wurde das Reaktionsgefäß mit einem Septum verschlossen und für 6 h bei 150°C in der Mikrowelle erhitzt. Nach Abkühlen wurde das Reaktionsgemisch mit Wasser versetzt. Es wurde 30 min verrührt und vom entstandenen Feststoff abfiltriert. Das Filtrat wurde mittels präparativer HPLC gereinigt (Acetonitril:Wasser (+ 0.1 % Trifluoressigsäure) - Gradient). Die Produktfraktion wurde in Dichlormethan gelöst und zweimal mit gesättigter, wässriger Natriumhydrogencarbonatlösung gewaschen. Die organische Phase wurde über Natriumsulfat getrocknet, filtriert und eingeengt. Es wurden 52 mg der Titelverbindung erhalten (46 % d. Th.; Reinheit 94%).
LC-MS (Methode 1): Rₜ = 0.71 min; MS (EIpos): m/z = 513 [M+H]⁺.
¹H-NMR (400 MHz, DMSO-d₆): δ [ppm] = 1.34 (s, 6H), 1.70-1.79 (m, 4H), 2.48-2.57 (m, verdeckt vom DMSO-Signal), 3.41-3.51 (m, 1H), 4.10-4.18 (m, 2H), 4.37-4.43 (m, 2H), 5.83 (s, 2H), 7.10-7.28 (m, 3H), 7.31-7.46 (m, 2H), 8.62-8.66 (m, 1H), 8.85-8.89 (m, 1H), 11.20 (s, 1H).

### Beispiel 106

### 4-(3-Aminopyrrolidin-1-yl)-2-[1-(2-fluorbenzyl)-1H-pyrazolo[3,4-b]pyridin-3-yl]-5,5-dimethyl-5,7-dihydro-6H-pyrrolo[2,3-d]pyrimidin-6-on (Racemat)

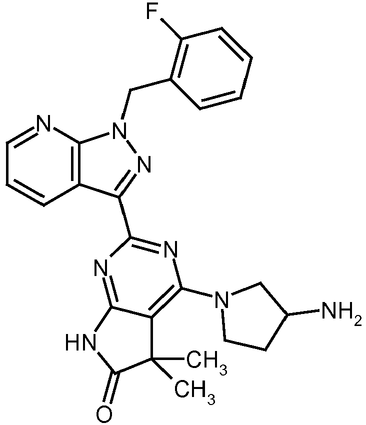

81 mg (0.24 mmol) tert.-Butyl-(1-{2-[1-(2-fluorbenzyl)-1H-pyrazolo[3,4-b]pyridin-3-yl]-5,5-dimethyl-6-oxo-6,7-dihydro-5H-pyrrolo[2,3-d]pyrimidin-4-yl}pyrrolidin-3-yl)carbamat (Racemat, Beispiel 61A) wurden mit 0.71 ml einer 2N Lösung von Chlorwasserstoff in Diethylether versetzt und für 4 h bei Raumtemperatur gerührt. Die Reaktionslösung wurde eingedampft und mittels päparativer HPLC gereinigt (Acetonitril/Wasser (+ 0.1 % Trifluoressigsäure) - Gradient). Die eingeengten Fraktionen wurden in Dichlormethan gelöst und zweimal mit gesättigter, wässriger Natriumhydrogencarbonatlösung gewaschen. Die vereinigten wässrigen Phasen wurden zweimal mit Dichlormethan extrahiert. Die vereinigten organischen Phasen wurden über Natriumsulfat getrocknet, filtriert und eingeengt. Es wurden 37 mg (55% d. Th.) der Zielverbindung erhalten.
LC-MS (Methode 1): Rₜ = 0.73 min; MS (ESIpos): m/z = 473 [M+H]⁺
¹H-NMR (400 MHz, DMSO-d₆): δ [ppm]= 1.42 (s, 6H), 1.70-1.79 (m, 1H), 2.06-2.16 (m, 1H), 3.39-3.45 (m, 1H), 3.58-3.63 (m, 1H), 3.73-3.82 (m, 1H), 3.83-3.94 (m, 2H), 5.83 (s, 2H), 7.10-7.26 (m, 3H), 7.31-7.39 (m, 1H), 7.42 (dd, 1H), 8.65 (dd, 1H), 8.88 (dd, 1H), 11.29 (br. s, 1H).

### Beispiel 107

### 4-(3-Aminoazetidin-1-yl)-2-[1-(2-fluorbenzyl)-1H-pyrazolo[3,4-b]pyridin-3-yl]-5,5-dimethyl-5,7-dihydro-6H-pyrrolo[2,3-d]pyrimidin-6-on

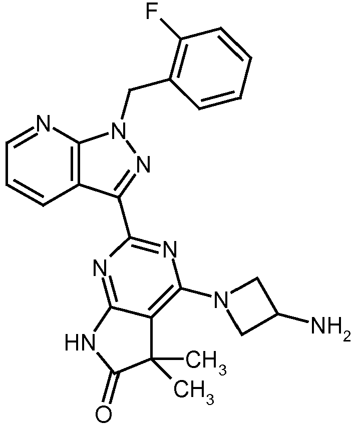

81 mg (0.13 mmol; Reinheit 91%) tert.-Butyl-(1-{2-[1-(2-fluorbenzyl)-1H-pyrazolo[3,4-b]pyridin-3-yl]-5,5-dimethyl-6-oxo-6,7-dihydro-5H-pyrrolo[2,3-d]pyrimidin-4-yl}-azetidin-3-yl)carbamat (Beispiel 62A) wurden mit 0.66 ml einer 2N Lösung von Chlorwasserstoff in Diethylether versetzt und für 4 h bei Raumtemperatur gerührt. Die Reaktionslösung wurde eingeengt und zweimal mittels päparativer HPLC gereinigt (Acetonitril/Wasser (+ 0.1 % Trifluoressigsäure) - Gradient). Die eingeengten Fraktionen wurden in Dichlormethan gelöst und zweimal mit gesättigter, wässriger Natriumhydrogencarbonatlösung gewaschen. Die vereinigten wässrigen Phasen wurden zweimal mit Dichlormethan extrahiert. Die vereinigten organischen Phasen wurden über Natriumsulfat getrocknet, filtriert und eingeengt. Es wurden 31 mg (49% d. Th.) der Zielverbindung erhalten.
LC-MS (Methode 1): Rₜ = 0.71 min; MS (ESIpos): m/z = 459 [M+H]⁺
¹H-NMR (400 MHz, DMSO-d₆): δ [ppm]= 1.33 (s, 6H), 2.22 (br. s, 2H), 3.83-3.98 (m, 3H), 4.46 (t, 2H), 5.82 (s, 2H), 7.10-7.27 (m, 3H), 7.31-7.38 (m, 1H), 7.42 (dd, 1H), 8.65 (dd, 1H), 8.88 (dd, 1H), 11.18 (s, 1H).

### Beispiel 108

### 2-[1-(2-Fluorbenzyl)-6-methyl-1H-pyrazolo[3,4-d]pyrimidin-3-yl]-5,5-dimethyl-4-[(3,3,3-trifluoropropyl)amino]-5,7-dihydro-6H-pyrrolo[2,3-d]pyrimidin-6-on

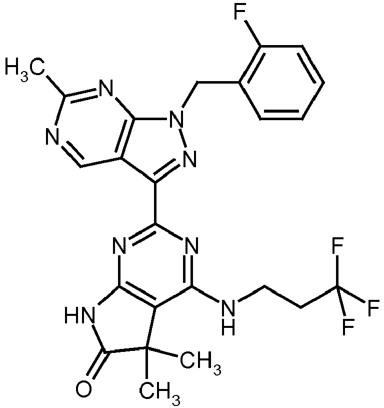

Die Titelverbindung wurde analog der Vorschrift für Beispiel 35 ausgehend von 100 mg (0.16 mmol) Beispiel 70A und 372 mg (3.29 mmol) 3,3,3-Trifluor-propylamin hergestellt. Ausb.: 38 mg (44% d. Th.)
LC-MS (Methode 1): Rₜ = 1.11 min; MS (ESIpos): m/z= 515 [M+H]⁺
¹H-NMR (400MHz, DMSO-d₆): δ [ppm]= 1.37 (s, 6H), 2.59 - 2.74 (m, 2H), 2.78 (s, 3H), 3.83 (q, 2H), 5.75 (s, 2H), 6.89 (t, 1H), 7.10 - 7.29 (m, 3H), 7.32 - 7.43 (m, 1H), 9.65 (s, 1H), 11.11 (s, 1H).

### Beispiel 109

### 2-[1-(2-Fluorbenzyl)-1H-pyrazolo[3,4-b]pyridin-3-yl]-4-hydroxy-5,5-dimethyl-5,7-dihydro-6H-pyrrolo[2,3-d]pyrimidin-6-on

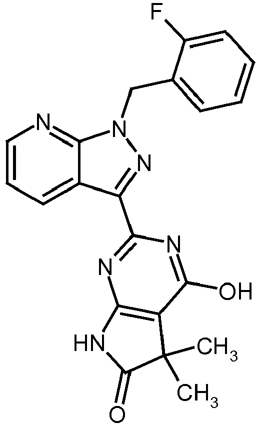

6.0 g (14.87 mmol) Beispiel 13A wurden in 60 ml Trifluoressigsäure gelöst (Erwärmung) und im Eisbad gekühlt. Unter Rühren wurden 6.7 ml Wasser und dann in kleinen Portionen innerhalb 1h 1.54 g (22.3 mmol) Natriumnitrit zugegeben. Dann wurde das Reaktionsgemisch in 250 ml Wasser gegossen und der ausgefallene Niederschlag abgesaugt. Der Feststoff wurde in 50 ml Wasser (mit konz. Natriumhydrogencarbonat-Lösung auf pH 6 eingestellt) ausgerührt, wiederum abgesaugt, mit Wasser gewaschen und getrocknet. Ausb.: 5.75 g (94% d.Th.)
LC-MS (Methode 1): Rₜ = 0.92 min; MS (ESIpos): m/z = 405 [M+H]⁺
¹H-NMR (400MHz, DMSO-d₆): δ [ppm]= 1.33 (s, 6H), 5.86 (s, 2H), 7.16 (t, 1H), 7.23 (t, 1H), 7.28 - 7.41 (m, 2H), 7.49 (dd, 1H), 8.61 - 8.85 (m, 2H), 11.11 (s, 1H), 12.12 (br. s, 0.2H), 12.44 (br. s, 0.8H).

### Beispiel 110

### 2-[1-(2,3-Difluorbenzyl)-5-fluoro-1H-pyrazolo[3,4-b]pyridin-3-yl]-5,5-dimethyl-4-(3,3,3-trifluoropropoxy)-5,7-dihydro-6H-pyrrolo[2,3-d]pyrimidin-6-on

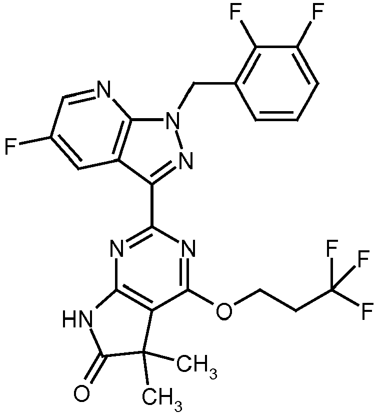

Unter Argon wurden 80 mg (0.18 mmol) der Verbindung aus Beispiel 116 in 2 ml THF und 0.4 ml DMF gelöst, mit 31 mg (0.27 mmol) 3,3,3-Trifluorpropan-1-ol, 71.5 mg (0.27 mmol) Triphenylphosphin und 57 µl (0.27 mmol) 94%-igem Diisopropylazodicarboxylat versetzt und über Nacht bei RT gerührt. Es wurden weitere 71.5 mg (0.27 mmol) Triphenylphosphin zugesetzt und 15 min im Ultraschallbad behandelt, dann weitere 57.5 µl (0.27 mmol) 94%-iges Diisopropylazodicarboxylat zugetropft und über Nacht bei RT gerührt. Danach wurden 31 mg (0.27 mmol) 3,3,3-Trifluorpropan-1-ol und 71.5 mg (0.27 mmol) Triphenylphosphin zugegeben, 15 min im Ultraschallbad behandelt, 57.5 µl (0.27 mmol) 94%-iges Diisopropylazodicarboxylat zugegeben und über Nacht bei RT gerührt. Die Reaktionsmischung wurde per präparativer HPLC (Chromatorex C18, Gradient aus Acetonitril/0.01% aq. Ameisensäure) gereinigt. Ausb.: 41 mg (42% d. Th.)
LC-MS (Methode 1): Rₜ = 1.46 min; MS (ESIpos): m/z = 633 [M+H]⁺
¹H-NMR (400MHz, DMSO-d₆): δ [ppm]= 1.35 (s, 6H), 2.93 (qt, 2H), 4.79 (t, 2H), 5.92 (s, 2H), 7.01 - 7.10 (m, 1H), 7.18 (t, 1H), 7.34 - 7.46 (m, 1H), 8.55 (dd, 1H), 8.76 (dd, 1H), 11.45 (s, 1H).

### Beispiel 111

### 2-[1-(2-Fluorbenzyl)-1H-pyrazolo[3,4-b]pyridin-3-yl]-5,5-dimethyl-4-propoxy-5,7-dihydro-6H-pyrrolo[2,3-d]pyrimidin-6-on

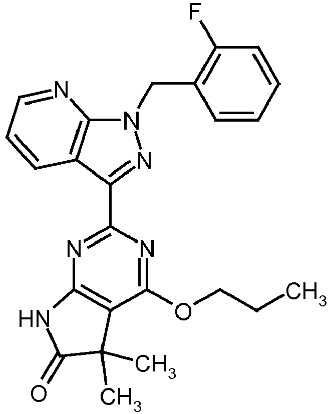

Unter Argon wurden 80 mg (0.20 mmol) der Verbindung aus Beispiel 109, 13 mg (16 µl, 0.22 mmol) n-Propanol und 57 mg (0.22 mmol) Triphenylphosphin in 0.8 ml THF suspendiert, 10 min im Ultraschallbad gemischt und schließlich mit 44 mg (43µl, 0.22 mmol) Diisopropylazodicarboxylat versetzt und 1h bei RT gerührt. Die Reaktionsmischung wurde mittels präparativer HPLC (Reprosil C18, Gradient aus Acetonitril/0.01% aq. Ameisensäure) gereinigt. Ausb.: 48 mg (55% d. Th.)
LC-MS (Methode 1): Rₜ = 1.28 min; MS (ESIpos): m/z = 447 [M+H]⁺
¹H-NMR (400MHz, DMSO-d₆): δ [ppm]= 1.05 (t, 3H), 1.36 (s, 6H), 1.77 - 1.91 (m, 2H), 4.54 (t, 2H), 5.86 (s, 2H), 7.11 - 7.27 (m, 3H), 7.32 - 7.40 (m, 1H), 7.45 (dd, 1H), 8.68 (dd, 1H), 8.85 (dd, 1H), 11.39 (s, 1H).

### Beispiel 112

### 4-Ethoxy-2-[1-(2-fluorbenzyl)-1H-pyrazolo[3,4-b]pyridin-3-yl]-5,5-dimethyl-5,7-dihydro-6H-pyrrolo[2,3-d]pyrimidin-6-on

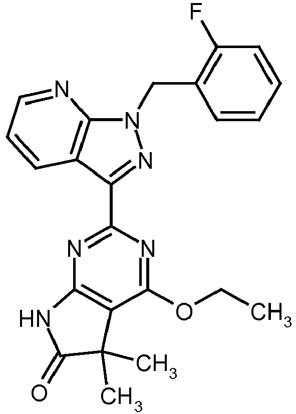

Analog der Vorschrift für Beispiel 111 wurden 150 mg (0.37 mmol) Beispiel 109, 24 µl (0.41 mmol) Ethanol und 107 mg (0.41 mmol) Triphenylphosphin wurden in 1.5 ml THF 10 min im Ultraschallbad gemischt, mit 82.5 mg (0.41 mmol) Diisopropylazodicarboxylat versetzt und über nacht bei RT gerührt. Dann wurden weitere 24 µl (0.41 mmol) Ethanol und 107 mg (0.41 mmol) Triphenylphosphin zugegeben, 5 min im Ultraschallbad gemischt mit 82.5 mg (0.41 mmol) Diisopropylazodicarboxylat versetzt und ca. 30 min bei RT gerührt. Die Reaktionsmischung wurde mittels präparativer HPLC (Reprosil C18, Gradient aus Acetonitril/0.01% aq. Ameisensäure) gereinigt. Ausb.: 25 mg (16% d. Th.).
LC-MS (Methode 1): Rₜ = 1.21 min; MS (ESIpos): m/z = 433 [M+H]⁺
¹H-NMR (400MHz, DMSO-d₆): δ [ppm]= 1.35 (s, 6H), 1.43 (t, 3H), 4.62 (q, 2H), 5.86 (s, 2H), 7.09 - 7.27 (m, 3H), 7.31 - 7.40 (m, 1H), 7.45 (dd, 1H), 8.68 (dd, 1H), 8.85 (dd, 1H), 11.39 (s, 1H).

### Beispiel 113

### 4-(Cyclopropylmethoxy)-2-[1-(2-fluorbenzyl)-1H-pyrazolo[3,4-b]pyridin-3-yl]-5,5-dimethyl-5,7-dihydro-6H-pyrrolo[2,3-d]pyrimidin-6-on

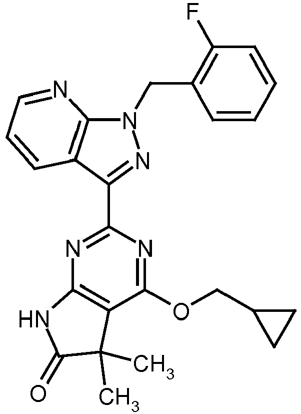

Analog der Vorschrift für Beispiel 111 wurden 200 mg (0.50 mmol) Beispiel 109, 39 mg (0.54 mmol) Cyclopropanmethanol und 143 mg (0.54 mmol) Triphenylphosphin wurden in 2 ml THF 10 min im Ultraschallbad gemischt, mit 110 mg (0.11 ml, 0.54 mmol) Diisopropylazodicarboxylat versetzt und über Nacht bei RT gerührt. Dann wurden weitere 14 mg (0.19 mmol) Cyclopropanmethanol, 48 mg (0.18 mmol) Triphenylphosphin und 35ml (0.17 mmol) Diisopropylazodicarboxylat zugegebent und 1,5 h bei RT gerührt. Die Reaktionsmischung wurde mittels präparativer HPLC (Chromatorex C18, Gradient aus Acetonitril/0.01% aq. Ameisensäure) gereinigt.. Ausb.: 52 mg (23% d. Th.)
LC-MS (Methode 1): Rₜ = 1.27 min; MS (ESIpos): m/z = 459 [M+H]⁺
¹H-NMR (400MHz, DMSO-d₆): δ [ppm]= 0.40 - 0.48 (m, 2H), 0.55 - 0.64 (m, 2H), 1.27 - 1.43 (m, 7H), 4.44 (d, 2H), 5.86 (s, 2H), 7.09 - 7.28 (m, 3H), 7.31 - 7.41 (m, 1H), 7.47 (dd, 1H), 8.68 (dd, 1H), 8.84 (dd, 1H), 11.41 (s, 1H).

### Beispiel 114

### 2-[3-(2-Fluorbenzyl)-1H-pyrazolo[4,3-b]pyridin-1-yl]-5,5-dimethyl-4-(3,3,3-trifluorpropoxy)-5,7-dihydro-6H-pyrrolo[2,3-d]pyrimidin-6-on

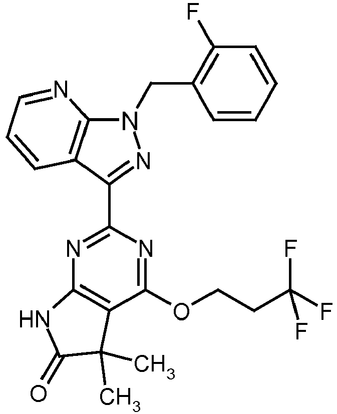

Unter Argon wurden 57 mg (0.22 mmol) Triphenylphosphin in 1.5 ml THF gelöst, mit 43 µl (0.22 mmol) Diisopropylazodicarboxylat und 25 mg (0.22 mmol) 3,3,3-Trifluorpropan-1-ol versetzt und 10 min gerührt. Dann wurde eine Suspension von 80 mg Beispiel 109 in 0.5 ml DMF, die 3 min im Ultraschallbad behandelt wurde, zugegeben und das reaktionsgemisch über Nacht bei RT gerührt (Lösung). Es wurden weitere 57 mg (0.22 mmol) Triphenylphosphin zugegeben, das Gemisch 10 min im Ultraschallbad behandelt, dann weitere 43 µl (0.22 mmol) Diisopropylazodicarboxylat zugegeben und über Nacht gerührt. Anschliessend wurden weitere 25 mg (0.22 mmol) 3,3,3-Trifluorpropan-1-ol, 57 mg (0.22 mmol) Triphenylphosphin zugegeben, das Reaktionsgemisch 10 min im Ultraschallbad behandelt, dann mit 43 µl (0.22 mmol) Diisopropylazodicarboxylat versetzt und eine weitere Nacht bei RT gerührt. Die Reaktionsmischung wurde mittels präparativer HPLC (Reprosil C18, Gradient aus Acetonitril/0.01% aq. Ameisensäure) gereinigt. Ausb.: 37 mg (37% d. Th.)
LC-MS (Methode 1): Rₜ = 1.21 min; MS (ESIpos): m/z = 501 [M+H]⁺
¹H-NMR (400MHz, DMSO-d₆): δ [ppm]= 1.35 (s, 6H), 2.92 (qt, 2H), 4.79 (t, 2H), 5.87 (s, 2H), 7.11 - 7.27 (m, 3H), 7.32 - 7.41 (m, 1H), 7.45 (dd, 1H), 8.68 (dd, 1H), 8.86 (dd, 1H), 11.45 (s, 1H).

### Beispiel 115

### 2-[1-(2-Fluorbenzyl)-1H-pyrazolo[3,4-b]pyridin-3-yl]-5,5-dimethyl-4-(2,2,2-trifluorethoxy)-5,7-dihydro-6H-pyrrolo[2,3-d]pyrimidin-6-on

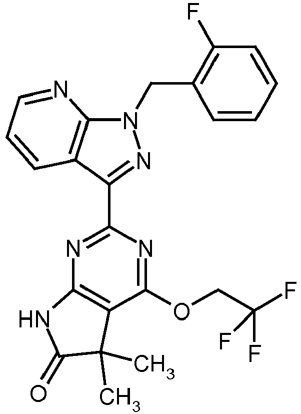

Zu einer Suspension von 200 mg (0.5 mmol) der Verbindung aus Beispiel 109 in DMF (1.97 ml) wurden 177 mg (0.54 mmol) Cäsiumcarbonat und 114 mg (0.54 mmol) 2,2,2-Trifluorethyliodid gegeben und über Nacht bei RT gerührt. Dann wurde in der Mikrowelle 1h auf 120°C erhitzt. Die Reaktionsmischung wurde mittels präparativer HPLC (Chromatorex C18, Gradient aus Acetonitril/0.01% aq. Ameisensäure) gereinigt. Ausb.: 14.5 mg (6% d. Th.).
LC-MS (Methode 1): Rₜ = 1.20 min; MS (ESIpos): m/z = 487 [M+H]⁺
¹H-NMR (400MHz, DMSO-d₆): δ [ppm]= 1.36 (s, 6H), 5.30 (q, 2H), 5.88 (s, 2H), 7.10 - 7.28 (m, 3H), 7.32 - 7.41 (m, 1H), 7.48 (dd, 1H), 8.69 (dd, 1H), 8.89 (dd, 1H), 11.61 (s, 1H).

### Beispiel 116

### 2-[1-(2,3-Difluorbenzyl)-5-fluor-1H-pyrazolo[3,4-b]pyridin-3-yl]-4-hydroxy-5,5-dimethyl-5,7-dihydro-6H-pyrrolo[2,3-d]pyrimidin-6-on

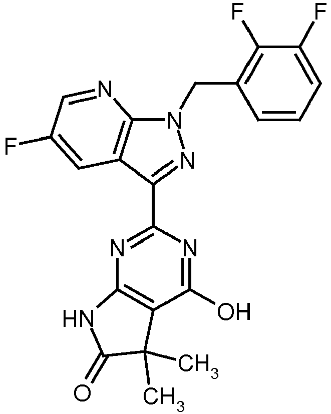

In Analogie zur Darstellung von Beispiel 16A wurden 5.11 g (11.629 mmol) Beispiel 69A umgesetzt. Es wurden 660 mg (12 % d. Th.) der Titelverbindung sowie 2.39 g von 2-[1-(2,3-Difluorbenzyl)-5-fluor-1H-pyrazolo[3,4-b]pyridin-3-yl]-4-iod-5,5-dimethyl-5,7-dihydro-6H-pyrrolo[2,3-d]pyrimidin-6-on (Beispiel 70A) erhalten.
LC-MS (Methode 1): Rₜ = 0.98 min; MS (ESIpos): m/z = 441 [M+H]⁺
¹H-NMR (400MHz, DMSO-d₆): δ [ppm]= 1.33 (s, 6H), 5.89 (s, 2H), 7.15 - 7.20 (m, 2H), 7.37 - 7.45 (m, 1H), 8.58 (br s, 1H), 8.79 (s, 1H), 11.08 (br s, 1H), 12.58 (br s, 1H).

### Beispiel 117

### 2-[1-(2-Fluorbenzyl)-1H-pyrazolo[3,4-b]pyridin-3-yl]-4-(2-hydroxyethoxy)-5,5-dimethyl-5,7-dihydro-6H-pyrrolo[2,3-d]pyrimidin-6-on

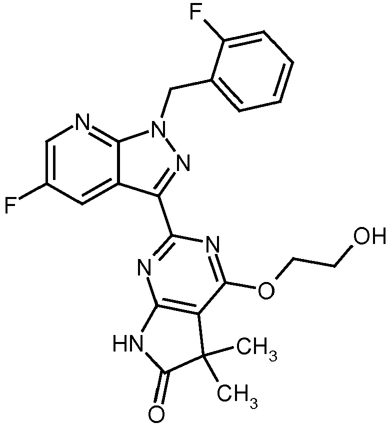

Analog zu Beispiel 1 wurden 100 mg (0.19 mmol) Beispiel 15A, 121 mg (1.94 mmol) Ethylenglycol, 9 mg (0.039 mmol) 3,4,7,8-Tetramethyl-1,10-phenanthrolin, 3.7 mg (0.019 mmol) Kupfer-(I)-iodid und 126 mg (0.39 mmol) Cäsiumcarbonat in 3 ml Toluol in 4 Zyklen für jeweils 2 h bei 140 °C in der Mikrowelle erhitzt. Ausb.: 24 mg (28% d. Th.)
LC-MS (Methode 1): Rₜ = 0.94 min; MS (EIpos): m/z = 449 [M+H]⁺.
¹H-NMR (400MHz, DMSO-d₆): δ [ppm] = 1.37 (s, 6H), 3.82 (q, 2H), 4.58 (t, 2H), 4.94 (t, 1H), 5.86 (s, 2H), 7.10 - 7.27 (m, 3H), 7.32 - 7.40 (m, 1H), 7.45 (dd, 1H), 8.68 (dd, 1H), 8.86 (dd, 1H), 11.41 (s, 1H).

### B. Bewertung der pharmakologischen Wirksamkeit

Im Folgenden werden die nachstehenden Abkürzungen verwendet:
- BSA: Bovines Serumalbumin
- EDTA: Ethylendiamintetraessigsäure
- µCi: Micro Curie
- Tris: Tris(hydroxymethyl)-aminomethan

Die pharmakologische Wirkung der erfindungsgemäßen Verbindungen kann in folgenden Assays gezeigt werden:

### B-1. Gefäßrelaxierende Wirkung in vitro

Kaninchen werden durch Nackenschlag betäubt und entblutet. Die Aorta wird entnommen, von anhaftendem Gewebe befreit, in 1.5 mm breite Ringe geteilt und einzeln unter einer Vorspannung in 5 ml-Organbäder mit 37°C warmer, Carbogen-begaster Krebs-Henseleit-Lösung folgender Zusammensetzung gebracht (jeweils mM): Natriumchlorid: 119; Kaliumchlorid: 4.8; Calciumchlorid-Dihydrat: 1; Magnesiumsulfat-Heptahydrat: 1.4; Kaliumdihydrogenphosphat: 1.2; Natriumhydrogencarbonat: 25; Glucose: 10. Die Kontraktionskraft wird mit Statham UC2-Zellen erfasst, verstärkt und über A/D-Wandler (DAS-1802 HC, Keithley Instruments München) digitalisiert sowie parallel auf Linienschreiber registriert. Zur Erzeugung einer Kontraktion wird Phenylephrin dem Bad kumulativ in ansteigender Konzentration zugesetzt. Nach mehreren Kontrollzyklen wird die zu untersuchende Substanz in jedem weiteren Durchgang in jeweils steigender Dosierung zugesetzt und die Höhe der Kontraktion mit der Höhe der im letzten Vordurchgang erreichten Kontraktion verglichen. Daraus wird die Konzentration errechnet, die erforderlich ist, um die Höhe des Kontrollwertes um 50% zu reduzieren (IC₅₀-Wert). Das Standardapplikationsvolumen beträgt 5 µl, der DMSO-Anteil in der Badlösung entspricht 0.1%.

Repräsentative IC₅₀-Werte für die erfindungsgemäßen Verbindungen sind in der nachstehenden Tabelle (Tabelle 1) wiedergegeben:

**Tabelle 1:**

| Beispiel Nr. | IC₅₀ [nM] | Beispiel Nr. | IC₅₀ [nM] |
|---|---|---|---|
| 1 | 114 | 32 | 9160 |
| 2 | 54 | 33 | 696 |
| 3 | 469 | 34 | 6150 |
| 4 | 62 | 35 | >10000 |
| 5 | 285 | 36 | 5490 |
| 6 | 680 | 37 | 3870 |
| 7 | 200 | 38 | 10000 |
| 8 | 309 | 39 | 618 |
| 9 | 21000 | 88 | 1670 |
| 10 | 552 | 89 | 3880 |
| 11 | 663 | 90 | 3072 |
| 12 | 631 | 91 | 517 |
| 13 | 4970 | 92 | 520 |
| 14 | 1030 | 93 | 984 |
| 15 | 453 | 94 | 277 |
| 16 | 126 | 95 | 228 |
| 17 | 200 | 96-1 | 1468 |
| 18 | 2720 | 96-2 | 601 |
| 19 | 1280 | 97 | 1350 |
| 20 | 1870 | 98 | 214 |
| 21 | 100 | 99 | 10000 |
| 22 | 530 | 100 | 4758 |
| 23 | 2864 | 102 | 156 |
| 24 | 573 | 103 | 551 |
| 25 | 501 | 104 | 482 |
| 26 | 674 | 105 | 478 |
| 27 | 1470 | 106 | 1454 |
| 28 | 98 | 107 | 700 |
| 29 | 11400 | 108 | 444 |
| 30 | >10000 | 109 | 81 |
| 31 | 142 | 110 | 10000 |
| | | 111 | 1620 |
| | | 112 | 1620 |
| | | 113 | 1960 |
| | | 114 | 1600 |
| | | 115 | 1634 |
| | | 117 | 38 |
| | | | |
| | | | |

### B-2. Wirkung an rekombinanter Guanylatcyclase-Reporterzelllinie

Die zelluläre Wirkung der erfindungsgemäßen Verbindungen wird an einer rekombinanten Guanylatcyclase-Reporterzelllinie, wie in F. Wunder et al., Anal. Biochem. 339, 104-112 (2005) beschrieben, bestimmt.

Repräsentative Werte (MEC = minimal effektive Konzentration) für die erfindungsgemäßen Verbindungen sind in der nachstehenden Tabelle (Tabelle 2) wiedergegeben:

**Tabelle 2:**

| **Beispiel** | **MEC [µM]** | **Beispiel** | **MEC [µM]** | **Beispiel** | **MEC [µM]** |
|---|---|---|---|---|---|
| 1 | 0.1 | 41 | 0.01 | 81 | 0.03 |
| 2 | 0.3 | 42 | 0.1 | 82 | 0.1 |
| 3 | 3.0 | 43 | 0.03 | 83 | 0.03 |
| 4 | 0.1 | 44 | 0.1 | 84 | 0.1 |
| 5 | 0.03 | 45 | 0.1 | 85 | 0.3 |
| 6 | 0.1 | 46 | 0.1 | 86 | 0.01 |
| 7 | 0.1 | 47 | 0.1 | 87 | 0.03 |
| 8 | 0.3 | 48 | 0.1 | 88 | 0.1 |
| 9 | 0.1 | 49 | 0.3 | 89 | 0.03 |
| 10 | 0.3 | 50 | 0.1 | 90 | 0.3 |
| 11 | 0.3 | 51 | 0.03 | 91 | 0.03 |
| 12 | 0.1 | 52 | 0.01 | 92 | 0.3 |
| 13 | 0.1 | 53 | 3.0 | 93 | 0.3 |
| 14 | 0.3 | 54 | 0.03 | 94 | 0.1 |
| 15 | 1.0 | 55 | 0.03 | 95 | 0.1 |
| 16 | 0.1 | 56 | 0.03 | 96-1 | 0.1 |
| 17 | 0.1 | 57 | 0.1 | 96-2 | 0.01 |
| 18 | 0.03 | 58 | 0.03 | 97 | 0.1 |
| 19 | 0.3 | 59 | 0.1 | 98 | 0.01 |
| 20 | 0.3 | 60 | 0.3 | 99 | 0.3 |
| 21 | 0.03 | 61 | 0.03 | 100 | 0.3 |
| 22 | 0.03 | 62 | 0.1 | 101 | 0.03 |
| 23 | 0.1 | 63 | 0.03 | 102 | 1 |
| 24 | 0.1 | 64 | 0.1 | 103 | 1 |
| 25 | 0.1 | 65 | 0.1 | 104 | 0.1 |
| 26 | 0.03 | 66 | 0.1 | 105 | 0.3 |
| 27 | 0.1 | 67 | 0.03 | 106 | 1 |
| 28 | 0.3 | 68 | 0.1 | 107 | 1 |
| 29 | 1.0 | 69 | 0.3 | 108 | 0.3 |
| 30 | 1.0 | 70 | 0.03 | 109 | 0.03 |
| 31 | 0.01 | 71 | 0.01 | 110 | 1.0 |
| 32 | 1.0 | 72 | 0.1 | 111 | 0.3 |
| 33 | 0.3 | 73 | 0.3 | 112 | 0.1 |
| 34 | 1.0 | 74 | 0.01 | 113 | 0.1 |
| 35 | 0.3 | 75 | 0.03 | 114 | 0.3 |
| 36 | 0.3 | 76 | 0.03 | 115 | 0.3 |
| 37 | 0.3 | 77 | 0.1 | 116 | 0.03 |
| 38 | 0.3 | 78 | 0.03 | 117 | 0.03 |
| 39 | 0.1 | 79 | 0.1 | | |
| 40 | 0.1 | 80 | 0.03 | | |
| | | | | | |

### B-3. Radiotelemetrische Blutdruckmessung an wachen, spontan hypertensiven Ratten

Für die im Folgenden beschriebene Blutdruckmessung an wachen Ratten wird ein im Handel erhältliches Telemetriesystem der Firma DATA SCIENCES INTERNATIONAL DSI, USA eingesetzt.

Das System besteht aus 3 Hauptkomponenten:
- Implantierbare Sender (Physiotel® Telemetrietransmitter)
- Empfänger (Physiotel® Receiver), die über einen Multiplexer (DSI Data Exchange Matrix) mit einem
- Datenakquisitionscomputer
verbunden sind.

Die Telemetrieanlage ermöglicht eine kontinuierliche Erfassung von Blutdruck Herzfrequenz und Körperbewegung an wachen Tieren in ihrem gewohnten Lebensraum.

### Tiermaterial

Die Untersuchungen werden an ausgewachsenen weiblichen spontan hypertensiven Ratten (SHR Okamoto) mit einem Körpergewicht von >200 g durchgeführt. SHR/NCrl von Okamoto Kyoto School of Medicine, 1963 wurden aus männlichen Wistar Kyoto Ratten mit stark erhöhtem Blutdruck und weiblichen mit leicht erhöhtem Blutdruck gekreuzt und in der F13 an die U.S. National Institutes of Health abgegeben.

Die Versuchstiere werden nach Senderimplantation einzeln in Makrolon - Käfigen Typ 3 gehalten. Sie haben freien Zugang zu Standardfutter und Wasser.

Der Tag - Nacht - Rhythmus im Versuchslabor wird per Raumbeleuchtung um 6:00 Uhr morgens und um 19:00 Uhr abends gewechselt.

### Senderimplantation

Die eingesetzten Telemetriesender TA11 PA - C40 werden den Versuchstieren mindestens 14 Tage vor dem ersten Versuchseinsatz unter aseptischen Bedingungen chirurgisch implantiert. Die so instrumentierten Tiere sind nach Abheilen der Wunde und Einwachsen des Implantats wiederholt einsetzbar.

Zur Implantation werden die nüchternen Tiere mit Pentobabital (Nembutal, Sanofi: 50mg/kg i.p.) narkotisiert und an der Bauchseite weiträumig rasiert und desinfiziert. Nach Eröffnung des Bauchraumes entlang der Linea alba wird der flüssigkeitsgefüllte Meßkatheter des Systems oberhalb der Bifurcation nach cranial in die Aorta descendens eingesetzt und mit Gewebekleber (VetBonD TM, 3M) befestigt. Das Sendergehäuse wird intraperitoneal an der Bauchwandmuskulatur fixiert und die Wunde wird schichtweise verschlossen.

Postoperativ wird zur Infektionsprophylaxe ein Antibiotikum verabreicht (Tardomyocel COMP Bayer 1ml/kg s.c.)

### Substanzen und Lösungen

Wenn nicht anders beschrieben werden die zu untersuchenden Substanzen jeweils einer Gruppe von Tieren (n = 6) per Schlundsonde oral verabreicht. Entsprechend einem Applikationsvolumen von 5 ml/kg Körpergewicht werden die Testsubstanzen in geeigneten Lösungsmittelgemischen gelöst oder in 0.5%-iger Tylose suspendiert.

Eine Lösungsmittel- behandelte Gruppe von Tieren wird als Kontrolle eingesetzt.

### Versuchsablauf

Die vorhandene Telemetrie - Meßeinrichtung ist für 24 Tiere konfiguriert. Jeder Versuch wird unter einer Versuchsnummer registiert (VJahr Monat Tag).

Den in der Anlage lebenden instrumentierten Ratten ist jeweils eine eigene Empfangsantenne zugeordnet (1010 Receiver, DSI).

Die implantierten Sender sind über einen eingebauten Magnetschalter von außen aktivierbar. Sie werden bei Versuchsvorlauf auf Sendung geschaltet. Die ausgestrahlten Signale können durch ein Datenakquisitionssystem (Dataquest TM A.R.T. for WINDOWS, DSI) online erfasst und entsprechend aufgearbeitet werden. Die Ablage der Daten erfolgt jeweils in einem hierfür eröffneten Ordner der die Versuchsnummer trägt.

Im Standardablauf werden über je 10 Sekunden Dauer gemessen:
- Systolischer Blutdruck (SBP)
- Diastolischer Blutdruck (DBP)
- Arterieller Mitteldruck (MAP)
- Herzfrequenz (HR)
- Aktivität (ACT).

Die Messwerterfassung wird rechnergesteuert in 5 Minuten Abständen wiederholt. Die als Absolutwert erhobenen Quelldaten werden im Diagramm mit dem aktuell gemessenen Barometerdruck (Ambient Pressure Reference Monitor; APR-1) korrigiert und in Einzeldaten abgelegt. Weitere technische Details sind der umfangreichen Dokumentation der Herstellerfirma (DSI) zu entnehmen.

Wenn nicht anders beschrieben erfolgt die Verabreichung der Prüfsubstanzen am Versuchstag um 9.00 Uhr. Im Anschluss an die Applikation werden die oben beschriebenen Parameter 24 Stunden gemessen.

### Auswertung

Nach Versuchsende werden die erhobenen Einzeldaten mit der Analysis-Software (DATAQUEST TM A. R.T. TM ANALYSIS) sortiert. Als Leerwert werden hier 2 Stunden vor Applikation angenommen, so dass der selektierte Datensatz den Zeitraum von 7:00 Uhr am Versuchstag bis 9:00 Uhr am Folgetag umfasst.

Die Daten werden über eine voreinstellbare Zeit durch Mittelwertbestimmung geglättet (15 Minuten Average) und als Textdatei auf einen Datenträger übertragen. Die so vorsortierten und komprimierten Messwerte werden in Excel-Vorlagen übertragen und tabellarisch dargestellt. Die Ablage der erhobenen Daten erfolgt pro Versuchstag in einem eigenen Ordner, der die Versuchsnummer trägt. Ergebnisse und Versuchsprotokolle werden in Papierform nach Nummern sortiert in Ordnern abgelegt.

### Literatur

Klaus Witte, Kai Hu, Johanna Swiatek, Claudia Müssig, Georg Ertl and Björn Lemmer: Experimental heart failure in rats: effects on cardiovascular circadian rhythms and on myocardial β-adrenergic signaling. Cardiovasc Res 47 (2): 203-405, 2000; Kozo Okamoto: Spontaneous hypertension in rats. Int Rev Exp Pathol 7: 227- 270, 1969; Maarten van den Buuse: Circadian Rhythms of Blood Pressure, Heart Rate, and Locomotor Activity in Spontaneously Hypertensive Rats as Measured With Radio-Telemetry. Physiology & Behavior 55(4): 783-787, 1994

### B-4. Bestimmung pharmakokinetischer Kenngrößen nach intravenöser und oraler Gabe

Die pharmakokinetischen Parameter der erfindungsgemäßen Verbindungen werden in männlichen CD-1-Mäusen, männlichen Wister-Ratten und weiblichen Beagle-Hunden bestimmt. Die intravenöse Gabe erfolgt bei Mäusen und Ratten mittels einer speziesspezifischen Plasma/DMSO-Formulierung und bei Hunden mittels einer Wasser/PEG400/Ethanol-Formulierung. Die orale Gabe der gelösten Substanz mittels Schlundsonde wird in allen Spezies basierend auf einer Wasser/PEG400/Ethanol-Formulierung durchgeführt. Den Ratten wird zur vereinfachten Blutabnahme vor der Substanzgabe ein Silikonkatheter in die rechte *Vena jugularis externa* gelegt. Die Operation erfolgt mindestens einen Tag vor dem Versuch unter Isofluran-Narkose und unter Gabe eines Analgetikums (Atropin/Rimadyl (3/1) 0.1 mL s.c.). Die Blutabnahme (in der Regel mehr als 10 Zeitpunkte) erfolgt in einem Zeitfenster, welches terminale Zeitpunkte von mindestens 24 bis maximal 72 Stunden nach Substanzgabe beinhaltet. Das Blut wird bei der Entnahme in heparinisierte Röhrchen geleitet. So dann wird mittels Zentrifugation das Blutplasma gewonnen und gegebenenfalls bis zur weiteren Bearbeitung bei -20°C gelagert.

Den Proben der erfindungsgemäßen Verbindungen, Kalibrierproben und Qualifier wird ein interner Standard zugesetzt (dies kann auch eine chemisch nicht verwandte Substanz sein) und es folgt eine Proteinfällung mittels Acetonitril im Überschuss. Nach Zugabe einer Puffer-Lösung, die an die LC-Bedingungen angepasst ist, und folgendem Vortexen wird bei 1000 g zentrifugiert. Der Überstand wird mittels LC-MS/MS unter Verwendung von C18-reversed-phase-Säulen und variablen Eluenten-Gemischen vermessen. Die Quantifizierung der Substanzen erfolgt anhand der Peakhöhen oder -flächen aus extrahierten Ionenchromatogrammen spezifischer selected ion monitoring-Experimente.

Aus den ermittelten Plasmakonzentration-Zeit-Verläufen werden die pharmakokinetischen Kenngrößen wie AUC, Cₘₐₓ, t_{1/2} (terminale Halbwertszeit), MRT (Mean Residence Time) und CL (Clearance) mittels eines validierten pharmakokinetischen Rechenprogramms berechnet.

Da die Substanzquantifizierung in Plasma durchgeführt wird, muss die Blut/Plasma-Verteilung der Substanz bestimmt werden, um die pharmakokinetischen Parameter entsprechend anpassen zu können. Dazu wird eine definierte Menge Substanz in heparinisiertem Vollblut der entsprechenden Spezies für 20 min im Taumelrollenmischer inkubiert. Nach Zentrifugation bei 1000g wird die Konzentration im Plasma gemessen (mittels LC-MS/MS; s.o.) und durch Quotientenbildung der C_{Blut}/C_{Plasma}-Wert ermittelt.

### B-5. Metabolismus-Untersuchung

Zur Bestimmung des Metabolismus-Profils der erfindungsgemäßen Verbindungen werden diese mit rekombinanten humanen Cytochrom P450 (CYP) Enzymen, Lebermikrosomen oder mit primären frischen Hepatozyten verschiedener Tierspezies (z.B. Ratte, Hund) als auch humanen Ursprungs inkubiert, um Informationen über einen möglichst kompletten hepatischen Phase I- und Phase II-Metabolismus sowie über die am Metabolismus beteiligten Enzyme zu erhalten und zu vergleichen.

Die erfindungsgemäßen Verbindungen wurden mit einer Konzentration von etwa 0.1-10 µM inkubiert. Dazu wurden Stammlösungen der erfindungsgemäßen Verbindungen mit einer Konzentration von 0.01-1 mM in Acetonitril hergestellt, und dann mit einer 1:100 Verdünnung in den Inkubationsansatz pipettiert. Die Lebermikrosomen und rekombinanten Enzyme wurden in 50 mM Kaliumphosphatpuffer pH 7.4 mit und ohne NADPH-generierendem System, bestehend aus 1 mM NADP⁺, 10 mM Glucose-6-phosphat und 1 Unit Glucose-6-phosphat Dehydrogenase, bei 37°C inkubiert. Primäre Hepatozyten wurden in Suspension in Williams E Medium ebenfalls bei 37°C inkubiert. Nach einer Inkubationszeit von 0 - 4h wurden die Inkubationsansätze mit Acetonitril abgestoppt (Endkonzentration ca. 30%) und das Protein bei ca. 15000 x g abzentrifugiert. Die so abgestoppten Proben wurden entweder direkt analysiert oder bis zur Analyse bei -20°C gelagert.

Die Analyse erfolgt mittels Hochleistungsflüssigkeits-Chromatographie mit Ultraviolett- und massenspektrometrischer Detektion (HPLC-UV-MS/MS). Dazu werden die Überstände der Inkubationsproben mit geeigneten C18-reversed-phase-Säulen und variablen Eluenten-Gemischen aus Acetonitril und 10 mM wässriger Ammoniumformiat-Lösung oder 0.05 % Ameisensäure chromatographiert. Die UV-Chromatogramme in Verbindung mit massenspektrometrischen Daten dienen zur Identifizierung, Strukturaufklärung und quantitativen Abschätzung der Metabolite, und der quantitativen metabolischen Abnahme der erfindungsgemäßen Verbindung in den Inkubationsansätzen.

### B-6. Inhibition der humanen Phosphodiesterase 5 (PDE 5)

PDE 5-Präparationen werden aus humanen Plättchen durch Aufschluss (Microfluidizer®, 800 bar, 3 Passagen), gefolgt von Zentrifugation (75000 g, 60 min, 4°C) und Ionenaustauscher-Chromatographie des Überstandes auf einer Mono Q 10/10 Säule (linearer Natriumchlorid-Gradient, Elution mit einer 0.2-0.3M Lösung von Natriumchlorid in Puffer (20 mM Hepes pH 7.2, 2 mM Magnesiumchlorid) gewonnen. Fraktionen, die PDE 5 Aktivität aufweisen, werden vereinigt (PDE 5-Präparat) und bei -80°C gelagert.

Die Testsubstanzen werden zur Bestimmung ihrer in vitro Wirkung an humaner PDE 5 in 100% DMSO aufgelöst und seriell verdünnt. Typischerweise werden Verdünnungsreihen (1:3) von 200 µM bis 0.091 µM hergestellt (resultierende Endkonzentrationen im Test: 4 µM bis 0.0018 µM). Jeweils 2 µL der verdünnten Substanzlösungen werden in die Vertiefungen von Mikrotiterplatten (Isoplate-96 /200W; Perkin Elmer) vorgelegt. Anschließend werden 50 µL einer Verdünnung des oben beschriebenen PDE 5-Präparats hinzugefügt. Die Verdünnung des PDE 5 Präparats wird so gewählt, dass während der späteren Inkubation weniger als 70% des Substrates umgesetzt wird (typische Verdünnung: 1: 100; Verdünnungspuffer: 50 mM Tris/Salzsäure pH 7.5, 8.3 mM Magnesiumchlorid, 1.7 mM EDTA, 0.2% BSA). Das Substrat [8-³H] cyclisches Guanosin-3',5'-monophosphat (1 µCi/µL; Perin Elmer) wird 1:2000 mit Assaypuffer (50 mM Tris/Salzsäure pH 7.5, 8.3 mM Magnesiumchlorid, 1.7 mM EDTA) auf eine Konzentration von 0.0005µCi/µL verdünnt. Durch Zugabe von 50 µL (0.025 µCi) des verdünnten Substrates wird die Enzymreaktion schließlich gestartet. Die Testansätze werden für 60 min bei Raumtemperatur inkubiert und die Reaktion durch Zugabe von 25 µL einer Suspension von 18 mg/mL Yttrium Scintillation Proximity Beads in Wasser (Phosphodiesterase beads für SPA Assays, RPNQ 0150, Perkin Elmer) gestoppt. Die Mikrotiterplatten werden mit einer Folie versiegelt und für 60 min bei Raumtemperatur stehengelassen. Anschließend werden die Platten für 30 s pro Vertiefung in einem Microbeta Szintillationszähler (Perkin Elmer) vermessen. IC₅₀-Werte werden anhand der graphischen Auftragung der Substanzkonzentration gegen die prozentuale PDE 5-Inhibition bestimmt.

Repräsentative Werte IC₅₀-Werte für die erfindungsgemäßen Verbindungen sind in der nachstehenden Tabelle (Tabelle 3) wiedergegeben:

**Tabelle 3:**

| Beispiel Nr. | IC₅₀ [nM] | Beispiel Nr. | IC₅₀ [nM] | Beispiel Nr. | IC₅₀ [nM] |
|---|---|---|---|---|---|
| 1 | 140 | 41 | 170 | 81 | 290 |
| 2 | 8.0 | 42 | 120 | 82 | |
| 3 | 46 | 43 | 410 | 83 | 230 |
| 4 | 6.0 | 44 | 86 | 84 | 19 |
| 5 | 130 | 45 | 78 | 85 | 4.2 |
| 6 | 120 | 46 | 35 | 86 | 330 |
| 7 | 110 | 47 | 83 | 87 | 210 |
| 8 | 400 | 48 | 54 | 88 | 150 |
| 9 | 230 | 49 | | 89 | 82 |
| 10 | 52 | 50 | 40 | 90 | 230 |
| 11 | 48 | 51 | 5.0 | 91 | 600 |
| 12 | 50 | 52 | 34 | 92 | 170 |
| 13 | 80 | 53 | 150 | 93 | 120 |
| 14 | 130 | 54 | | 94 | 140 |
| 15 | 430 | 55 | 130 | 95 | 4.0 |
| 16 | 13 | 56 | 34 | 96-1 | 150 |
| 17 | 24 | 57 | 16 | 96-2 | 26 |
| 18 | 120 | 58 | 270 | 97 | 63 |
| 19 | 280 | 59 | 8.0 | 98 | 4.0 |
| 20 | 900 | 60 | 430 | 99 | 440 |
| 21 | 400 | 61 | 100 | 100 | 2800 |
| 22 | 580 | 62 | 480 | 101 | 870 |
| 23 | 1600 | 63 | 230 | 102 | 74 |
| 24 | 100 | 64 | 980 | 103 | 400 |
| 25 | 320 | 65 | 430 | 104 | 80 |
| 26 | 12 | 66 | 16 | 105 | 980 |
| 27 | 4.0 | 67 | | 106 | 12 |
| 28 | 110 | 68 | 35 | 107 | 100 |
| 29 | >4000 | 69 | 46 | 108 | 25 |
| 30 | >4000 | 70 | 80 | 109 | 50 |
| 31 | 500 | 71 | 42 | 110 | 840 |
| 32 | 720 | 72 | 110 | 111 | 320 |
| 33 | 570 | 73 | 16 | 112 | 210 |
| 34 | 550 | 74 | 110 | 113 | 120 |
| 35 | 110 | 75 | 210 | 114 | 200 |
| 36 | 89 | 76 | 130 | 115 | 250 |
| 37 | 100 | 77 | | 116 | 83 |
| 38 | 41 | 78 | 330 | 117 | 52 |
| 39 | 88 | 79 | | | |
| 40 | 11 | 80 | | | |

### B-7. Bestimmung der Organ-protektiven Wirkungen im Langzeitversuch an Ratten.

Die Organ-protektiven Wirkungen der sGC Stimulatoren wurden in einem therapeutisch relevanten "low nitric oxide (NO) / high renin" Hypertoniemodell an der Ratten gezeigt. Die Studie wurde in Anlehnung an die kürzlich erschienene Publikation durchgeführt (Sharkovska Y, Kalk P, Lawrenz B, Godes M, Hoffmann LS, Wellkisch K, Geschka S, Relle K, Hocher B, Stasch JP. NOindependent stimulation of soluble guanylate cyclase reduces target organ damage in lowand high-renin models of hypertension. J. Hypertension. 2010; 28: 1666-1675). Dabei wurden Renin-transgene Ratten (TGR(mRen2)27), denen der NO-Synthase-Inhibitor L-NAME über das Trinkwasser verabreicht wurde, gleichzeitig mit einem sGC Stimualtor oder Vehikel über mehrere Wochen behandelt. Haemodynamische und renale Parameter wurden während des Behanlungszeitraums bestimmt. Am Ende der Langzeitstudie wurde die Organprotektion (Niere, Lunge, Herz, Aorta) durch histopathologische Untersuchungen, Biomarker, Expressionsanalysen und kardiovaskuläre Plasmaparameter gezeigt.

### C. Ausführungsbeispiele für pharmazeutische Zusammensetzungen

Die erfindungsgemäßen Verbindungen können folgendermaßen in pharmazeutische Zubereitungen überführt werden:

### Tablette:

### Zusammensetzung:

100 mg der erfindungsgemäßen Verbindung, 50 mg Lactose (Monohydrat), 50 mg Maisstärke (nativ), 10 mg Polyvinylpyrrolidon (PVP 25) (Fa. BASF, Ludwigshafen, Deutschland) und 2 mg Magnesiumstearat.

Tablettengewicht 212 mg. Durchmesser 8 mm, Wölbungsradius 12 mm.

### Herstellung:

Die Mischung aus erfindungsgemäßer Verbindung, Lactose und Stärke wird mit einer 5%-igen Lösung (m/m) des PVPs in Wasser granuliert. Das Granulat wird nach dem Trocknen mit dem Magnesiumstearat 5 Minuten gemischt. Diese Mischung wird mit einer üblichen Tablettenpresse verpresst (Format der Tablette siehe oben). Als Richtwert für die Verpressung wird eine Presskraft von 15 kN verwendet.

### Oral applizierbare Suspension:

### Zusammensetzung:

1000 mg der erfindungsgemäßen Verbindung, 1000 mg Ethanol (96%), 400 mg Rhodigel^{®} (Xanthan gum der Firma FMC, Pennsylvania, USA) und 99 g Wasser.

Einer Einzeldosis von 100 mg der erfindungsgemäßen Verbindung entsprechen 10 ml orale Suspension.

### Herstellung:

Das Rhodigel wird in Ethanol suspendiert, die erfindungsgemäße Verbindung wird der Suspension zugefügt. Unter Rühren erfolgt die Zugabe des Wassers. Bis zum Abschluß der Quellung des Rhodigels wird ca. 6 h gerührt.

### Oral applizierbare Lösung:

### Zusammensetzung:

500 mg der erfindungsgemäßen Verbindung, 2.5 g Polysorbat und 97 g Polyethylenglycol 400. Einer Einzeldosis von 100 mg der erfindungsgemäßen Verbindung entsprechen 20 g orale Lösung.

### Herstellung:

Die erfindungsgemäße Verbindung wird in der Mischung aus Polyethylenglycol und Polysorbat unter Rühren suspendiert. Der Rührvorgang wird bis zur vollständigen Auflösung der erfindungsgemäßen Verbindung fortgesetzt.

### i.v.-Lösung:

Die erfindungsgemäße Verbindung wird in einer Konzentration unterhalb der Sättigungslöslichkeit in einem physiologisch verträglichen Lösungsmittel (z.B. isotonische Kochsalzlösung, Glucoselösung 5% und/oder PEG 400-Lösung 30%) gelöst. Die Lösung wird steril filtriert und in sterile und pyrogenfreie Injektionsbehältnisse abgefüllt.

### Substituierte annellierte Pyrimidine und ihre Verwendung

### Zusammenfassung

Die vorliegende Anmeldung betrifft neue substituierte annellierte Pyrimidine, Verfahren zu ihrer Herstellung, ihre Verwendung allein oder in Kombinationen zur Behandlung und/oder Prophylaxe von Krankheiten sowie ihre Verwendung zur Herstellung von Arzneimitteln zur Behandlung und/oder Prophylaxe von Krankheiten, insbesondere zur Behandlung und/oder Prophylaxe von Herz-Kreislauf-Erkrankungen.

## Patentansprüche

1. Verbindungen der allgemeinen Formel (I) 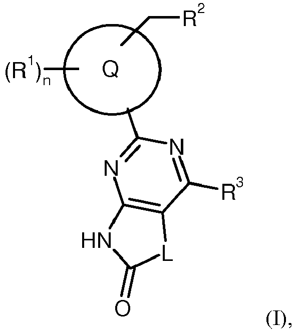 in welcher
L für eine Gruppe #¹-CR^{7A}R^{7B}-(CR^{8A}R^{8B})ₘ-#² steht,
wobei
#¹ für die Anknüpfstelle an die Carbonylgruppe steht,
#² für die Anknüpfstelle an den Pyrimidinring steht,
m für eine Zahl 0 steht,
R^{7A} für Wasserstoff, Fluor, Methyl, Hydroxy steht,
R^{7B} für Wasserstoff, Fluor, Methyl oder Trifluormethyl steht,
oder
R^{7A} und R^{7B} zusammen mit dem Kohlenstoffatom, an das sie gebunden sind, einen Tetrahydrofuranyl-Ring bilden,
der Ring Q für eine Gruppe der Formel 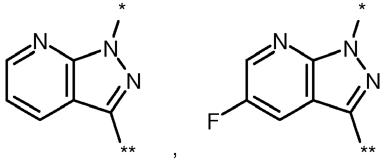 steht, wobei
* für die Anknüpfungsstelle an -CH₂-R² steht,
** für die Anknüpfungsstelle an das Pyrimidin steht,
R³ für -OR⁴ oder -NR⁵R⁶ steht,
wobei
R⁴ für (C₁-C₆)-Alkyl oder Pyrazolyl steht,
worin (C₁-C₆)-Alkyl mit 1 bis 3 Substituenten unabhängig voneinander ausgewählt aus der Gruppe Fluor, Trifluormethyl, -(C=O)ₚ-OR⁹ und -(C=O)ₚ-NR⁹R¹⁰ substituiert sein kann,
worin
p die Zahl 0 oder 1 bedeutet,
R⁹ und R¹⁰ unabhängig voneinander jeweils für Wasserstoff oder Methyl stehen,
und
worin Pyrazolyl mit 1 oder 2 Substituenten unabhängig voneinander ausgewählt aus der Gruppe Fluor, Difluormethyl, Trifluormethyl, 2,2,2-Trifluorethyl, 1,1,2,2,2-Pentafluorethyl, Methyl, Cyclopropyl, Cyclobutyl, Cyclopentyl substituiert sein kann,
R⁵ für Wasserstoff, Methyl oder Ethyl steht,
R⁶ für (C₁-C₆)-Alkyl, Oxetanyl, Azetidinyl, Tetrahydrofuranyl, Pyrrolidinyl, Tetrahydropyranyl, Piperidinyl, Piperazinyl, Morpholinyl, Phenyl, Pyrazolyl, Oxazolyl, Thiazolyl, Triazolyl, Oxadiazolyl, Thiadiazolyl, Pyridyl oder Pyrimidinyl steht,
worin (C₁-C₆)-Alkyl mit 1 oder 2 Substituenten unabhängig voneinander ausgewählt aus der Gruppe Fluor, Difluormethyl, Trifluormethyl Cyclopropyl, Cyclobutyl, Cyclopentyl, Trifluormethoxy, -(C=O)ₚ-OR⁹, -(C=O)ₚ-NR⁹R¹⁰, Tetrahydrofuranyl, Pyrrolidinyl, Tetrahydropyranyl, Phenyl, Furanyl, Pyrazolyl, Imidazolyl, Triazolyl und Pyridyl substituiert ist,
worin
p die Zahl 0 oder 1 bedeutet,
R⁹ und R¹⁰ unabhängig voneinander jeweils für Wasserstoff, Methyl, Ethyl, Trifluormethyl, 2,2,2-Trifluorethyl, 1,1,2,2,2-Pentafluorethyl, Cyclopropyl oder Cyclobutyl stehen,
und
worin Cyclopropyl, Cyclobutyl, Cyclopentyl, Tetrahydrofuranyl, Pyrrolidinyl, Tetrahydropyranyl, Phenyl, Furanyl, Pyrazolyl, Imidazolyl, Triazolyl und Pyridyl ihrerseits mit 1 oder 2 Substituenten unabhängig voneinander ausgewählt aus der Gruppe Fluor, Chlor, Cyano, Difluormethyl, Trifluormethyl, Methyl, Ethyl, Oxo und Hydroxy sein können,
worin Oxetanyl, Azetidinyl, Tetrahydrofuranyl, Pyrrolidinyl, Tetrahydropyranyl, Piperidinyl, Piperazinyl, Morpholinyl, Phenyl, Pyrazolyl, Oxazolyl, Thiazolyl, Triazolyl, Oxadiazolyl, Thiadiazolyl, Pyridyl und Pyrimidinyl mit 1 oder 2 Substituenten unabhängig voneinander ausgewählt aus der Gruppe Fluor, Difluormethyl, Trifluormethyl, Methyl, Ethyl, Oxo, Azetidinyl und Pyrrolidinyl substituiert sein können,
oder
R⁵ und R⁶ bilden zusammen mit dem Stickstoffatom, an das sie gebunden sind, einen Azetidinyl-, Pyrrolidinyl-, Imidazolidinyl-, Piperidinyl-, Dihydropiperdinyl-, Piperazinyl-, Morpholinyl-, Pyrazolyl- oder Imidazolyl-Ring, worin der Azetidinyl-, Pyrrolidinyl-, Imidazolidinyl-, Piperidinyl-, Dihydropiperdinyl-, Piperazinyl-, Morpholinyl-, Pyrazolyl- und Imidazolyl-Ring mit 1 oder 2 Substituenten unabhängig voneinander ausgewählt aus der Gruppe Fluor, Cyano, Difluormethyl, Trifluormethyl, 2,2,2-Trifluorethyl, 1,1,2,2,2-Pentafluorethyl, Methyl, Ethyl, 1-Hydroxyethyl, Cyclopropyl, Cyclobutyl, Cyclopentyl, Hydroxy, Oxo, Methoxy, Ethoxy, Difluormethoxy und Trifluormethoxy substituiert sein können,
R² für 2-Fluorphenyl, 2,3-Difluorphenyl oder 3-Fluorpyrid-2-yl steht,
sowie ihre Salze, Solvate und Solvate der Salze.

2. Verbindungen der Formel (I) gemäß Anspruch 1, in welcher
L für eine Gruppe #¹-CR^{7A}R^{7B}-(CR^{8A}R^{8B})ₘ-#² steht,
wobei
#¹ für die Anknüpfstelle an die Carbonylgruppe steht,
#² für die Anknüpfstelle an den Pyrimidinring steht,
m für eine Zahl 0 steht,
R^{7A} für Wasserstoff, Fluor, Methyl, Hydroxy steht,
R^{7B} für Wasserstoff, Fluor, Methyl oder Trifluormethyl steht, oder
R^{7A} und R^{7B} zusammen mit dem Kohlenstoffatom, an das sie gebunden sind, einen Tetrahydrofuranyl-Ring bilden,
der Ring Q für eine Gruppe der Formel 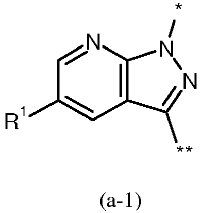 steht, wobei
* für die Anknüpfungsstelle an -CH₂-R² steht,
** für die Anknüpfungsstelle an das Pyrimidin steht,
R¹ für Wasserstoff oder Fluor steht,
R³ für -NR⁵R⁶ steht,
wobei
R⁵ für Wasserstoff oder Methyl steht,
R⁶ für (C₁-C₆)-Alkyl steht,
worin (C₁-C₆)-Alkyl mit 1 oder 2 Substituenten unabhängig voneinander ausgewählt aus der Gruppe Fluor, Difluormethyl und Trifluormethyl substituiert ist,
R² für 2-Fluorphenyl oder 3-Fluorpyrid-2-yl steht,
sowie ihre Salze, Solvate und Solvate der Salze.

3. 2-[5-Fluor-1-(2-fluorbenzyl)-1H-pyrazolo[3,4-b]pyridin-3-yl]-5,5-dimethyl-4-[(3,3,3-trifluorpropyl)amino]-5,7-dihydro-6H-pyrrolo[2,3-d]pyrimidin-6-on gemäß Ansprüchen 1 und 2, der folgenden Formel 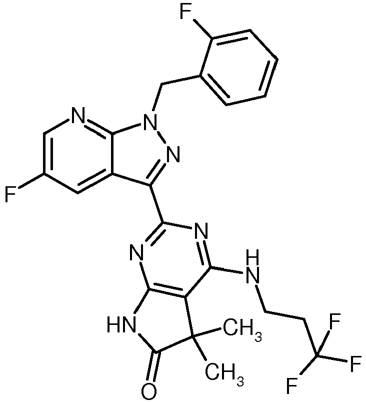 sowie dessen Salze, Solvate und Solvate der Salze.

4. Verfahren zur Herstellung von Verbindungen der Formel (I), wie in den Ansprüchen 1 bis 3 definiert, **dadurch gekennzeichnet, dass** man eine Verbindung der Formel (II) 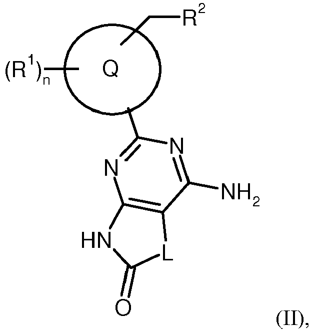 in welcher n, L, Q, R¹ und R² jeweils die in den Ansprüchen 1 bis 3 genannten Bedeutungen haben,
in einem inerten Lösungsmittel mit iso-Pentylnitrit und einem Halogen-Äquivalent in eine Verbindung der Formel (III) 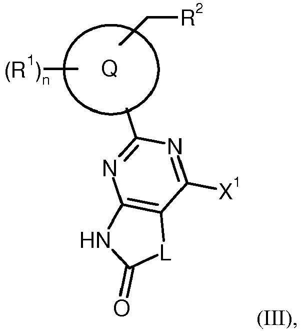 in welcher n, L, Q, R¹ und R² jeweils die in den Ansprüchen 1 bis 3 angegebenen Bedeutungen haben und
X¹ für Brom oder Iod steht
überführt, und diese im Anschluss in einen inerten Lösungsmittel gegebenenfalls in Gegenwart einer geeigneten Base mit einer Verbindung der Formel (IV)
R³-H (IV),
in welcher R³ die in den Ansprüchen 1 bis 3 angegebene Bedeutung hat,
zu einer Verbindung der Formel (I) 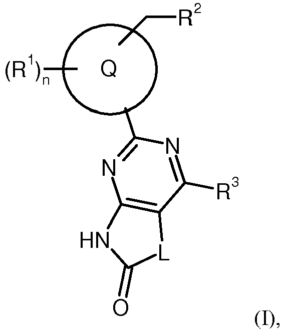 in welcher n, L, Q, R¹, R² und R³ jeweils die in den Ansprüchen 1 bis 3 angegebenen Bedeutungen haben,
umsetzt, und gegebenenfalls die resultierenden Verbindungen der Formel (I) gegebenenfalls mit den entsprechenden (i) Lösungsmitteln und/oder (ii) Säuren oder Basen in ihre Solvate, Salze und/oder Solvate der Salze überführt.

5. Verbindung der Formel (I), wie in einem der Ansprüche 1 bis 3 definiert, zur Behandlung und/oder Prophylaxe von Krankheiten.

6. Verwendung einer Verbindung der Formel (I), wie in einem der Ansprüche 1 bis 3 definiert, zur Herstellung eines Arzneimittels zur Behandlung und/oder Prophylaxe von Herzinsuffizienz, Angina pectoris, Hypertonie, pulmonaler Hypertonie, Ischämien, Gefäßerkrankungen, Niereninsuffizienz, thromboembolischen Erkrankungen, fibrotischen Erkrankungen und Arteriosklerose.

7. Verbindung der Formel (I), wie in einem der Ansprüche 1 bis 3 definiert, zur Verwendung in einem Verfahren zur Behandlung und/oder Prophylaxe von Herzinsuffizienz, Angina pectoris, Hypertonie, pulmonaler Hypertonie, Ischämien, Gefäßerkrankungen, Niereninsuffizienz, thromboembolischen Erkrankungen, fibrotischen Erkrankungen und Arteriosklerose.

8. Arzneimittel enthaltend eine Verbindung der Formel (I), wie in einem der Ansprüche 1 bis 3 definiert, in Kombination mit einem inerten, nicht-toxischen, pharmazeutisch geeigneten Hilfsstoff.

9. Arzneimittel enthaltend eine Verbindung der Formel (I), wie in einem der Ansprüche 1 bis 3 definiert, in Kombination mit einem weiteren Wirkstoff ausgewählt aus der Gruppe bestehend aus organischen Nitraten, NO-Donatoren, cGMP-PDE-Inhibitoren, antithrombotisch wirkenden Mitteln, den Blutdruck senkenden Mitteln sowie den Fettstoffwechsel verändernden Mitteln.

10. Arzneimittel nach Anspruch 8 oder 9 zur Behandlung und/oder Prophylaxe von Herzinsuffizienz, Angina pectoris, Hypertonie, pulmonaler Hypertonie, Ischämien, Gefäßerkrankungen, Niereninsuffizienz, thromboembolischen Erkrankungen, fibrotischen Erkrankungen und Arteriosklerose.

## Claims

1. Compounds of general formula (I) 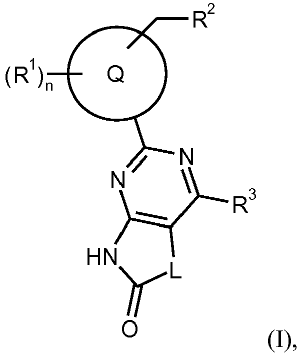 in which
L stands for a group #¹-CR^{7A}R^{7B}-(CR^{8A}R^{8B})ₘ-#²,
wherein
#¹ stands for the point of attachment to the carbonyl group,
#² stands for the point of attachment to the pyrimidine ring,
m stands for a number 0,
R^{7A} stands for hydrogen, fluorine, methyl, hydroxy,
R^{7B} stands for hydrogen, fluorine, methyl or trifluoromethyl,
or
R^{7A} and R^{7B} together with the carbon atom to which they are bound, form a tetrahydrofuranyl ring,
the ring Q stands for a group of formula 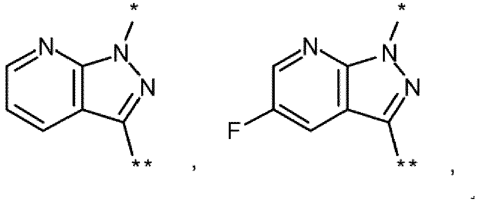 wherein
* stands for the point of attachment to -CH₂-R²,
** stands for the point of attachment to the pyrimidine,
R³ stands for -OR⁴ or -NR⁵R⁶,
wherein
R⁴ stands for (C₁-C₆)-alkyl or pyrazolyl,
in which (C₁-C₆)-alkyl can be substituted with 1 to 3 substituents selected independently of one another from the group fluorine, trifluoromethyl, -(C=O)ₚ-OR⁹ and -(C=O)ₚ-NR⁹R¹⁰,
in which
p denotes the number 0 or 1,
R⁹ and R¹⁰ independently of one another stand in each case for hydrogen or methyl,
and
in which pyrazolyl can be substituted with 1 or 2 substituents selected independently of one another from the group fluorine, difluoromethyl, trifluoromethyl, 2,2,2-trifluoroethyl, 1,1,2,2,2-pentafluoroethyl, methyl, cyclopropyl, cyclobutyl, cyclopentyl,
R⁵ stands for hydrogen, methyl or ethyl,
R⁶ stands for (C₁-C₆)-alkyl, oxetanyl, azetidinyl, tetrahydrofuranyl, pyrrolidinyl, tetrahydropyranyl, piperidinyl, piperazinyl, morpholinyl, phenyl, pyrazolyl, oxazolyl, thiazolyl, triazolyl, oxadiazolyl, thiadiazolyl, pyridyl or pyrimidinyl,
in which (C₁-C₆)-alkyl is substituted with 1 or 2 substituents selected independently of one another from the group fluorine, difluoromethyl, trifluoromethyl cyclopropyl, cyclobutyl, cyclopentyl, trifluoromethoxy, -(C=O)ₚ-OR⁹, -(C=O)ₚ-NR⁹R¹⁰, tetrahydrofuranyl, pyrrolidinyl, tetrahydropyranyl, phenyl, furanyl, pyrazolyl, imidazolyl, triazolyl and pyridyl,
in which
p denotes the number 0 or 1,
R⁹ and R¹⁰ each stand, independently of one another, for hydrogen, methyl, ethyl, trifluoromethyl, 2,2,2-trifluoroethyl, 1,1,2,2,2-pentafluoroethyl, cyclopropyl or cyclobutyl,
and
in which cyclopropyl, cyclobutyl, cyclopentyl, tetrahydrofuranyl, pyrrolidinyl, tetrahydropyranyl, phenyl, furanyl, pyrazolyl, imidazolyl, triazolyl and pyridyl for their part can be substituted with 1 or 2 substituents selected independently of one another from the group fluorine, chlorine, cyano, difluoromethyl, trifluoromethyl, methyl, ethyl, oxo and hydroxy,
in which oxetanyl, azetidinyl, tetrahydrofuranyl, pyrrolidinyl, tetrahydropyranyl, piperidinyl, piperazinyl, morpholinyl, phenyl, pyrazolyl, oxazolyl, thiazolyl, triazolyl, oxadiazolyl, thiadiazolyl, pyridyl and pyrimidinyl can be substituted with 1 or 2 substituents selected independently of one another from the group fluorine, difluoromethyl, trifluoromethyl, methyl, ethyl, oxo, azetidinyl and pyrrolidinyl,
or
R⁵ and R⁶ form, together with the nitrogen atom to which they are bound, an azetidinyl, pyrrolidinyl, imidazolidinyl, piperidinyl, dihydropiperdinyl, piperazinyl, morpholinyl, pyrazolyl or imidazolyl ring,
in which the azetidinyl, pyrrolidinyl, imidazolidinyl, piperidinyl, dihydropiperdinyl, piperazinyl, morpholinyl, pyrazolyl and imidazolyl ring can be substituted with 1 or 2 substituents selected independently of one another from the group fluorine, cyano, difluoromethyl, trifluoromethyl, 2,2,2-trifluoroethyl, 1,1,2,2,2-pentafluoroethyl, methyl, ethyl, 1-hydroxyethyl, cyclopropyl, cyclobutyl, cyclopentyl, hydroxy, oxo, methoxy, ethoxy, difluoromethoxy and trifluoromethoxy,
R² stands for 2-fluorophenyl, 2,3-difluorophenyl or 3-fluoropyrid-2-yl,
and their salts, solvates and solvates of the salts.

2. Compounds of formula (I) according to Claim 1, in which
L stands for a group #¹-CR^{7A}R^{7B}-(CR^{8A}R^{8B})ₘ-#², wherein
#¹ stands for the point of attachment to the carbonyl group,
#² stands for the point of attachment to the pyrimidine ring,
m stands for a number 0,
R^{7A} stands for hydrogen, fluorine, methyl, hydroxy,
R^{7B} stands for hydrogen, fluorine, methyl or trifluoromethyl,
or
R^{7A} and R^{7B} together with the carbon atom to which they are bound, form a tetrahydrofuranyl ring,
the ring Q stands for a group of formula 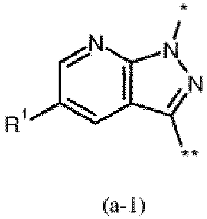 wherein
* stands for the point of attachment to -CH₂-R²,
** stands for the point of attachment to the pyrimidine,
R¹ stands for hydrogen or fluorine,
R³ stands for -NR⁵R⁶,
wherein
R⁵ stands for hydrogen or methyl,
R⁶ stands for (C₁-C₆)-alkyl,
in which (C₁-C₆)-alkyl is substituted with 1 or 2 substituents selected independently of one another from the group fluorine, difluoromethyl and trifluoromethyl,
R² stands for 2-fluorophenyl or 3-fluoropyrid-2-yl,
and their salts, solvates and solvates of the salts.

3. 2-[5-Fluoro-1-(2-fluorobenzyl)-1H-pyrazolo[3,4-b]pyridin-3-yl]-5,5-dimethyl-4-[(3,3,3-trifluoropropyl)amino]-5,7-dihydro-6H-pyrrolo[2,3-d]pyrimidin-6-one according to Claims 1 and 2, of the following formula: 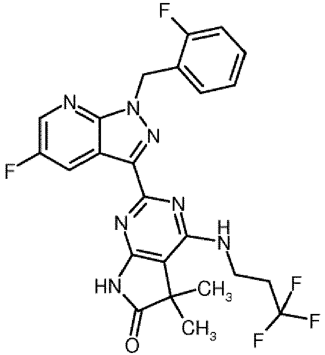 and its salts, solvates and solvates of the salts.

4. Method of production of compounds of formula (I), as defined in Claims 1 to 3, **characterized in that** a compound of formula (II) 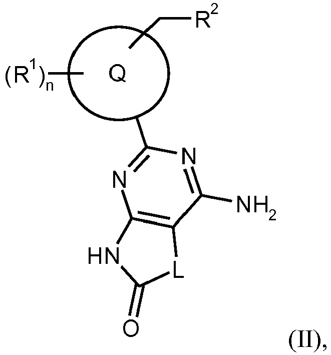 in which n, L, Q, R¹ and R² in each case have the meanings stated in Claims 1 to 3,
is converted in an inert solvent with isopentyl nitrite and a halogen equivalent to a compound of formula (III) 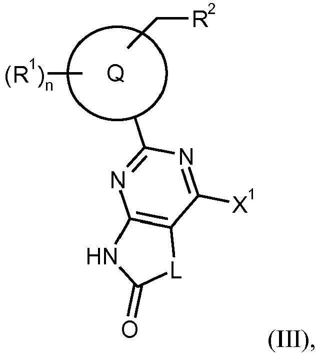 in which n, L, Q, R¹ and R² in each case have the meanings given in Claims 1 to 3 and
X¹ stands for bromine or iodine
and this is then reacted in an inert solvent optionally in the presence of a suitable base with a compound of formula (IV)
R³-H (IV),
in which R³ has the meaning given in Claims 1 to 3,
to a compound of formula (I) 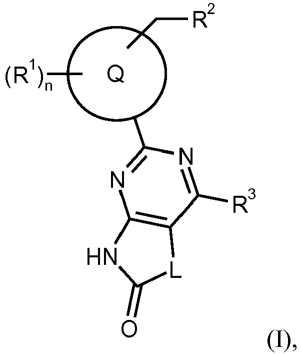 in which n, L, Q, R¹, R² and R³ in each case have the meanings given in Claims 1 to 3,
and optionally the resultant compounds of formula (I) optionally with the corresponding (i) solvents and/or (ii) acids or bases are transformed to their solvates, salts and/or solvates of the salts.

5. Compound of formula (I), as defined in one of Claims 1 to 3, for treating and/or preventing diseases.

6. Use of a compound of formula (I), as defined in one of Claims 1 to 3, for producing a medicinal product for treating and/or preventing heart failure, angina pectoris, hypertension, pulmonary hypertension, ischaemias, vascular diseases, renal insufficiency, thromboembolic diseases, fibrotic diseases and arteriosclerosis.

7. Compound of formula (I), as defined in one of Claims 1 to 3, for use in a method of treating and/or preventing heart failure, angina pectoris, hypertension, pulmonary hypertension, ischaemias, vascular diseases, renal insufficiency, thromboembolic diseases, fibrotic diseases and arteriosclerosis.

8. Medicinal product containing a compound of formula (I), as defined in one of Claims 1 to 3, in combination with an inert, non-toxic, pharmaceutically suitable excipient.

9. Medicinal product containing a compound of formula (I), as defined in one of Claims 1 to 3, in combination with another active substance selected from the group consisting of organic nitrates, NO-donors, cGMP-PDE inhibitors, antithrombotic agents, agents for lowering blood pressure and agents for altering fat metabolism.

10. Medicinal product according to Claim 8 or 9 for treating and/or preventing heart failure, angina pectoris, hypertension, pulmonary hypertension, ischaemias, vascular diseases, renal insufficiency, thromboembolic diseases, fibrotic diseases and arteriosclerosis.

## Revendications

1. Composés de formule générale (I) 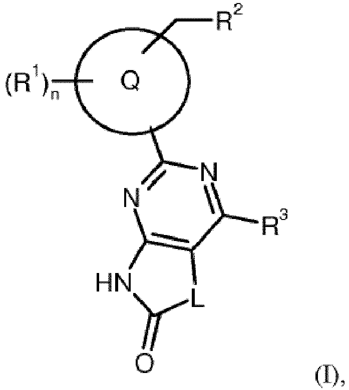 dans lesquels
L représente un groupe #¹-CR^{7A}R^{7B}-(CR^{8A}R^{8B})ₘ-#²,
#¹ représentant l'emplacement de liaison au groupe carbonyle,
#² représentant l'emplacement de liaison au cycle pyrimidine,
m représentant un nombre 0,
R^{7A} représentant hydrogène, fluor, méthyle, hydroxy,
R^{7B} représentant hydrogène, fluor, méthyle ou trifluorométhyle,
ou
R^{7A} et R^{7B} formant ensemble avec l'atome de carbone auquel ils sont reliés un cycle tétrahydrofuranyle,
le cycle Q représente un groupe de formule 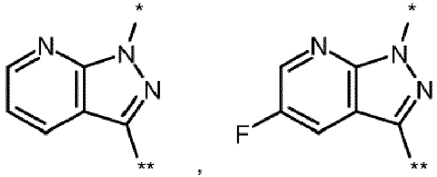 dans lesquelles
* représente l'emplacement de liaison à -CH₂-R²,
** représente l'emplacement de liaison à la pyrimidine,
R³ représente -OR⁴ ou -NR⁵R⁶,
R⁴ représentant alkyle en (C₁-C₆) ou pyrazolyle,
l'alkyle en (C₁-C₆) pouvant être substitué avec 1 à 3 substituants choisis indépendamment les uns des autres dans le groupe constitué par fluor, trifluorométhyle, -(C=O)ₚ-OR⁹ et - (C=O)ₚ-NR⁹R¹⁰,
p signifiant le nombre 0 ou 1,
R⁹ et R¹⁰ représentant indépendamment l'un de l'autre hydrogène ou méthyle,
et
le pyrazolyle pouvant être substitué avec 1 ou 2 substituants choisis indépendamment l'un de l'autre dans le groupe constitué par fluor, difluorométhyle, trifluorométhyle, 2,2,2-trifluoroéthyle, 1,1,2,2,2-pentafluoroéthyle, méthyle, cyclopropyle, cyclobutyle, cyclopentyle,
R⁵ représentant hydrogène, méthyle ou éthyle,
R⁶ représentant alkyle en (C₁-C₆), oxétanyle, azétidinyle, tétrahydrofuranyle, pyrrolidinyle, tétrahydropyranyle, pipéridinyle, pipérazinyle, morpholinyle, phényle, pyrazolyle, oxazolyle, thiazolyle, triazolyle, oxadiazolyle, thiadiazolyle, pyridyle ou pyrimidinyle,
l'alkyle en (C₁-C₆) étant substitué avec 1 ou 2 substituants choisis indépendamment l'un de l'autre dans le groupe constitué par fluor, difluorométhyle, trifluorométhyle, cyclopropyle, cyclobutyle, cyclopentyle, trifluorométhoxy, -(C=O)ₚ-OR⁹, -(C=O)ₚ-NR⁹R¹⁰, tétrahydrofuranyle, pyrrolidinyle, tétrahydropyranyle, phényle, furanyle, pyrazolyle, imidazolyle, triazolyle et pyridiyle,
p signifiant le nombre 0 ou 1,
R⁹ et R¹⁰ représentant chacun indépendamment l'un de l'autre hydrogène, méthyle, éthyle, trifluorométhyle, 2,2,2-trifluoroéthyle, 1,1,2,2,2-pentafluoroéthyle, cyclopropyle ou cyclobutyle,
et
le cyclopropyle, le cyclobutyle, le cyclopentyle, le tétrahydrofuranyle, le pyrrolidinyle, le tétrahydropyranyle, le phényle, le furanyle, le pyrazolyle, l'imidazolyle, le triazolyle et le pyridiyle pouvant de leur côté être substitués avec 1 ou 2 substituants choisis indépendamment l'un de l'autre dans le groupe constitué par fluor, chlore, cyano, difluorométhyle, trifluorométhyle, méthyle, éthyle, oxo et hydroxy,
l'oxétanyle, l'azétidinyle, le tétrahydrofuranyle, le pyrrolidinyle, le tétrahydropyranyle, le pipéridinyle, le pipérazinyle, le morpholinyle, le phényle, le pyrazolyle, l'oxazolyle, le thiazolyle, le triazolyle, l'oxadiazolyle, le thiadiazolyle, le pyridyle ou le pyrimidinyle pouvant être substitués avec 1 ou 2 substituants choisis indépendamment l'un de l'autre dans le groupe constitué par fluor, difluorométhyle, trifluorométhyle, méthyle, éthyle, oxo, azétidinyle et pyrrolidinyle,
ou
R⁵ et R⁶ formant ensemble avec l'atome d'azote auquel ils sont reliés un cycle azétidinyle, pyrrolidinyle, imidazolidinyle, pipéridinyle, dihydropipéridinyle, pipérazinyle, morpholinyle, pyrazolyle ou imidazolyle,
le cycle azétidinyle, pyrrolidinyle, imidazolidinyle, pipéridinyle, dihydropipéridinyle, pipérazinyle, morpholinyle, pyrazolyle et imidazolyle pouvant être substitué avec 1 ou 2 substituants choisis indépendamment l'un de l'autre dans le groupe constitué par fluor, cyano, difluorométhyle, trifluorométhyle, 2,2,2-trifluoroéthyle, 1,1,2,2,2-pentafluoroéthyle, méthyle, éthyle, 1-hydroxyéthyle, cyclopropyle, cyclobutyle, cyclopentyle, hydroxy, oxo, méthoxy, éthoxy, difluorométhoxy et trifluorométhoxy,
R² représente 2-fluorophényle, 2,3-difluorophényle ou 3-fluoropyrid-2-yle,
ainsi que leurs sels, solvates et solvates des sels.

2. Composés de formule (I) selon la revendication 1, dans lesquels
L représente un groupe #¹-CR^{7A}R^{7B}-(CR^{8A}R^{8B})ₘ-#²,
#¹ représentant l'emplacement de liaison au groupe carbonyle,
#² représentant l'emplacement de liaison au cycle pyrimidine,
m représentant un nombre 0,
R^{7A} représentant hydrogène, fluor, méthyle, hydroxy,
R^{7B} représentant hydrogène, fluor, méthyle ou trifluorométhyle,
ou
R^{7A} et R^{7B} formant ensemble avec l'atome de carbone auquel ils sont reliés un cycle tétrahydrofuranyle,
le cycle Q représente un groupe de formule 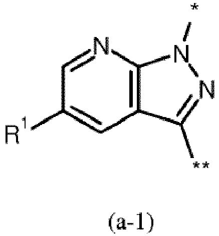 dans laquelle
* représente l'emplacement de liaison à -CH₂-R²,
** représente l'emplacement de liaison à la pyrimidine,
R¹ représente hydrogène ou fluor,
R³ représente -NR⁵R⁶,
R⁵ représentant hydrogène ou méthyle,
R⁶ représentant alkyle en (C₁-C₆),
l'alkyle en (C₁-C₆) étant substitué avec 1 ou 2 substituants choisis indépendamment l'un de l'autre dans le groupe constitué par fluor, difluorométhyle et trifluorométhyle,
R² représente 2-fluorophényle ou 3-fluoropyrid-2-yle, ainsi que leurs sels, solvates et solvates des sels.

3. 2-[5-Fluoro-1-(2-fluorobenzyl)-1H-pyrazolo[3,4-b]pyridin-3-yl]-5,5-diméthyl-4-[(3,3,3-trifluoropropyl)amino]-5,7-dihydro-6H-pyrrolo[2,3-d]pyrimidin-6-one selon les revendications 1 et 2, de la formule suivante : 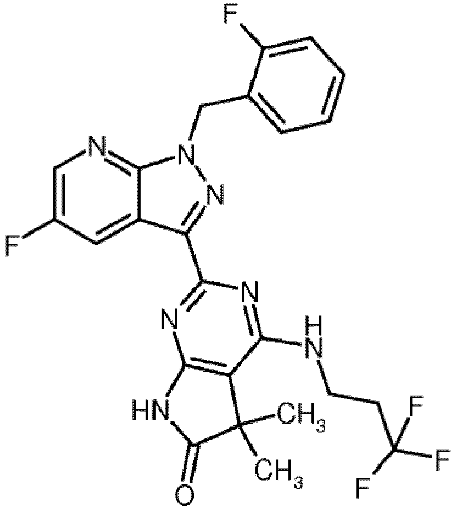 ainsi que ses sels, solvates et solvates des sels.

4. Procédé de fabrication de composés de formule (I), tels que définis dans les revendications 1 à 3, **caractérisé en ce qu'**un composé de formule (II) 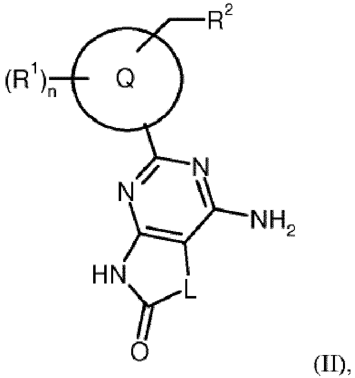 dans laquelle n, L, Q, R¹ et R² ont chacun les significations indiquées dans les revendications 1 à 3, est transformé dans un solvant inerte avec de l'isopentylnitrite et un équivalent d'halogène en un composé de formule (III) 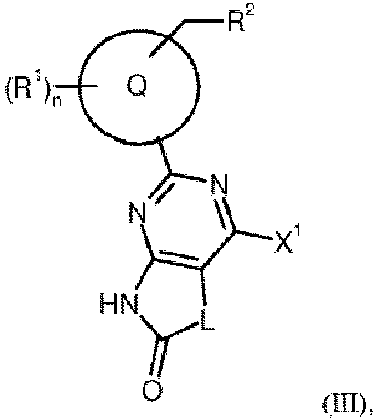
dans laquelle n, L, Q, R¹ et R² ont chacun les significations indiquées dans les revendications 1 à 3, et
X¹ représente brome ou iode,
puis celui-ci est mis en réaction dans un solvant inerte éventuellement en présence d'une base appropriée avec un composé de formule (IV)
R³-H (IV)
dans laquelle R³ a la signification indiquée dans les revendications 1 à 3,
pour former un composé de formule (I) 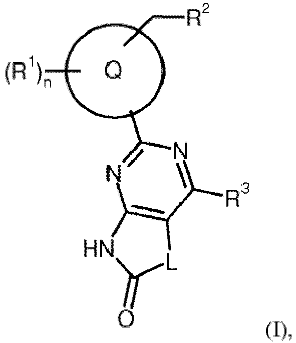 dans laquelle n, L, Q, R¹, R² et R³ ont chacun les significations indiquées dans les revendications 1 à 3, et les composés de formule (I) résultants sont éventuellement transformés avec (i) les solvants et/ou (ii) les acides ou les bases appropriés en leurs solvates, sels et/ou solvates des sels.

5. Composé de formule (I), tel que défini dans l'une quelconque des revendications 1 à 3, pour le traitement et/ou la prophylaxie de maladies.

6. Utilisation d'un composé de formule (I), tel que défini dans l'une quelconque des revendications 1 à 3, pour la fabrication d'un médicament pour le traitement et/ou la prophylaxie de l'insuffisance cardiaque, de l'angine de poitrine, de l'hypertension, de l'hypertension pulmonaire, des ischémies, des maladies vasculaires, de l'insuffisance rénale, des maladies thromboemboliques, des maladies fibrotiques et de l'artériosclérose.

7. Composé de formule (I), tel que défini dans l'une quelconque des revendications 1 à 3, destiné à une utilisation dans un procédé de traitement et/ou de prophylaxie de l'insuffisance cardiaque, de l'angine de poitrine, de l'hypertension, de l'hypertension pulmonaire, des ischémies, des maladies vasculaires, de l'insuffisance rénale, des maladies thromboemboliques, des maladies fibrotiques et de l'artériosclérose.

8. Médicament, contenant un composé de formule (I), tel que défini dans l'une quelconque des revendications 1 à 3, en combinaison avec un adjuvant inerte, non toxique, pharmaceutiquement approprié.

9. Médicament, contenant un composé de formule (I), tel que défini dans l'une quelconque des revendications 1 à 3, en combinaison avec un autre agent actif choisi dans le groupe constitué par les nitrates organiques, les donneurs de NO, les inhibiteurs de cGMP-PDE, les agents à effet antithrombotique, les agents abaissant la tension artérielle, ainsi que les agents modifiant le métabolisme lipidique.

10. Médicament selon la revendication 8 ou 9, pour le traitement et/ou la prophylaxie de l'insuffisance cardiaque, de l'angine de poitrine, de l'hypertension, de l'hypertension pulmonaire, des ischémies, des maladies vasculaires, de l'insuffisance rénale, des maladies thromboemboliques, des maladies fibrotiques et de l'artériosclérose.
